(19) Europäisches Patentamt
European Patent Office
Office européen des brevets

(11) EP 2 135 620 A1

(12) # EUROPEAN PATENT APPLICATION
published in accordance with Art. 153(4) EPC

(43) Date of publication:
**23.12.2009 Bulletin 2009/52**

(21) Application number: **08739138.9**

(22) Date of filing: **28.03.2008**

(51) Int Cl.:
***A61K 45/00*** (2006.01) ***A61K 31/44*** (2006.01)
***A61K 31/443*** (2006.01) ***A61K 31/4433*** (2006.01)
***A61K 31/4436*** (2006.01) ***A61K 31/4439*** (2006.01)
***A61K 31/444*** (2006.01) ***A61K 31/5377*** (2006.01)
***A61P 27/02*** (2006.01) ***A61P 43/00*** (2006.01)
***C07D 213/81*** (2006.01) ***C07D 401/12*** (2006.01)
***C07D 405/12*** (2006.01) ***C07D 405/14*** (2006.01)
***C07D 409/12*** (2006.01) ***C07D 413/12*** (2006.01)
***C07D 417/12*** (2006.01)

(86) International application number:
**PCT/JP2008/056014**

(87) International publication number:
**WO 2008/123395 (16.10.2008 Gazette 2008/42)**

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **28.03.2007 JP 2007084356**

(71) Applicant: **Santen Pharmaceutical Co., Ltd**
**Osaka-shi**
**Osaka 533-8651 (JP)**

(72) Inventors:
- **YONEDA, Shinji**
**Ikoma-shi**
**Nara 630-0101 (JP)**
- **FUJISAWA, Koushi**
**Ikoma-shi**
**Nara 630-0101 (JP)**
- **WATANABE, Katsuhiko**
**Ikoma-shi**
**Nara 630-0101 (JP)**
- **FUJIKAWA, Junko**
**Ikoma-shi**
**Nara 630-0101 (JP)**
- **SHIMAZAKI, Atsushi**
**Ikoma-shi**
**Nara 630-0101 (JP)**
- **KIRIHARA, Tomoko**
**Ikoma-shi**
**Nara 630-0101 (JP)**
- **TAJIMA, Hisashi**
**Ikoma-shi**
**Nara 630-0101 (JP)**

(74) Representative: **Perrey, Ralf et al**
**Müller-Boré & Partner**
**Grafinger Strasse 2**
**81671 München (DE)**

(54) **OCULAR HYPOTENSIVE AGENT COMPRISING COMPOUND CAPABLE OF INHIBITING HISTONE DEACETYLASE AS ACTIVE INGREDIENT**

(57) An object of the present invention is to find a novel pharmacological effect of a compound having an HDAC inhibitory effect. The compound having an HDAC inhibitory effect of the invention has an excellent effect of cell morphological change on trabecular meshwork cells and/or effect of intraocular pressure reduction, and is therefore useful as a preventive and/or therapeutic agent for a disease considered to be associated with aqueous humor circulation and/or intraocular pressure, particularly as a preventive and/or therapeutic agent for glaucoma or ocular hypertension.



Printed by Jouve, 75001 PARIS (FR)

## Description

### Technical Field

The present invention relates to an intraocular pressure-lowering agent comprising at least one pharmaceutically useful compound having a histone deacetylase (hereinafter referred to as "HDAC") inhibitory effect as an active ingredient. The compound has an effect of morphological change on trabecular meshwork cells and/or an effect of intraocular pressure reduction, and is therefore useful as a preventive and/or therapeutic agent for a disease considered to be associated with aqueous humor circulation and/or intraocular pressure.

### Background Art

The aqueous humor circulation in the eye is closely related to intraocular pressure, and the hindrance of the aqueous humor circulation has a considerable effect on the intraocular pressure. In particular, when the aqueous humor circulation is hindered, the intraocular pressure is increased to cause a disease considered to be associated with aqueous humor circulation or intraocular pressure such as glaucoma or ocular hypertension.

In general, aqueous humor is produced by filtration or active transport of plasma components and most aqueous humor flows out of the eyeball through the trabecular outflow pathway. That is, it becomes possible to prevent and/or treat a disease considered to be associated with aqueous humor circulation or intraocular pressure by changing the morphology of trabecular meshwork cells with a medicinal agent or the like to reduce the resistance to aqueous humor outflow thereby enhancing aqueous humor outflow.

For example, as a medicinal agent for changing the morphology of trabecular meshwork cells to enhance aqueous humor outflow, latrunculin A (an actin polymerization inhibitor), H-7 (a myosin light-chain kinase (MLCK) inhibitor), Y-39983 (a Rho-kinase inhibitor) (WO 1997/030701 and WO 2000/009162) and the like are known.

On the other hand, eukaryotic chromosomal DNA wraps around core histone proteins, histones H2A, H2B, H3 and H4 to form a basic structure called nucleosome. Further, the nucleosome structures assemble to form a chromatin structure. Post-translational modifications of histones are closely related to the constitution of the chromatin structure, and as the post-translational modification, acetylation, methylation, phosphorylation, ubiquitylation and the like are known.

For example, it is considered that histone acetylation is related to gene transcriptional induction, replication, repair and the like.

The histone acetylation is reversibly regulated by a histone acetyltransferase (hereinafter referred to as "HAT") and a histone deacetylase.

It is believed that if HDAC is inhibited, histone acetylation by HAT is enhanced and subsequent gene transcriptional induction, replication, repair and the like are activated, and therefore, various diseases considered to be associated with cell proliferation, senescence, etc. such as cancer, autoimmune diseases, neurodegenerative diseases and infectious diseases can be prevented and/or treated (Protein, Nucleic Acid and Enzyme, Vol. 51. No. 14 (2006), JP-A-2005-272419 and JP-T-2006-517532).

As typical examples of the compound having an HDAC inhibitory effect, butyric acid which has an effect of cell cycle arrest, an effect of normalization and differentiation of transformed cells and the like (J. Biol. Chem., 254, 1716-1723 (1979) ), trichostatin A which is a microbial metabolite and has an effect of cell cycle arrest, an effect of induction of differentiation and the like (Cancer Res., 47, 3688-3691 (1987), Exp. Cell Res., 177, 122-131 (1988) and J. Biol. Chem., 265, 17174-17179 (1990)), trapoxin which is a microbial metabolite and has an inhibitory effect of cell proliferation (J. Antibiotics, 43, 1524-1534 (1990) and J. Biol. Chem., 268, 22429-22435 (1993) and the like are known. Further, as an anticancer agent, compounds having an HDAC inhibitory effect such as SAHA, FK-228, MS 275, PXD-101, MGCD-0103, LBH-589, NSC-696085, CG-781 and CRA-026440 have been developed or on sale.

However, the relationship between the compound having an HDAC inhibitory effect and the morphological change of trabecular meshwork cells and the relationship between the compound having an HDAC inhibitory effect and the effect of intraocular pressure reduction are completely unknown.

### Disclosure of the Invention

#### Problems to be Solved

It is a very interesting subject to find a novel pharmacological effect of a compound having an HDAC inhibitory effect.

Means for Solving the Problems

The present inventors conducted intensive studies for finding a novel pharmacological effect of a compound having an HDAC inhibitory effect, and as a result, they found that the compound has an effect of morphological change on trabecular meshwork cells.

Further, the present inventors conducted a test for evaluation of intraocular-lowering effect by intracameral administration of the compound using male Japanese White rabbits, and as a result, they confirmed that the compound has an effect of intraocular pressure reduction, and thus completed the present invention.

Accordingly, the compound having an HDAC inhibitory effect can be used as an intraocular pressure-lowering agent and is useful as a preventive and/or therapeutic agent for a disease considered to be associated with aqueous humor circulation and/or intraocular pressure.

The invention is directed to:

[1] an intraocular pressure-lowering agent comprising at least one compound having an HDAC inhibitory effect as an active ingredient;

[2] the intraocular pressure-lowering agent according to [1], wherein the compound having an HDAC inhibitory effect is a compound selected from the following compounds or a salt thereof

·(2E,4E,6R)-7-(4-Dimethylaminophenyl)-N-hydroxy-4,6-dimethyl-7-oxo-2,4-heptadienamide (Trichostatin A),

· N-Hydroxy-N'-phenyloctanediamide (SAHA),

· 4-Dimethylamino-N-(6-hydroxycarbamoylhexyl)benzamide (M 344),

· (E)-N-Hydroxy-5-[3-(phenylsulfonylamino)phenyl]-2-penten-4-ynamide (Oxamflatin),

· N-Hydroxy-3-[3-hydroxyamino-3-oxo-1-propenyl]benzamide (CBHA),

· (E)-N-Hydroxy-3-[1-methyl-4-(4-methylbenzoyl)-1H-pyrrol-2-yl]-2-propenamide (MC1293),

· (1R)-[(2S,3S)-3-[(E)-2-[(2S,3S)-3-[(1R)-Hydroxyethyl]oxiran-2-yl] vinyl]oxiran-2-yl]-2-propen-1-ol (Depudecin),

· N-(2-Aminophenyl)-4-(pyridin-3-ylmethyloxycarbonylaminomethyl)benzamide (MS 275),

· *cyclo*[L-(2-Amino-8-oxodecanoyl)-(1'-methoxy-L-tryptophyl)-L-isoleucyl-D-pipecolinyl] (Apicidin); and

· N-(2-Aminophenyl)-4-[4-(pyridin-3-yl)pyrimidin-2-ylaminomethyl]benzamide (free base of MGCD-0103).

or a salt thereof; and

[3] the intraocular pressure-lowering agent according to [1], wherein the compound having an HDAC inhibitory effect is a compound represented by the following general formula (1) or a salt thereof.

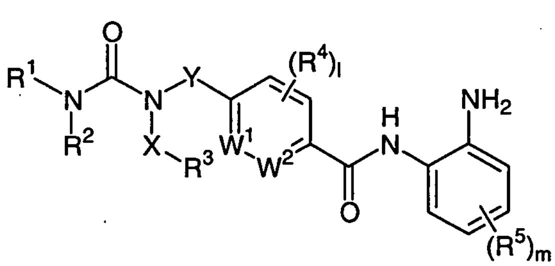

( 1 )

[wherein $R^1$ and $R^2$ are the same or different and represent a hydrogen atom, a lower alkyl group which may have a substituent, a lower alkenyl group which may have a substituent, a lower alkynyl group which may have a substituent or a group represented by the following general formula (2);

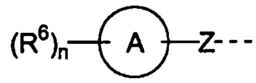 (2)

$R^3$ represents a hydroxy group, a lower alkoxy group which may have a substituent, a lower cycloalkyloxy group which may have a substituent, an aryloxy group which may have a substituent, a carboxy group, a lower alkoxycarbonyl group which may have a substituent, $-OCONR^aR^b$, $-NR^cR^d$ or a group represented by the following general formula (3);

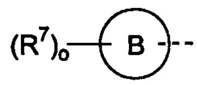 (3)

$R^4$ and $R^5$ are the same or different and represent a halogen atom, a lower alkyl group which may have a substituent, a hydroxy group, or a lower alkoxy group which may have a substituent;
$R^6$ represents a halogen atom, a lower alkyl group which may have a substituent, a lower cycloalkyl group which may have a substituent, an aryl group which may have a substituent, a heterocyclic group which may have a substituent, a hydroxy group, a lower alkoxy group which may have a substituent, a lower cycloalkyloxy group which may have a substituent, an aryloxy group which may have a substituent, a mercapto group, a lower alkylthio group which may have a substituent, a lower cycloalkylthio group which may have a substituent, an arylthio group which may have a substituent, a formyl group, a lower alkylcarbonyl group which may have a substituent, a carboxy group, a lower alkoxycarbonyl group which may have a substituent, a nitro group, a cyano group or $-NR^eR^f$;
$R^7$ represents a lower alkyl group which may have a substituent, a lower cycloalkyl group which may have a substituent, an aryl group which may have a substituent, a hydroxy group, a lower alkoxy group which may have a substituent, a lower cycloalkyloxy group which may have a substituent or an aryloxy group which may have a substituent;
$R^a$ and $R^b$ are the same or different and represent a hydrogen atom, a lower alkyl group which may have a substituent, a lower cycloalkyl group which may have a substituent, an aryl group which may have a substituent or a heterocyclic group which may have a substituent;
$R^c$, $R^d$, Re and $R^f$ are the same or different and represent a hydrogen atom, a lower alkyl group which may have a substituent, a lower cycloalkyl group which may have a substituent or an aryl group which may have a substituent;
the ring A represents a cyclic hydrocarbon or a heterocyclic ring;
the ring B represents a heterocyclic ring having one or plural heteroatoms selected from the group consisting of a nitrogen atom and an oxygen atom in the ring;
X represents a lower alkylene group which may have a substituent;
Y and Z are the same or different and represent a single bond or a lower alkylene group which may have a substituent;
$W^1$-$W^2$ represents N-C or C-N; and
1, m, n and o are the same or different and represent 0, 1, 2 or 3. The same shall apply hereinafter.].

The compound represented by the following general formula (1) or a salt thereof (hereinafter referred to as "the present compound") is a novel compound which has not been written in any documents.

Advantageous Effects of the Invention

The present invention provides intraocular pressure-lowering agent comprising a compound having an HDAC inhibitory effect as an active ingredient. The compound having an HDAC inhibitory effect has an effect of morphological change on trabecular meshwork cells and/or an effect of intraocular pressure reduction, and is therefore useful as a preventive and/or therapeutic agent for a disease associated with aqueous humor circulation and/or intraocular pressure, particularly as a therapeutic agent for glaucoma and/or ocular hypertension.

Best Mode for Carrying Out the Invention

Hereinafter, definitions of terms and phrases (activities, atoms, groups, rings and the like) to be used in the present specification and claims will be described in detail.
The "compound having an HDAC inhibitory effect" in the present invention refers to any compounds which can inhibit HDAC including families or subfamilies thereof and is not limited. Preferred compounds are exemplified by those

which exhibit an HDAC inhibitory effect in "Test for Evaluation of an HDAC Inhibitory Effect" under the item of "Pharmacological Tests".

Specific examples thereof include compounds disclosed in JP-A-2002-332267, JP-A-2001-64177, JP-A-2001-81031, JP-A-10-152462, JP-A-11-335375, JP-A-11-269140, JP-A-11-269146, JP-A-11-302173, JP-A-11-335373, US 20040162317, WO 2004069133, WO 2004052838, WO 2004087693, WO 2003087057, WO 2003092686, WO 2004035525, WO 2004069823, JP 2001316283, WO 2002030879, WO 2002022577, WO 2004092115, WO 2005019174, JP2007001885, Journal of Medicinal Chemistry (2002), 45, 753, European Journal of Medicinal Chemistry (2006), 41, 697 and the like, and compounds having an HDAC inhibitory effect under clinical development as an anticancer agent such as FK-228, PXD-101, MGCD-0103, LBH-589, NSC-696085, CG-781 and CRA-026440. The contents of the above-mentioned documents are incorporated by reference into this specification.

More specifically,

· (2E,4E,6R)-7-(4-Dimethylaminophenyl)-N-hydroxy-4,6-dimethyl-7-oxo-2,4-heptadienamide (Trichostatin A, CAS Registry No.58880-19-6)

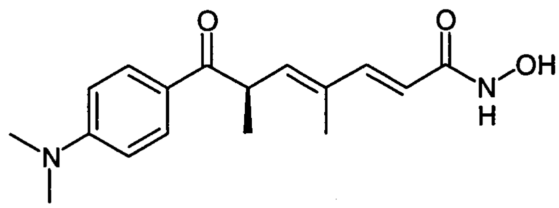

· N-Hydroxy-N'-phenyloctanediamide (SAHA, CAS Registry No.149647-78-9)

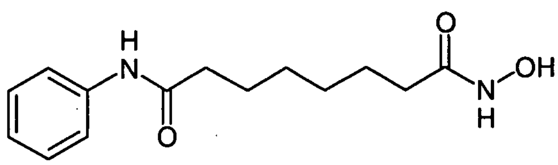

· 4-Dimethylamino-N-(6-hydroxycarbamoylhexyl)benzamide (M 344, CAS Registry No.251456-60-7)

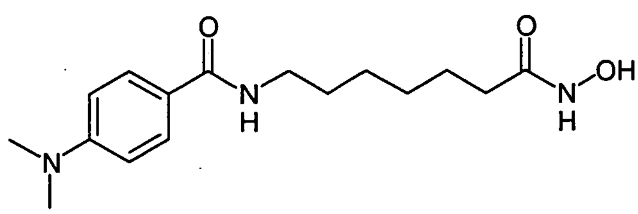

· (E)-N-Hydroxy-5-[3-(phenylsulfonylamino)phenyl]-2-penten-4-ynamide (Oxamflatin, CAS Registry No. 151720-43-3)

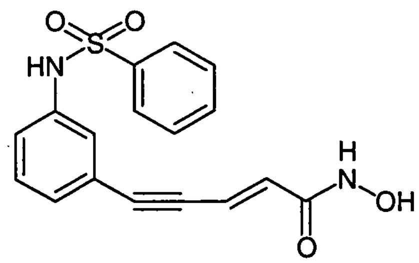

· N-Hydroxy-3-[3-hydroxyamino-3-oxo-1-propenyl]benzamide (CBHA, CAS Registry No.174664-65-4)

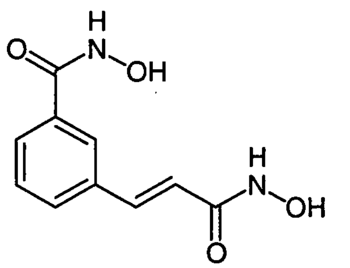

· (E)-N-Hydroxy-3-[1-methyl-4-(4-methylbenzoyl)-1H-pyrrol-2-yl]-2-propenamide (MC1293, CAS Registry No. 428872-06-4)

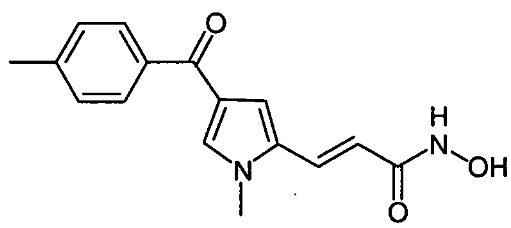

· (1R)-[(2S,3S)-3-[(E)-2-[(2S,3S)-3-[(1R)-Hydroxyethyl]oxiran-2-yl]vinyl]oxiran-2-yl]-2-propen-1-ol (Depudecin, CAS Registry No.139508-73-9)

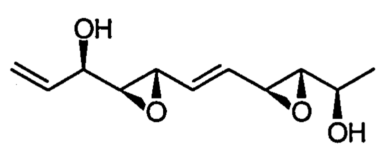

N-(2-Aminophenyl)-4-(pyridin-3-ylmethyloxycarbonylaminomethyl)benzamide (MS 275, CAS Registry No. 209783-80-2)

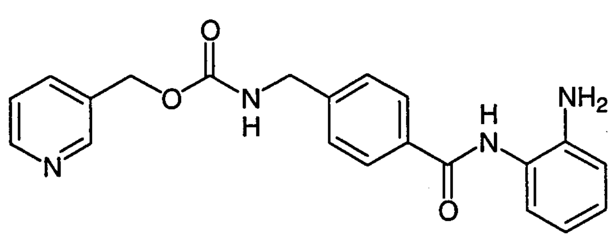

· *cyclo*[L-(2-Amino-8-oxodecanoyl)-(1'-methoxy-L-tryptophyl)-L-isoleucyl-D-pipecolinyl] (Apicidin, CAS Registry No. 183506-66-3)

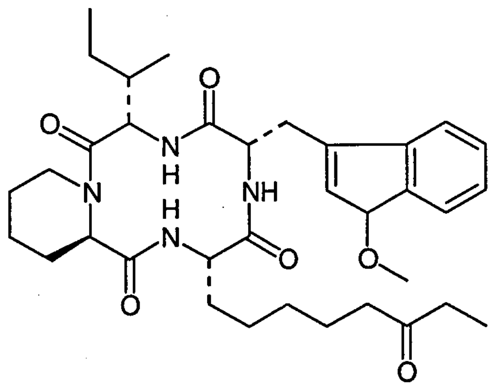

· N-(2-Aminophenyl)-4-[4-(pyridin-3-yl)pyrimidin-2-ylaminomethyl]benzamide (free base of MGCD-0103, CAS Registry No.726169-73-9)

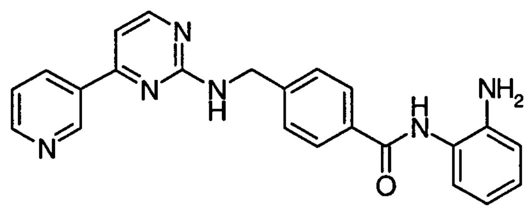

and the compounds represented by the above-mentioned general formula (1) and salts thereof are included.

The "halogen atom" refers to a fluorine, chlorine, bromine or iodine atom.

The "lower alkyl group" refers to a straight-chain or branched alkyl group having 1 to 8, preferably 1 to 6 carbon atoms.

Specific examples thereof include methyl, ethyl, n-propyl, n-butyl, n-pentyl, n-hexyl, n-heptyl, n-octyl, isopropyl, isobutyl, sec-butyl, tert-butyl and isopentyl groups.

The "lower alkenyl group" refers to a straight -chain or branched alkenyl group having 2 to 8, preferably 2 to 6 carbon atoms. Specific examples thereof include vinyl, propenyl, butenyl, pentenyl, hexenyl, heptenyl, octenyl, isopropenyl, 2-methyl-1-propenyl and 2-methyl-2-butenyl groups.

The "lower alkynyl group" refers to a straight-chain or branched alkynyl group having 2 to 8, preferably 2 to 6 carbon atoms. Specific examples thereof include ethynyl, propynyl, butynyl, pentynyl, hexynyl, heptynyl, octynyl isobutynyl and isopentynyl groups.

The "lower cycloalkyl group" refers to a cycloalkyl group having 3 to 8, preferably 3 to 6 carbon atoms. Specific examples thereof include cyclopropyl, cyclobutyl, cyclopentyl, cyclohexyl, cycloheptyl and cyclooctyl groups.

The "aryl group" refers to a residue formed by removing one hydrogen atom from a monocyclic aromatic hydrocarbon group, or a bicyclic or tricyclic condensed polycyclic aromatic hydrocarbon having 6 to 14 carbon atoms. Specific examples thereof include phenyl, naphthyl, anthryl and phenanthryl groups.

The "lower alkoxy group" refers to a group formed by substituting the hydrogen atom of a hydroxy group with a lower alkyl group. Specific examples thereof include methoxy, ethoxy, n-propoxy, n-butoxy, n-pentyloxy, n-hexyloxy, n-heptyloxy, n-octyloxy, isopropoxy, isobutoxy, sec-butoxy, tert-butoxy and isopentyloxy groups.

The "lower cycloalkyloxy group" refers to a group formed by substituting the hydrogen atom of a hydroxy group with a lower cycloalkyl group. Specific examples thereof include cyclopropyloxy, cyclobutyloxy, cyclopentyloxy, cyclohexyloxy, cycloheptyloxy and cyclooctyloxy groups.

The "aryloxy group" refers to a group formed by substituting the hydrogen atom of a hydroxy group with an aryl group. Specific examples thereof include phenoxy, naphthoxy, anthryloxy and phenanthryloxy groups.

The "lower alkylthio group" refers to a group formed by substituting the hydrogen atom of a mercapto group with a lower alkyl group. Specific examples thereof include methylthio, ethylthio, n-propylthio, n-butylthio, n-pentylthio,

n-hexylthio, n-heptylthio, n-octylthio, isopropylthio, isobutylthio, sec-butylthio, tert-butylthio and isopentylthio groups.

The "lower cycloalkylthio group" refers to a group formed by substituting the hydrogen atom of a mercapto group with a lower cycloalkyl group. Specific examples thereof include cyclopropylthio, cyclobutylthio, cyclopentylthio, cyclohexylthio, cycloheptylthio and cyclooctylthio groups.

The "arylthio group" refers to a group formed by substituting the hydrogen atom of a mercapto group with an aryl group. Specific examples thereof include phenylthio, naphthylthio, anthrylthio and phenanthrylthio groups.

The "lower alkylcarbonyl group" refers to a group formed by substituting the hydrogen atom of a formyl group with a lower alkyl group. Specific examples thereof include methylcarbonyl, ethylcarbonyl, n-propylcarbonyl, n-butylcarbonyl, n-pentylcarbonyl, n-hexylcarbonyl, n-heptylcarbonyl, n-octylcarbonyl, isopropylcarbonyl, isobutylcarbonyl, sec-butylcarbonyl, tert-butylcarbonyl and isopentylcarbonyl groups.

The "lower alkoxycarbonyl group" refers to a group formed by substituting the hydrogen atom of a formyl group with a lower alkoxy group. Specific examples thereof include methoxycarbonyl, ethoxycarbonyl, n-propoxycarbonyl, n-butoxycarbonyl, n-pentyloxycarbonyl, n-hexyloxycarbonyl, n-heptyloxycarbonyl, n-octyloxycarbonyl, isopropoxycarbonyl, isobutoxycarbonyl, sec-butoxycarbonyl, tert-butoxycarbonyl and isopentyloxycarbonyl groups.

The "heterocyclic ring" refers to a saturated or unsaturated monocyclic heterocyclic ring, or bicyclic or tricyclic condensed polycyclic heterocyclic ring having one or plural heteroatoms selected from a nitrogen atom, an oxygen atom and a sulfur atom in the ring.

Specific examples of the saturated monocyclic heterocyclic ring include aziridine, azetidine, pyrrolidine, pyrazolidine, imidazolidine, triazolidine, piperidine, hexahydropyridazine, hexahydropyrimidine, piperazine, homopiperidine and homopiperazine, having a nitrogen atom in the ring; tetrahydrofuran, tetrahydropyran, 1,4-dioxane and 1,2-dioxirane, having an oxygen atom in the ring; tetrahydrothiophene and tetrahydrothiopyran, having a sulfur atom in the ring; oxazolidine, isoxazolidine and morpholine, having a nitrogen atom and an oxygen atom in the ring; and thiazolidine, isothiazolidine and thiomorpholine, having a nitrogen atom and a sulfur atom in the ring.

Further, such a saturated monocyclic heterocyclic ring may be condensed with a benzene ring or the like to form a bicyclic or tricyclic condensed polycyclic heterocyclic ring such as dihydroindole, dihydroindazole, dihydrobenzimidazole, tetrahydroquinoline, tetrahydroisoquinoline, tetrahydrocinnoline, tetrahydrophthalazine, tetrahydroquinazoline, tetrahydroquinoxaline, dihydrobenzofuran, dihydroisobenzofuran, chroman, isochroman, benzo[1,3]dioxole, 2,3-dihydrobenzo[1,4]dioxin, dihydrobenzothiophene, dihydroisobenzothiophene, thiochroman, isothiochroman, dihydrobenzoxazole, dihydrobenzisoxazole, dihydrobenzoxazine, dihydrobenzothiazole, dihydrobenzoisothiazole, dihydrobenzothiazine, xanthene, 4a-carbazole or perimidine.

Specific examples of the unsaturated monocyclic heterocyclic ring include dihydropyrrole, pyrrole, dihydropyrazole, pyrazole, dihydroimidazole, imidazole, dihydrotriazole, triazole, tetrahydropyridine, dihydropyridine, pyridine, tetrahydropyridazine, dihydropyridazine, pyridazine, tetrahydropyrimidine, dihydropyrimidine, pyrimidine, tetrahydropyrazine, dihydropyrazine and pyrazine, having a nitrogen atom in the ring; dihydrofuran, furan, dihydropyran and pyran, having an oxygen atom in the ring; dihydrothiophene, thiophene, dihydrothiopyran and thiopyran, having a sulfur atom in the ring; dihydrooxazole, oxazole, dihydroisoxazole, isoxazole, dihydrooxazine and oxazine, having a nitrogen atom and an oxygen atom in the ring; dihydrothiazole, thiazole, dihydroisothiazole, isothiazole, dihydrothiazine and thiazine, having a nitrogen atom and a sulfur atom in the ring.

Further, such an unsaturated monocyclic heterocyclic ring may be condensed with a benzene ring or the like to form a bicyclic or tricyclic condensed polycyclic heterocyclic ring such as indole, indazole, benzimidazole, benzotriazole, dihydroquinoline, quinoline, dihydroisoquinoline, isoquinoline, phenanthridine, dihydrocinnoline, cinnoline, dihydrophthalazine, phthalazine, dihydroquinazoline, quinazoline, dihydroquinoxaline, quinoxaline, benzofuran, isobenzofuran, chromen, isochromen, benzothiophene, isobenzothiophene, thiochromen, isothiochromen, benzoxazole, benzisoxazole, benzoxazine, benzothiazole, benzoisothiazole, tetrahydrobenzothiazole, benzothiazine, phenoxanthine, carbazole, 6-carboline, phenanthridine, acridine, phenanthroline, phenazine, phenothiazine or phenoxazine.

Further, among these heterocyclic rings, in the case of a heterocyclic ring having two hydrogen atoms on the same carbon atom, these hydrogen atoms may be substituted with an oxo group to form a heterocyclic ketone such as 2-pyrrolidone, 4-piperidone, 4-thiazolidone, pyran-4-(4H)-one or pyrazin-2-(3H)-one, and these heterocyclic ketones are also included in the scope of the heterocyclic ring of the invention.

The "heterocyclic ring having one or plural heteroatoms selected from the group consisting of a nitrogen atom and an oxygen atom in the ring" refers to a heterocyclic ring having one or plural nitrogen atoms and/or oxygen atoms in the ring among the above-mentioned heterocyclic rings.

The "heterocyclic group" refers to a residue formed by removing one hydrogen atom from a heterocyclic ring.

The "cyclic hydrocarbon" refers to a saturated or unsaturated monocyclic hydrocarbon, or bicyclic or tricyclic hydrocarbon having 3 to 10 carbon atoms.

Specific examples of the saturated monocyclic hydrocarbon include cyclopropane, cyclobutane, cyclopentane, cyclohexane, cycloheptane and cyclooctane.

Specific examples of the saturated bicyclic hydrocarbon include octahydropentalene, octahydroindene and

decahydronaphthalene.

Specific examples of the saturated tricyclic hydrocarbon include bicyclo[2.2.1]heptane.

Specific examples of the unsaturated monocyclic hydrocarbon include cyclopentene, cyclohexene, cyclopentadiene, cyclohexadiene and benzene.

Specific examples of the unsaturated bicyclic hydrocarbon include indan, 1,2,3,4-tetrahydronaphthalene and naphthalene.

The "lower alkylene group" refers to a straight-chain or branched alkylene group having 1 to 8, preferably 1 to 6 carbon atoms. Specific examples thereof include methylene, ethylene, trimethylene, tetramethylene, pentamethylene, hexamethylene, heptamethylene, octamethylene, methylmethylene and ethylmethylene.

The "lower alkyl group which may have a substituent", "lower alkenyl group which may have a substituent", "lower alkynyl group which may have a substituent", "lower alkoxy group which may have a substituent", "lower alkylthio group which may have a substituent", "lower alkylcarbonyl group which may have a substituent", "lower alkoxycarbonyl group which may have a substituent" and/or "lower alkylene group which may have a substituent" refers to a "lower alkyl group", a "lower alkenyl group", a "lower alkynyl group", a "lower alkoxy group", a "lower alkylthio group", a "lower alkylcarbonyl group", a "lower alkoxycarbonyl group" and/or a "lower alkylene group" which may have one or plural substituents selected from the group consisting of a halogen atom, a lower cycloalkyl group, an aryl group, a heterocyclic group, a nitro group, a cyano group, an oxo group, $-OR^p$, $-SR^q$, $-COR^r$, $-COOR^s$, $-CONR^tR^u$ and $-NR^vR^w$.

The "lower cycloalkyl group which may have a substituent", "aryl group which may have a substituent", "heterocyclic group which may have a substituent", "lower cycloalkyloxy group which may have a substituent" and/or "aryloxy group which may have a substituent" refers to a "lower cycloalkyl group", an "aryl group", a "heterocyclic group", a "lower cycloalkyloxy group" and/or an "aryloxy group" which may have one or plural substituents selected from the group consisting of a halogen atom, a lower alkyl group, a lower alkenyl group, a lower alkynyl group, a lower cycloalkyl group, an aryl group, a heterocyclic group, a nitro group, a cyano group, an oxo group, $-OR^p$, $-SR^q$,- $COR^q$, $-COOR^r$, $-CONR^sR^t$ and $-NR^uR^v$.

Here, $R^p$, $R^q$, $R^r$, $R^s$, $R^t$, $R^u$, $R^v$ and $R^w$ are the same or different and represent a group selected from the group consisting of a hydrogen atom, a lower alkyl group, a lower alkenyl group, a lower alkynyl group, a lower cycloalkyl group, an aryl group and a heterocyclic group.

With regard to the term "plural groups" as used herein, the respective groups may be the same or different, and the number of the groups is preferably 2 or 3, particularly preferably 2. Further, a hydrogen atom and a halogen atom are also included in the concept of the "group".

In the invention, when "l", "m", "n" and/or "o" represents 2 or 3, the respective plural groups represented by $R^4$, $R^5$, $R^6$ or $R^7$ may be the same or different. Incidentally, when "l", "m", "n" and/or "o" represents 0, the respective groups represented by $R^4$, $R^5$, $R^6$ and/or $R^7$ do not exist. That is, it shows that the compound does not have the substituents.

The active ingredient of the invention may be in the form of a salt, and the salt is not particularly limited as long as it is a pharmaceutically acceptable salt. Examples thereof include salts with an inorganic acid such as hydrochloric acid, hydrobromic acid, hydroiodic acid, nitric acid, sulfuric acid or phosphoric acid; salts with an organic acid such as acetic acid, fumaric acid, maleic acid, succinic acid, citric acid, tartaric acid, adipic acid, gluconic acid, glucoheptonic acid, glucuronic acid, terephthalic acid, methanesulfonic acid, lactic acid, hippuric acid, 1,2-ethanedisulfonic acid, isethionic acid, lactobionic acid, oleic acid, pamoic acid, polygalacturonic acid, stearic acid, tannic acid, trifluoromethanesulfonic acid, benzenesulfonic acid, p-toluenesulfonic acid, lauryl sulfate, methyl sulfate, naphthalenesulfonic acid or sulfosalicylic acid; quaternary ammonium salts with methyl bromide, methyl iodide or the like; salts with a halogen ion such as a bromine ion, a chlorine ion or an iodine ion; salts with an alkali metal such as lithium, sodium or potassium; salts with an alkaline earth metal such as calcium or magnesium; salts with a metal such as iron or zinc; salts with ammonia; and salts with an organic amine such as triethylenediamine, 2-aminoethanol, 2,2-iminobis(ethanol), 1-deoxy-1-(methylamino)-2-D-sorbitol, 2-amino-2-(hydroxymethyl)-1,3-propanediol, procaine or N,N-bis(phenylmethyl)-1,2-ethanediamine.

In the case where there are geometric isomers or optical isomers in the active ingredient of the invention, these isomers are also included in the scope of the invention.

Further, the active ingredient of the invention may be in the form of a hydrate or a solvate.

Further, in the case where there is proton tautomerism in the active ingredient of the invention, the tautomeric isomers thereof are also included in the invention.

In the case where there are crystalline polymorphisms and crystalline polymorphism groups (crystalline polymorphism systems) in the active ingredient of the invention, these crystalline polymorphisms and crystalline polymorphism groups (crystalline polymorphism systems) are also included in the invention. Here, the crystalline polymorphism groups (crystalline polymorphism systems) mean individual crystal forms in respective stages when the crystal forms are changed by conditions for the production, crystallization, storage or the like of these crystals and/or states thereof (the states also include a formulated state) and/or all the processes thereof.

(a) Preferred examples of the present compound include compounds in which the respective groups are as defined below and salts thereof in the compounds represented by the general formula (1) and salts thereof:

(a1) $R^1$ and $R^2$ are the same or different and represent a hydrogen atom, a lower alkyl group, a lower alkyl group having a halogen atom as a substituent, a lower alkyl group having a methoxy group as a substituent, a lower alkyl group having a methylthio group as a substituent, a lower alkyl group having a cyano group as a substituent, a lower alkyl group having a methylaminocarbonyl group as a substituent, a lower alkyl group having a diisopropylamino group as a substituent, a lower alkenyl group, a lower alkynyl group or a group represented by the following general formula (2); and/or

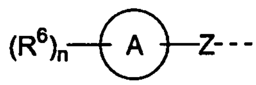 (2)

(a2) $R^3$ represents a hydroxy group, a lower alkoxy group, a lower cycloalkyloxy group, an aryloxy group, a carboxy group, a lower alkoxycarbonyl group, $-OCONR^aR^b$, $-NR^cR^d$ or a group represented by the following general formula (3); and/or

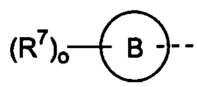 (3)

(a3) $R^4$ and $R^5$ are the same or different and represent a halogen atom, a lower alkyl group, a hydroxy group or a lower alkoxy group; and/or

(a4) $R^6$ represents a halogen atom, a lower alkyl group, a lower alkyl group having a halogen atom as a substituent, a lower alkyl group having a cyano group as a substituent, a lower cycloalkyl group, an aryl group, a heterocyclic group, a hydroxy group, a lower alkoxy group, a lower alkoxy group having a halogen atom as a substituent, a lower alkoxy group having an aryl group as a substituent, a lower cycloalkyloxy group, an aryloxy group, a mercapto group, a lower alkylthio group, a lower cycloalkylthio group, an arylthio group, a formyl group, a lower alkylcarbonyl group, a carboxy group, a lower alkoxycarbonyl group, a nitro group, a cyano group or $-NR^eR^f$; and/or

(a5) $R^7$ represents a lower alkyl group, a lower cycloalkyl group, an aryl group, a hydroxy group, a lower alkoxy group, a lower cycloalkyloxy group or an aryloxy group; and/or

(a6) $R^a$ and $R^b$ are the same or different and represent a hydrogen atom, a lower alkyl group, a lower cycloalkyl group, an aryl group or a heterocyclic group; and/or

(a7) $R^c$, $R^d$, $R^e$ and $R^f$ are the same or different and represent a hydrogen atom, a lower alkyl group, a lower cycloalkyl group or an aryl group; and/or

(a8) the ring A represents a cyclic hydrocarbon or a heterocyclic ring; and/or

(a9) the ring B represents a heterocyclic ring having one or plural heteroatoms selected from the group consisting of a nitrogen atom and an oxygen atom in the ring; and/or

(a10) X represents a lower alkylene group; and/or

(a11) Y and Z are the same or different and represent a single bond, a lower alkylene group or a lower alkylene group having an oxo group as a substituent; and/or

(a12) $W^1$-$W^2$ represents N-C or C-N; and/or

(a13) 1, m, n and o are the same or different and represent 0, 1, 2 or 3.

That is, preferred examples of the present compound include, in the compounds represented by the general formula (1), compounds which comprise one or a combination of two or more selected from the group consisting of the above-mentioned (a1), (a2), (a3), (a4), (a5), (a6), (a7), (a8), (a9), (a10), (a11), (a12) and (a13) and salts thereof.

(b) More preferred examples of the present compound include compounds in which the respective groups are as defined below and salts thereof in the compounds represented by the general formula (1) and salts thereof:

(b1) $R^1$ represents a lower alkyl group, a lower alkyl group having a halogen atom as a substituent, a lower alkyl group having a methoxy group as a substituent, a lower alkyl group having a methylthio group as a

substituent, a lower alkyl group having a cyano group as a substituent, a lower alkyl group having a methylaminocarbonyl group as a substituent, a lower alkyl group having a diisopropylamino group as a substituent, a lower alkynyl group or a group represented by the following general formula (2); and/or

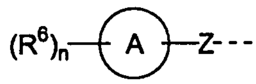 (2)

(b2) $R^2$ represents a hydrogen atom; and/or
(b3) $R^3$ represents a hydroxy group, a carboxy group, a lower alkoxycarbonyl group, $-OCONR^aR^b$, $-NR^cR^d$ or a group represented by the following general formula (3); and/or

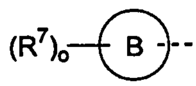 (3)

(b4) $R^6$ represents a halogen atom, a lower alkyl group, a lower alkyl group having a halogen atom as a substituent, a lower alkyl group having a cyano group as a substituent, an aryl group, a morpholino group, a hydroxy group, a lower alkoxy group, a lower alkoxy group having a halogen atom as a substituent, a lower alkoxy group having an aryl group as a substituent, a lower alkylthio group, a lower alkylcarbonyl group, a nitro group, a cyano group or $-NR^eR^f$; and/or
(b5) $R^7$ represents a lower alkyl group or a lower alkoxy group; and/or
(b6) $R^a$ and $R^b$ are the same or different and represent a hydrogen atom or a heterocyclic group; and/or
(b7) $R^c$, $R^d$, $R^e$ and $R^f$ represent a lower alkyl group; and/or
(b8) the ring A represents a cyclic hydrocarbon or a heterocyclic ring; and/or
(b9) the ring B represents a heterocyclic ring having plural heteroatoms selected from the group consisting of a nitrogen atom and an oxygen atom in the ring; and/or
(b10) X and Y represent a lower alkylene group; and/or
(b11) Z represents a single bond, a lower alkylene group or a lower alkylene group substituted with an oxo group; and/or
(b12) $W^1$-$W^2$ represents C-N or N-C; and/or
(b13) 1 and m represent 0; and/or
(b14) o represents 0 or 1; and/or
(b15) n represents 0, 1 or 2.
That is, more preferred examples of the present compound include, in the compounds represented by the general formula (1), compounds which comprise one or a combination of two or more selected from the group consisting of the above-mentioned (b1), (b2), (b3), (b4), (b5), (b6), (b7), (b8), (b9), (b10), (b11), (b12), (b13), (b14) and (b15) and salts thereof.

(c) Preferred examples of the ring A include the following rings.
The ring A represents benzene, indan, thiophene, benzo[1,3]dioxole, 2,3-dihydrobenzofuran, 1H-benzimidazole, isoxazole, thiazole, benzothiazole, 2,3-dihydrobenzo[1,4]dioxin or pyridine.
Further, compounds which have the ring A and satisfy the requirements of the above-mentioned (a), (b), and/or the following (d) and salts thereof are particularly preferred.
(d) Other preferred examples of the ring B include the following rings.
The ring B represents pyrrolidine or morpholine.
Further, compounds which have the ring B and satisfy the requirements of the above-mentioned (a), (b), and/or (c) and salts thereof are particularly preferred.
(e) Particularly preferred specific examples of the present compound include the following compounds and salts thereof.

· N-(2-Aminophenyl)-5-[3-(2,3-dihydrobenzo[1,4]dioxin-6-yl)-1-(3-dimethylaminopropyl)ureidomethyl]pyridine-2-carboxylic acid amide,
· N-(2-Aminophenyl)-5-[3-(4-dimethylaminophenyl)-1-(3-(morpholin-4-yl) propyl)ureidomethyl]pyridine-2-carboxylic acid amide,

· N-(2-Aminophenyl)-5-[1-(3-(morpholin-4-yl)propyl)-3-phenethylureidomethyl] pyridine-2-carboxylic acid amide,
· N-(2-Aminophenyl)-5-[3-(3,4-difluorophenyl)-1-(3-dimethylaminopropyl) ureidomethyl]pyridine-2-carboxylic acid amide,
· N-(2-Aminophenyl)-5-[1-(3-dimethylaminopropyl)-3-(4-methoxyphenyl) ureidomethyl]pyridine-2-carboxylic acid amide,
· N-(2-Aminophenyl)-5-[3-(4-diethylaminophenyl)-1-(3-dimethylaminopropyl) ureidomethyl]pyridine-2-carboxylic acid amide,
· N-(2-Aminophenyl)-5-[1-(3-dimethylaminopropyl)-3-(3-fluorophenyl) ureidomethyl]pyridine-2-carboxylic acid amide,
· N-(2-Aminophenyl)-5-[1-(3-dimethylaminopropyl)-3-(3-fluoro-4-methylphenyl)ureidomethyl]pyridine-2-carboxylic acid amide,
· N-(2-Aminophenyl)-5-[1-(3-dimethylaminopropyl)-3-(4-fluoro-3-methylphenyl)ureidomethyl]pyridine-2-carboxylic acid amide,
· N-(2-Aminophenyl)-5-[3-(4-cyanophenyl)-1-(3-dimethylaminopropyl) ureidomethyl]pyridine-2-carboxylic acid amide,
· N-(2-Aminophenyl)-5-[1-(3-dimethylaminopropyl)-3-(4-trifluoromethoxyphenyl) ureidomethyl]pyridine-2-carboxylic acid amide,
· N-(2-Aminophenyl)-5-[3-(benzo[1,3]dioxol-5-yl)-1-(3-hydroxypropyl) ureidomethyl]pyridine-2-carboxylic acid amide,
· N-(2-Aminophenyl)-5-[3-(4-dimethylaminophenyl)-1-(2-ethoxycarbonylethyl)ureidomethyl]pyiidine-2-carboxylic acid amide,
· N-(2-Aminophenyl)-5-[3-(1,3-benzothiazol-2-yl)-1-(3-dimethylaminopropyl)ureidomethyl]pyridine-2-carboxylic acid amide,
· N-(2-Aminophenyl)-5-[3-(1H-benzimidazol-2-yl)-1-(3-dimethylaminopropyl)ureidomethyl]pyridine-2-carboxylic acid amide,
· N-(2-Aminophenyl)-5-[3-(2,3-dihydro-1-benzofuran-5-yl)-1-(3-(morpholin-4-yl)propyl)ureidomethyl]pyridine-2-carboxylic acid amide,
· N-(2-Aminophenyl)-5-[3-(2,3-dihydrobenzo[1,4]dioxin-6-yl)-1-(2-hydroxyethyl)ureidomethyl]pyridine-2-carboxylic acid amide,
· N-(2-Aminophenyl)-5-[1-(3-dimethylaminopropyl)-3-(phenylcarbonylmethyl)ureidomethyl]pyridine-2-carboxylic acid amide,
· N-(2-Aminophenyl)-5-[1-(3-dimethylaminopropyl)-3-(1,3-thiazol-2-yl)ureidomethyl]pyridine-2-carboxylic acid amide,
· N-(2-Aminophenyl)-5-[1-(3-dimethylaminopropyl)-3-(6-methoxy-1,3-benzothiazol-2-yl)ureidomethyl]pyridine-2-carboxylic acid amide,
· N-(2-Aminophenyl)-5-[1-(3-dimethylaminopropyl)-3-(6-fluoro-1,3-benzothiazol-2-yl)ureidomethyl]pyridine-2-carboxylic acid amide,
· N-(2-Aminophenyl)-5-[1-(3-diethylaminopropyl)-3-(3,4-difluorophenyl) ureidomethyl]pyridine-2-carboxylic acid amide,
· N-(2-Aminopheny])-5-[1-(3-(morpholin-4-yl)propyl)-3-(pyridin-3-yl)ureidomethyl]pyridine-2-carboxylic acid amide,
· N-(2-Aminophenyl)-5-[1-(3-dimethylaminopropyl)-3-(thiophen-3-yl)ureidomethyl]pyridine-2-carboxylic acid amide,
· N-(2-Aminophenyl)-5-[1-(3-dimethylaminopropyl)-3-(3-fluoro-4-methoxyphenyl)ureidomethyl]pyridine-2-carboxylic acid amide,
· N-(2-Aminophenyl)-5-[3-(3-chloro-4-fluorophenyl)-1-(3-diethylaminopropyl)ureidomethyl]pyridine-2-carboxylic acid amide,
· N-(2-Aminophenyl)-5-[1-(3-diethylaminopropyl)-3-(4-fluoro-3-nitrophenyl)ureidomethyl]pyridine-2-carboxylic acid amide,
· N-(2-Aminophenyl)-5-[3-(2,3-dihydrobenzo[1,4]dioxin-6-yl)-1-(2-dimethylaminoethyl)ureidomethyl]pyridine-2-carboxylic acid amide,
· N-(2-Aminophenyl)-5-[1-(2-dimethylaminoethyl)-3-(3-methoxyphenyl) ureidomethyl]pyridine-2-carboxylic acid amide,
· N-(2-Aminophenyl)-5-[1-(2-dimethylaminoethyl)-3-(3-fluorophenyl) ureidomethyl]pyridine-2-carboxylic acid amide,
· N-(2-Aminophenyl)-5-[1-(2-dimethylaminoethyl)-3-(thiophen-3-yl) ureidomethyl]pyridine-2-carboxylic acid amide,

· N-(2-Aminophenyl)-5-[3-(pyridin-3-yl)-1-[3-(pyrrolidin-1-yl)propyl] ureidomethyl]pyridine-2-carboxylic acid amide,
· N-(2-Aminophenyl)-5-[1-(3-(morpholin-4-yl)propyl)-3-(phenylcarbonylmethyl) ureidomethyl]pyridine-2-carboxylic acid amide,
· N-(2-Aminophenyl)-5-[3-(3-chlorophenyl)-1-(3-(morpholin-4-yl)propyl) ureidomethyl]pyridine-2-carboxylic acid amide,
· N-(2-Aminophenyl)-5-[3-(4-fluorophenethyl)-1-(3-(morpholin-4-yl) propyl)ureidomethyl]pyridine-2-carboxylic acid amide,
· N-(2-Aminophenyl)-5-[3-(4-cyanophenyl)-1-(3-hydroxypropyl) ureidomethyl]pyridine-2-carboxylic acid amide,
· N-(2-Aminophenyl)-5-[3-(3-fluorophenyl)-1-[2-(4-methylpiperazin-1-yl)ethyl]ureidomethyl]pyridine-2-carboxylic acid amide,
· N-(2-Aminophenyl)-5-[3-(2,3-dihydrobenzo[1,4]dioxin-6-yl)-1-[3-(4-methylpiperazin-1-yl)propyl]ureidomethyl] pyridine-2-carboxylic acid amide,
· N-(2-Aminophenyl)-5-[1-(2-dimethylaminoethyl)-3-(3-methylphenyl) ureidomethyl]pyridine-2-carboxylic acid amide;
· N-(2-Aminophenyl)-5-[3-(2,3-dihydrobenzo[1,4]dioxin-6-yl)-1-[4-(morpholin-4-yl)butyl]ureidomethyl]pyridine-2-carboxylic acid amide,
· N-(2-Aminophenyl)-5-[1-(2-dimethylaminoethyl)-3-(1,3-thiazol-2-yl)ureidomethyl]pyridine-2-carboxylic acid amide,
· N-(2-Aminophenyl)-5-[3-(2-methoxyphenyl)-1-[4-(morpholin-4-yl)butyl]ureidomethyl]pyridine-2-carboxylic acid amide,
· N-(2-Aminophenyl)-5-[3-(3-methylphenyl)-1-[3-(4-methylpiperidin-1-yl)propyl]ureidomethyl]pyridine-2-carboxylic acid amide; and
· N-(2-Aminophenyl)-5-[3-cyclopentyl-1-[5-(morpholin-4-yl)pentyl] ureidomethyflpyridine-2-carboxyhc acid amide.

The present compounds can be prepared according to the following methods. Each specific process for preparing this present compounds are described in detail in the following examples (section of Production Examples). Additionally, the term "Boc" used in the following synthetic routes represents a tert-butoxycarbonyl group. In the case that an oxygen atom, a nitrogen atom, a sulfur atom, and so on are contained in $R^1$, $R^2$, $R^3$, $R^4$, $R^5$ of the following scheme, they will be able to be protected or deprotected by generally used methods.
The processes for preparing the present compounds are divided roughly into the methods described below, and the suitable method can be chosen according to the kind of substituent.
1) The present compound (I) can be synthesized according to the synthetic route 1. That is, the compound (I) can be given by the treatment of the compound (II) in an organic solvent such as methanol in the presence of an acid such as hydrogen chloride-ethyl acetate solution at 0˚C to room temperature for 30 minutes to 24 hours.

Synthetic Route 1

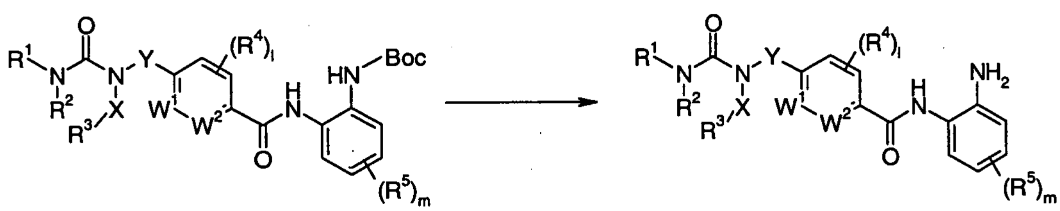

(II) (I)

The compound (IIa, $R^2$=H) can be synthesized according to the synthetic route 1-1. That is, it can be given by the reaction of the compound (III) with an isocyanate (IV) in an organic solvent such as dichloromethane at 0˚C to room temperature for 30 minutes to 24 hours.

Synthetic Route 1-1

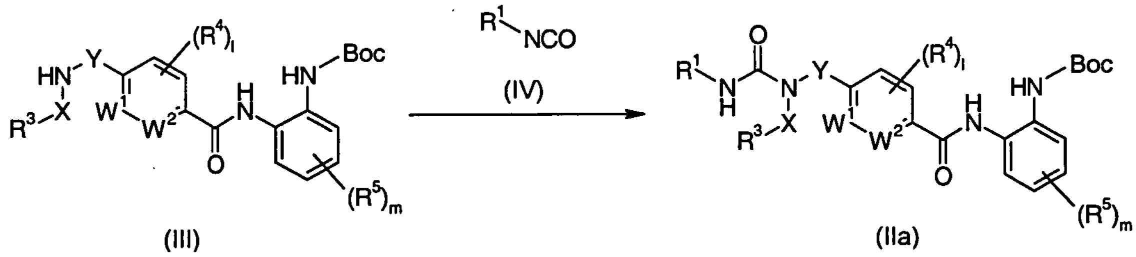


(III) (IIa)

The compound (III) can be synthesized according to the synthetic route 1-2. That is, it can be given by the reaction of the sulfonate (V) with an amine (VI) at 0˚C to room temperature for 30 minutes to 24 hours.

Synthetic Route 1-2

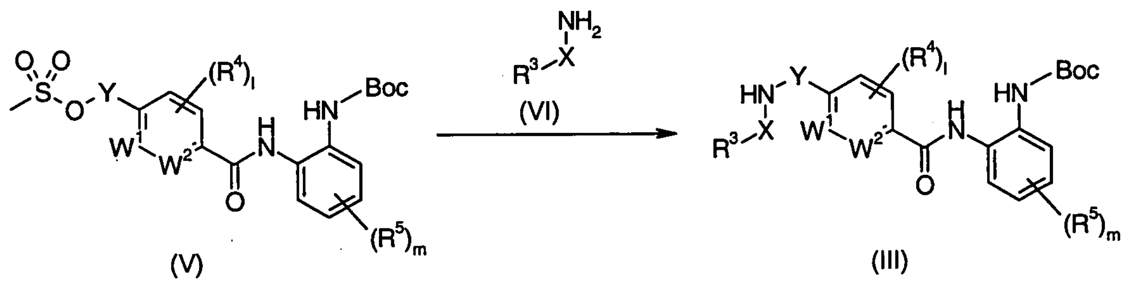


(V) (III)

The compound (V) can be synthesized according to the synthetic route 1-3. That is, it can be given by the reaction of the compound (VII) with methanesulfonyl chloride (VIII) in an organic solvent such as dichloromethane in the presence of a base such as triethylamine at 0˚C to room temperature for 30 minutes to 3 hours.

Synthetic Route 1-3

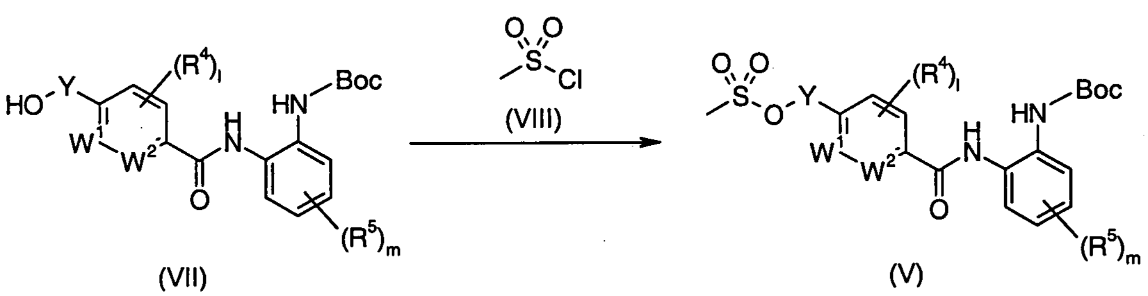


(VII) (V)

The compound (VIIa, Y=$CH_2$) can be synthesized according to the synthetic route 1-4. That is, it can be given by the treatment of the compound (IX) in an organic solvent such as tetrahydrofuran (hereinafter referred to as THF) in the presence of a reducing reagent such as lithium borohydride at 0˚C to room temperature for 30 minutes to 24 hours.

Synthetic Route 1-4

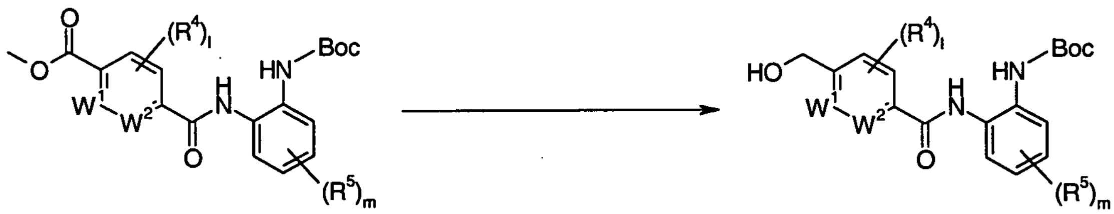

(IX) (VIIa)

The compound (IX) can be synthesized according to the synthetic route 1-5. That is, it can be given by the reaction of the compound (X) with the compound (XI) in an organic solvent such as N,N-dimethylformamide (hereinafter referred to as DMF) in the presence of a condensing reagent such as O-(7-azabenzotriazol-1-yl)-1,1,3,3-tetramethyluronium hexafluorophosphate (hereinafter referred to as HATU) and a base such as N,N-diisopropylethylamine at room temperature for 1 hour to 24 hours.

Synthetic Route 1-5

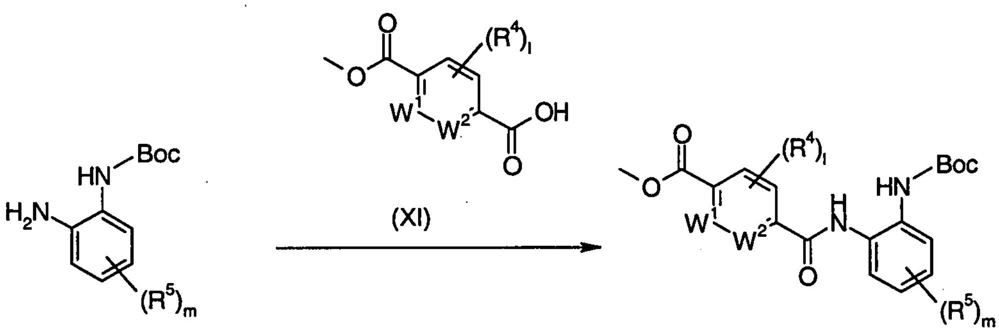

(XI)

(X) (IX)

The compound (X) can be synthesized according to the synthetic route 1-6. That is, it can be given by the reaction of the compound (XII) with di-tert-butyl dicarbonate (XIII) in an organic solvent such as THF in the presence of a base such as triethylamine at room temperature for 1 hour to 24 hours.

Synthetic Route 1-6

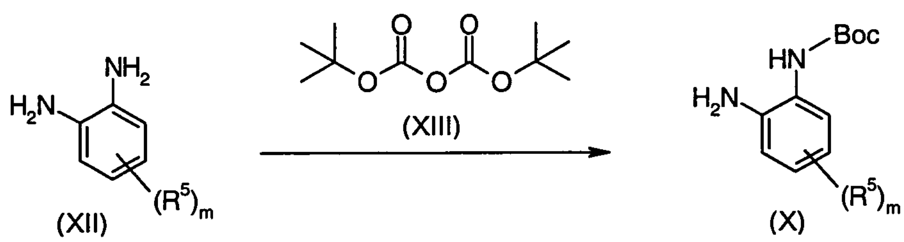

(XIII)

(XII) (X)

2) The compound (II) can be synthesized according to the synthetic route 2. That is, it can be given by the reaction of the compound (III) with an amine (XIV) in an organic solvent such as THF in the presence of a reagent for urea formation such as 1,1'-carbonyldiimidazole at 0°C to 60°C for 30 minutes to 24 hours.

Synthetic Route 2

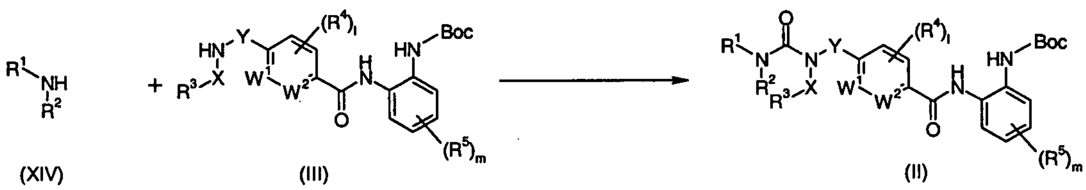

(XIV) (III) (II)

Although a pharmacological effect of the present compound will be described in detail in the following Examples under the item of "Pharmacological Tests", the present inventors studied an effect of morphological change on trabecular meshwork cells by a large number of compounds having an HDAC inhibitory effect and different chemical structures. That is, they studied an effect of cell morphological change by the compounds on trabecular meshwok cells in an evaluation system using the cell shape index (hereinafter referred to as "CSI") reported in The Journal of Clinical Investigation, 103, 1141-1150 (1999) as an index, and as a result, they found that the compounds have an excellent effect of morphological change of trabecular meshwork cells.

Further, they studied an effect of intraocular pressure reduction of the compounds by intracameral administration using male Japanese White rabbits in order to confirm an actual effect of intraocular pressure reduction of the compounds, and as a result, they confirmed that the compounds have an effect of intraocular pressure reduction.

Accordingly, the compounds having an HDAC inhibitory effect can be used as an intraocular pressure-lowering agent and are useful as a preventive and/or therapeutic agent for a disease considered to be associated with aqueous humor circulation and/or intraocular pressure and are particularly useful as a preventive and/or therapeutic agent for glaucoma or ocular hypertension.

The intraocular pressure-lowering agent of the invention can be administered orally or parenterally. Examples of the dosage form include a tablet, a capsule, a granule, a powder, an injection and an eye drop, and such a preparation can be prepared by a widely used technique.

For example, an oral preparation such as a tablet, a capsule, a granule or a powder can be prepared by optionally adding a necessary amount of an excipient such as lactose, mannitol, starch, crystalline cellulose, light silicic anhydride, calcium carbonate or calcium hydrogen phosphate; a lubricant such as stearic acid, magnesium stearate or talc; a binder such as starch, hydroxypropyl cellulose, hydroxypropylmethyl cellulose or polyvinylpyrrolidone; a disintegrant such as carboxymethyl cellulose, low-substituted hydroxypropylmethyl cellulose or calcium citrate; a coating agent such as hydroxypropylmethyl cellulose, macrogol or a silicone resin; a stabilizer such as ethyl parahydroxybenzoate or benzyl alcohol; a corrigent such as a sweetener, a sour agent or a flavor; or the like.

Further, a parenteral preparation such as an injection or an eye drop can be prepared by optionally adding a necessary amount of a tonicity agent such as sodium chloride, concentrated glycerin, propylene glycol, polyethylene glycol, potassium chloride, sorbitol or mannitol; a buffer such as sodium phosphate, sodium hydrogen phosphate, sodium acetate, citric acid, glacial acetic acid or trometamol; a surfactant such as polysorbate 80, polyoxy 40 stearate or polyoxyethylene hydrogenated castor oil 60; a stabilizer such as sodium citrate or sodium edetate; a preservative such as benzalkonium chloride, paraben, benzethonium chloride, parahydroxybenzoate ester, sodium benzoate, chlorobutanol or sorbic acid; a pH adjusting agent such as hydrochloric acid, citric acid, phosphoric acid, glacial acetic acid, sodium hydroxide, sodium carbonate or sodium hydrogen carbonate; a soothing agent such as benzyl alcohol; or the like.

The invention also relates to a method for changing morphology of trabecular meshwork cells and/or a method for lowering intraocular pressure, comprising administering an effective amount of the present compound to a patient; a method for preventing and/or treating a disease associated with aqueous humor circulation and/or intraocular pressure, comprising administering an effective amount of the present compound to a patient; and a method for preventing and/or treating glaucoma and/or ocular hypertension, comprising administering an effective amount of the present compound to a patient.

The dose of the active ingredient of the intraocular pressure-lowering agent can be appropriately selected and employed depending on the symptoms, age, dosage form or the like. For example, in the case of an oral preparation, it can be administered in an amount of generally from 0.01 to 1000 mg, preferably from 1 to 100 mg per day in a single

dose or several divided doses. Further, in the case of an eye drop, a preparation containing the present compound at a concentration of generally from 0.0001 to 10% (w/v), preferably from 0.01 to 5% (w/v) can be administered in a single dose or several divided doses.

Hereinafter, Production Examples of the active ingredient of the intraocular pressure-lowering agent, Preparation Examples and results of Pharmacological Tests will be described. However, these examples are described for the purpose of understanding the invention better and are not meant to limit the scope of the present invention.

Incidentally, the following compounds i) to ix) are commercially available compounds having an HDAC inhibitory effect, and further, the compound ii) can also be prepared according to the method described in JP-A-2003-226680.

i) (2E,4E,6R)-7-(4-Dimethylaminophenyl)-N-hydroxy-4,6-dimethyl-7-oxo-2,4-heptadienamide (Trichostatin A, product of Wako Pure Chemical).
ii) N-Hydroxy-N'-phenyloctanediamide (SAHA, product of ALEXIS Biochemicals).
iii) 4-Dimethylamino-N-(6-hydroxycarbamoylhexyl)benzamide (M 344, A, product of Wako Pure Chemical).
iv) (E)-N-Hydroxy-5-[3-(phenylsulfonylamino)phenyl]-2-penten-4-ynamide (Oxamflatin, product of ALEXIS Biochemicals).
v) N-Hydroxy-3-[3-hydroxyamino-3-oxo-1-propenyl]benzamide (CBHA, product of CALBIOCHEM).
vi) (E)-N-Hydroxy-3-[1-methyl-4-(4-methylbenzoyl)-1H-pyrrol-2-yl]-2-propenamide (MC1293, product of Wako Pure Chemical).
vii) (1R)-[(2S,3S)-3-[(E)-2-[(2S,3S)-3-[(1R)-Hydroxyethyl]oxiran-2-yl] vinyl]oxiran-2-yl]-2-propen-1-ol (Depudecin, product of SIGMA).
viii) N-(2-Aminophenyl)-4-(pyridin-3-ylmethyloxycarbonylaminomethyl) benzamide (MS 275, product of CALBIOCHEM).
ix) *cyclo*[L-(2-Amino-8-oxodecanoyD-(1'-methoxy-L-tryptophyl)-L-isoleucyl-D-pipecolinyl] (Apicidin, product of ALEXIS Biochemicals).

[Production Examples]

Reference Example 1

2-Aminophenylcarbamic acid t-butyl ester (Reference Compound No.1-1)

A solution of di-t-butyl dicarbonate (44 g, 200 mmol) in THF (50 mL) was added dropwise to a solution of o-phenylenediamine (22 g, 200 mmol) and triethylamine (30 mL, 210 mmol) in THF (150 mL), and then the mixture was stirred at room temperature for 15 hours. The reaction mixture was concentrated, the obtained solid was filtered with ethyl acetate, and then the solid was dried under reduced pressure to give 21 g of the title reference compound as a white solid. Additionally, another solid which was obtained by concentration of the filtrate was collected by filtration with ethyl acetate, and then the solid was dried under reduced pressure to give 11 g of the title reference compound as a white solid. (Yield 76%)

| | |
|---|---|
| 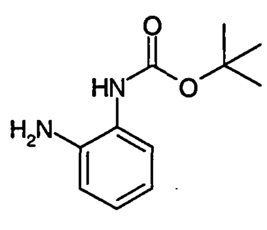 | $^1$H-NMR (400 MHz, DMSO-$d_6$)<br>δ 1.48 (s, 9H), 4.84 (s, 2H), 6.55 (td, J = 7.6, 1.4 Hz, 1H), 6.70 (dd, J = 7.6, 1.4 Hz, 1H), 6.86 (td, J = 7.6, 1.4 Hz, 1H), 7.20 (d, J = 7.6 Hz, 1H), 8.30 (br s, 1H) |

Reference Example 2

N-(2-t-Butoxycarbonylaminophenyl)-5-methoxycarbonylpyridine-2-carboxylic acid amide

(Reference Compound No.2-1)

HATU (21 g, 55 mmol) was added to a solution of 2-aminophenylcarbamic acid t-butyl ester (Reference Compound No.1-1, 10 g, 50 mmol), 5-methoxycarbonylpyridine-2-carboxylic acid (10 g, 55 mmol), and N-methylmorpholine (11 mL, 100 mmol) in DMF (100 mL), and then the reaction mixture was stirred at room temperature for 20 hours. Water

(300 mL) was added thereto, and then the whole was extracted with ethyl acetate (300 mL) three times. The organic layer was washed with brine (200 mL), and then dried over anhydrous magnesium sulfate. The solvent was evaporated under reduced pressure, and then the obtained solid was collected by filtration to give 15 g of the title reference compound as a pale brown solid. (Yield 79%)

| | |
|---|---|
| 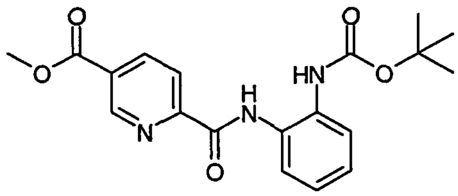 | $^1$H-NMR (400 MHz, $CDCl_3$)<br>δ 1.53 (s, 9H), 4.01 (s, 3H), 6.90 (br s, 1H), 7.20-7.25 (m, 2H), 7.51 (m, 1H), 7.82 (m, 1H), 8.38 (dd, J = 8.1, 0.7 Hz, 1H), 8.50 (dd, J = 8.1, 2.1 Hz, 1H), 9.18 (dd, J = 2.1, 0.7 Hz, 1H), 10.28 (br s, 1H) |

Reference Example 3

N-(2-t-Butoxycarbonylaminophenyl)-5-hydroxymethylpyridine-2-carboxylic acid amide

(Reference Compound No.3-1)

Under ice cooling, lithium tetrahydroborate (2.1 g, 100 mmol) was added to a solution of N-(2-t-butoxycarbonylaminophenyl)-5-methoxycarbonylpyridine-2-carboxylic acid amide (Reference Compound No.2-1, 38 g, 100 mmol) in THF (400 mL), and then the reaction mixture was stirred for 3 hours. Under ice cooling, water (200 mL) and 1.0 M aqueous hydrochloric acid (300 mL) were added thereto, the whole was extracted with ethyl acetate (300 mL), and then the organic layer was washed with water (500 mL) and brine (400 mL). After the organic layer was dried over anhydrous magnesium sulfate, the solvent was evaporated under reduced pressure. Ethyl acetate (20 mL) and hexane (30 mL) were added to the residue, and then the resulting solid was collected by filtration to give 18 g of the title reference compound as a white solid. (Yield 53%)

| | |
|---|---|
| 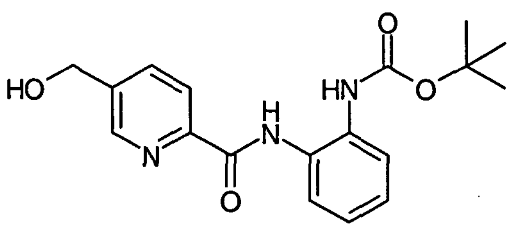 | $^1$H-NMR (400 MHz, DMSO-$d_6$)<br>δ 1.50 (s, 9H), 4.66 (d, J = 5.1 Hz, 2H), 5.52 (t, J = 5.1 Hz, 1H), 7.15 (dd, J = 7.6, 1.5 Hz, 1H), 7.24-7.27 (m, 2H), 7.98 (dd, J = 7.9, 1.5 Hz, 1H), 8.02 (d, J = 7.6 Hz, 1H), 8.14 (d, J = 7.9 Hz, 1H), 8.58 (br s, 1H), 9.11 (br s, 1H), 10.47 (br s, 1H) |

Reference Example 4

N-(2-t-Butoxycarbonylaminophenyl)-5-methanesulfonyloxymethylpyridine-2-carboxylic acid amide (Reference Compound No.4-1)

Under ice cooling, methanesulfonyl chloride (4.2 mL, 54 mmol) was added to a solution of N-(2-t-butoxycarbonylaminophenyl)-5-hydroxymethylpyridine-2-carboxylic acid amide (Reference Compound No.3-1, 16 g, 45 mmol) and triethylamine (16 mL, 110 mmol) in dichloromethane (150 mL), and then the reaction mixture was stirred for 1 hour. Water (400 mL) was added thereto, and then the whole was extracted with ethyl acetate (500 mL) five times. The organic layer was washed with water (500 mL), dried over anhydrous magnesium sulfate, and then the solvent was evaporated under reduced pressure. The resulting solid was collected by filtration with hexane (80 mL) and ethyl acetate (20 mL) to give 18 g of the title reference compound as a white solid. (Yield 94%)

| | |
|---|---|
| 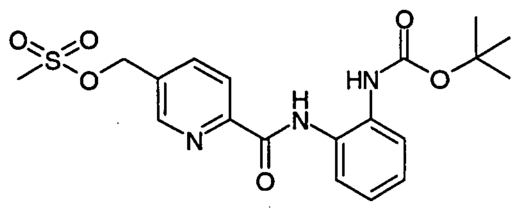 | $^1$H-NMR (500 MHz, $CDCl_3$) δ 1.58 (s, 9H), 3.06 (s, 3H), 5.35 (s, 2H), 6.92 (br s, 1H), 7.21-7.24 (m, 2H), 7.53 (br s, 1H), 7.78 (br s, 1H), 7.98 (dd, J = 8.2, 2.1 Hz, 1H), 8.35 (d, J = 8.2 Hz, 1H), 8.65 (d, J = 2.1 Hz, 1H), 10.21 (br s, 1H) |

Reference Example 5

N-(2-t-Butoxycarbonylaminophenyl)-5-(3-dimethylaminopropylaminomethyl)pyridine-2-carb oxylic acid amide (Reference Compound No.5-1)

N-(2-t-Butoxycarbonylaminophenyl)-5-methanesulfonyloxymethylpyridine-2-carboxylic acid amide (Reference Compound No.4-1, 4.9 g, 12 mmol) was added to a suspension of N,N-dimethyl-1,3-propanediamine (3.0 mL, 24 mmol) and potassium carbonate (3.3 g, 24 mmol) in DMF (40 mL), and then the reaction mixture was stirred at room temperature for 6 hours. Saturated aqueous sodium hydrogen carbonate (100 mL) was added thereto, and then the whole was extracted with ethyl acetate (100 mL) twice. The organic layer was washed with water (100 mL) three times, dried over anhydrous magnesium sulfate, and then the solvent was evaporated under reduced pressure. The residue was purified by silica gel column chromatography (chloroform-methanol) to give 1.9 g of the title reference compound as a yellow amorphous product. (Yield 38%)

| | |
|---|---|
| 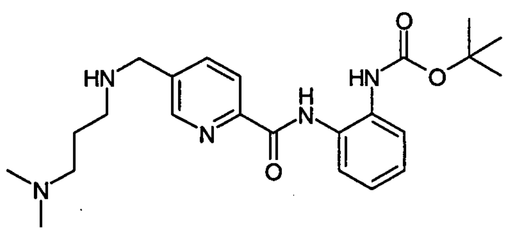 | $^1$H-NMR (500 MHz, $CDCl_3$) δ 1.52 (s, 9H), 1.69 (m, 2H), 2.22 (s, 6H), 2.34 (t, J = 7.0 Hz, 2H), 2.69 (t, J = 7.0 Hz, 2H), 3.90 (s, 2H), 7.15 (br s, 1H), 7.18-7.24 (m, 2H), 7.59 (br s, 1H), 7.71 (br s, 1H), 7.88 (dd, J = 7.9, 1.8 Hz, 1H), 8.25 (d, J = 7.9 Hz, 1H), 8.55 (d, J = 1.8 Hz, 1H), 10.17 (br s, 1H) |

By using any compounds selected from Reference Compound No.4-1 and commercially available compounds, the following Reference Compounds No.5-2~5-19 were obtained by a method similar to that of Reference Compound No. 5-1. Additionally, by using any compounds selected from Reference Compound No.11-1 and commercially available compounds, the following Reference Compound No.5-20 was obtained by a method similar to that of Reference Compounds No.4-1 and No.5-1.

| | |
|---|---|
| N-(2-t-Butoxycarbonylaminophenyl)-5-(3-diethylaminopropylaminomethyl)pyridine-2-carboxylic acid amide (Reference Compound No.5-2) 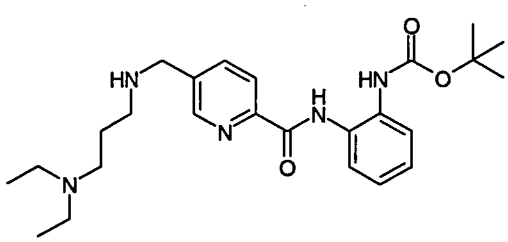 | $^1$H-NMR (500 MHz, DMSO-$d_6$) δ 0.92 (t, J = 7.0 Hz, 6H), 1.49 (s, 9H), 1.53 (m, 2H), 2.38-2.43 (m, 6H), 3.32 (m, 2H), 3.80 (s, 2H), 7.15 (td, J = 7.6, 1.4 Hz, 1H), 7.24-7.27 (m, 2H), 7.98 (dd, J = 7.9, 1.7 Hz, 1H), 8.01 (d, J = 7.6 Hz, 1H), 8.11 (d, J = 7.9 Hz, 1H), 8.57 (d, J = 1.7 Hz, 1H), 9.10 (br s, 1H), 10.46 (br s, 1H) |
| N-(2-t-Butoxycarbonylaminophenyl)-5-(t-butoxycarbonylmethylaminomethyl)pyridine-2-carboxylic acid amide (Reference Compound No.5-3) | $^1$H-NMR (500 MHz, $CDCl_3$) δ 1.48 (s, 9H), 1.52 (s, 9H), 3.32 (s, 2H), 3.92 (s, 2H), 7.08 (br s, 1H), 7.18-7.24 (m, 2H), 7.60 (d, J = 6.1 Hz, 1H), 7.71 (d, J = |

(continued)

| | |
|---|---|
| 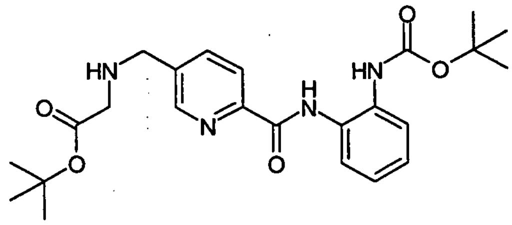 | 6.1 Hz, 1H), 7.91 (dd, J = 8.1, 1.7 Hz, 1H), 8.26 (dd, J = 8.1, 0.6 Hz, 1H), 8.56 (d, J = 1.7 Hz, 1H), 10.17 (br s, 1H) |
| N-(2-t-Butoxycarbonylaminophenyl)-5-(3-hydroxypropylaminomethyl)pyridine-2-carboxylic acid amide (Reference Compound No.5-4) 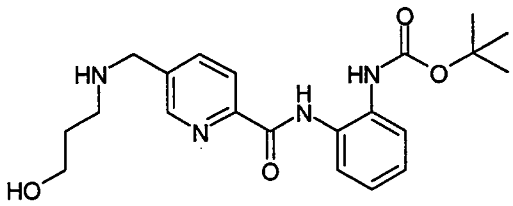 | $^1$H-NMR (400 MHz, $CDCl_3$) δ 1.52 (s, 9H), 1.77 (m, 2H), 2.91 (t, J = 5.9 Hz, 2H), 3.82 (t, J = 5.4 Hz, 2H), 3.91 (s, 2H), 7.08 (br s, 1H), 7.18-7.25 (m, 2H), 7.59 (d, J = 6.3 Hz, 1H), 7.71 (d, J = 6.3 Hz, 1H), 7.86 (dd, J = 8.1, 2.2 Hz, 1H), 8.27 (dd, J = 8.1, 0.6 Hz, 1H), 8.54 (d, J = 2.2 Hz, 1H), 10.17 (br s, 1H) |
| N-(2-t-Butoxycarbonylaminophenyl)-5-(2-ethoxycarbonylethylaminomethyl)pyridine-2-carboxylic acid amide (Reference Compound No.5-5) 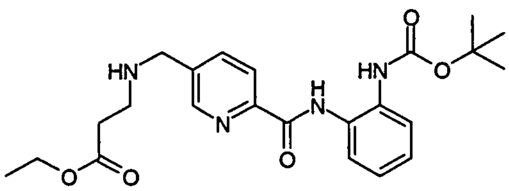 | $^1$H-NMR (400 MHz, DMSO-$d_6$) δ 1.18 (t, J = 7.1 Hz, 3H), 1.49 (s, 9H), 2.45 (t, J = 6.7 Hz, 2H), 2.72 (m, 2H), 3.83 (s, 2H), 4.05 (q, J = 7.1 Hz, 2H), 7.15 (td, J = 7.5, 1.7 Hz, 1H), 7.24-7.27 (m, 2H), 7.98 (dd, J = 8.1, 1.7 Hz, 1H), 8.02 (d, J = 7.5 Hz, 1H), 8.12 (d, J = 8.1 Hz, 1H), 8.57 (d, J = 1.7 Hz, 1H), 9.11 (br s, 1H), 10.47 (br s, 1H) |
| N-(2-t-Butoxycarbonylaminophenyl)-5-(2-hydroxyethylaminomethyl)pyridine-2-carboxylic acid amide (Reference Compound No.5-6) 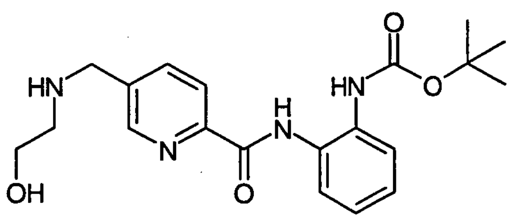 | $^1$H-NMR (400 MHz, $CDCl_3$) δ 1.51 (s, 9H), 2.83 (m, 2H), 3.70 (m, 2H), 3.93 (s, 2H), 7.14 (br s, 1H), 7.18-7.24 (m, 2H), 7.58 (d, J = 6.6 Hz, 1H), 7.72 (d, J = 6.1 Hz, 1H), 7.87 (dd, J = 8.1, 2.2 Hz, 1H), 8.24 (dd, J = 8.1, 0.7 Hz ,1H), 8.54 (dd, J = 2.2, 0.7 Hz, 1H), 10.18 (br s, 1H) |
| N-(2-t-Butoxycarbonylaminophenyl)-5-(2-dimethylaminoethylaminomethyl)pyridine-2-carboxylic acid amide (Reference Compound No.5-7) 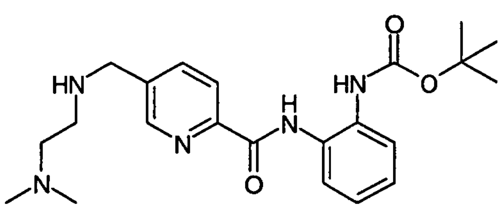 | $^1$H-NMR (400 MHz, $CDCl_3$) δ 1.51 (s, 9H), 1.94 (s, 1H), 2.23 (s, 6H), 2.44 (t, J = 5.9 Hz, 2H), 2.66 (t, J = 5.9 Hz, 2H), 3.91 (s, 2H), 7.15 (br s, 1H), 7.16-7.22 (m, 2H), 7.58 (s, 1H), 7.70 (s, 1H), 7.89 (dd, J = 7.8, 2.1 Hz, 1H), 8.24 (d, J = 7.8 Hz, 1H), 8.55 (d, J = 2.1 Hz, 1H), 10.17 (s, 1H) |

(continued)

| | |
|---|---|
| N-(2-t-Butoxycarbonylaminophenyl)-5-[3-(pyrrolidin-1-yl) propylaminomethyl]pyridine-2-carboxylic acid amide (Reference Compound No.5-8) 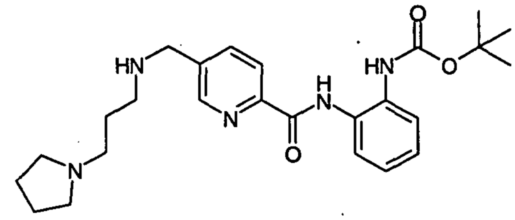 | $^1$H-NMR (500 MHz, $CDCl_3$) δ 1.52 (s, 9H), 1.72-1.80 (m, 6H), 2.48-2.55 (m, 6H), 2.70 (t, J = 6.9 Hz, 2H), 3.90 (s, 2H), 7.18-7.27 (m, 3H), 7.59 (br s, 1H), 7.71 (br s, 1H), 7.88 (dd, J = 7.9, 1.8 Hz, 1H), 8.25 (d, J = 7.9 Hz, 1H), 8.54 (d, J = 1.8 Hz, 1H), 10.17 (br s, 1H) |
| N-(2-t-Butoxycarbonylaminophenyl)-5-(2-t-butoxycarbonylethylaminomethyl)pyridine-2-carboxylic acid amide (Reference Compound No.5-9) 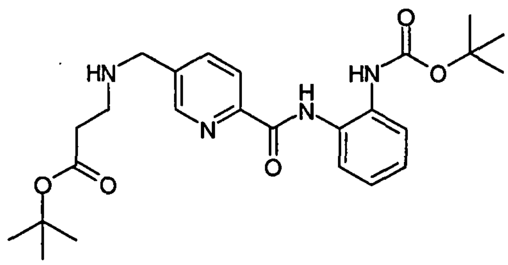 | $^1$H-NMR (500 MHz, $CDCl_3$) δ 1.46 (s, 9H), 1.52 (s, 9H), 2.46 (t, J = 6.4 Hz, 2H), 2.86 (t, J = 6.4 Hz, 2H), 3.92 (s, 2H), 7.09 (br s, 1H), 7.18-7.24 (m, 2H), 7.61 (d, J = 6.1 Hz, 1H), 7.70 (d, J = 6.1 Hz, 1H), 7.89 (dd, J = 7.9, 1.8 Hz, 1H), 8.26 (dd, J = 7.9, 0.6 Hz, 1H), 8.56 (d, J = 1.8 Hz, 1H), 10.16 (br s, 1H) |
| N-(2-t-Butoxycarbonylaminophenyl)-5-[3-(morpholin-4-yl)propylaminomethyl)]pyridine-2-carboxylic acid amide (Reference Compound No.5-10) 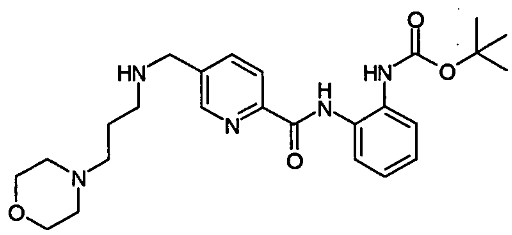 | $^1$H-NMR (400 MHz, $CDCl_3$) δ 1.52 (s, 9H), 1.72 (m, 2H), 2.40-2.44 (m, 6H), 2.70 (t, J = 6.8 Hz, 2H), 3.70 (m, 4H), 3.91 (s, 2H), 7.11 (br s, 1H), 7.18-7.25 (m, 2H), 7.59 (br s, 1H), 7.71 (br s, 1H), 7.88 (dd, J = 8.1, 2.2 Hz, 1H), 8.26 (dd, J = 8.1, 0.7 Hz, 1H), 8.55 (dd, J = 2.2, 0.7 Hz, 1H), 10.17 (br s, 1H) |
| N-(2-t-Butoxycarbonylaminophenyl)-5-[2-(4-methylpiperazin-1-yl)ethylaminomethyl]pyridine-2-carboxylic acid amide (Reference Compound No.5-11) 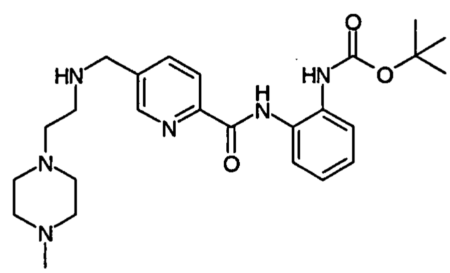 | $^1$H-NMR (400 MHz, $CDCl_3$) δ 1.52 (s, 9H), 2.29 (s, 3H), 2.46 (br s, 8H), 2.52 (t, J = 6.0 Hz, 2H), 2.71 (t, J = 6.0 Hz, 2H), 3.92 (s, 2H), 7.09 (br s, 1H), 7.17-7.25 (m, 2H), 7.60 (d, J = 7.3 Hz, 1H), 7.70 (d, J = 7.1 Hz, 1H), 7.89 (dd, J = 7.9, 2.2 Hz, 1H), 8.26 (d, J = 7.9 Hz, 1H), 8.55 (d, J = 2.2 Hz, 1H), 10.16 (s, 1H) |

(continued)

| | |
|---|---|
| N-(2-t-Butoxycarbonylaminophenyl)-5-[3-(4-methylpiperazin-1-yl)propylaminomethyl]pyridine-2-carboxylic acid amide (Reference Compound No.5-12)<br>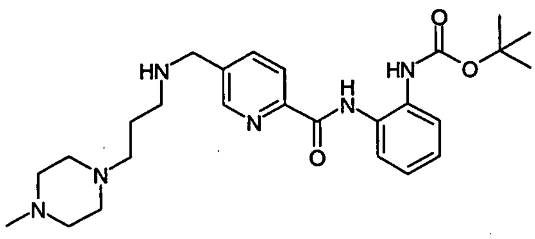 | $^1$H-NMR (400 MHz, $CDCl_3$)<br>δ 1.52 (s, 9H), 1.72 (m, 2H), 2.28 (s, 3H), 2.43 (t, J = 6.9 Hz, 2H), 2.46 (br s, 8H), 2.69 (t, J = 6.9 Hz, 2H), 3.90 (s, 2H), 7.09 (br s, 1H), 7.17-7.25 (m, 2H), 7.60 (d, J = 6.6 Hz, 1H), 7.70 (d, J = 6.6 Hz, 1H), 7.88 (dd, J = 7.9, 2.0 Hz, 1H), 8.26 (d, J = 7.9 Hz, 1H), 8.55 (d, J = 2.0 Hz, 1H), 10.16 (s, 1H) |
| N-(2-t-Butoxycarbonylaminophenyl)-5-[4-(pyrrolidin-1-yl) butylaminomethyl]pyridine-2-carboxylic acid amide (Reference Compound No.5-13)<br>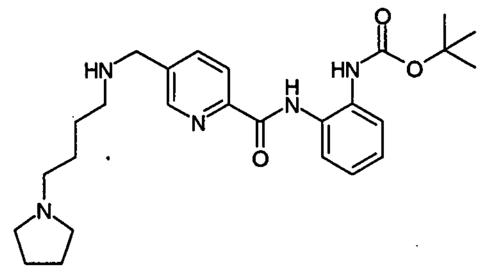 | $^1$H-NMR (500 MHz, $CDCl_3$) δ 1.52 (s, 9H), 1.55-1.65 (m, 4H), 1.80-1.86 (m, 4H), 2.54 (t, J = 7.4 Hz, 2H), 2.59-2.64 (m, 4H), 2.67 (t, J = 6.8 Hz, 2H), 3.90 (s, 2H), 7.13 (br s, 1H), 7.18-7.25 (m, 2H), 7.59 (d, J = 5.5 Hz, 1H), 7.71 (d, J = 6.1 Hz, 1H), 7.90 (dd, J = 7.9, 1.8 Hz, 1H), 8.25 (d, J = 7.9 Hz, 1H), 8.55 (d, J = 1.8 Hz, 1H), 10.17 (s, 1H) |
| N-(2-t-Butoxycarbonylaminophenyl)-5-(2-diethylaminoethylaminomethyl)pyridine-2-carboxylic acid amide (Reference Compound No.5-14)<br>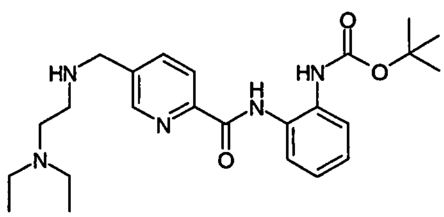 | $^1$H-NMR (400 MHz, $CDCl_3$)<br>δ 1.01 (t, J = 7.2 Hz, 6H), 1.52 (s, 9H), 2.52 (q, J = 7.2 Hz, 4H), 2.58 (t, J = 5.7 Hz, 2H), 2.68 (t, J = 5.7 Hz, 2H), 3.92 (s, 2H), 7.09 (br s, 1H), 7.18-7.23 (m, 2H), 7.60 (d, J = 6.6 Hz, 1H), 7.70 (d, J = 6.3 Hz, 1H), 7.89 (dd, J = 8.1, 2.2 Hz, 1H), 8.26 (dd, J = 8.1, 0.6 Hz, 1H), 8.56 (dd, J = 2.2, 0.6 Hz, 1H), 10.17 (s, 1H) |
| N-(2-t-Butoxycarbonylaminophenyl)-5-(4-dimethylaminobutylaminomethyl)pyridine-2-carboxylic acid amide (Reference Compound No.5-15)<br>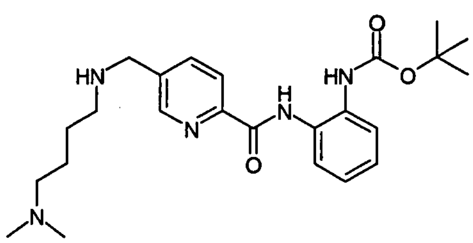 | $^1$H-NMR (400 MHz, $CDCl_3$)<br>δ 1.52 (s, 9H), 1.63-1.69 (m, 4H), 2.36 (s, 6H), 2.51 (m, 2H), 2.71 (t, J = 6.5 Hz, 2H), 3.94 (s, 2H), 7.10 (br s, 1H), 7.18-7.25 (m, 2H), 7.60 (m, 1H), 7.71 (m, 1H), 7.97 (dd, J = 7.9, 2.2 Hz, 1H), 8.27 (dd, J = 7.9, 1.1 Hz, 1H), 8.59 (d, J = 2.2, 1.1 Hz, 1H), 10.17 (s, 1H) |

(continued)

| | |
|---|---|
| N-(2-t-Butoxycarbonylaminophenyl)-5-[4-(morpholin-4-yl)butylaminomethyl]pyridine-2-carboxylic acid amide (Reference Compound No.5-16) 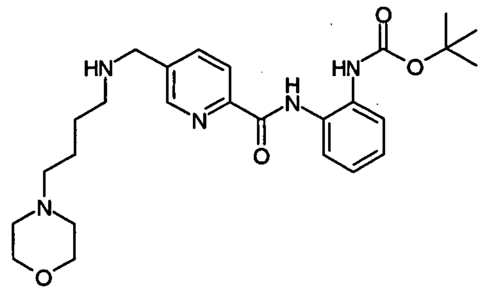 | $^1$H-NMR (500 MHz, $CDCl_3$) δ 1.52 (s, 9H), 1.54-1.60 (m, 4H), 2.35 (t, J = 7.0 Hz, 2H), 2.43 (br s, 4H), 2.66 (t, J = 6.7 Hz, 2H), 3.71 (t, J = 4.7 Hz, 4H), 3.91 (s, 2H), 7.08 (br s, 1H), 7.18-7.24 (m, 2H), 7.60 (d, J = 6.3 Hz, 1H), 7.70 (d, J = 6.3 Hz, 1H), 7.89 (dd, J = 7.9, 2.1 Hz, 1H), 8.26 (d, J = 7.9 Hz, 1H), 8.56 (d, J = 1.5 Hz, 1H), 10.16 (s, 1H) |
| N-(2-t-Butoxycarbonylaminophenyl)-5-(5-dimethylaminopentylaminomethyl)pyridine-2-carboxylic acid amide (Reference Compound No.5-17) 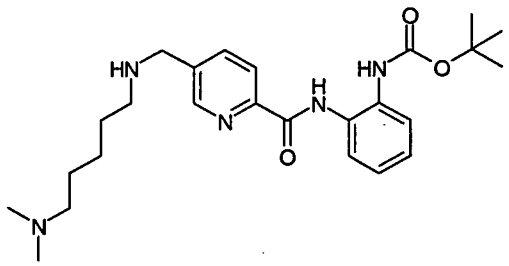 | $^1$H-NMR (400 MHz, $CDCl_3$) δ 1.38 (m, 2H), 1.52 (s, 9H), 1.52-1.59 (m, 4H), 2.31 (s, 6H), 2.37 (t, J = 7.6 Hz, 2H), 2.65 (t, J = 7.1 Hz, 2H), 3.90 (s, 2H), 7.11 (br s, 1H), 7.17-7.24 (m, 2H), 7.60 (d, J = 5.8 Hz, 1H), 7.70 (d, J = 5.8 Hz, 1H), 7.89 (dd, J = 7.9, 2.1 Hz, 1H), 8.26 (dd, J = 7.9, 0.7 Hz, 1H), 8.56 (dd, J = 2.1, 0.7 Hz, 1H), 10.17 (s, 1H) |
| N-(2-t-Butoxycarbonylaminophenyl)-5-[3-(4-methylpiperidin-1-yl)propylaminomethyl]pyridine-2 -carboxylic acid amide (Reference Compound No.5-18) 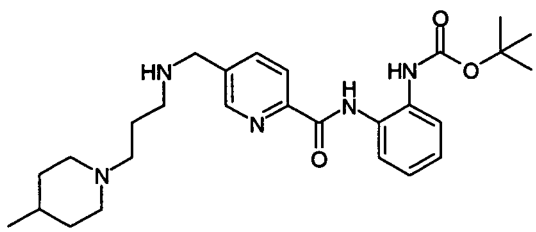 | $^1$H-NMR (500 MHz, $CDCl_3$) δ 0.91 (d, J = 6.4 Hz, 3H), 1.21 (m, 2H), 1.35 (m, 1H), 1.52 (s, 9H), 1.62 (m, 2H), 1.73 (m, 2H), 1.89 (m, 2H), 2.39 (t, J = 7.3 Hz, 2H), 2.68 (t, J = 6.7 Hz, 2H), 2.90 (d, J = 11.6 Hz, 2H), 3.90 (s, 2H), 7.10 (br s, 1H), 7.18-7.24 (m, 2H), 7.61 (d, J = 6.1 Hz, 1H), 7.70 (d, J = 6.4 Hz, 1H), 7.88 (dd, J = 7.9, 2.1 Hz, 1H), 8.26 (d, J = 7.9 Hz, 1H), 8.55 (d, J = 2.1 Hz, 1H), 10.16 (s, 1H) |
| N-(2-t-Butoxycarbonylaminophenyl)-5-[5-(morpholin-4-yl)pentylaminomethyl]pyridine-2-carboxylic acid amide (Reference Compound No.5-19) 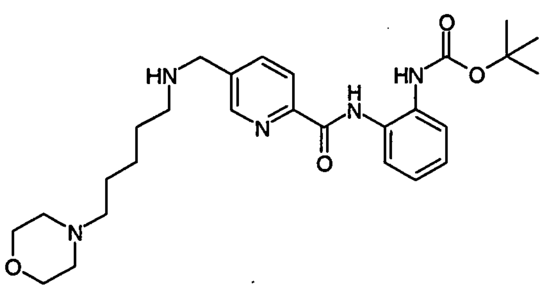 | $^1$H-NMR (400 MHz, $CDCl_3$) δ 1.36 (m, 2H), 1.47-1.56 (m, 4H), 1.52 (s, 9H), 2.33 (t, J = 7.5 Hz, 2H), 2.43 (br s, 4H), 2.64 (t, J = 7.1 Hz, 2H), 3.71 (t, J = 4.8 Hz, 4H), 3.90 (s, 2H), 7.09 (br s, 1H), 7.18-7.25 (m, 2H), 7.60 (d, J = 6.6 Hz, 1H), 7.71 (d, J = 6.3 Hz, 1H), 7.88 (dd, J = 8.1, 2.2 Hz, 1H), 8.26 (d, J = 8.1 Hz, 1H), 8.55 (d, J = 2.2 Hz, 1H), 10.17 (s, 1H) |

(continued)

| | |
|---|---|
| N-(2-t-Butoxycarbonylaminophenyl)-6-(2-dimethylaminoethylaminomethyl)pyridine-3-carboxylic acid amide (Reference Compound No.5-20) 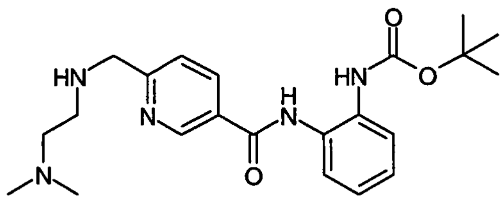 | $^1$H-NMR (400 MHz, $CDCl_3$) δ 1.52 (s, 9H), 2.23 (s, 6H), 2.35 (t, J = 6.2 Hz, 2H), 2.76 (m, 2H), 4.01 (s, 2H), 7.02 (s, 1H), 7.18 (td, J = 7.8, 1.5 Hz, 1H), 7.22-7.25 (m, 2H), 7.44 (d, J = 8.2 Hz, 1H), 7.82 (d, J = 7.8 Hz, 1H), 8.20 (dd, J = 8.2, 2.1 Hz, 1H), 9.12 (d, J = 2.1 Hz, 1H), 9.51 (s, 1H) |

Reference Example 6

N-(2-t-Butoxycarbonylaminophenyl)-5-[1-(3-dimethylaminopropyl)-3-phenethylureidomethyl] pyridine-2-carboxylic acid amide (Reference Compound No.6-1)

Phenethylisocyanate (15 μL, 0.11 mmol) was added to a solution of N-(2-t-butoxycarbonylaminophenyl)-5-(3-dimethylaminopropylaminomethyl)pyridine-2-carbo xylic acid amide (Reference Compound No.5-1, 27 mg, 0.060 mmol) in dichloromethane (1.0 mL), and then the mixture was stirred at room temperature for 2 hours. The reaction mixture was concentrated, and then the residue was purified by silica gel column chromatography (chloroform-methanol) to give 32 mg of the title reference compound as a colorless amorphous product. (Yield 88%)

| | |
|---|---|
| 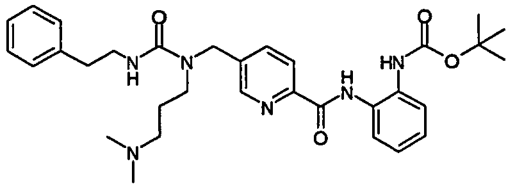 | $^1$H-NMR (500 MHz, DMSO-$d_6$) δ 1.48 (s, 9H), 1.53 (m, 2H), 2.04 (s, 6H), 2.13 (t, J = 6.4 Hz, 2H), 2.74 (t, J = 7.2 Hz, 2H), 3.13 (t, J = 6.4 Hz, 2H), 3.31 (m, 2H), 4.54 (s, 2H), 7.12-7.30 (m, 9H), 7.84 (dd, J = 7.9, 1.8 Hz, 1H), 8.02 (d, J = 7.3 Hz, 1H), 8.12 (d, J = 7.9 Hz, 1H), 8.48 (d, J = 1.8 Hz, 1H), 9.11 (br s, 1H), 10.46 (br s, 1H) |

By using any compounds selected from Reference Compounds No.5-1~5-20, commercially available compounds, and known compounds, the following Reference Compounds No.6-2~6-141 were obtained by a method similar to that of Reference Compound No.6-1.

| | |
|---|---|
| N-(2-t-Butoxycarbonylaminophenyl)-5-[3-(2,3-dihydrobenzo[1,4]dioxin-6-yl)-1-(3-dimethylaminopropyl)ureidomethyl]pyridine-2-carboxylic acid amide (Reference Compound No.6-2) 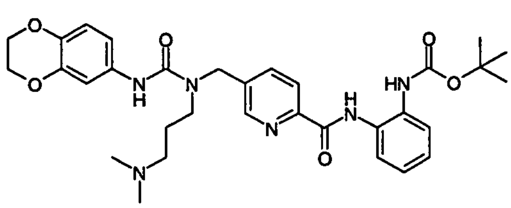 | $^1$H-NMR (500 MHz, DMSO-$d_6$) δ 1.47 (s, 9H), 1.67 (m, 2H), 2.17 (s, 6H), 2.25 (t, J = 6.3 Hz, 2H), 3.31 (m, 2H), 4.17-4.21 (m, 4H), 4.60 (s, 2H), 6.73 (d, J = 8.9 Hz, 1H), 6.77 (dd, J = 8.9, 2.4 Hz, 1H), 7.07 (d, J = 2.4 Hz, 1H), 7.15 (td, J = 7.0, 1.2 Hz, 1H), 7.23-7.27 (m, 2H), 7.96 (dd, J = 8.2, 1.8 Hz, 1H), 8.01 (d, J = 7.0 Hz, 1H), 8.14 (d, J = 8.2 Hz, 1H), 8.56 (br s, 1H), 9.11 (br s, 1H), 9.42 (br s, 1H), 10.46 (br s, 1H) |

(continued)

| | |
|---|---|
| N-(2-t-Butoxycarbonylaminophenyl)-5-[3-(3,4-difluorophenyl)-1-(3-dimethylaminopropyl)ureido methyl] pyridine-2-carboxylic acid amide (Reference Compound No.6-3)<br>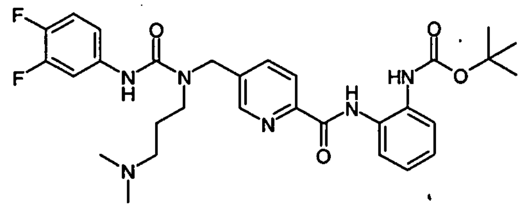 | $^{1}$H-NMR (500 MHz, DMSO-$d_6$)<br>δ 1.47 (s, 9H), 1.70 (m, 2H), 2.19 (s, 6H), 2.26 (t, J = 6.3 Hz, 2H), 3.35 (m, 2H), 4.63 (s, 2H), 7.07 (m, 1H), 7.15 (td, J = 7.3, 1.5 Hz, 1H), 7.23-7.27 (m, 2H), 7.34 (dd, J = 19.7, 9.3 Hz, 1H), 7.68 (ddd, J = 13.7, 7.6, 2.4 Hz, 1H), 7.98 (dd, J = 7.9, 1.8 Hz, 1H), 8.01 (d,J = 7.3 Hz, 1H), 8.14 (d, J = 7.9 Hz, 1H). 8.58 (d, J = 1.8 Hz, 1H), 9.12 (br s, 1H), 9.43 (br s, 1H), 10.02 (br s, 1H) |
| 5-[3-(Benzo[1,3]dioxol-5-yl)-1-(3-dimethylaminopropyl) ureidomethyl]-N-(2-t-butoxycarbonylaminophenyl) pyridine-2-carboxylic acid amide (Reference Compound No.6-4)<br>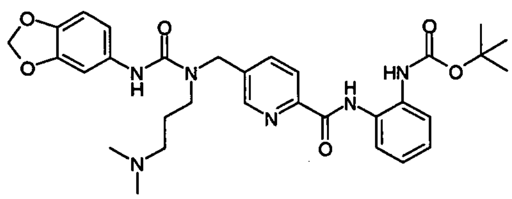 | $^{1}$H-NMR (500 MHz, DMSO-$d_6$)<br>δ 1.47 (s, 9H), 1.68 (m, 2H), 2.18 (s, 6H), 2.25 (t, J = 6.3 Hz, 2H), 3.32 (m, 2H), 4.63 (s, 2H), 5.95 (s, 2H), 6.72 (dd, J = 8.2, 2.1 Hz, 1H), 6.80 (d, J = 8.2 Hz, 1H), 7.15 (td, J = 7.6, 1.5 Hz, 1H), 7.18 (d, J = 2.1 Hz, 1H), 7.23-7.27 (m, 2H), 7.97 (dd, J = 7.9, 1.8 Hz, 1H), 8.01 (d, J = 7.6 Hz, 1H), 8.14 (d, J = 7.9 Hz, 1H), 8.57 (d, J = 1.8 Hz, 1H), 9.11 (br s, 1H), 9.51 (br s, 1H), 10.47 (br s, 1H) |
| N-(2-t-Butoxycarbonylaminophenyl)-5-[1-(3-dimethylaminopropyl)-3-(4-methoxyphenyl) ureidomethyl]pyridine-2-carboxylic acid amide (Reference Compound No.6-5)<br>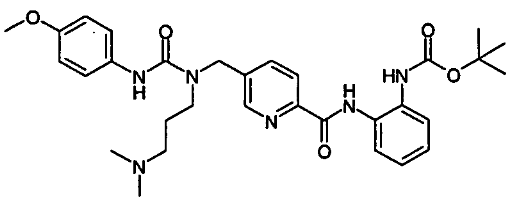 | $^{1}$H-NMR (400 MHz, DMSO-$d_6$)<br>δ 1.47 (s, 9H), 1.68 (m, 2H), 2.18 (s, 6H), 2.26 (t, J = 6.3 Hz, 2H), 3.33 (m, 2H), 3.70 (s, 3H), 4.62 (s, 2H), 6.80 (d, J = 9.0 Hz, 2H), 7.15 (td, J = 8.3, 1.5 Hz, 1H), 7.23-7.27 (m, 2H), 7.33 (d, J = 9.0 Hz, 2H), 7.97 (dd, J = 8.1, 1.8 Hz, 1H), 8.01 (d, J = 8.3 Hz, 1H), 8.14 (d, J = 8.1 Hz, 1H), 8.57 (d, J = 1.8 Hz, 1H), 9.12 (br s, 1H), 9.42 (br s, 1H), 10.47 (br s, 1H) |
| 5-[3-(Benzo[1,3]dioxol-5-yl)-1-(3-diethylaminopropyl) ureidomethyl]-N-(2-t-butoxycarbonylaminophenyl) pyridine-2-carboxylic acid amide (Reference Compound No.6-6)<br>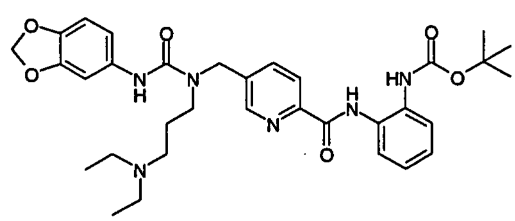 | $^{1}$H-NMR (500 MHz, $CDCl_3$)<br>δ 1.02 (t, J = 7.2 Hz, 6H), 1.51 (s, 9H), 1.74 (m, 2H), 2.51 (t, J = 6.0 Hz, 2H), 2.62 (q, J = 7.2 Hz, 4H), 3.37 (t, J = 5.7 Hz, 2H), 4.61 (s, 2H), 5.92 (s, 2H), 6.71-6.72 (m, 2H), 7.06 (br s, 1H), 7.14 (s, 1H), 7.17-7.24 (m, 2H), 7.60 (d, J = 7.2 Hz, 1H), 7.69 (d, J = 7.2 Hz, 1H), 7.93 (dd, J = 7.9, 2.0 Hz, 1H), 8.25 (d, J = 7.9 Hz, 1H), 8.58 (d, J = 2.0 Hz, 1H), 9.69 (s, 1H), 10.15 (s, 1H) |

(continued)

| | |
|---|---|
| N-(2-t-Butoxycarbonylaminophenyl)-5-[1-(3-dimethylaminopropyl)-3-(2-fluorophenyl)ureidom ethyl] pyridine-2-carboxylic acid amide (Reference Compound No.6-7)<br>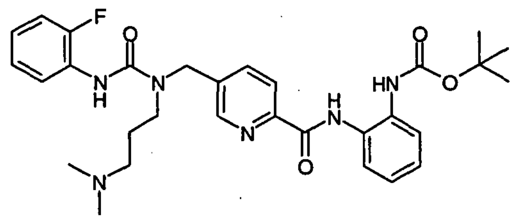 | $^1$H-NMR (500 MHz, DMSO-$d_6$)<br>δ 1.47 (s, 9H), 1.75 (m, 2H), 2.16 (s, 6H), 2.28 (t, J = 6.1 Hz, 2H), 3.36 (t, J = 5.6 Hz, 2H), 4.63 (s, 2H), 7.04 (m, 1H), 7.11 (m, 1H), 7.14-7.21 (m, 2H), 7.24 (d, J = 7.7 Hz, 1H), 7.26 (m, 1H), 7.88 (t, J = 7.7 Hz, 1H), 7.99 (dd, J = 7.7, 1.5 Hz, 1H), 8.02 (d, J = 7.1 Hz, 1H), 8.14 (d, J = 7.7 Hz, 1H), 8.59 (d, J = 1.5 Hz, 1H), 9.10 (s, 1H), 9.87 (s, 1H), 10.47 (s, 1H) |
| N-(2-t-Butoxycarbonylaminophenyl)-5-[1-(3-dimethylaminopropyl)-3-(3-fluorophenyl)ureidomethyl] pyridine-2-carboxylic acid amide (Reference Compound No.6-8)<br>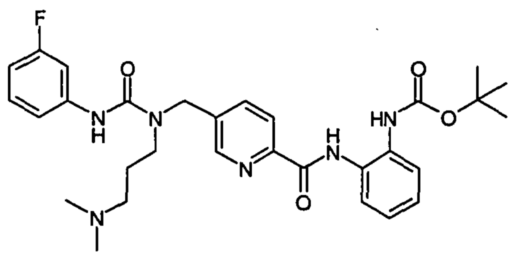 | $^1$H-NMR (500 MHz, DMSO-$d_6$)<br>δ 1.46 (s, 9H), 1.71 (m, 2H), 2.20 (s, 6H), 2.28 (t, J = 6.3 Hz, 2H), 3.36 (t, J = 6.3 Hz, 2H), 4.63 (s, 2H), 6.75 (td, J = 8.2, 2.3 Hz, 1H), 7.07 (dd, J = 8.2, 1.2 Hz, 1H), 7.15 (td, J = 8.2, 1.5 Hz, 1H), 7.23-7.30 (m, 3H), 7.49 (ddd, J = 12.2, 2.3, 1.5 Hz, 1H), 7.98 (dd, J = 8.2, 2.3 Hz, 1H), 8.02 (d, J = 8.2 Hz, 1H), 8.14 (d, J = 8.2 Hz, 1H), 8.59 (d, J = 2.3 Hz, 1H), 9.11 (s, 1H), 10.00 (s, 1H), 10.47 (s, 1H) |
| N-(2-t-Butoxycarbonylaminophenyl)-5-[3-(2,5-difluorophenyl)-1-(3-dimethylaminopropyl)ureidomethyl] pyridine-2-carboxylic acid amide (Reference Compound No.6-9)<br>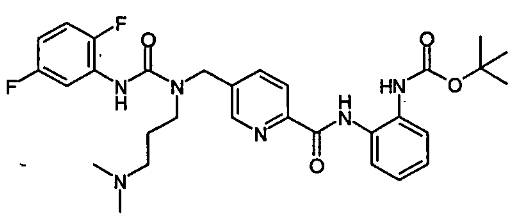 | $^1$H-NMR (500 MHz, DMSO-$d_6$)<br>δ 1.46 (s, 9H), 1.75 (m, 2H), 2.15 (s, 6H), 2.28 (t, J = 6.1 Hz, 2H), 3.43 (t, J = 6.1 Hz, 2H), 4.62 (s, 2H), 6.81 (m, 1H), 7.15 (td, J = 7.7, 1.5 Hz, 1H), 7.22-7.28 (m, 3H), 7.93 (m, 1H), 7.99-8.02 (m, 2H), 8.14 (dd, J = 7.7, 0.6 Hz, 1H), 8.60 (d, J = 1.5 Hz, 1H), 9.11 (s, 1H), 10.42 (s, 1H), 10.48 (s, 1H) |
| N-(2-t-Butoxycarbonylaminophenyl)-5-[3-(3,5-difluorophenyl)-1-(3-dimethylaminopropyl)ureidomethyl] pyridine-2-carboxylic acid amide (Reference Compound No.6-10)<br>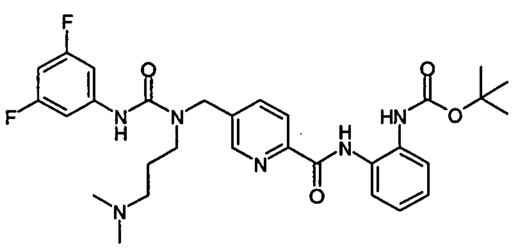 | $^1$H-NMR (500 MHz, DMSO-$d_6$)<br>δ 1.47 (s, 9H), 1.71 (m, 2H), 2.20 (s, 6H), 2.27 (t, J = 6.4 Hz, 2H), 3.36 (t, J = 6.1 Hz, 2H), 4.64 (s, 2H), 6.76 (tt, J = 9.2, 2.2 Hz, 1H), 7.14-7.17 (m, 3H), 7.23-7.27 (m, 2H), 7.98 (dd, J = 7.9, 2.2 Hz, 1H), 8.01 (d, J = 7.9 Hz, 1H), 8.14 (d, J = 7.9 Hz, 1H), 8.58 (s, 1H), 9.11 (s, 1H), 10.13 (s, 1H), 10.47 (s, 1H) |

(continued)

| | |
|---|---|
| N-(2-t-Butoxycarbonylaminophenyl)-5-[1-(3-dimethylaminopropyl)-3-(3-fluoro-4-methylphenyl) ureidomethyl]pyridine-2-carboxylic acid amide (Reference Compound No.6-11) 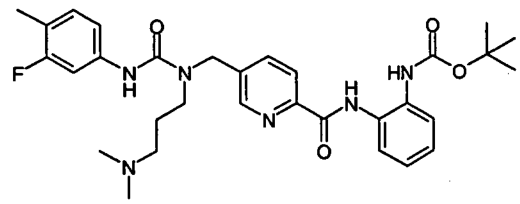 | $^{1}$H-NMR (500 MHz, DMSO-$d_6$) δ 1.47 (s, 9H), 1.70 (m, 2H), 2.15 (s, 3H), 2.20 (s, 6H), 2.26 (t, J = 6.4 Hz, 2H), 3.35 (t, J = 6.1 Hz, 2H), 4.62 (s, 2H), 6.98 (dd, J = 8.1, 2.1 Hz, 1H), 7.11-7.17 (m, 2H), 7.23-7.27 (m, 2H), 7.43 (dd, J = 12.8, 2.1 Hz, 1H), 7.98 (dd, J = 8.1, 2.1 Hz, 1H), 8.01 (d, J = 8.1 Hz, 1H), 8.14 (d, J = 8.1 Hz, 1H), 8.58 (d, J = 2.1 Hz, 1H), 9.11 (s, 1H), 9.82 (s, 1H), 10.47 (s, 1H) |
| N-(2-t-Butoxycarbonylaminophenyl)-5-[1-(3-dimethylaminopropyl)-3-(4-fluoro-3-methylphenyl) ureidomethyl]pyridine-2-carboxylic acid amide (Reference Compound No.6-12) 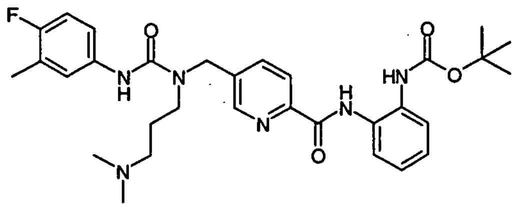 | $^{1}$H-NMR (500 MHz, DMSO-$d_6$) δ 1.47 (s, 9H), 1.69 (m, 2H), 2.18 (s, 6H), 2.20 (s, 3H), 2.26 (t, J = 6.4 Hz, 2H), 3.35 (t, J = 6.1 Hz, 2H), 4.62 (s, 2H), 7.01 (t, J = 9.2 Hz, 1H), 7.15 (td, J = 7.7, 1.5, 1H), 7.20-7.28 (m, 3H), 7.34 (dd, J = 7.0, 2.4 Hz, 1H), 7.97 (dd, J = 8.1, 2.0 Hz, 1H), 8.01 (d, J = 7.0 Hz, 1H), 8.14 (d, J = 8.1 Hz, 1H), 8.57 (s, 1H), 9.11 (s, 1H), 9.55 (s, 1H), 10.47 (s, 1H) |
| N-(2-t-Butoxycarbonylaminophenyl)-5-(1-(t-butoxycarbonylmethyl)-3-(4-dimethylaminophenyl) ureidomethyl]pyridine-2-carboxylic acid amide (Reference Compound No.6-13) 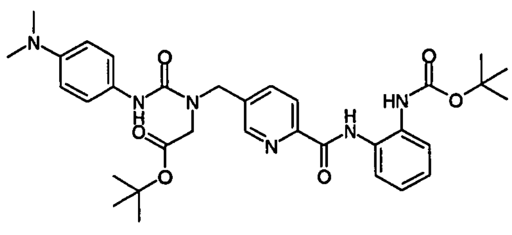 | $^{1}$H-NMR (400 MHz, DMSO-$d_6$) δ 1.34 (s, 9H), 1.48 (s, 9H), 2.82 (s, 6H), 4.11 (s, 2H), 4.70 (s, 2H), 6.66 (d, J = 9.3 Hz, 2H), 7.13-7.27 (m, 5H), 7.99 (dd, J = 8.1, 2.0 Hz, 1H), 8.01 (m, 1H), 8.13 (d, J = 8.1 Hz, 1H), 8.30 (br s, 1H), 8.60 (d, J = 2.0 Hz, 1H), 9.11 (br s, 1H), 10.48 (br s, 1H) |
| 5-[3-(Benzo[1,3]dioxol-5-yl)-1-(2-t-butoxycarbonylethyl) ureidomethyl]-N-(2-t-butoxycarbonylaminophenyl) pyridine-2-carboxylic acid amide (Reference Compound No.6-14) 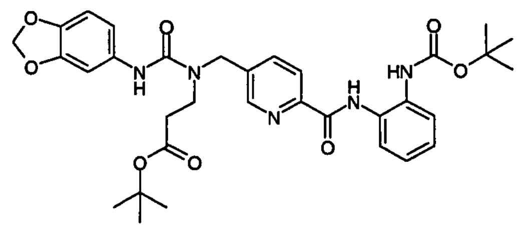 | $^{1}$H-NMR (500 MHz, DMSO-$d_6$) δ 1.38 (s, 9H), 1.47 (s, 9H), 2.52 (m, 2H), 3.55 (t, J = 7.0 Hz, 2H), 4.70 (s, 2H), 5.95 (s, 2H), 6.81 (m, 2H), 7.12 (m, 1H), 7.15 (td, J = 7.6, 1.5 Hz, 1H), 7.23-7.27 (m, 2H), 7.93 (dd, J = 8.2, 1.8 Hz, 1H), 8.01 (d, J = 7.6 Hz, 1H), 8.15 (d, J = 8.2 Hz, 1H), 8.47 (br s, 1H), 8.52 (d, J = 1.8 Hz, 1H), 9.12 (br s, 1H), 10.47 (br s, 1H) |

(continued)

| | |
|---|---|
| N-(2-t-Butoxycarbonylaminophenyl)-5-[1-(3-dimethylaminopropyl)-3-(indan-5-yl)ureidomethyl] pyridine-2-carboxylic acid amide (Reference Compound No.6-15)<br>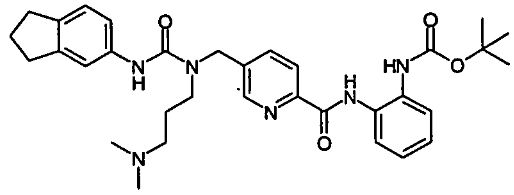 | $^1$H-NMR (400 MHz, DMSO-$d_6$)<br>δ 1.47 (s, 9H), 1.69 (br s, 2H), 1.99 (m, 2H), 2.19 (s, 6H), 2.27 (t, J = 6.2 Hz, 2H), 2.76-2.83 (m, 4H), 3.36 (m, 2H), 4.61 (s, 2H), 7.08 (s, 1H), 7.09 (d, J = 7.6 Hz, 1H), 7.15 (td, J = 7.6, 1.6 Hz, 1H), 7.24 (d, J = 7.6 Hz, 1H), 7.25 (m, 1H), 7.37 (m, 1H), 7.97 (dd, J = 8.1, 1.8 Hz, 1H), 8.01 (d, J = 7.6 Hz, 1H), 8.14 (d, J = 8.1 Hz, 1H), 8.58 (d, J = 1.8 Hz, 1H), 9.13 (br s, 1H), 9.51 (br s, 1H), 10.47 (br s, 1H) |
| 5-[3-(Biphenyl-4-yl)-1-(3-dimethylaminopropyl) ureidomethyl]-N-(2-t-butoxycarbonylaminophenyl) pyridine-2-carboxylic acid amide (Reference Compound No.6-16)<br>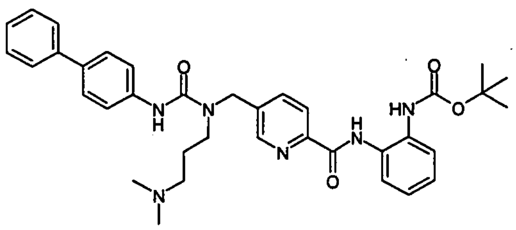 | $^1$H-NMR (500 MHz, DMSO-$d_6$)<br>δ 1.47 (s, 9H), 1.72 (m, 2H), 2.23 (s, 6H), 2.29 (t, J = 6.3 Hz, 2H), 3.37 (t, J = 6.3 Hz, 2H), 4.65 (s, 2H), 7.15 (td, J = 7.6, 1.4 Hz, 1H), 7.25 (m, 1H), 7.31 (m, 1H), 7.43 (t, J = 7.6 Hz, 2H), 7.52-7.56 (m, 3H), 7.57-7.60 (m, 2H), 7.62-7.65 (m, 2H), 7.99-8.02 (m, 2H), 8.15 (d, J = 7.9 Hz, 1H), 8.60 (d, J = 1.7 Hz, 1H), 9.11 (br s, 1H), 9.84 (br s, 1H), 10.47 (br s, 1H) |
| N-(2-t-Butoxycarbonylaminophenyl)-5-[1-(3-dimethylaminopropyl)-3-(4-trifluoromethylphenyl) ureidomethyl]pyridine-2-carboxylic acid amide (Reference Compound No.6-17)<br>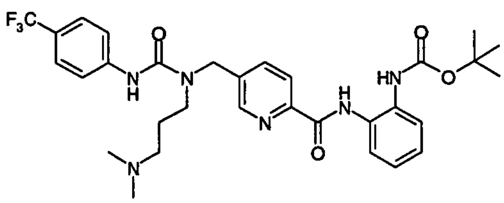 | $^1$H-NMR (500 MHz, DMSO-$d_6$)<br>δ 1.47 (s, 9H), 1.71 (m, 2H), 2.06 (s, 6H), 2.28 (t, J = 6.1 Hz, 2H), 3.36 (t, J = 6.1, Hz, 2H), 4.65 (s, 2H), 7.15 (td, J = 7.9, 1.5 Hz, 1H), 7.25 (m, 1H), 7.39 (td, J = 7.9, 1.5 Hz, 1H), 7.65-7.72 (m, 4H), 7.95 (dd, J = 8.1, 1.8 Hz, 1H), 8.03 (dd, J = 7.9, 1.5 Hz, 1H), 8.14 (d, J = 8.1 Hz, 1H), 8.52 (d, J = 1.8 Hz, 1H), 9.12 (br s, 1H), 9.61 (br s, 1H), 10.46 (br s, 1H) |
| N-(2-t-Butoxycarbonylaminophenyl)-5-[3-(4-cyanophenyl)-1-(3-dimethylaminopropyl)ureidomethyl] pyridine-2-carboxylic acid amide (Reference Compound No.6-18)<br>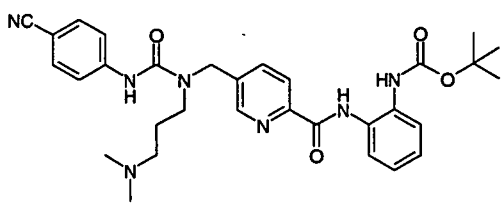 | $^1$H-NMR (400 MHz, DMSO-$d_6$)<br>δ 1.46 (s, 9H), 1.72 (m, 2H), 2.21 (s, 6H), 2.28 (t, J = 6.0 Hz, 2H), 3.37 (t, J = 6.0 Hz, 2H), 4.64 (s, 2H), 7.15 (td, J = 7.6, 1.7 Hz, 1H), 7.23-7.28 (m, 2H), 7.61 (m, 2H), 7.72 (m, 2H), 7.98-8.02 (m, 2H), 8.15 (d, J = 8.1 Hz, 1H), 8.59 (d, J = 1.8 Hz, 1H), 9.12 (br s, 1H), 10.17 (br s, 1H), 10.47 (br s, 1H) |

(continued)

| | |
|---|---|
| N-(2-t-Butoxycarbonylaminophenyl)-5-[1-(3-dimethylaminopropyl)-3-(4-trifluoromethoxyphenyl) ureidomethyl]pyridine-2-carboxylic acid amide (Reference Compound No.6-19)<br>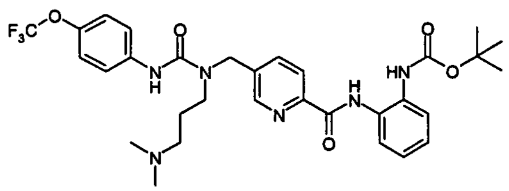 | $^1$H-NMR (400 MHz, DMSO-$d_6$)<br>δ 1.46 (s, 9H), 1.71 (m, 2H), 2.20 (s, 6H), 2.27 (t, J = 6.1 Hz, 2H), 3.37 (t, J = 6.1 Hz, 2H), 4.64 (s, 2H), 7.15 (td, J = 7.6, 1.6 Hz, 1H), 7.23-7.30 (m, 3H), 7.51-7.56 (m, 3H), 7.98 (dd, J = 8.1, 1.8 Hz, 1H), 8.01 (d, J = 7.6 Hz, 1H), 8.14 (d, J = 8.1 Hz, 1H), 8.58 (d, J = 1.8 Hz, 1H), 9.13 (br s, 1H), 9.83 (br s, 1H), 10.47 (br s, 1H) |
| N-(2-t-Butoxycarbonylaminophenyl)-5-[3-(4-dimethylaminophenyl)-1-(3-hydroxypropyl)ureidomethyl] pyridine-2-carboxylic acid amide (Reference Compound No.6-20)<br>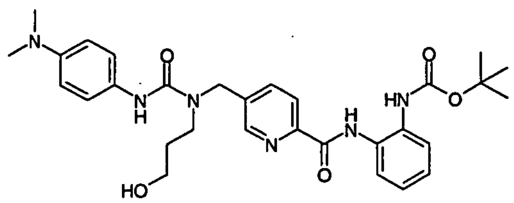 | $^1$H-NMR (400 MHz, DMSO-$d_6$)<br>δ 1.47 (s, 9H), 1.67 (m, 2H), 2.82 (s, 6H), 3.39 (t, J = 6.5 Hz, 2H), 3.46 (m, 2H), 4.66 (s, 2H), 4.85 (br s, 1H), 6.66 (d, J = 9.0 Hz, 2H), 7.15 (td, J = 7.6, 1.6 Hz, 1H), 7.20-7.27 (m, 4H), 7.94 (dd, J = 8.1, 1.8 Hz, 1H), 8.01 (d, J = 7.6 Hz, 1H), 8.14 (d, J = 8.1 Hz, 1H), 8.31 (br s, 1H), 8.54 (d, J = 1.8 Hz, 1H), 9.12 (br s, 1H), 10.47 (br s, 1H) |
| N-(2-t-Butoxycarbonylaminophenyl)-5-[3-(2,3-dihydrobenzo[1,4]dioxin-6-yl)-1-(3-hydroxypropyl) ureidomethyl]pyridine-2-carboxylic acid amide (Reference Compound No.6-21)<br>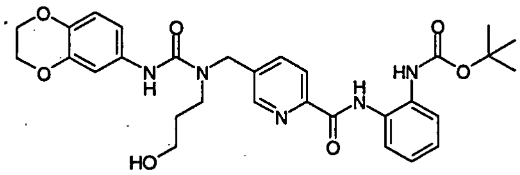 | $^1$H-NMR (400 MHz, DMSO-$d_6$)<br>δ 1.47 (s, 9H), 1.66 (m, 2H), 3.38 (t, J = 6.4 Hz, 2H), 3.46 (m, 2H), 4.16-4.21 (m, 4H), 4.65 (s, 2H), 4.91 (burs, 1H), 6.72 (d, J = 8.8 Hz, 1H). 6.84 (dd, J = 8.8, 2.4 Hz, 1H), 7.03 (d, J = 2.4 Hz, 1H), 7.15 (td, J = 7.7, 1.7 Hz, 1H), 7.23-7.27 (m, 2H), 7.94 (dd, J = 8.1, 1.7 Hz, 1H), 8.01 (d, J = 7.7 Hz, 1H), 8.14 (d, J = 8.1 Hz, 1H), 8.45 (br s, 1H), 8.54 (d, J = 1.7 Hz, 1H), 9.12 (br s, 1H), 10.47 (br s, 1H) |
| N-(2-t-Butoxycarbonylaminophenyl)-5-[3-(4-cyanophenyl)-1-(3-hydroxcypropyl)ureidomethyl] pyridine-2-carboxylic acid amide (Reference Compound No.6-22)<br>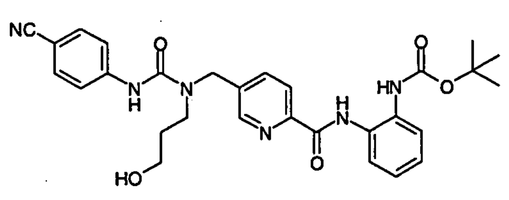 | $^1$H-NMR (400 MHz, DMSO-$d_6$)<br>δ 1.46 (s, 9H), 1.69 (m, 2H), 3.33 (br s, 2H), 3.45 (m, 2H), 4.70 (s, 2H), 5.03 (br s, 1H), 7.15 (td, J = 7.7, 1.7 Hz, 1H), 7.23-7.27 (m, 2H), 7.65 (d, J = 9.0 Hz, 2H), 7.71 (d, J = 9.0 Hz, 2H), 7.97 (dd, J = 8.1, 1.7 Hz, 1H), 8.00 (d, J = 7.7 Hz, 1H), 8.14 (d, J = 8.1 Hz, 1H), 8.56 (d, J = 1.7 Hz, 1H), 9.13 (br s, 1H), 9.15 (br s, 1H), 10.47 (br s, 1H) |

(continued)

| | |
|---|---|
| 5-[3-(Benzo(1,3]dioxol-5-yl)-1-(3-hydroxypropyl) ureidomethyl]-N-(2-t-butoxycarbonylaminophenyl) pyridine-2-carboxylic acid amide (Reference Compound No.6-23) 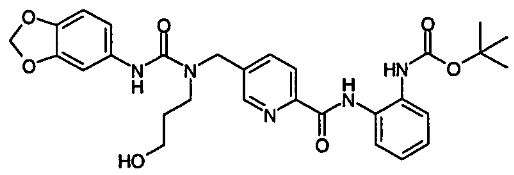 | $^1$H-NMR (400 MHz, DMSO-$d_6$) δ 1.47 (s, 9H), 1.67 (m, 2H), 3.39 (t, J = 6.6 Hz, 2H), 3.47 (m, 2H), 4.66 (s, 2H), 4.92 (br s, 1H), 5.95 (s, 2H), 6.79-6.82 (m, 2H), 7.12 (m, 1H), 7.15 (td, J = 7.8, 1.6 Hz, 1H), 7.23-7.27 (m, 2H), 7.95 (dd, J = 8.1, 2.0 Hz, 1H), 8.01 (d, J = 7.8 Hz, 1H), 8.15 (d, J = 8.1 Hz, 1H), 8.53 (br s, 1H), 8.56 (d, J = 2.0 Hz, 1H), 9.13 (br s, 1H), 10.47 (br s, 1H) |
| N-(2-t-Butoxycarbonylaminophenyl)-5-[1-(3-hydroxypropyl)-3-(4-methoxyphenyl)ureidomethyl] pyridine-2-carboxylic acid amide (Reference Compound No.6-24) 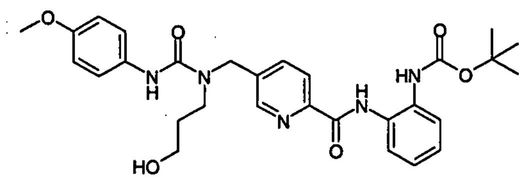 | $^1$H-NMR (400 MHz, DMSO-$d_6$) δ 1.47 (s, 9H), 1.68 (m, 2H), 3.40 (t, J = 6.6 Hz, 2H), 3.47 (m, 2H), 3.71 (s, 3H), 4.66 (s, 2H), 4.89 (br s, 1H), 6.84 (d, J = 9.3 Hz, 2H), 7.15 (td, J = 7.7, 1.6 Hz, 1H), 7.23-7.27 (m, 2H), 7.32 (d, J = 9.3 Hz, 2H), 7.95 (dd, J = 8.1, 1.8 Hz, 1H), 8.01 (d, J = 7.7 Hz, 1H), 8.15 (d, J = 8.1 Hz, 1H), 8.47 (br s, 1H), 8.55 (d, J = 1.8 Hz, 1H), 9.13 (br s, 1H), 10.47 (br s, 1H) |
| N-(2-t-Butoxycarbonylaminophenyl)-5-[3-(4-dimethylaminophenyl)-1-(2-ethoxycarbonylethyl) ureidomethyl]pyridine-2-carboxylic acid amide (Reference Compound No.6-25) 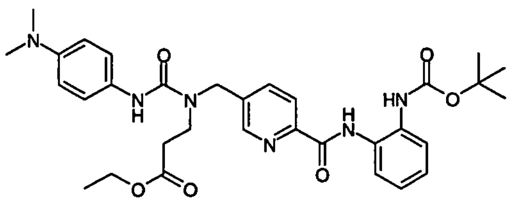 | $^1$H-NMR (400 MHz, DMSO-$d_6$) δ 1.16 (m, 3H), 1.47 (s, 9H), 2.60 (t, J = 7.1 Hz, 2H), 2.82 (s, 6H), 3.58 (t, J = 7.1 Hz, 2H), 4.03 (q, J = 7.1 Hz, 2H), 4.70 (s, 2H), 6.66 (d, J = 9.0 Hz, 2H), 7.15 (td, J = 7.8, 1.6 Hz, 1H), 7.21-7.27 (m, 4H), 7.93 (dd, J = 8.1, 1.7 Hz, 1H), 8.01 (d, J = 7.8 Hz, 1H), 8.15 (d, J = 8.1 Hz, 1H), 8.30 (br s, 1H), 8.52 (d, J = 1.7 Hz, 1H), 9.12 (br s, 1H), 10.47 (br s, 1H) |
| 5-[3-(Benzo[1,3]dioxol-5-yl)-1-(2-ethoxycarbonylethyl) ureidomethyl]-N-(2-t-butoxycarbonylaminophenyl) pyridine-2-carboxylic acid amide (Reference Compound No.6-26) 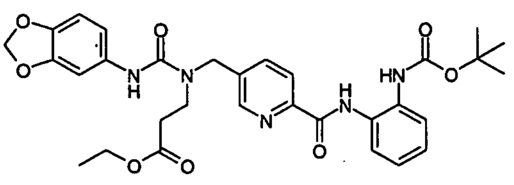 | $^1$H-NMR (400 MHz, DMSO-$d_6$) δ 1.16 (m, 3H), 1.47 (s, 9H), 2.60 (t, J = 7.1 Hz, 2H), 3.59 (t, J.= 7.1 Hz, 2H), 4.03 (q, J = 7.1 Hz, 2H), 4.71 (s, 2H), 5.95 (s, 2H), 6.81 (m, 2H), 7.12 (m, 1H), 7.15 (td, J = 7.6, 1.6 Hz, 1H), 7.23-7.27 (m, 2H), 7.93 (dd, J = 8.1, 1.8 Hz, 1H), 8.01 (d, J = 7.6 Hz, 1H), 8.15 (d, J = 8.1 Hz, 1H), 8.48 (br s, 1H), 8.52 (d, J = 1.8 Hz, 1H), 9.13 (br s, 1H), 10.47 (br s, 1H) |

(continued)

| | |
|---|---|
| N-(2-t-Butoxycarbonylaminophenyl)-5-[1-(2-ethoxycarbonylethyl)-3-(4-methoxyphenyl)ureidomethyl] pyridine-2-carboxylic acid amide (Reference Compound No.6-27) 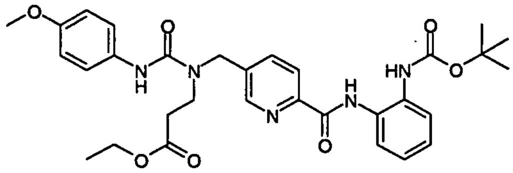 | $^1$H-NMR (400 MHz, DMSO-$d_6$) δ 1.16 (m, 3H), 1.47 (s, 9H), 2.61 (t, J = 7.1 Hz, 2H), 3.59 (t, J = 7.1 Hz, 2H), 3.71 (s, 3H), 4.03 (q, J = 7.2 Hz, 2H), 4.71 (s, 2H), 6.84 (d, J = 9.0 Hz, 2H), 7.15 (td, J = 7.6, 1.3 Hz, 1H), 7.23-7.27 (m, 2H), 7.33 (d, J = 9.0 Hz, 2H), 7.93 (dd, J = 8.1, 1.8 Hz, 1H), 8.01 (d, J = 7.6 Hz, 1H), 8.15 (d, J = 8.1 Hz, 1H), 8.44 (br s, 1H), 8.52 (d, J = 1.8 Hz, 1H), 9.12 (br s, 1H), 10.47 (br s, 1H) |
| N-(2-t-Butoxycarbonylaminophenyl)-5-[1-(3-dimethylaminopropyl)-3-(thiophen-2-yl)ureidomethyl] pyridine-2-carboxylic acid amide (Reference Compound No.6-28) 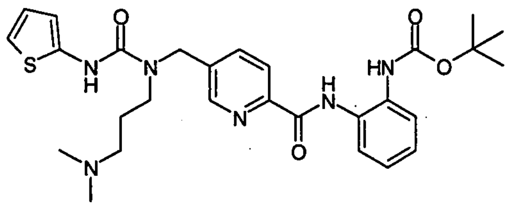 | $^1$H-NMR (400 MHz, DMSO-$d_6$) δ 1.47 (s, 9H), 1.68 (m, 2H), 2.21 (br s, 6H), 2.27 (t, J = 6.1 Hz, 2H), 3.32 (m, 2H), 4.64 (s, 2H), 6.52 (dd, J = 3.4, 1.7 Hz, 1H), 6.78-6.82 (m, 2H), 7.15 (m, 1H), 7.23-7.27 (m, 2H), 7.96 (dd, J = 8.1, 1.8 Hz, 1H), 8.01 (d, J = 7.8 Hz, 1H), 8.15 (d, J = 8.1 Hz, 1H), 8.57 (d, J = 1.8 Hz, 1H), 9.12 (br s, 1H), 10.47 (br s, 1H), 11.09 (br s, 1H) |
| N-(2-t-Butoxycarbonylaminophenyl)-5-[1-(3-dimethylaminopropyl)-3-(thiophen-3-yl)ureidomethyl] pyridine-2-carboxylic acid amide (Reference Compound No.6-29) 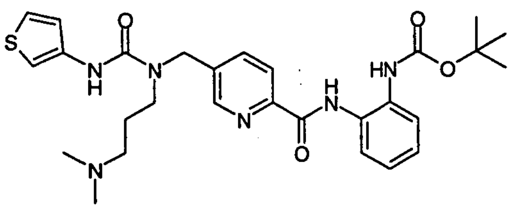 | $^1$H-NMR (400 MHz, DMSO-$d_6$) δ 1.47 (s, 9H), 1.68 (m, 2H), 2.19 (s, 6H), 2.25 (t, J = 6.1 Hz, 2H), 3.33 (m, 2H), 4.63 (s, 2H), 7.04 (dd, J = 5.1, 1.2 Hz, 1H), 7.15 (td, J = 7.7, 1.3 Hz, 1H), 7.23-7.28 (m, 3H), 7.40 (dd, J = 5.1, 3.2 Hz, 1H), 7.96 (dd, J = 8.1, 1.3 Hz, 1H), 8.01 (d, J = 7.7 Hz, 1H), 8.14 (d, J = 8.1 Hz, 1H), 8.57 (d, J = 1.3 Hz, 1H), 9.12 (br s, 1H), 10.07 (br s, 1H), 10.47 (br s, 1H) |
| N-(2-t-Butoxycarbonylaminophenyl)-5-[3-(2,3-dihydrobenzo[1,4]dioxin-6-yl)-1-[3-(morpholin-4-yl) propyl]ureidomethyl]pyridine-2-carboxylic acid amide (Reference Compound No.6-30) 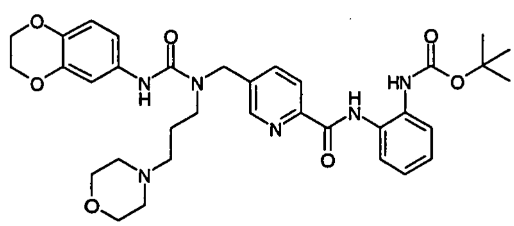 | $^1$H-NMR (400 MHz, DMSO-$d_6$) δ 1.47 (s, 9H), 1.68 (m, 2H), 2.26-2.30 (m, 6H), 3.34 (m, 2H), 3.53-3.55 (m, 4H), 4.17-4.22 (m, 4H), 4.67 (s, 2H), 6.72-6.78 (m, 2H), 6.87 (dd, J = 8.6, 2.6 Hz, 1H), 7.05 (m, 1H), 7.15 (dd, J = 7.6, 1.6 Hz, 1H), 7.24 (m, 1H), 7.94 (dd, J = 8.1, 1.7 Hz, 1H), 8.01 (d, J = 7.6 Hz, 1H), 8.14 (d, J = 8.1 Hz, 1H), 8.48 (br s, 1H), 8.54 (d, J = 1.7 Hz, 1H), 9.12 (br s, 1H), 10.47 (br s, 1H) |

(continued)

| | |
|---|---|
| 5-[3-(Benzo[1,3]dioxol-5-yl)-1-[3-(morpholin-4-yl)propyl] ureidomethyl]-N-(2-t-butoxycarbonylaminophenyl) pyridine-2-carboxylic acid amide (Reference Compound No.6-31) 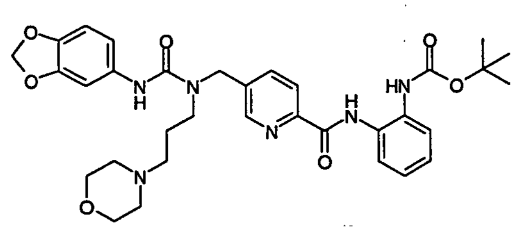 | $^1$H-NMR (400 MHz, DMSO-$d_6$) δ 1.47 (s, 9H), 1.68 (m, 2H), 2.26-2.30 (m, 6H), 3.35 (m, 2H), 3.52-3.54 (m, 4H), 4.67 (s, 2H), 5.95 (s, 2H), 6.82 (m, 2H), 7.14-7.19 (m, 2H), 7.23-7.27 (m, 2H), 7.95 (dd, J = 8.1, 1.6 Hz, 1H), 8.01 (d, J = 7.6 Hz, 1H), 8.15 (d, J = 8.1 Hz, 1H), 8.54 (d, J = 1.6 Hz, 1H), 8.57 (br s, 1H), 9.13 (br s, 1H), 10.47 (br s, 1H) |
| N-(2-t-Butoxycarbonylaminophenyl)-5-[3-(4-methoxyphenyl)-1-[3-(morpholin-4-yl)propyl] ureidomethyl]pyridine-2-carboxylic acid amide (Reference Compound No.6-32) 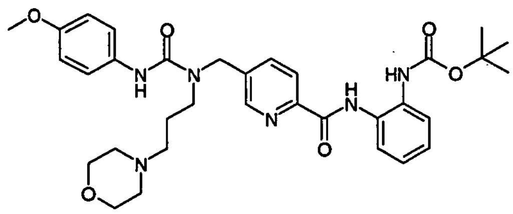 | $^1$H-NMR (400 MHz, DMSO-$d_6$) δ 1.47 (s, 9H), 1.69 (m, 2H), 2.27-2.30 (m, 6H), 3.36 (m, 2H), 3.52-3.54 (m, 4H), 3.71 (s, 3H), 4.68 (s, 2H), 6.85 (d, J = 9.1 Hz, 2H), 7.15 (td, J = 7.6, 1.6 Hz, 1H), 7.24 (d, J = 7.6 Hz, 1H), 7.25 (m, 1H), 7.34 (d, J = 9.1 Hz, 2H), 7.95 (dd, J = 8.1, 1.6 Hz, 1H), 8.01 (d, J = 7.6 Hz, 1H), 8.15 (d, J = 8.1 Hz, 1H), 8.54 (d, J = 1.6 Hz, 1H), 8.55 (br s, 1H), 9.12 (br s, 1H), 10.47 (br s, 1H) |
| N-(2-t-Butoxycarbonylaminophenyl)-5-[3-(2,3-dihydro-1-benzofuran-5-yl)-1-(3-dimethylaminopropyl) ureidomethyl]pyridine-2-carboxylic acid amide (Reference Compound No.6-33) 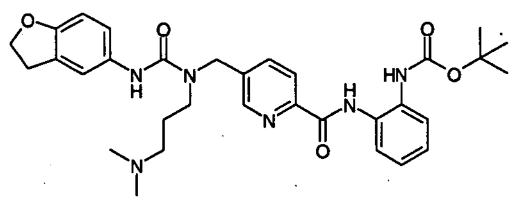 | $^1$H-NMR (400 MHz, DMSO-$d_6$) δ 1.48 (s, 9H), 1.68 (m, 2H), 2.17 (s, 6H), 2.25 (t, J = 6.3 Hz, 2H), 2.50 (m, 2H), 3.14 (t, J = 8.7 Hz, 2H), 4.47 (t, J = 8.7 Hz, 2H), 4.61 (s, 2H), 6.64 (d, J = 8.4 Hz, 1H), 7.01 (dd, J = 8.4, 2.1 Hz, 1H), 7.15 (td, J = 7.7, 1.6 Hz, 1H), 7.23-7.27 (m, 2H), 7.35 (d, J = 2.1 Hz, 1H), 7.96 (dd, J = 8.1, 2.0 Hz, 1H), 8.01 (d, J = 7.7 Hz, 1H), 8.14 (dd, J = 8.1, 0.5 Hz, 1H), 8.57 (d, J = 2.0 Hz, 1H), 9.13 (br s, 1H), 9.32 (br s, 1H), 10.47 (br s, 1H) |
| N-(2-t-Butoxycarbonylaminophenyl)-5-[3-(2,3-dihydro-1-benzofuran-5-yl)-1-[3-(morpholin-4-yl) propyl] ureidomethyl]pyridine-2-carboxylic acid amide (Reference Compound No.6-34) 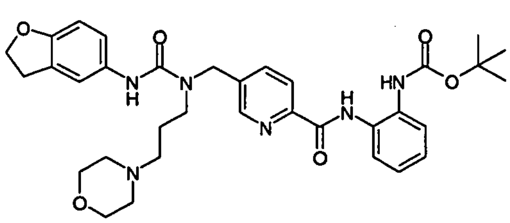 | $^1$H-NMR (400 MHz, DMSO-$d_6$) δ 1.48 (s, 9H), 1.68 (m, 2H), 2.27-2.32 (m, 6H), 3.14 (t, J = 8.5 Hz, 2H), 3.33 (m, 2H), 3.52-3.54 (m, 4H), 4.48 (t, J = 8.5 Hz, 2H), 4.67 (s, 2H), 6.65 (d, J = 8.5 Hz, 1H), 7.04 (dd, J = 8.5, 2.0 Hz, 1H), 7.15 (td, J = 7.6, 1.3 Hz, 1H), 7.23-7.27 (m, 2H), 7.32 (d, J = 2.0 Hz, 1H), 7.95 (dd, J = 8.1, 1.5 Hz, 1H), 8.01 (d, J = 7.6 Hz, 1H), 8.15 (d, J = 8.1 Hz, 1H), 8.50 (br s, 1H), 8.55 (d, J = 1.5 Hz, 1H), 9.13 (br s, 1H), 10.47 (br s, 1H) |

(continued)

| | |
|---|---|
| N-(2-t-Butoxycarbonylaminophenyl)-5-[1-(3-diethylaminopropyl)-3-(4-fluorophenyl)ureidometh yl] pyridine-2-carboxylic acid amide (Reference Compound No.6-35) 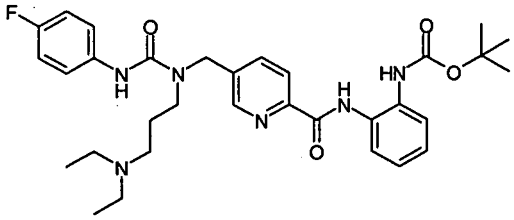 | $^1$H-NMR (400 MHz, DMSO-$d_6$) δ 0.92 (t, J = 7.1 Hz, 6H), 1.47 (s, 9H), 1.67 (m, 2H), 2.37 (t, J = 6.3 Hz, 2H), 2.47 (m, 4H), 3.35 (m, 2H), 4.66 (s, 2H), 7.10 (t, J = 8.9 Hz, 2H), 7.15 (td, J = 7.5, 1.5 Hz, 1H), 7.23-7.27 (m, 2H), 7.43 (m, 2H), 7.95 (dd, J = 8.1, 1.6 Hz, 1H), 8.01 (d, J = 7.5 Hz, 1H), 8.15 (d, J = 8.1 Hz, 1H), 8.56 (d, J = 1.6 Hz, 1H), 9.11 (br s, 1H), 10.47 (br s, 1H) |
| N-(2-t-Butoxycarbonylaminophenyl)-5-[1-(3-diethylaminopropyl)-3-(3,4-difluorophenyl)ureidomethyl] pyridine-2-carboxylic acid amide (Reference Compound No.6-36) 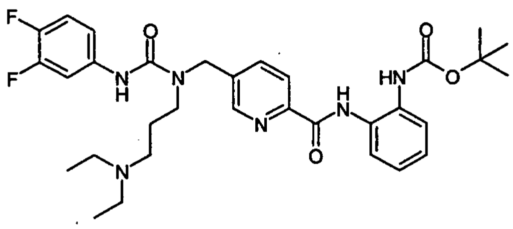 | $^1$H-NMR (400 MHz, DMSO-$d_6$) δ 0.92 (t, J = 7.1 Hz, 6H), 1.46 (s, 9H), 1.67 (m, 2H), 2.36 (t, J = 6.3 Hz, 2H), 2.46 (m, 4H), 3.35 (m, 2H), 4.67 (s, 2H), 7.13-7.17 (m, 2H), 7.23-7.28 (m, 2H), 7.33 (dd, J = 19.7, 9.2 Hz, 1H), 7.65 (ddd, J = 13.7, 7.6, 2.4 Hz, 1H), 7.97 (dd, J = 8.1, 2.0 Hz, 1H), 8.01 (dd, J = 8.1, 1.2 Hz, 1H), 8.15 (d, J = 8.1 Hz, 1H), 8.56 (d, J = 2.0 Hz, 1H), 9.13 (br s, 1H), 9.30 (br s, 1H), 10.47 (br s, 1H) |
| N-(2-t-Butoxycarbonylaminophenyl)-5-[1-(3-diethylaminopropyl)-3-(3,5-difluorophenyl)ureidomethyl] pyridine-2-carboxylic acid amide (Reference Compound No.6-37) 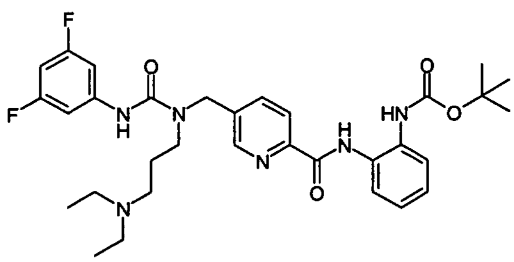 | $^1$H-NMR (400 MHz, DMSO-$d_6$) δ 0.93 (t, J = 7.1 Hz, 6H), 1.46 (s, 9H), 1.67 (m, 2H), 2.36 (t, J = 6.6 Hz, 2H), 2.48 (m, 4H), 3.36 (t, J = 6.6 Hz, 2H), 4.68 (s, 2H), 6.77 (tt, J = 9.3, 2.4 Hz, 1H), 7.13-7.28 (m, 5H), 7.97 (dd, J = 8.1, 1.7 Hz, 1H), 8.01 (dd, J = 8.2, 1.7 Hz, 1H), 8.15 (d, J = 8.1 Hz, 1H), 8.57 (d, J = 1.7 Hz, 1H), 9.13 (br s, 1H), 9.47 (br s, 1H), 10.47 (br s, 1H) |
| N-(2-t-Butoxycarbonylaminophenyl)-5-[3-(3-chloro-4-fluorophenyl)-1-(3-diethylaminopropyl)ureidomethyl] pyridine-2-carboxylic acid amide (Reference Compound No.6-38) 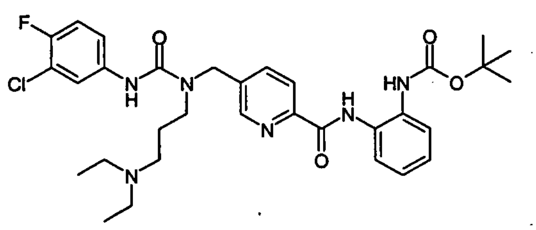 | $^1$H-NMR (400 MHz, DMSO-$d_6$) δ 0.92 (t, J = 7.1 Hz, 6H), 1.46 (s, 9H), 1.67 (m, 2H), 2.36 (t, J = 6.7 Hz, 2H), 2.47 (m, 4H), 3.36 (t, J = 6.7 Hz, 2H), 4.67 (s, 2H), 7.15 (td, J = 7.6, 1.5 Hz, 1H), 7.22-7.28 (m, 2H), 7.31-7.35 (m, 2H), 7.78 (m, 1H), 7.97 (dd, J = 8.1, 1.7 Hz, 1H), 8.01 (d, J = 7.6, 1.5 Hz, 1H), 8.15 (d, J = 8.1 Hz, 1H), 8.56 (d, J = 1.7 Hz, 1H), 9.12 (br s, 1H), 9.25 (br s, 1H), 10.47 (br s, 1H) |

(continued)

| | |
|---|---|
| N-(2-t-Butoxycarbonylaminophenyl)-5-[1-(3-diethylaminopropyl)-3-(3-fluorophenyl)ureidomethyl] pyridine-2-carboxylic acid amide (Reference Compound No.6-39) 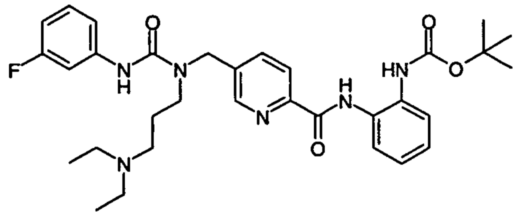 | $^1$H-NMR (400 MHz, DMSO-$d_6$) δ 0.94 (t, J = 7.2 Hz, 6H), 1.46 (s, 9H), 1.68 (m, 2H), 2.37 (t, J = 6.5 Hz, 2H), 2.48 (m, 4H), 3.36 (t, J = 6.5 Hz, 2H), 4.67 (s, 2H), 6.77 (m, 1H), 7.12-7.17 (m, 2H), 7.23-7.32 (m, 3H), 7.46 (m, 1H), 7.98 (dd, J = 8.2, 2.0 Hz, 1H), 8.01 (dd, J = 8.1, 1.0 Hz, 1H), 8.15 (d, J = 8.2 Hz, 1H), 8.57 (d, J = 2.0 Hz, 1H), 9.12 (br s, 1H), 9.36 (br s, 1H), 10.47 (br s, 1H) |
| N-(2-t-Butoxycarbonylaminophenyl)-5-[1-(3-diethlaminopropyl)-3-(4-fluoro-3-methylphenyl) ureidomethyl]pyridine-2-carboxylic acid amide (Reference Compound No.6-40) 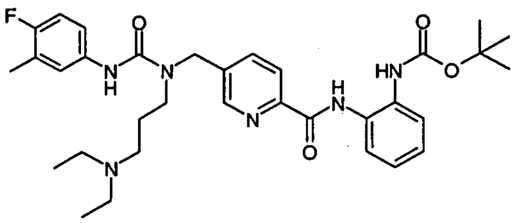 | $^1$H-NMR (400 MHz, $CDCl_3$) δ 1.03 (t, J = 7.2 Hz, 6H), 1.51 (s, 9H), 1.76 (m, 2H), 2.25 (d, J = 2.0 Hz, 3H), 2.51 (t, J = 6.1 Hz, 2H), 2.63 (q, J = 7.2 Hz, 4H), 3.38 (t, J = 5.9 Hz, 2H), 4.62 (s, 2H), 6.91 (t, J = 9.0 Hz, 1H), 7.05 (br s, 1H), 7.10 (m, 1H), 7.17-7.25 (m, 2H), 7.34 (m, 1H), 7.60 (d, J = 7.3 Hz, 1H), 7:69 (d, J = 7.3 Hz, 1H), 7.93 (dd, J = 8.1, 2.2 Hz, 1H), 8.26 (d, J = 8.1 Hz, 1H), 8.58 (d, J = 2.2 Hz, 1H), 9.73 (s, 1H), 10.15 (s, 1H) |
| N-(2-t-Butoxycarbonylaminophenyl)-5-[1-(3-diethylaminopropyl)-3-(4-fluoro-3-nitrophenyl) ureidomethyl]pyridine-2-carboxylic acid amide (Reference Compound No.6-41) 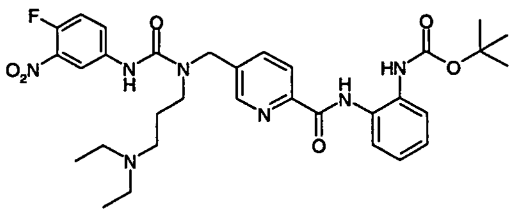 | $^1$H-NMR (500 MHz, $CDCl_3$) δ 1.08 (t, J = 7.2 Hz, 6H), 1.52 (s, 9H), 1.80 (m, 2H), 2.54 (t, J = 6.1 Hz, 2H), 2.68 (q, J = 7.2 Hz, 4H), 3.40 (t, J = 5.7 Hz, 2H), 4.63 (s, 2H), 7.02 (br s, 1H), 7.18-7.24 (m, 2H), 7.58 (d, J = 6.4 Hz, 1H), 7.62-7.76 (m, 1H), 7.71 (d, J = 6.4 Hz, 1H), 7.88-7.93 (m, 2H), 8.03 (dd, J = 6.4, 2.7 Hz, 1H), 8.27 (d, J = 7.9 Hz, 1H), 8.59 (d, J = 1.5 Hz, 1H), 10.16 (s, 1H), 10.53 (s, 1H) |
| N-(2-t-Butoxycarbonylaminophenyl)-5-[1-(3-diethylaminopropyl)-3-(3-ethoxyphenyl)ureidomethyl] pyridine-2-carboxylic acid amide (Reference Compound No.6-42) 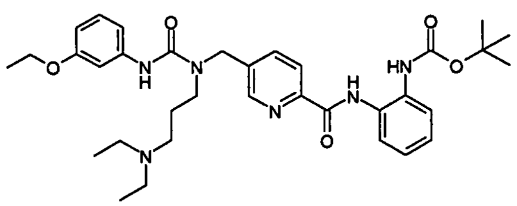 | $^1$H-NMR (400 MHz, $CDCl_3$) δ 1.06 (t, J = 7.2 Hz, 6H), 1.40 (t, J = 7.0 Hz, 3H), 1.51 (s, 9H), 1.76 (m, 2H), 2.51 (t, J = 6.1 Hz, 2H), 2.66 (q, J = 7.2 Hz, 4H), 3.38 (t, J = 5.6 Hz, 2H), 4.03 (q, J = 7.0 Hz, 2H), 4.63 (s, 2H), 6.56 (m, 1H), 6.90 (m, 1H), 7.05 (br s, 1H), 7.15 (t, J = 8.2 Hz, 1H), 7.19-7.23 (m, 3H), 7.60 (d, J = 6.9 Hz, 1H), 7.69 (d, J = 6.9 Hz, 1H), 7.93 (dd, J = 8.1, 2.2 Hz, 1H), 8.26 (d, J = 8.1 Hz, 1H), 8.58 (d, J = 2.2 Hz, 1H), 9.79 (s, 1H), 10.15 (s, 1H) |

(continued)

| | |
|---|---|
| N-(2-t-Butoxycarbonylaminophenyl)-5-[3-(2,3-dihydrobenzo[1,4]dioxin-6-yl)-1-(2-dimethylaminoethyl) ureidomethyl]pyridine-2-carboxylic acid amide (Reference Compound No.6-43) 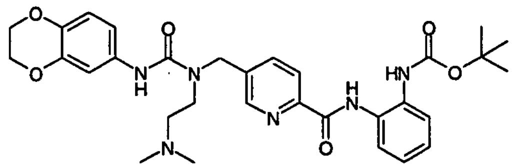 | $^1$H-NMR (400 MHz, $CDCl_3$) δ 1.52 (s, 9H), 2.38 (s, 6H), 2.51 (t, J = 4.3 Hz, 2H), 3.31 (t, J = 4.3 Hz, 2H, 4.21-4.25 (m, 4H), 4.64 (s, 2H), 6.76-6.82 (m, 2H), 6.93 (d, J = 2.0 Hz, 1H), 7.08 (br s, 1H), 7.18-7.23 (m, 2H), 7.58 (br s, 1H), 7.72 (br s, 1H), 7.91 (dd, J = 8.1, 2.2 Hz, 1H), 8.26 (d, J = 8.1 Hz, 1H), 8.56 (d, J = 2.2 Hz, 1H), 10.16 (s, 1H), 10.85 (s, 1H) |
| N-(2-t-Butoxycarbonylaminophenyl)-5-(1-(2-dimethylaminoethyl)-3-(3-methoxyphenyl)ureidomethyl] pyridine-2-carboxylic acid amide (Reference Compound No.6-44) 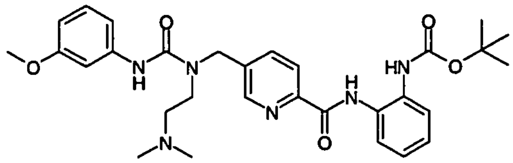 | $^1$H-NMR (400 MHz, $CDCl_3$) δ 1.51 (s, 9H), 2.40 (s, 6H), 2.53 (t, J = 4.3 Hz, 2H), 3.33 (t, J = 4.3 Hz, 2H), 3.81 (s, 3H), 4.66 (s, 2H), 6.56 (ddd, J = 7.8, 2.7, 0.8 Hz, 1H), 6.84 (dd, J = 7.8, 1.2 Hz, 1H), 7.07 (s, 1H), 7.14-7.22 (m, 4H), 7.58 (br s, 1H), 7.73 (br s, 1H), 7.91 (dd, J = 7.8, 2.1 Hz, 1H), 8.26 (d, J = 7.8 Hz, 1H), 8.57 (d, J = 2.1 Hz, 1H), 10.17 (s, 1H), 11.12 (s, 1H) |
| N-(2-t-Butoxycarbonylaminophenyl)-5-[1-(2-dimethylaminoethyl)-3-(3-fluorophenyl)ureidomethyl] pyridine-2-carboxylic acid amide (Reference Compound No.6-45) 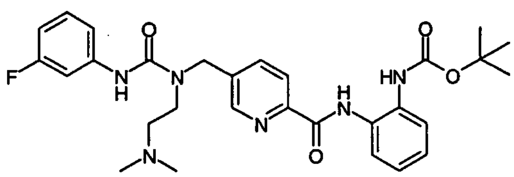 | $^1$H-NMR (400 MHz, $CDCl_3$) δ 1.52 (s, 9H), 2.41 (s, 6H), 2.55 (t, J = 4.3 Hz, 2H), 3.33 (t, J = 4.3 Hz, 2H), 4.66 (s, 2H), 6.68 (td, J = 8.4, 1.6 Hz, 1H), 6.98-7.07 (m, 2H), 7.18-7.29 (m, 4H), 7.58 (br s, 1H), 7.73 (br s, 1H), 7.91 (dd, J = 8.1, 2.2 Hz, 1H), 8.28 (d, J = 8.1 Hz, 1H), 8.58 (d, J = 2.2 Hz, 1H), 10.17 (s, 1H), 11.33 (s, 1H) |
| N-(2-t-Butoxycarbonylaminophenyl)-5-[1-(2-dimethylaminoethyl)-3-(thiophen-3-yl)ureidomethyl] pyridine-2-carboxylic acid amide (Reference Compound No.6-46) 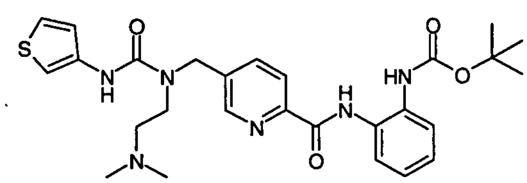 | $^1$H-NMR (400 MHz, $CDCl_3$) δ 1.51 (s, 9H), 2.39 (s, 6H), 2.53 (t, J = 4.3 Hz, 2H), 3.31 (t, J = 4.3 Hz, 2H), 4.67 (s, 2H), 6.87 (dd, J = 5.1, 1.2 Hz, 1H), 7.00 (s, 1H), 7.18-7.22 (m, 3H), 7.30 (dd, J = 5.1, 1.3 Hz, 1H), 7.58 (br s, 1H), 7.71 (br s, 1H), 7.91 (dd, J = 7.9, 2.2 Hz, 1H), 8.26 (d, J = 7.9 Hz, 1H), 8.56 (d, J = 2.2 Hz, 1H), 10.16 (s, 1H), 11.49 (s, 1H) |

(continued)

| | |
|---|---|
| N-(2-t-Butoxycarbonylaminophenyl)-5-[3-(3,4-difluorophenyl)-1-[3-(pyrrolidin-1-yl)propyl]ureidomethyl] pyridine-2-carboxylic acid amide (Reference Compound No.6-47) 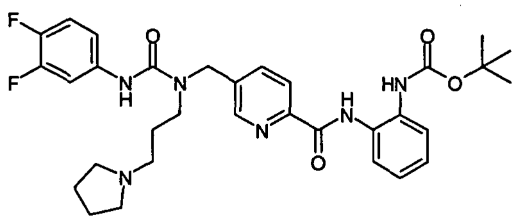 | $^1$H-NMR (400 MHz, DMSO-$d_6$) δ 1.47 (s, 9H), 1.69-1.74 (m, 6H), 2.40-2.44 (m, 6H), 3.40 (t, J = 6.2 Hz, 2H), 4.65 (s, 2H), 7.10 (m, 1H), 7.15 (td, J = 7.6, 1.5 Hz, 1H), 7.23-7.27 (m, 2H), 7.35 (dd, J = 19.8, 9.3 Hz, 1H), 7.65 (ddd, J = 13.7, 7.6, 2.4 Hz, 1H), 7.97 (dd, J = 8.1, 1.8 Hz, 1H), 8.01 (d, J = 8.1 Hz, 1H), 8.14 (dd, J = 8.1, 0.5 Hz, 1H), 8.57 (d, J = 1.8 Hz, 1H), 9.12 (br s, 1H), 9.55 (br s, 1H), 10.47 (br s, 1H) |
| N-(2-t-Butoxycarbonylaminophenyl)-5-[3-(pyridin-3-yl)-1-[3-(pyrrolidin-1-yl)propyl]ureidomethyl]pyridine-2-carboxylic acid amide (Reference Compound No.6-48) 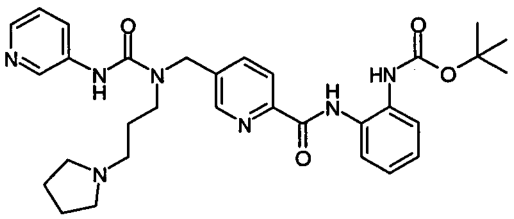 | $^1$H-NMR (500 MHz, DMSO-$d_6$) δ 1.47 (s, 9H), 1.72-1.74 (m, 6H), 2.42-2.46 (m, 6H), 3.43 (t, J = 6.3 Hz, 2H), 4.67 (s, 2H), 7.15 (td, J = 7.5, 1.5 Hz, 1H), 7.24-7.27 (m, 2H), 7.30 (dd, J = 8.2, 4.7 Hz, 1H), 7.89 (ddd, J = 8.2, 2.4, 1.5 Hz, 1H), 7.98 (dd, J = 7.9, 1.7 Hz, 1H), 8.01 (d, J = 7.5 Hz, 1H), 8.14 (d, J = 7.9 Hz, 1H), 8.18 (dd, J = 4.7, 1.5 Hz, 1H), 8.56 (d, J = 2.4 Hz, 1H), 8.57 (d, J = 1.7 Hz, 1H), 9.11 (br s, 1H), 9.58 (br s, 1H), 10.47 (br s, 1H) |
| N-(2-t-Butoxycarbonylaminophenyl)-5-[3-(3-chlorophenyl)-1-[3-(morpholin-4-yl)propyl]ureidomethyl] pyridine-2-carboxylic acid amide (Reference Compound No.6-49) 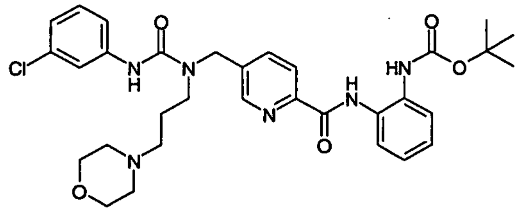 | $^1$H-NMR (500 MHz, $CDCl_3$) δ 1.52 (s, 9H), 1.80 (m, 2H), 2.45-2.52 (m, 6H), 3.39 (t, J = 5.7 Hz, 2H), 3.69-3.74 (m, 4H), 4.64 (s, 2H), 7.02 (br s, 1H), 7.09 (d, J = 8.1 Hz, 1H), 7.18-7.25 (m, 3H), 7.31 (d, J = 8.1 Hz, 1H), 7.55-7.61 (m, 2H), 7.71 (d, J = 6.4 Hz, 1H), 7.91 (dd, J = 8.1, 1.8 Hz, 1H), 8.26 (d, J = 8.1 Hz, 1H), 8.58 (d, J = 1.8 Hz, 1H), 9.04 (s, 1H), 10.16 (s, 1H) |
| N-(2-t-Butoxycarbonylaminophenyl)-5-[3-(2-fluorophenethyl)-1-[3-(morpholin-4-yl)propyl] ureidomethyl]pyridine-2-carboxylic acid amide (Reference Compound No.6-50) 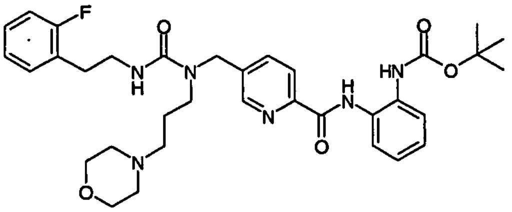 | $^1$H-NMR (400 MHz, $CDCl_3$) δ 1.52 (s, 9H), 1.62 (m, 2H), 2.27-2.36 (m, 6H), 2.92 (t, J = 6.6 Hz, 2H), 3.14 (t, J = 5.6 Hz, 2H), 3.49 (td, J = 6.6, 6.6 Hz, 2H), 3.56 (br s, 4H), 4.57 (s, 2H), 7.00-7.11 (m, 3H), 7.17-7.25 (m, 4H), 7.34 (br s, 1H), 7.61 (br s, 1H), 7.69 (br s, 1H), 7.78 (dd, J = 8.0, 1.8 Hz, 1H), 8.24 (dd, J = 8.0, 0.6 Hz, 1H); 8.50 (d, J = 1.8 Hz, 1H), 10.15 (s, 1H) |

(continued)

| | |
|---|---|
| N-(2-t-Butoxycarbonylaminophenyl)-5-[3-(4-fluorophenethyl)-1-[3-(morpholin-4-yl)propyl]ureidomethyl]pyridine-2-carboxylic acid amide (Reference Compound No.6-51) 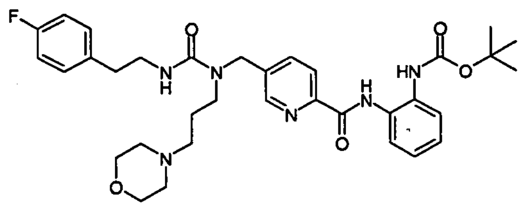 | 1-NMR (500 MHz, $CDCl_3$) δ 1.51 (s, 9H), 1.63 (m, 2H), 2.27-2.36 (m, 6H), 2.84 (t, J = 6.8 Hz, 2H), 3.15 (t, J = 5.5 Hz, 2H), 3.45 (td, J = 6.8, 6.8 Hz, 2H), 3.57 (br s, 4H), 4.57 (s, 2H), 7.00 (t, J = 8.7 Hz, 2H), 7.08 (br s, 1H), 7.16 (dd, J = 8.7, 5.4 Hz, 2H), 7.19-7.25 (m, 3H), 7.62 (br s, 1H), 7.69 (br s, 1H), 7.82 (dd, J = 8.0, 1.8 Hz, 1H), 8.26 (dd, J = 8.0, 0.6 Hz, 1H), 8.47 (d, J = 1.8 Hz, 1H), 10.15 (s, 1H) |
| N-(2-t-Butoxycarbonylaminophenyl)-5-[1-[3-(morpholin-4-yl)propyl]-3-phenethylureidomethyl] pyridine-2-carboxylic acid amide (Reference Compound No.6-52) 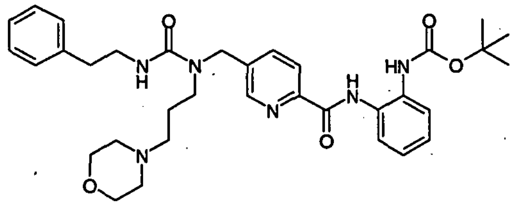 | $^1$H-NMR (400 MHz, $CDCl_3$) δ 1.53 (s, 9H), 1.61 (m, 2H), 2.27-2.32 (m, 6H), 2.86 (t, J = 6.7 Hz, 2H), 3.15 (t, J = 5.6 Hz, 2H), 3.45-3.52 (m, 6H), 4.58 (s, 2H), 7.14 (br s, 1H), 7.18-7.23 (m, 5H), 7.26-7.32 (m, 3H), 7.60 (br s, 1H), 7.69 (br s, 1H), 7.81 (dd, J = 8.2, 2.2 Hz, 1H), 8.25 (d, J = 8.2 Hz, 1H), 8.51 (d, J = 2.2 Hz, 1H), 10.15 (s, 1H) |
| N-(2-t-Butoxycarbonylaminophenyl)-5-[3-(3-fluorophenyl)-1-[2-(4-methylpiperazin-1-yl)ethyl] ureidomethyl]pyridine-2-carboxylic acid amide (Reference Compound No.6-53) 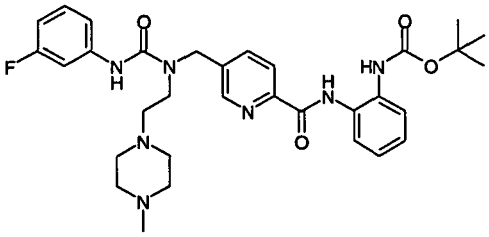 | $^1$H-NMR (400 MHz, $CDCl_3$) δ 1.52 (s, 9H), 2.33 (s, 3H), 2.54 (br s, 4H), 2.60 (t, J = 4.3 Hz, 2H), 2.68 (br s, 4H), 3.37 (t, J = 4.3 Hz, 2H), 4.65 (s, 2H), 6.74 (m, 1H), 7.03 (br s, 1H), 7.17-7.28 (m, 4H), 7.37 (m, 1H), 7.58 (d, J = 6.1 Hz, 1H), 7.72 (d, J = 6.1 Hz, 1H), 7.92 (dd, J = 8.1, 2.2 Hz, 1H), 8.27 (d, J = 8.1 Hz, 1H), 8.58 (d, J = 2.2 Hz, 1H), 10.17 (s, 1H), 10.19 (s, 1H) |
| N-(2-t-Butoxycarbonylaminophenyl)-5-[3-(3,4-difluorophenyl)-1-[2-(4-methylpiperazin-1-yl)ethyl] ureidomethyl]pyridine-2-carboxylic acid amide (Reference Compound No.6-54) 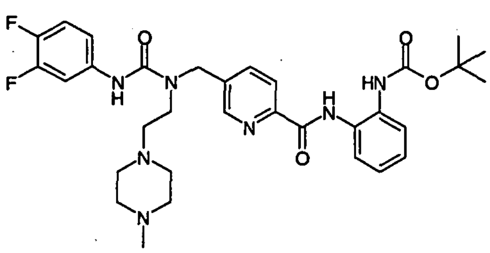 | $^1$H-NMR (400 MHz, $CDCl_3$) δ 1.52 (s, 9H), 2.32 (s, 3H), 2.51 (br s, 4H), 2.60 (t, J = 4.2 Hz, 2H), 2.67 (br s, 4H), 3.36 (t, J = 4.2 Hz, 2H), 4.64 (s, 2H), 7.02 (br s, 1H), 7.07-7.10 (m, 2H), 7.18-7.25 (m, 2H), 7.47 (m, 1H), 7.58 (d, J = 6.3 Hz, 1H), 7.72 (d, J = 6.3 Hz, 1H), 7.91 (dd, J = 8.1, 1.7 Hz, 1H), 8.28 (d, J = 8.1 Hz, 1H), 8.57 (d, J = 1.7 Hz, 1H), 10.17 (s, 1H), 10.23 (s, 1H) |

(continued)

| | |
|---|---|
| N-(2-t-Butoxycarbonylaminophenyl)-5-[3-(2,3-dihydrobenzo[1 ,4]dioxin-6-yl)-1-[2-(4-methylpiperazin-1-yl)ethyl]ureidomethyl]pyddine-2-carbox ylic acid amide (Reference Compound No.6-55) 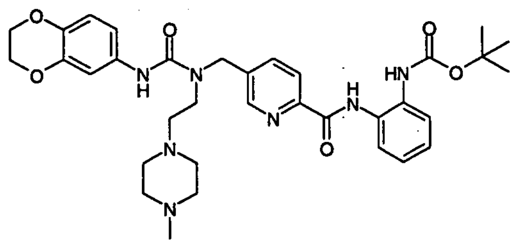 | $^1$H-NMR (400 MHz, $CDCl_3$) δ 1.52 (s, 9H), 2.31 (s, 3H), 2.52 (br s, 4H), 2.56 (t, J = 4.2 Hz, 2H), 2.64 (br s, 4H), 3.34 (t, J = 4.2 Hz, 2H), 4.22-4.26 (m, 4H), 4.64 (s, 2H), 6.80 (d, J = 8.7 Hz, 1H), 6.88 (dd, J = 8.7, 2.6 Hz, 1H), 7.03 (d, J = 2.6 Hz, 1H), 7.05 (br s, 1H), 7.18-7.25 (m, 2H), 7.59 (d, J = 6.8 Hz, 1H), 7.71 (d, J = 6.8 Hz, 1H), 7.92 (dd, J = 8.1, 2.1 Hz, 1H), 8.26 (d, J = 8.1 Hz, 1H), 8.56 (d, J = 2.1 Hz, 1H), 9.86 (s, 1H), 10.16 (s, 1H) |
| N-(2-t-Butoxycarbonylaminophenyl)-5-[3-(3-fluorophenyl)-1-[3-(4-methylpiperazin-1-yl)propyl] ureidomethyl]pyridine-2-carboxylic acid amide (Reference Compound No.6-56) 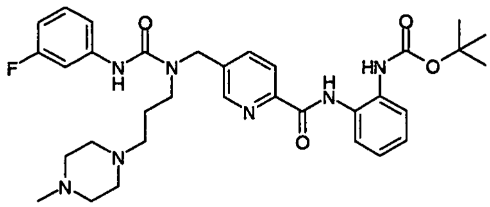 | $^1$H-NMR (400 MHz, $CDCl_3$) δ 1.51 (s, 9H), 1.79 (m, 2H), 2.29 (s, 3H), 2.46 (br s, 4H), 2.48 (t, J = 5.7 Hz, 2H), 2.52 (br s, 4H), 3.37 (t, J = 5.7 Hz, 2H), 4.64 (s, 2H), 6.78 (m, 1H), 7.04 (br s, 1H), 7.17-7.28 (m, 4H), 7.38 (m, 1H), 7.59 (d, J = 6.6 Hz, 1H), 7.70 (d, J = 6.6 Hz, 1H), 7.92 (dd, J = 8.1, 2.2 Hz, 1H), 8.25 (d, J = 8.1 Hz, 1H), 8.58 (d, J = 2.2 Hz, 1H), 9.20 (s, 1H), 10.16 (s, 1H) |
| N-(2-t-Butoxycarbonylaminophenyl)-5-[3-(3,4-diflorophenyl)-1-[3-(4-methylpiperazin-1-yl)propyl] ureidomethyl]pyridine-2-carboxylic acid amide (Reference Compound No.6-57) 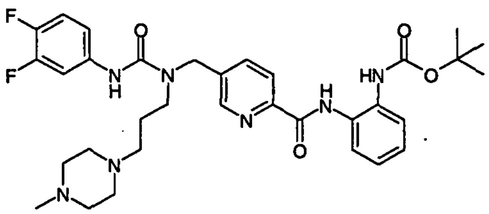 | $^1$H-NMR (500 MHz, $CDCl_3$) δ 1.51 (s, 9H), 1.79 (m, 2H), 2.26 (s, 3H), 2.40 (br s, 4H), 2.48 (t, J = 5.7 Hz, 2H), 2.49 (br s, 4H), 3.36 (t, J = 5.7 Hz, 2H), 4.62 (s, 2H), 7.03 (br s, 1H), 7.05-7.11 (m, 2H), 7.18-7.24 (m, 2H), 7.46 (m, 1H), 7.59 (d, J = 6.7 Hz, 1H), 7.70 (d, J = 6.7 Hz, 1H), 7.91 (dd, J = 7.9, 2.1 Hz, 1H), 8.25 (d, J = 7.9 Hz, 1H), 8.57 (d, J = 2.1 Hz, 1H), 9.31 (s, 1H), 10.15 (s, 1H) |
| N-(2-t-Butoxycarbonylaminophenyl)-5-[3-(2,3-dihydrobenzo[1,4]dioxin-6-yl)-1-[3-(4-methylpiperazin-1-yl)propyl]ureidomethyl]pyridine-2-carbo xylic acid amide (Reference Compound No.6-58) 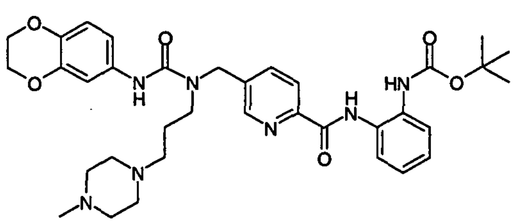 | $^1$H-NMR (400 MHz, $CDCl_3$) δ 1.51 (s, 9H), 1.76 (m, 2H), 2.24 (s, 3H), 2.39 (br s, 4H), 2.47 (t, J = 5.6 Hz, 2H), 2.48 (br s, 4H), 3.35 (t, J = 5.6 Hz, 2H), 4.24-4.25 (m, 4H), 4.62 (s, 2H), 6.78-6.86 (m, 2H), 7.02 (d, J = 2.2 Hz, 1H), 7.07 (br s, 1H), 7.17-7.24 (m, 2H), 7.60 (d, J = 6.6 Hz, 1H), 7.68 (d, J = 6.6 Hz, 1H), 7.93 (d, J = 8.1 Hz, 1H), 8.24 (d, J = 8.1 Hz, 1H), 8.56 (s, 1H), 9.06 (s, 1H), 10.14 (s, 1H) |

(continued)

| | |
|---|---|
| N-(2-t-Butoxycarbonylaminophenyl)-5-[3-(3-fluorophenyl)-1-[4-(pyrrolidin-1-yl)butyl]ureidomethyl] pyridine-2-carboxylic acid amide (Reference Compound No.6-59) 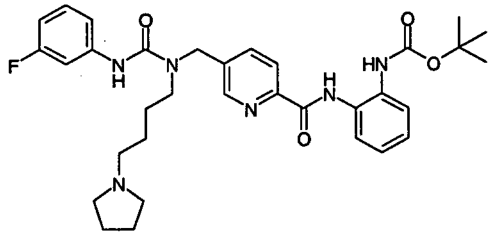 | $^1$H-NMR (400 MHz, $CDCl_3$) δ 1.51 (s, 9H), 1.56 (m, 2H), 1.71-1.78 (m, 6H), 2.49-2.53 (m, 6H), 3.30 (t, J = 8.3 Hz, 2H), 4.69 (s, 2H), 6.78 (m, 1H), 7.05 (m, 1H), 7.07 (br s, 1H), 7.19-7.28 (m, 4H), 7.34 (s, 1H), 7.59 (d, J = 6.8 Hz, 1H), 7.71 (d, J = 6.8 Hz, 1H), 7.89 (dd, J = 8.1, 2.2 Hz, 1H), 8.26 (d, J = 8.1 Hz, 1H), 8.57 (d, J = 2.2 Hz, 1H), 10.17 (s, 1H) |
| N-(2-t-Butoxycarbonylaminophenyl)-5-[3-(3,4-difluorophenyl)-1-[4-(pyrrolidin-1-yl)butyl]ureidomethyl] pyridine-2-carboxylic acid amide (Reference Compound No.6-60) 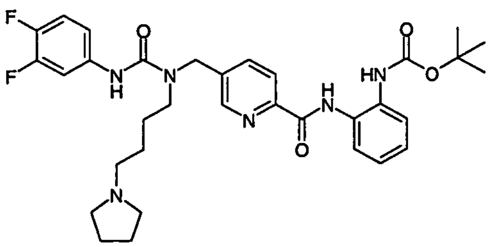 | $^1$H-NMR (400 MHz, $CDCl_3$) δ 1.51 (s, 9H), 1.55 (m, 2H), 1.66-1.77 (m, 6H), 2.48-2.54 (m, 6H), 3.28 (t, J = 8.2 Hz, 2H), 4.67 (s, 2H), 6.97 (m, 1H), 7.07 (m, 1H), 7.08 (br s, 1H), 7.18-7.25 (m, 2H), 7.34 (m, 1H), 7.55 (s, 1H), 7.58 (d, J = 7.0 Hz, 1H), 7.71 (d, J = 7.0 Hz, 1H), 7.88 (dd, J = 8.1, 2.1 Hz, 1H), 8.26 (d, J = 8.1 Hz, 1H), 8.56 (d, J = 2.1 Hz, 1H), 10.17 (s, 1H) |
| N-(2-t-Butoxycarbonylaminophenyl)-5-[3-(2,3-dihydrobenzo[1,4]dioxin-6-yl)-1-[4-(pyrrolidin-1-yl)butyl] ureidomethyl]pyridine-2-carboxylic acid amide (Reference Compound No.6-61) 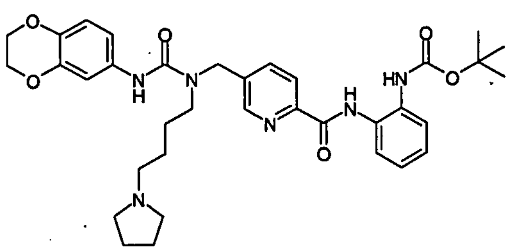 | $^1$H-NMR (400 MHz, $CDCl_3$) δ 1.52 (s, 9H), 1.53 (m, 2H), 1.64-1.68 (m, 4H), 1.72 (t, J = 8.3 Hz, 2H), 2.46-2.53 (m, 6H), 3.27 (t, J = 8.3 Hz, 2H), 4.23-4.24 (m, 4H), 4.67 (s, 2H), 6.75 (dd, J = 8.3, 2.2 Hz, 1H), 6.79 (d, J = 8.3 Hz, 1H), 6.89 (d, J = 2.2 Hz, 1H), 7.08 (br s, 1H), 7.17-7.25 (m, 2H), 7.37 (s, 1H), 7.60 (d, J = 7.1 Hz, 1H), 7.70 (d, J = 7.1 Hz, 1H), 7.89 (dd, J = 8.1, 2.0 Hz, 1H), 8.25 (d, J = 8.1 Hz, 1H), 8.56 (d, J = 2.0 Hz, 1H), 10.16 (s, 1H) |
| N-(2-t-Butoxycarbonylaminophenyl)-5-[1-(2-diethylaminoethyl)-3-(3-fluorophenyl)ureidomethyl] pyridine-2-carboxylic acid amide (Reference Compound No.6-62) 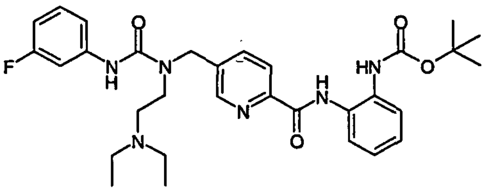 | s$^1$H-NMR (400 MHz, $CDCl_3$) δ 1.10 (t, J = 7.2 Hz, 6H), 1.52 (s, 9H), 2.61 (t, J = 4.0 Hz, 2H), 2.68 (q, J = 7.2 Hz, 4H), 3.35 (t, J = 4.0 Hz, 2H), 4.66 (s, 2H), 6.69 (m, 1H), 7.02 (m, 1H), 7.02 (br, 1H), 7.18-7.29 (m, 4H), 7.59 (d, J = 7.1 Hz, 1H), 7.72 (d, J = 7.1 Hz, 1H), 7.92 (dd, J = 8.1, 2.0 Hz, 1H), 8.27 (d, J = 8.1 Hz, 1H), 8.58 (d, J = 2.0 Hz, 1H), 10.17 (s, 1H), 11.23 (s, 1H) |

(continued)

| | |
|---|---|
| N-(2-t-Butoxycarbonylaminophenyl)-5-[1-(2-diethylaminoethyl)-3-(3,4-difluorophenyl)ureidomethyl] pyridine-2-carboxylic acid amide (Reference Compound No.6-63) 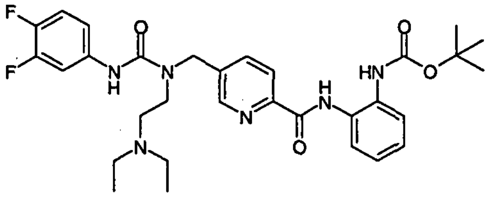 | $^1$H-NMR (500 MHz, $CDCl_3$) δ 1.09 (t, J = 7.1 Hz, 6H), 1.52 (s, 9H), 2.61 (t, J = 4.3 Hz, 2H), 2.67 (q, J = 7.1 Hz, 4H), 3.35 (t, J = 4.3 Hz, 2H), 4.65 (s, 2H), 6.92 (m, 1H), 7.03 (br s, 1H), 7.05 (m, 1H), 7.19-7.24 (m, 2H), 7.39 (m, 1H), 7.58 (d, J = 6.7 Hz, 1H), 7.72 (d, J =6.7 Hz, 1H), 7.91 (dd, J = 7.9, 2.1 Hz, 1H), 8.28 (d, J = 7.9 Hz, 1H), 8.58 (d, J = 2.1 Hz, 1H), 10.17 (s, 1H), 11.23 (s, 1H) |
| -(2-t-Butoxycarbonylaminophenyl)-5-[1-(2-diethylaminoethyl)-3-(2,3-dihydrobenzo[1,4]dioxin-6-yl) ureidomethyl]pyridine-2-carboxylic acid amide (Reference Compound No.6-64) 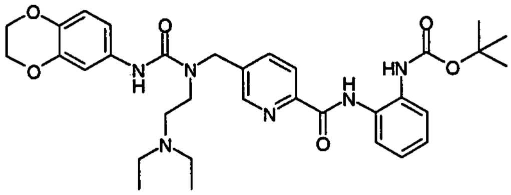 | $^1$H-NMR (400 MHz, $CDCl_3$) δ 1.08 (t, J = 7.2 Hz, 6H), 1.52 (s, 9H), 2.57 (t, J = 4.0 Hz, 2H), 2.64 (q, J = 7.2 Hz, 4H), 3.33 (t, J = 4.0 Hz, 2H), 4.21-4.25 (m, 4H), 4.65 (s, 2H), 6.80 (d, J = 8.5 Hz, 1H), 6.82 (dd, J = 8.5, 2.0 Hz, 1H), 6.93 (d, J = 2.0 Hz, 1H), 7.05 (br s, 1H), 7.17-7.25 (m, 2H), 7.60 (d, J = 6.8 Hz, 1H), 7.71 (d, J = 6.8 Hz, 1H), 7.93 (dd, J = 8.1, 2.2 Hz, 1H), 8.26 (d, J = 8.1 Hz, 1H), 8.57 (d, J = 2.2 Hz, 1H), 10.16 (s, 1H), 10.79 (s, 1H) |
| N-(2-t-Butoxycarbonylaminophenyl)-5-[3-(2,3-dihydrobenzo[1,4]dioxin-6-yl)-1-(4-dimethylaminobutyl) ureidomethyl]pyridine-2-carboxylic acid amide (Reference Compound No.6-65) 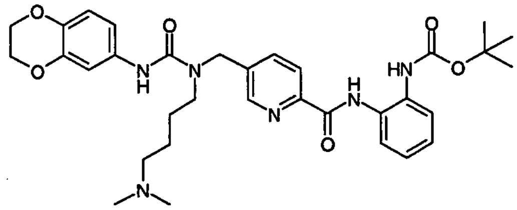 | $^1$H-NMR (500 MHz, $CDCl_3$) δ 1.52 (s, 9H), 1.54 (m, 2H), 1.69 (m, 2H), 2.21 (s, 6H), 2.35 (t, J = 6.4 Hz, 2H), 3.23 (t, J = 8.2 Hz, 2H), 4.23 (br s, 4H), 4.68 (s, 2H), 6.78 (d, J = 2.0 Hz, 1H), 6.79 (d, J = 0.8 Hz, 1H), 6.90 (dd, J = 2.0, 0.8 Hz, 1H), 7.08 (br s, 1H), 7.18-7.24 (m, 2H), 7.60 (d, J = 5.5 Hz, 1H), 7.70 (d, J = 6.2 Hz, 1H), 7.90 (dd, J = 8.2, 2.1 Hz, 1H), 8.25 (d, J = 8.2 Hz, 1H), 8.56 (d, J = 2.1 Hz, 1H), 10.16 (s, 1H) |
| N-(2-t-Butoxycarbonylaminophenyl)-5-[1-(4-dimethylaminobutyl)-3-(3-fluorophenyl)ureidomethyl] pyridine-2-carboxylic acid amide (Reference Compound No.6-66) 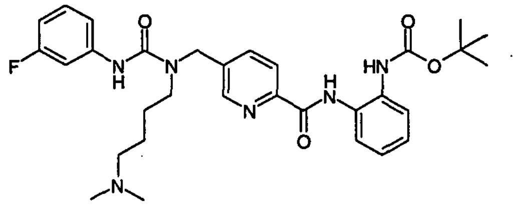 | $^1$H-NMR (500 MHz, $CDCl_3$) δ 1.52 (s, 9H), 1.55 (m, 2H), 1.72 (m, 2H), 2.24 (s, 6H), 2.36 (t, J = 6.5 Hz, 2H), 3.26 (t, J = 8.4 Hz, 2H), 4.69 (s, 2H), 6.77 (tdd, J = 8.4, 2.4, 0.8 Hz, 1H), 7.06 (d, J = 8.4 Hz, 1H), 7.07 (br s, 1H), 7.18-7.24 (m, 4H), 7.60 (d, J = 6.7 Hz, 1H), 7.70 (d, J = 6.1 Hz, 1H), 7.90 (dd, J = 8.1, 2.1 Hz, 1H), 7.99 (s, 1H), 8.26 (d, J = 8.1 Hz, 1H), 8.55 (d, J = 2.1 Hz, 1H), 10.17 (s, 1H) |

(continued)

| | |
|---|---|
| N-(2-t-Butoxycarbonylaminophenyl)-5-[1-(2-dimethylaminoethyl)-3-(3-methylphenyl)ureidomethyl] pyridine-2-carboxylic acid amide (Reference Compound No.6-67) 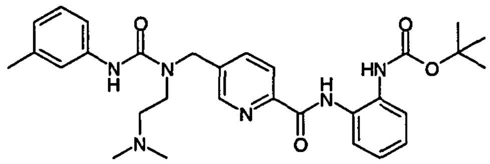 | $^{1}$H-NMR (500 MHz, $CDCl_3$) δ 1.52 (s, 9H), 2.34 (s, 3H), 2.40 (s, 6H), 2.53 (t, J = 4.4 Hz, 2H), 3.33 (t, J = 4.4 Hz, 2H), 4.66 (s, 2H), 6.82 (d, J = 7.3 Hz, 1H), 7.03 (br s, 1H), 7.05 (m, 1H), 7.15-7.24 (m, 3H), 7.29 (s, 1H), 7.59 (d, J = 6.7 Hz, 1H), 7.71 (d, J = 6.7 Hz, 1H), 7.92 (dd, J = 7.9, 2.1 Hz, 1H), 8.27 (d, J = 7.9 Hz, 1H), 8.57 (d, J = 2.1 Hz, 1H), 10.16 (s, 1H), 10.96 (s, 1H) |
| N-(2-t-Butoxycarbonylaminophenyl)-5-[3-(3-chlorophenyl)-1-(2-dimethylaminoethyl)ureidomethyl] pyridine-2-carboxylic acid amide (Reference Compound No.6-68) 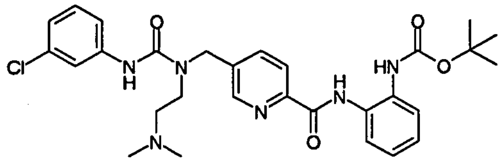 | $^{1}$H-NMR (500 MHz, $CDCl_3$) δ 1.52 (s, 9H), 2.41 (s, 6H), 2.55 (t, J = 4.3 Hz, 2H), 3.33 (t, J = 4.3 Hz, 2H), 4.66 (s, 2H), 6.96 (dt, J = 7.3, 1.8 Hz, 1H), 7.02 (br s, 1H), 7.15-7.24 (m, 4H), 7.47 (t, J = 1.8 Hz, 1H), 7.58 (d, J = 5.8 Hz, 1H), 7.72 (d, J = 6.1 Hz, 1H), 7.91 (dd, J = 7.9, 2.1 Hz, 1H), 8.27 (d, J = 7.9 Hz, 1H), 8.58 (d, J = 2.1 Hz, 1H), 10.17 (s, 1H), 11.30 (s, 1H) |
| N-(2-t-Butoxycarbonylaminophenyl)-5-[1-(2-dimethylaminoethyl)-3-(pyridin-3-yl)ureidomethyl] pyridine-2-carboxylic acid amide (Reference Compound No.6-69) 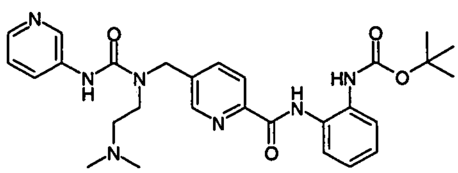 | $^{1}$H-NMR (500 MHz, $CDCl_3$) δ 1.52 (s, 9H), 2.42 (s, 6H), 2.58 (t, J = 4.3 Hz, 2H), 3.36 (t, J = 4.3 Hz, 2H), 4.67 (s, 2H), 7.03 (br s, 1H), 7.19-7.26 (m, 3H), 7.58 (d, J = 6.4 Hz, 1H), 7.72 (d, J = 5.8 Hz, 1H), 7.92 (dd, J = 7.9, 2.0 Hz, 1H), 8.09 (m, 1H), 8.23 (dd, J = 4.7, 1.4 Hz, 1H), 8.28 (d, J = 7.9 Hz, 1H), 8.32 (d, J = 2.4 Hz, 1H), 8.58 (d, J = 2.0 Hz, 1H), 10.17 (s, 1H), 11.53 (s, 1H) |
| N-(2-t-Butoxycarbonylaminophenyl)-5-[3-(3-chlorophenyl)-1-(3-dimethylaminopropyl)ureidomethyl] pyridine-2-carboxylic acid amide (Reference Compound No.6-70) 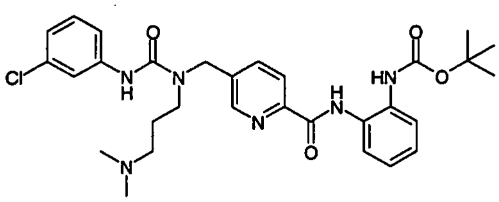 | $^{1}$H-NMR (400 MHz, DMSO-$d_6$) δ 1.47 (s, 9H), 1.71 (m, 2H), 2.20 (s, 6H), 2.27 (t, J = 6.3 Hz, 2H), 3.35 (m, 2H), 4.63 (s, 2H), 6.99 (ddd, J = 7.8, 2.0, 1.0 Hz, 1H), 7.15 (td, J = 8.1, 1.5 Hz, 1H), 7.19-7.30 (m, 4H), 7.73 (t, J = 2.1 Hz, 1H), 7.98 (dd, J = 8.1, 1.8 Hz, 1H), 8.02 (dd, J = 8.1, 1.0 Hz, 1H), 8.14 (d, J = 8.1 Hz, 1H), 8.58 (d, J = 1.8 Hz, 1H), 9.13 (br s, 1H), 9.95 (br s, 1H), 10.48 (br s, 1H) |

(continued)

| | |
|---|---|
| N-(2-t-Butoxycarbonylaminophenyl)-5-[1-(3-dimethylaminopropyl)-3-(3-methylphenyl)ureidomethyl] pyridine-2-carboxylic acid amide (Reference Compound No.6-71)<br>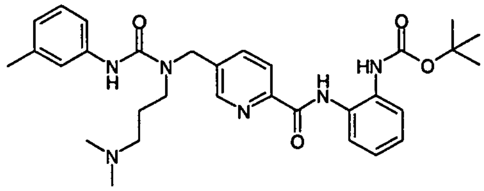 | $^1$H-NMR (400 MHz, DMSO-$d_6$)<br>δ 1.47 (s, 9H), 1.70 (t, J = 6.1 Hz, 2H), 2.20 (s, 6H), 2.26 (s, 3H), 2.28 (m, 2H), 3.35 (m, 2H), 4.62 (s, 2H), 6.76 (d, J = 7.3 Hz, 1H), 7.11-7.28 (m, 6H), 7.98 (dd, J = 8.1, 1.8 Hz, 1H), 8.01 (dd, J = 8.1, 1.0 Hz, 1H), 8.14 (d, J = 8.0 Hz, 1H), 8.58 (d, J = 1.8 Hz, 1H), 9.12 (br s, 1H), 9.61 (br s, 1H), 10.47 (br s, 1H) |
| N-(2-t-Butoxycarbonylaminophenyl)-5-[3-(4-dimethylaminophenyl)-1-[4-(morpholin-4-yl)butyl] ureidomethyl]pyridine-2-carboxylic acid amide (Reference Compound No.6-72)<br>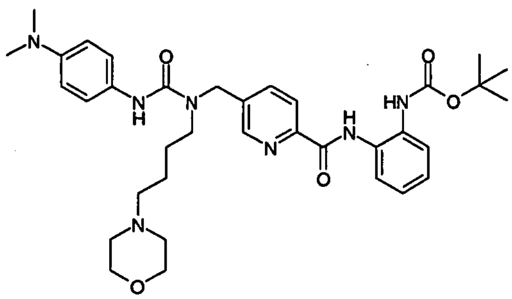 | $^1$H-NMR (500 MHz, $CDCl_3$)<br>δ 1.52 (s, 9H), 1.54 (m, 2H), 1.70 (m, 2H), 2.39 (t, J = 6.9 Hz, 2H), 2.43 (m, 4H), 2.92 (s, 6H), 3.30 (t, J = 8.1 Hz, 2H), 3.63 (t, J = 4.3 Hz, 4H), 4.68 (s, 2H), 6.61 (s, 1H), 6.71 (d, J = 8.9 Hz, 2H), 7.06 (br s, 1H), 7.17(d, J = 8.9 Hz, 2H), 7.19-7.22 (m, 2H), 7.59 (d, J = 5.8 Hz, 1H), 7.71 (d, J = 6.1 Hz, 1H), 7.90 (dd, J = 7.9, 1.8 Hz, 1H), 8.27 (d, J = 7.9 Hz, 1H), 8.56 (d, J = 1.8 Hz, 1H), 10.16 (s, 1H) |
| N-(2-t-Butoxycarbonylaminophenyl)-5-[3-(3-fluorophenyl)-1-[4-(morpholin-4-yl)butyl]ureidomethyl] pyridine-2-carboxylic acid amide (Reference Compound No.6-73)<br>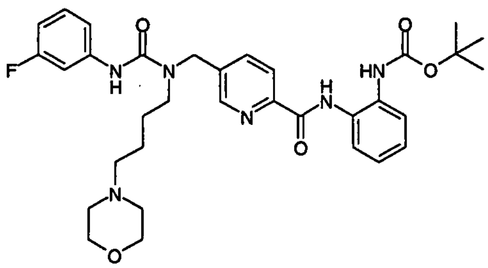 | $^1$H-NMR (500 MHz, $CDCl_3$)<br>δ 1.52 (s, 9H), 1.55 (m, 2H), 1.71 (m, 2H), 2.39 (t, J = 7.0 Hz, 2H), 2.43 (m, 4H), 3.33 (t, J = 7.9 Hz, 2H), 3.67 (t, J = 4.7 Hz, 4H), 4.68 (s, 2H), 6.70 (s, 1H), 6.78 (td, J = 8.4, 2.4 Hz, 1H), 7.01 (dd, J = 7.9, 1.8 Hz, 1H), 7.05 (br s, 1H), 7.19-7.25 (m, 3H), 7.31 (dt, J = 10.8, 2.4 Hz, 1H), 7.56 (d, J = 6.1 Hz, 1H), 7.73 (d, J = 5.2 Hz, 1H), 7.87 (dd, J = 7.9, 2.1 Hz, 1H), 8.26 (d, J = 7.9 Hz, 1H), 8.55 (d, J = 2.1 Hz, 1H), 10.18 (s, 1H) |

(continued)

| | |
|---|---|
| N-(2-t-Butoxycarbonylaminophenyl)-5-[1-[4-(morpholin-4-yl)butyl]-3-(thiophen-3-yl)ureidomethyl]pyridine-2-carboxylic acid amide (Reference Compound No.6-74) 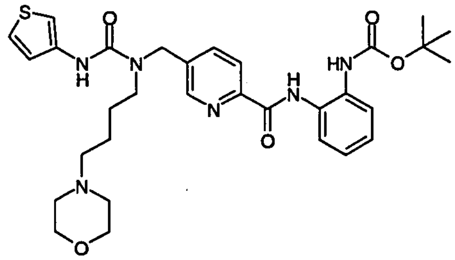 | $^1$H-NMR (500 MHz, $CDCl_3$) δ 1.51 (s, 9H), 1.54 (m, 2H), 1.70 (m, 2H), 2.38 (t, J = 7.0 Hz, 2H), 2.43 (t, J = 4.5 Hz, 4H), 3.31 (t, J = 7.9 Hz, 2H), 3.67 (t, J = 4.5 Hz, 4H), 4.69 (s, 2H), 6.86 (s, 1H), 6.98 (dd, J = 5.2, 1.5 Hz, 1H), 7.05 (br s, 1H), 7.20-7.25 (m, 3H), 7.27 (dd, J = 3.2, 1.5 Hz, 1H), 7.58 (d, J = 6.1 Hz, 1H), 7.72 (d, J = 6.4 Hz, 1H), 7.87 (dd, J = 7.9, 2.0 Hz, 1H), 8.26 (d, J = 7.9 Hz, 1H), 8.55 (d, J = 2.0 Hz, 1H), 10.16 (s, 1H) |
| N-(2-t-Butoxycarbonylaminophenyl)-5-[3-(2,3-dihydrobenzo[1,4]dioxin-6-yl)-1-[4-(morpholin-4-yl)butyl] ureidomethyl]pyridine-2-carboxylic acid amide (Reference Compound No.6-75) 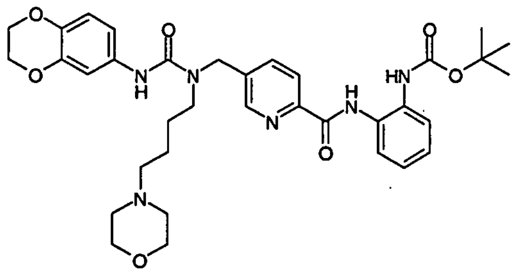 | $^1$H-NMR (500 MHz, $CDCl_3$) δ 1.52 (s, 9H), 1.54 (m, 2H), 1.70 (m, 2H), 2.39 (t, J = 7.0 Hz, 2H), 2.43 (m, 4H), 3.29 (t, J = 7.9 Hz, 2H), 3.64 (t, J = 4.6 Hz, 4H), 4.24 (br s, 4H), 4.67 (s, 2H), 6.61 (s, 1H), 6.75 (dd, J = 8.6, 2.4 Hz, 1H), 6.80 (d, J = 8.6 Hz, 1H), 6.92 (d, J = 2.4 Hz, 1H), 7.05 (br s, 1H), 7.19-7.24 (m, 2H), 7.59 (d, J = 6.1 Hz, 1H), 7.72 (d, J = 5.5 Hz, 1H), 7.89 (dd, J = 7.9, 1.8 Hz, 1H), 8.27 (d, J = 7.9 Hz, 1H), 8.56 (d, J = 1.8 Hz, 1H), 10.16 (s, 1H) |
| N-(2-t-Butoxycarbonylaminophenyl)-5-[1-[4-(morpholin-4-yl)butyl]-3-(pyridin-3-yl)ureidomethyl]pyridine-2-carboxylic acid amide (Reference Compound No.6-76) 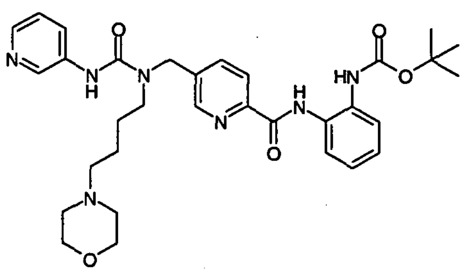 | $^1$H-NMR (500 MHz, $CDCl_3$) δ 1.51 (s, 9H), 1.57 (m, 2H), 1.74 (m, 2H), 2.41 (t, J = 7.0 Hz, 2H), 2.43 (t, J = 4.6 Hz, 4H), 3.36 (t, J = 8.1 Hz, 2H), 3.66 (t, J = 4.6 Hz, 4H), 4.70 (s, 2H), 6.86 (s, 1H), 7.05 (br s, 1H), 7.19-7.25 (m, 2H), 7.28 (dd, J = 8.7, 4.6 Hz, 1H), 7.55 (d, J = 6.4 Hz, 1H), 7.74 (d, J = 5.8 Hz, 1H), 7.87 (dd, J = 8.0, 2.0 Hz, 1H), 7.98 (ddd, J = 8.7, 2.4, 1.3 Hz, 1H), 8.26 (d, J = 8.0 Hz, 1H), 8.34 (dd, J = 4.6, 1.3 Hz, 1H), 8.45 (d, J = 2.4 Hz, 1H), 8.56 (d, J = 2.0 Hz, 1H), 10.19 (s, 1H) |

(continued)

| | |
|---|---|
| N-(2-t-Butoxycarbonylaminophenyl)-5-[1-[4-(morpholin-4-yl)butyl]-3-phenethylureidomethyl]pyridine-2-carboxylic acid amide (Reference Compound No.6-77) 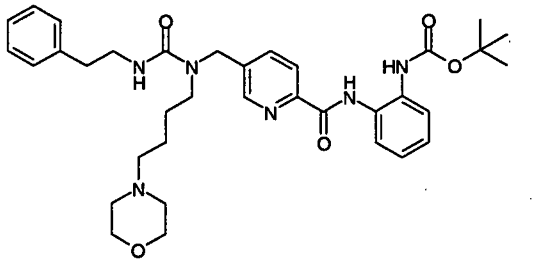 | $^1$H-NMR (500 MHz, $CDCl_3$) δ 1.40 (m, 2H), 1.49 (m, 2H), 1.52 (s, 9H), 2.25 (t, J = 7.0 Hz, 2H), 2.33 (m, 4H), 2.86 (t, J = 6.7 Hz, 2H), 3.09 (t, J = 7.9 Hz, 2H), 3.55 (td, J = 6.7, 5.7 Hz, 2H), 3.65 (t, J = 4.6 Hz, 4H), 4.58 (s, 2H), 4.75 (t, J = 5.7 Hz, 1H), 7.07 (br s, 1H), 7.17 (m, 2H), 7.19-7.25 (m, 3H), 7.27-7.30 (m, 2H), 7.59 (d, J = 6.7 Hz, 1H), 7.71 (d, J = 6.4 Hz, 1H), 7.76 (dd, J = 8.1, 1.8 Hz, 1H), 8.23 (d, J = 8.1 Hz, 1H), 8.47 (d, J = 1.8 Hz, 1H), 10.15 (s, 1H) |
| N-(2-t-Butoxycarbonylaminophenyl)-5-[3-(3-me thoxyphenyl)-1-[4-(morpholin-4-yl)butyl]ureido methyl] pyridine-2-carboxylic acid amide (Reference Compound No.6-78) 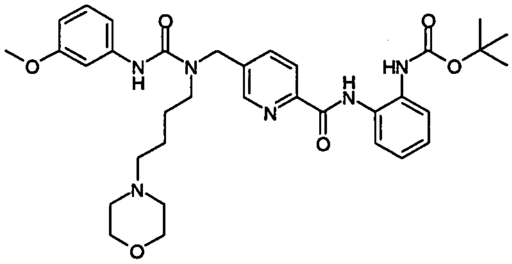 | $^1$H-NMR (500 MHz, $CDCl_3$) δ 1.51 (s, 9H), 1.54 (m, 2H), 1.71 (m, 2H), 2.38 (t, J = 7.0 Hz, 2H), 2.42 (m, 4H), 3.33 (t, J = 7.9 Hz, 2H), 3.67 (t, J = 4.6 Hz, 4H), 3.81 (s, 3H), 4.70 (s, 2H), 6.59 (s, 1H), 6.64 (dd, J = 8.0, 2.1 Hz, 1H), 6.84 (dd, J = 8.0, 2.1 Hz, 1H), 7.04 (br s, 1H), 7.11 (t, J = 2.1 Hz, 1H), 7.18-7.24 (m, 2H), 7.20 (t, J = 8.0 Hz, 1H), 7.58 (d, J = 6.1 Hz, 1H), 7.73 (d, J = 6.1 Hz, 1H), 7.89 (dd, J = 8.1, 2.0 Hz, 1H), 8.28 (d, J = 8.1 Hz, 1H), 8.57 (d, J = 2.0 Hz, 1H), 10.16 (s, 1H) |
| N-(2-t-Butoxycarbonylaminophenyl)-5-[3-(2,3-dihydrobenzo[1,4]dioxin-6-yl)-1-(5-dimethylaminopentyl) ureidomethyl]pyridine-2-carboxylic acid amide (Reference Compound No.6-79) 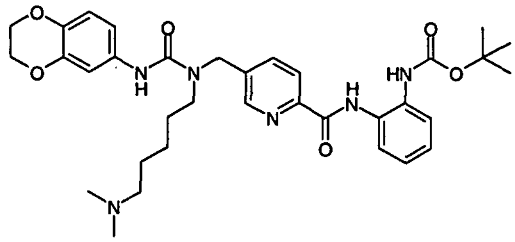 | $^1$H-NMR (500 MHz, $CDCl_3$) δ 1.38 (m, 2H), 1.50 (m, 2H), 1.52 (s, 9H), 1.67 (m, 2H), 2.20 (s, 6H), 2.25 (t, J = 7.0 Hz, 2H), 3.27 (t, J = 7.8 Hz, 2H), 4.21-4.27 (m, 4H), 4.67 (s, 2H), 6.53 (s, 1H), 6.74 (dd, J = 8.7, 2.4 Hz, 1H), 6.80 (d, J = 8.7 Hz, 1H), 6.95 (d, J = 2.4 Hz, 1H), 7.08 (br s, 1H), 7.18-7.24 (m, 2H), 7.59 (d, J = 6.1 Hz, 1H), 7.70 (d, J = 6.7 Hz, 1H), 7.87 (dd, J = 7.9, 2.1 Hz, 1H), 8.26 (d, J = 7.9 Hz, 1H), 8.55 (d, J = 2.1 Hz, 1H), 10.16 (s, 1H) |
| N-(2-t-Butoxycarbonylaminophenyl)-5-[1-(5-dimethylaminopentyl)-3-(3-fluorophenyl)ureidomethyl] pyridine-2-carboxylic acid amide (Reference Compound No.6-80) 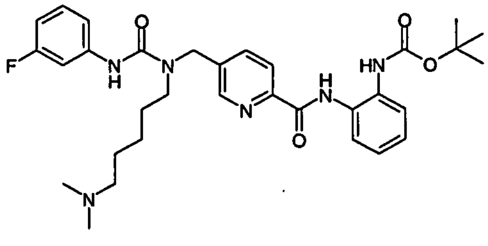 | $^1$H-NMR (500 MHz, $CDCl_3$) δ 1.40 (m, 2H), 1.50 (m, 2H), 1.52 (s, 9H), 1.68 (m, 2H), 2.20 (s, 6H), 2.26 (t, J = 7.0 Hz, 2H), 3.31 (t, J = 7.8 Hz, 2H), 4.69 (s, 2H), 6.73 (s, 1H), 6.78 (td, J = 8.4, 2.3 Hz, 1H), 7.02 (m, 1H), 7.06 (br s, 1H), 7.18-7.23 (m, 3H), 7.34 (dt, J = 11.1, 2.3 Hz, 1H), 7.56 (d, J = 6.1 Hz, 1H), 7.71 (d, J = 5.8 Hz, 1H), 7.87 (dd, J = 7.9, 1.8 Hz, 1H), 8.26 (d, J = 7.9 Hz, 1H), 8.56 (d, J = 1.8 Hz, 1H), 10.18 (s, 1H) |

(continued)

| | |
|---|---|
| N-(2-t-Butoxycarbonylaminophenyl)-5-[1-(3-dimethylaminopropyl)-3-(pyridin-3-yl)ureidomethyl] pyridine-2-carboxylic acid amide (Reference Compound No.6-81)<br>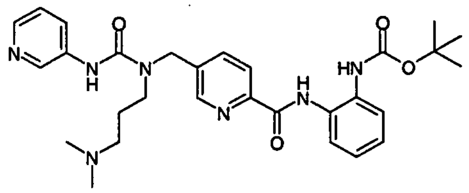 | $^1$H-NMR (500 MHz, DMSO-$d_6$)<br>δ 1.47 (s, 9H), 1.71 (m, 2H), 2.20 (s, 6H), 2.28 (t, J = 6.3 Hz, 2H), 3.38 (t, J = 6.3 Hz, 2H), 4.65 (s, 2H), 7.15 (t, J = 8.1 Hz, 1H), 7.23-7.25 (m, 2H), 7.29 (dd, J = 8.4, 4.7 Hz, 1H), 7.90 (ddd, J = 8.4, 2.6, 1.5 Hz, 1H), 7.99 (dd, J = 7.9, 2.0 Hz, 1H), 8.01 (d, J = 8.1 Hz, 1H), 8.15 (m, 1H), 8.16 (dd, J = 4.7, 1.5 Hz, 1H), 8.58 (d, J = 2.6 Hz, 1H), 8.59 (d, J = 2.0 Hz, 1H), 9.12 (br s, 1H), 9.93 (br s, 1H), 10.47 (br s, 1H) |
| N-(2-t-Butoxycarbonylaminophenyl)-5-[1-(3-dimethylaminopropyl)-3-(3-nitrophenyl)ureidomethyl] pyridine-2-carboxylic acid amide (Reference Compound No.6-82)<br>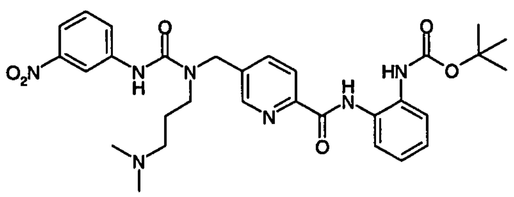 | $^1$H-NMR (400 MHz, DMSO-$d_6$)<br>δ 1.46 (s, 9H), 1.73 (m, 2H), 2.21 (s, 6H), 2.29 (t, J = 6.2 Hz, 2H), 3.39 (t, J = 6.1 Hz, 2H), 4.67 (s, 2H), 7.15 (td, J = 7.8, 1.5 Hz, 1H), 7.23-7.29 (m, 2H), 7.55 (t, J = 8.2 Hz, 1H), 7.71 (dd, J = 7.8, 1.5 Hz, 1H), 7.81 (m, 1H), 7.99-8.02 (m, 2H), 8.15 (d, J = 8.1 Hz, 1H), 8.56 (m, 1H), 8.60 (d, J = 1.2 Hz, 1H), 9.13 (br s, 1H), 10.27 (br s, 1H), 10.48 (br s, 1H) |
| 5-[3-(3-Acetylphenyl)-1-(3-dimethylaminopropyl) ureidomethyl]-N-(2-t-butoxycarbonylaminophenyl) pyridine-2-carboxylic acid amide (Reference Compound No.6-83)<br>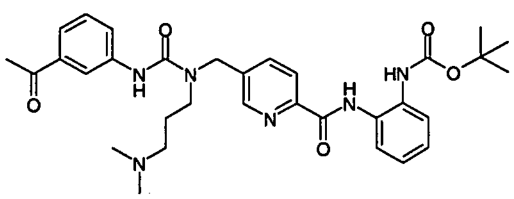 | $^1$H-NMR (400 MHz, DMSO-$d_6$)<br>δ 1.46 (s, 9H), 1.71 (m, 2H), 2.21 (s, 6H), 2.28 (t, J = 6.2 Hz, 2H), 2.56 (s, 3H), 3.38 (t, J = 6.1 Hz, 2H), 4.65 (s, 2H), 7.15 (t, J = 7.6 Hz, 1H), 7.23-7.27 (m, 2H), 7.41 (t, J = 7.8 Hz, 1H), 7.57 (m, 1H), 7.70 (m, 1H), 7.98-8.03 (m, 3H), 8.15 (d, J = 7.8 Hz, 1H), 8.59 (d, J = 1.5 Hz, 1H), 9.12 (br s, 1H), 9.90 (br s, 1H), 10.48 (br s, 1H) |
| N-(2-t-Butoxycarbonylaminophenyl)-5-[1-(3-dimethylaminopropyl)-3-(3-methylthiophenyl) ureidomethyl]pyridine-2-carboxylic acid amide (Reference Compound No.6-84)<br>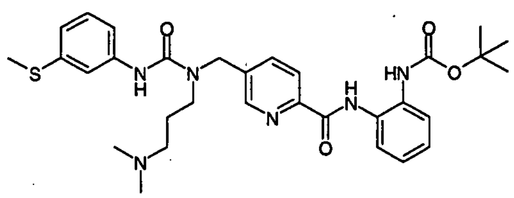 | $^1$H-NMR (400 MHz, DMSO-$d_6$)<br>δ 1.47 (s, 9H), 1.70 (m, 2H), 2.20 (s, 6H), 2.27 (t, J = 6.2 Hz, 2H), 2.45 (s, 3H), 3.35 (m, 2H), 4.62 (s, 2H), 6.84 (ddd, J = 7.6, 1.8, 1.1 Hz, 1H), 7.12-7.28 (m, 5H), 7.45 (t, J = 1.8 Hz, 1H), 7.98 (dd, J = 8.1, 1.8 Hz, 1H), 8.01 (d, J = 7.1 Hz, 1H), 8.15 (d, J = 8.1 Hz, 1H), 8.58 (d, J = 1.8 Hz, 1H), 9.13 (br s, 1H), 9.75 (br s, 1H), 10.48 (br s, 1H) |

(continued)

| | |
|---|---|
| N-(2-t-Butoxycarbonylaminophenyl)-5-[3-(3-fluorophenyl)-1-[3-(morpholin-4-yl)propyl]ureidomethyl] pyridine-2-carboxylic acid amide (Reference Compound No.6-85) 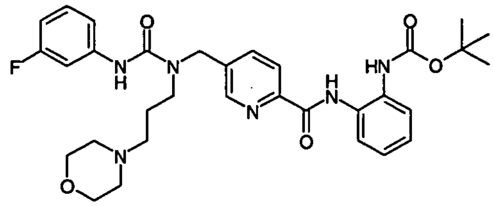 | $^1$H-NMR (400 MHz, $CDCl_3$) δ 1.51 (s, 9H), 1.80 (m, 2H), 2.43-2.54 (m, 6H), 3.39 (t, J = 5.6 Hz, 2H), 3.72 (t, J = 4.6 Hz, 4H), 4.65 (s, 2H), 6.79 (tdd, J = 8.4, 2.4, 0.8 Hz, 1H), 7.03 (br s, 1H), 7.13 (m, 1H), 7.17-7.30 (m, 3H), 7.42 (m, 1H), 7.59 (d, J = 6.3 Hz, 1H), 7.70 (d, J = 7.3 Hz, 1H), 7.92 (dd, J = 8.1, 2.1 Hz, 1H), 8.26 (dd, J = 8.1, 0.5 Hz, 1H), 8.58 (dd, J = 2.1, 0.5 Hz, 1H), 9.05 (s, 1H), 10.16 (s, 1H) |
| N-(2-t-Butoxycarbonylaminophenyl)-5-[3-(3-fluoro-4-methylphenyl)-1-[3-(morpholin-4-yl)propyl]ureidomethyl] pyridine-2-carboxylic acid amide (Reference Compound No.6-86) 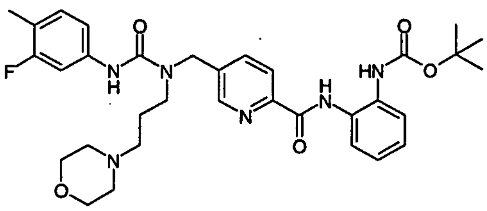 | $^1$H-NMR (400 MHz, $CDCl_3$) δ 1.51 (s, 9H), 1.79 (m, 2H), 2.23 (d, J = 1.7 Hz, 3H), 2.44-2.51 (m, 6H), 3.37 (t, J = 5.5 Hz, 2H), 3.69 (t, J = 4.5 Hz, 4H), 4.64 (s, 2H), 7.01 (dd, J = 8.1, 1.7 Hz, 1H), 7.04 (br s, 1H), 7.10 (t, J = 8.1 Hz, 1H), 7.17-7.25 (m, 2H), 7.31 (m, 1H), 7.59 (d, J = 6.1 Hz, 1H), 7.70 (d, J = 6.6 Hz, 1H), 7.92 (dd, J = 8.1, 2.2 Hz, 1H), 8.25 (dd, J = 8.1, 0.7 Hz, 1H), 8.57 (dd, J = 2.2, 0.7 Hz, 1H), 9.01 (s, 1H), 10.15 (s, 1H) |
| N-(2-t-Butoxycarbonylaminophenyl)-5-[1-[3-(morpholin-4-yl)propyl]-3-(thiophen-3-yl)ureidomethyl]pyridine-2-carboxylic acid amide (Reference Compound No.6-87) 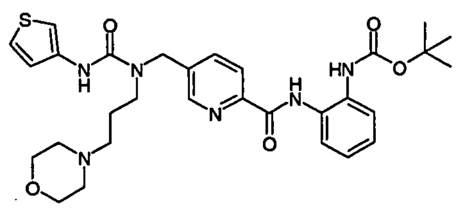 | $^1$H-NMR (400 MHz, $CDCl_3$) δ 1.51 (s, 9H), 1.78 (m, 2H), 2.41-2.52 (m, 6H), 3.36 (t, J = 5.6 Hz, 2H), 3.72 (t, J = 4.6 Hz, 4H), 4.65 (s, 2H), 7.05 (br s, 1H), 7.10 (dd, J = 5.1, 1.4 Hz, 1H), 7.17-7.25 (m, 2H), 7.25 (dd, J = 5.1, 3.4 Hz, 1H), 7.31 (dd, J = 3.4, 1.4 Hz, 1H), 7.60 (d, J = 7.1 Hz, 1H), 7.69 (d, J = 6.6 Hz, 1H), 7.92 (dd, J = 8.1, 2.2 Hz, 1H), 8.25 (dd, J = 8.1, 0.7 Hz, 1H), 8.57 (dd, J = 2.2, 0.7 Hz, 1H), 9.25 (s, 1H), 10.15 (s, 1H) |
| N-(2-t-Butoxycarbonylaminophenyl)-5-[3-(4-me thoxyphenyl)-1-[4-(morpholin-4-yl)butyl]ureido methyl] pyridine-2-carboxylic acid amide (Reference Compound No.6-88) 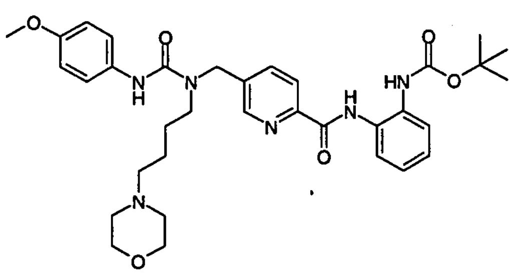 | $^1$H-NMR (500 MHz, $CDCl_3$) δ 1.52 (s, 9H), 1.55 (m, 2H), 1.72 (m, 2H), 2.39 (t, J = 6.9 Hz, 2H), 2.42 (m, 4H), 3.31 (t, J = 7.9 Hz, 2H), 3.63 (t, J = 4.6 Hz, 4H), 3.80 (s, 3H), 4.68 (s, 2H), 6.68 (s, 1H), 6.87 (d, J = 9.2 Hz, 2H), 7.05 (br s, 1H), 7.18-7.24 (m, 2H), 7.23 (d, J = 9.2 Hz, 2H), 7.58 (d, J = 5.8 Hz, 1H), 7.72 (d, J = 6.1 Hz, 1H), 7.89 (dd, J = 7.9, 1.8 Hz, 1H), 8.27 (dd, J = 7.9, 0.6 Hz, 1H), 8.56 (dd, J = 1.8, 0.6 Hz, 1H), 10.16 (s, 1H) |

(continued)

| | |
|---|---|
| N-(2-t-Butoxycarbonylaminophenyl)-5-[3-(4-methylphenyl)-1-[4-(morpholin-4-yl)butyl]ureidomethyl] pyridine-2-carboxylic acid amide (Reference Compound No.6-89) 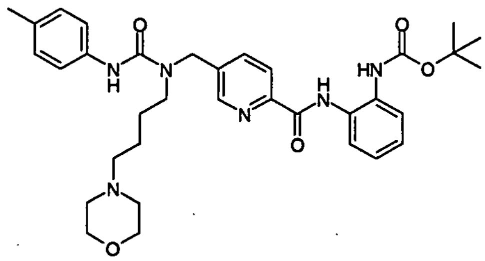 | $^1$H-NMR (400 MHz, $CDCl_3$)<br>δ 1.51 (s, 9H), 1.56 (m, 2H), 1.71 (m, 2H), 2.31 (s, 3H), 2.38 (t, J = 7.0 Hz, 2H), 2.42 (t, J = 4.5 Hz, 4H), 3.32 (t, J = 7.9 Hz, 2H), 3.65 (t, J = 4.5 Hz, 4H), 4.68 (s, 2H), 6.62 (s, 1H), 7.06 (br s, 1H), 7.12 (d, J = 8.1 Hz, 2H), 7.20-7.25 (m, 2H), 7.22 (d, J = 8.1 Hz, 2H), 7.58 (d, J = 6.6 Hz, 1H), 7.72 (d, J = 6.1 Hz, 1H), 7.89 (dd, J = 8.1, 2.2 Hz, 1H), 8.26 (dd, J = 8.1, 0.7 Hz, 1H), 8.56 (dd, J = 2.2, 0.7 Hz, 1H), 10.16 (s, 1H) |
| N-(2-t-Butoxycarbonylaminophenyl)-5-[3-(3,4-dimethoxyphenyl)-1-[4-(morpholin-4-yl)butyl] ureidomethyl]pyridine-2-carboxylic acid amide (Reference Compound No.6-90) 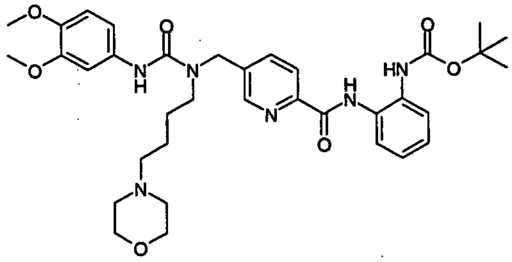 | $^1$H-NMR (500 MHz, $CDCl_3$)<br>δ 1.51 (s, 9H), 1.53 (m, 2H), 1.71 (m, 2H), 2.39 (t, J = 7.0 Hz, 2H), 2.41 (m, 4H), 3.32 (t, J = 7.9 Hz, 2H), 3.64 (t, J = 4.5 Hz, 4H), 3.86 (s, 3H), 3.90 (s, 3H), 4.70 (s, 2H), 6.64 (s, 1H), 6.71 (dd, J = 8.5, 2.4 Hz, 1H), 6.80 (d, J = 8.5 Hz, 1H), 7.04 (br s, 1H), 7.13 (d, J = 2.4 Hz, 1H), 7.19-7.25 (m, 2H), 7.58 (d, J = 5.5 Hz, 1H), 7.72 (d, J = 6.1 Hz, 1H), 7.89 (dd, J = 7.9, 2.0 Hz, 1H), 8.28 (d, J = 7.9 Hz, 1H), 8.57 (d, J = 2.0 Hz, 1H), 10.16 (s, 1H) |
| N-(2-t-Butoxycarbonylaminophenyl)-5-[3-(3-ethoxyphenyl)-1-[4-(morpholin-4-yl)butyl]ureidomethyl] pyridine-2-carboxylic acid amide (Reference Compound No.6-91) 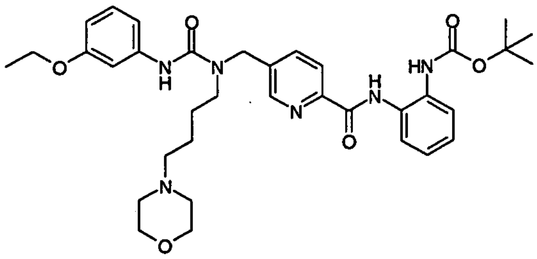 | $^1$H-NMR (400 MHz, $CDCl_3$)<br>δ 1.40 (t, J = 7.0 Hz, 3H), 1.51 (s, 9H), 1.54 (m, 2H), 1.71 (m, 2H), 2.38 (t, J = 7.1 Hz, 2H), 2.42 (t, J = 4.4 Hz, 4H), 3.32 (t, J = 7.8 Hz, 2H), 3.67 (t, J = 4.4 Hz, 4H), 4.03 (q, J = 7.0 Hz, 2H), 4.69 (s, 2H), 6.60 (s, 1H), 6.63 (ddd, J = 8.2, 2.3, 0.9 Hz, 1H), 6.83 (ddd, J = 8.2, 2.3, 0.9 Hz, 1H), 7.06 (br s, 1H), 7.10 (t, J = 2.3 Hz, 1H), 7.17 (t, J = 8.2 Hz, 1H), 7.20-7.23 (m, 2H), 7.57 (d, J = 6.3 Hz, 1H), 7.73 (d, J = 6.3 Hz, 1H), 7.88 (dd, J = 8.1, 2.2 Hz, 1H), 8.26 (dd, J = 8.1, 0.7 Hz, 1H), 8.55 (dd, J = 2.2, 0.7 Hz, 1H), 10.17 (s, 1H) |

(continued)

| | |
|---|---|
| N-(2-t-Butoxycarbonylaminophenyl)-5-[3-(3-methylphenyl)-1-[4-(morpholin-4-yl)butyl]ureidomethyl] pyridine-2-carboxylic acid amide (Reference Compound No.6-92) 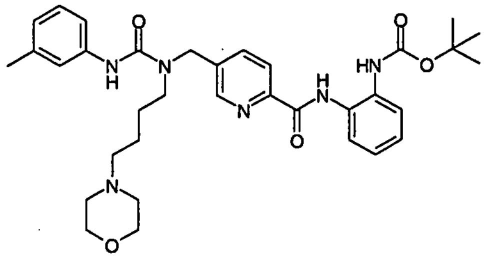 | $^1$H-NMR (500 MHz, $CDCl_3$) δ 1.52 (s, 9H), 1.54 (m, 2H), 1.72 (m, 2H), 2.34 (s, 3H), 2.39 (t, J = 7.0 Hz, 2H), 2.44 (t, J = 4.7 Hz, 4H), 3.33 (t, J = 7.9 Hz, 2H), 3.67 (t, J = 4.7 Hz, 4H), 4.69 (s, 2H), 6.57 (s, 1H), 6.91 (d, J = 7.8 Hz, 1H), 7.04 (br s, 1H), 7.12 (d, J = 7.8 Hz, 1H), 7.18-7.24 (m, 3H), 7.20 (t, J = 7.8 Hz, 1H), 7.58 (d, J = 5.8 Hz, 1H), 7.72 (d, J = 5.5 Hz, 1H), 7.89 (dd, J = 7.9, 2.1 Hz, 1H), 8.28 (dd, J = 7.9, 0.7 Hz, 1H), 8.57 (dd, J = 2.1, 0.7 Hz, 1H), 10.16 (s, 1H) |
| 5-[3-(3-Acetylphenyl)-1-[4-(morpholin-4-yl)butyl] ureidomethyl]-N-(2-t-butoxycarbonylaminophenyl) pyridine-2-carboxylic acid amide (Reference Compound No.6-93) 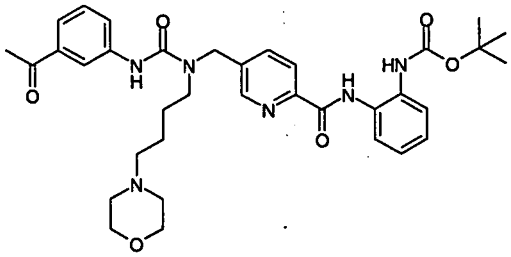 | $^1$H-NMR (400 MHz, $CDCl_3$) δ 1.51 (s, 9H), 1.55 (m, 2H), 1.74 (m, 2H), 2.41 (t, J = 7.0 Hz, 2H), 2.44 (t, J = 4.6 Hz, 4H), 2.61 (s, 3H), 3.35 (t, J = 7.9 Hz, 2H), 3.66 (t, J = 4.6 Hz, 4H), 4.70 (s, 2H), 6.83 (s, 1H), 7.05 (br s, 1H), 7.18-7.25 (m, 2H), 7.43 (t, J = 7.9 Hz, 1H), 7.55 (d, J = 6.3 Hz, 1H), 7.67 (ddd, J = 7.9, 1.8, 1.1 Hz, 1H), 7.72 (d, J = 6.8 Hz, 1H), 7.78 (ddd, J = 7.9, 1.8, 1.1 Hz, 1H), 7.82 (t, J = 1.8 Hz, 1H), 7.89 (dd, J = 8.0, 2.2 Hz, 1H), 8.28 (dd, J = 8.0, 0.6 Hz, 1H), 8.57 (dd, J = 2.2, 0.6 Hz, 1H), 10.17 (s, 1H) |
| N-(2-t-Butoxycarbonylaminophenyl)-5-[3-(2-methoxyphenyl)-1-[4-(morpholin-4-yl)butyl] ureidomethyl]pyridine-2-carboxylic acid amide (Reference Compound No.6-94) 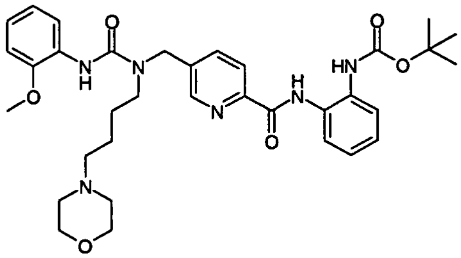 | $^1$H-NMR (400 MHz, $CDCl_3$) δ 1.51 (s, 9H), 1.56 (m, 2H), 1.73 (m, 2H), 2.37 (t, J = 7.3 Hz, 2H), 2.42 t, J = 4.6 Hz, 4H), 3.38 (t, J = 7.6 Hz, 2H), 3.69 (t, J = 4.6 Hz, 4H), 3.82 (s, 3H), 4.70 (s, 2H), 6.86 (dd, J = 7.2, 2.4 Hz, 1H), 6.98 (td, J = 7.2, 2.4 Hz, 1H), 7.01 (td, J = 7.2, 2.4 Hz, 1H), 7.05 (br s, 1H), 7.19-7.23 (m, 2H), 7.57 (d, J = 5.9 Hz, 1H), 7.73 (d, J = 5.6 Hz, 1H), 7.90 (dd, J = 8.1, 2.2 Hz, 1H), 8.13 (dd, J = 7.2, 2.4 Hz, 1H), 8.28 (dd, J = 8.1, 0.7 Hz, 1H), 8.57 (dd, J = 2.2, 0.7 Hz, 1H), 10.17 (s, 1H) |
| N-(2-t-Butoxycarbonylaminophenyl)-5-[3-(3-methylphenyl)-1-[3-(4-methylpiperidin-1-yl)propyl] ureidomethyl]pyridine-2-carboxylic acid amide (Reference Compound No.6-95) 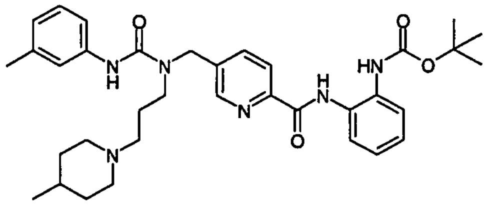 | $^1$H-NMR (500 MHz, $CDCl_3$) δ 0.90 (d, J = 6.4 Hz, 3H), 1.21 (m, 2H), 1.38 (m, 1H), 1.51 (s, 9H), 1.59 (m, 2H), 1.76 (m, 2H), 1.92 (m, 2H), 2.34 (s, 3H), 2.42 (t, J = 6.0 Hz, 2H), 2.88 (d, J = 11.6 Hz, 2H), 3.37 (t, J = 5.7 Hz, 2H), 4.63 (s, 2H), 6.89 (d, J = 7.6 Hz, 1H), 7.06 (s, 1H), 7.17-7.24 (m, 3H), 7.28-7.30 (m, 2H), 7.61 (d, J = 7.3 Hz, 1H), 7.68 (d, J = 7.0 Hz, 1H), 7.93 (dd, J = 7.9, 2.1 Hz, 1H), 8.24 (d, J = 7.9 Hz, 1H), 8.57 (d, J = 2.1 Hz, 1H), 9.34 (s, 1H), 10.14 (s, 1H) |

(continued)

| | |
|---|---|
| N-(2-t-Butoxycarbonylaminophenyl)-5-[3-(3,4-difluorophenyl)-1-[3-(4-methylpiperidin-1-yl)propyl] ureidomethyl]pyridine-2-carboxylic acid amide (Reference Compound No.6-96) 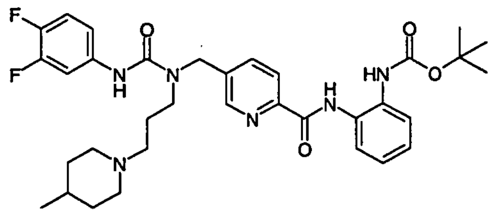 | $^1$H-NMR (500 MHz, $CDCl_3$) δ 0.90 (d, J = 6.4 Hz, 3H), 1.12 (m, 2H), 1.40 (m, 1H), 1.51 (s, 9H), 1.60 (m, 2H), 1.77 (m, 2H), 1.95 (m, 2H), 2.42 (t, J = 6.1 Hz, 2H), 2.86 (d, J = 11.6 Hz, 2H), 3.36 (t, J = 5.7 Hz, 2H), 4.61 (s, 2H), 7.03-7.10 (m, 3H), 7.18-7.24 (m, 2H), 7.45 (m, 1H), 7.59 (d, J = 6.4 Hz, 1H), 7.70 (d, J = 6.7 Hz, 1H), 7.91 (dd, J = 7.9, 2.1 Hz, 1H), 8.25 (d, J = 7.9 Hz, 1H), 8.57 (d, J = 2.1 Hz, 1H), 9.70 (s, 1H), 10.15 (s, 1H) |
| N-(2-t-Butoxycarbonylaminophenyl)-5-[3-(2,3-dihydrobenzo[1,4]dioxin-6-yl)-1-[3-(4-methylpiperidin--yl)propyl]ureidomethyl]pyridine-2-carboxylic acid amide (Reference Compound No.6-97) 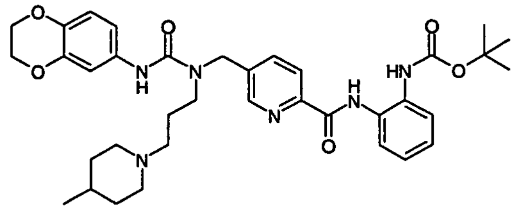 | $^1$H-NMR (400 MHz, $CDCl_3$) δ 0.87 (d, J = 6.3 Hz, 3H), 1.13 (m, 2H), 1.36 (m, 1H), 1.51 (s, 9H), 1.54 (m, 2H), 1.74 (m, 2H), 1.91 (m, 2H), 2.41 (t, J = 6.0 Hz, 2H), 2.85 (d, J = 11.5 Hz, 2H), 3.34 (t, J = 5.4 Hz, 2H), 4.23-4.24 (m, 4H), 4.61 (s, 2H), 6.79 (d, J = 8.7 Hz, 1H), 6.85 (dd, J = 8.7, 2.4 Hz, 1H), 7.01 (d, J = 2.4 Hz, 1H), 7.08 (br s, 1H), 7.17-7.24 (m, 2H), 7.61 (d, J = 7.1 Hz, 1H), 7.68 (d, J = 6.3 Hz, 1H), 7.93 (dd, J = 8.1, 2.2 Hz, 1H), 8.24 (d, J = 8.1 Hz, 1H), 8.56 (d, J = 2.2 Hz, 1H), 9.42 (s, 1H), 10.14 (s, 1H) |
| N-(2-t-Butoxycarbonylaminophenyl)-5-[3-(4-fluorophenyl)-1-[3-(morpholin-4-yl)propyl]ureidomethyl] pyridine-2-carboxylic acid amide (Reference Compound No.6-98) 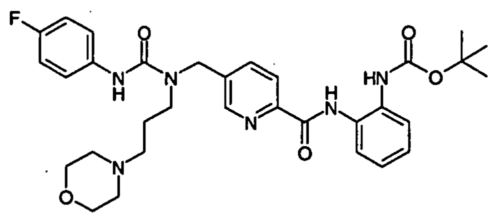 | $^1$H-NMR (500 MHz, $CDCl_3$) δ 1.51 (s, 9H), 1.79 (m, 2H), 2.45 (br s, 4H), 2.49 (t, J = 6.0 Hz, 2H), 3.39 (t, J = 5.5 Hz, 2H), 3.64 (t, J = 4.4 Hz, 4H), 4.64 (s, 2H), 7.03 (t, J = 8.7 Hz, 2H), 7.03 (br s, 1H), 7.18-7.24 (m, 2H), 7.38 (dd, J = 8.7, 4.9 Hz, 2H), 7.59 (d, J = 7.3 Hz, 1H), 7.69 (d, J = 7.0 Hz, 1H), 7.92 (dd, J = 8.1, 2.1 Hz, 1H), 8.25 (dd, J = 8.1, 0.6 Hz, 1H), 8.57 (dd, J = 2.1, 0.6 Hz, 1H), 9.08 (s, 1H), 10.14 (s, 1H) |
| N-(2-t-Butoxycarbonylaminophenyl)-5-[3-(4-fluoro-3-methylphenyl)-1-[3-(morpholin-4-yl)propyl]ureidomethyl] pyridine-2-carboxylic acid amide (Reference Compound No.6-99) 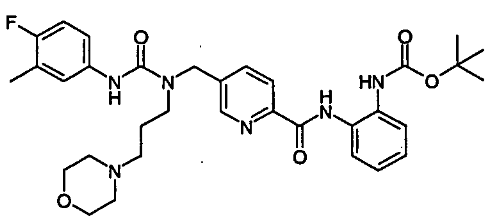 | $^1$H-NMR (500 MHz, $CDCl_3$) δ 1.51 (s, 9H), 1.79 (m, 2H), 2.27 (d, J = 1.8 Hz, 3H), 2.45 (br s, 4H), 2.49 (t, J = 6.0 Hz, 2H), 3.38 (t, J = 5.7 Hz, 2H), 3.64 (t, J = 4.6 Hz, 4H), 4.64 (s, 2H), 6.95 (t, J = 8.8 Hz, 1H), 7.05 (br s, 1H), 7.13 (ddd, J = 8.8, 4.3, 2.7 Hz, 1H), 7.18-7.24 (m, 2H), 7.30 (dd, J = 6.9, 2.7 Hz, 1H), 7.60 (d, J = 6.4 Hz, 1H), 7.69 (d, J = 7.3 Hz, 1H), 7.92 (dd, J = 7.9, 2.0 Hz, 1H), 8.25 (d, J = 7.9 Hz, 1H), 8.57 (d, J = 2.0 Hz, 1H), 9.00 (s, 1H), 10.14 (s, 1H) |

(continued)

| | |
|---|---|
| N-(2-t-Butoxycarbonylaminophenyl)-5-[3-(3,4-difluorophenyl)-1-[3-(morpholin-4-yl)propyl] ureidomethyl]pyridine-2-carboxylic acid amide (Reference Compound No.6-100)<br>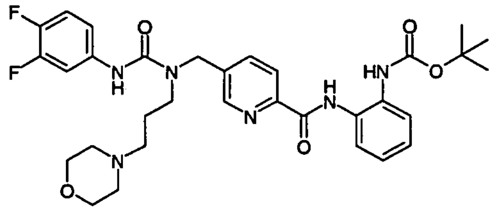 | $^1$H-NMR (500 MHz, $CDCl_3$)<br>δ 1.51 (s, 9H), 1.80 (m, 2H), 2.46-2.50 (m, 6H), 3.38 (t, J = 5.7 Hz, 2H), 3.67 (t, J = 4.7 Hz, 4H), 4.63 (s, 2H), 6.95-7.06 (m, 2H), 7.10 (m, 1H), 7.17-7.25 (m, 2H), 7.50 (ddd, J = 12.2, 7.3, 2.4 Hz, 1H), 7.58 (d, J = 6.1 Hz, 1H), 7.71 (d, J = 5.8 Hz, 1H), 7.91 (dd, J = 7.9, 2.1 Hz, 1H), 8.26 (dd, J = 7.9, 0.6 Hz, 1H), 8.57 (dd, J = 2.1, 0.6 Hz, 1H), 9.15 (s, 1H), 10.15 (s, 1H) |
| N-(2-t-Butoxycarbonylaminophenyl)-5-[3-(3-methylphenyl)-1-[3-(morpholin-4-yl)propyl]ureidomethyl] pyridine-2-carboxylic acid amide (Reference Compound No.6-101)<br>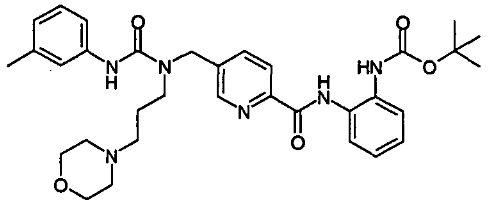 | $^1$H-NMR (500 MHz, $CDCl_3$)<br>δ 1.51 (s, 9H), 1.79 (m, 2H), 2.35 (s, 3H), 2.46 (br s, 4H), 2.49 (t, J = 6.1 Hz, 2H), 3.39 (t, J = 5.7 Hz, 2H), 3.70 (t, J = 4.6 Hz, 4H), 4.65 (s, 2H), 6.91 (m, 1H), 7.05 (br s, 1H), 7.17-7.25 (m, 4H), 7.33 (s, 1H), 7.60 (d, J = 7.0 Hz, 1H), 7.69 (d, J = 6.7 Hz, 1H), 7.93 (dd, J = 8.2, 1.9 Hz, 1H), 8.25 (d, J = 8.2 Hz, 1H), 8.57 (d, J = 1.9 Hz, 1H), 8.86 (s, 1H), 10.15 (s, 1H) |
| 5-[3-(3-Acetylphenyl)-1-[3-(morpholin-4-yl)propyl] ureidomethyl]-N-(2-t-butoxycarbonylaminophenyl) pyridine-2-carboxylic acid amide (Reference Compound No.6-102)<br>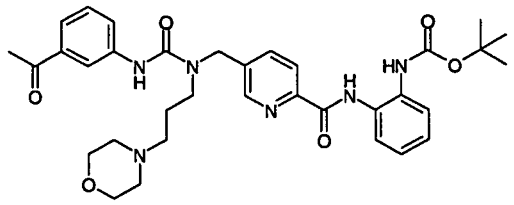 | $^1$H-NMR (500 MHz, $CDCl_3$)<br>δ 1.51 (s, 9H), 1.83 (m, 2H), 2.49 (br s, 4H), 2.51 (t, J = 6.1 Hz, 2H), 2.62 (s, 3H), 3.42 (t, J = 5.5 Hz, 2H), 3.72 (t, J = 4.6 Hz, 4H), 4.66 (s, 2H), 7.03 (br s, 1H), 7.17-7.24 (m, 2H), 7.44 (t, J = 7.9 Hz, 1H), 7.59 (d, J = 6.1 Hz, 1H), 7.67 (d, J = 7.9 Hz, 1H), 7.70 (m, 1H), 7.80 (dd, J = 7.9, 1.8 Hz, 1H), 7.93 (dd, J = 8.0, 1.9 Hz, 1H), 7.99 (t, J = 1.8 Hz, 1H), 8.26 (d, J = 8.0 Hz, 1H), 8.58 (d, J = 1.9 Hz, 1H), 9.21 (s, 1H), 10.15 (s, 1H) |
| N-(2-t-Butoxycarbonylaminophenyl)-5-[3-(3-methylphenyl)-1-[5-(morpholin-4-yl)pentyl]ureidomethyl] pyridine-2-carboxylic acid amide (Reference Compound No.6-103)<br>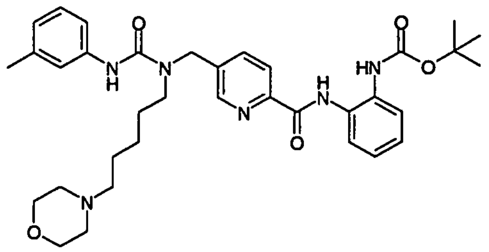 | $^1$H-NMR (500 MHz, $CDCl_3$)<br>δ 1.38 (m, 2H), 1.50-1.56 (m, 4H), 1.51 (s, 9H), 1.68 (m, 2H), 2.32 (t, J = 7.6 Hz, 2H), 2.34 (s, 3H), 2.41 (br s, 4H), 3.31 (t, J = 7.6 Hz, 2H), 3.70 (t, J = 4.6 Hz, 4H), 4.69 (s, 2H), 6.89 (d, J = 7.6 Hz, 1H), 7.04 (br s, 1H), 7.12 (d, J = 8.6 Hz, 1H), 7.17-7.25 (m, 3H), 7.58 (d, J = 6.1 Hz, 1H), 7.72 (d, J = 6.1 Hz, 1H), 7.88 (dd, J = 7.9, 2.1 Hz, 1H), 8.28 (d, J = 7.9 Hz, 1H), 8.56 (d, J = 2.1 Hz, 1H), 10.17 (s, 1H) |

(continued)

| | |
|---|---|
| N-(2-t-Butoxycarbonylaminophenyl)-5-[3-(3,4-difluorophenyl)-1-[5-(morpholin-4-yl)pentyl] ureidomethyl]pyridine-2-carboxylic acid amide (Reference Compound No.6-104)<br>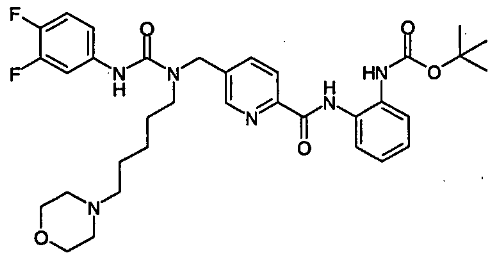 | $^1$H-NMR (500 MHz, $CDCl_3$)<br>δ 1.37 (m, 2H), 1.51 (s, 9H), 1.53 (m, 2H), 1.67 (m, 2H), 2.32 (t, J = 7.6 Hz, 2H), 2.41 (br s, 4H), 3.30 (t, J = 7.6 Hz, 2H), 3.70 (t, J = 4.6 Hz, 4H), 4.67 (s, 2H), 6.41 (s, 1H), 6.94 (m, 1H), 7.04-7.10 (m, 2H), 7.19-7.24 (m, 2H), 7.46 (m, 1H), 7.56 (br s, 1H), 7.74 (br s, 1H), 7.85 (dd, J = 7.9, 2.1 Hz, 1H), 8.25 (d, J = 7.9 Hz, 1H), 8.54 (d, J = 2.1 Hz, 1H), 10.17 (s, 1H) |
| N-(2-t-Butoxycarbonylaminophenyl)-5-[3-(2,3-dihydrobenzo[1,4]dioxin-6-yl)-1-[5-(morpholin-4-yl) pentyl]ureidomethyl]pyridine-2-carboxylic acid amide (Reference Compound No.6-105)<br>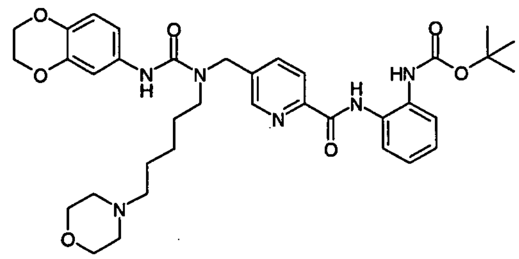 | $^1$H-NMR (500 MHz, $CDCl_3$)<br>δ 1.37 (m, 2H), 1.52 (s, 9H), 1.53 (m, 2H), 1.66 (m, 2H), 2.31 (t, J = 7.5 Hz, 2H), 2.41 (br s, 4H), 3.28 (t, J = 7.6 Hz, 2H), 3.70 (t, J = 4.6 Hz, 4H), 4.21-4.25 (m, 4H), 4.67 (s, 2H), 6.20 (s, 1H), 6.76 (dd, J = 8.6, 2.1 Hz, 1H), 6.79 (d, J = 8.6 Hz, 1H), 6.96 (d, J = 2.1 Hz, 1H), 7.07 (br s, 1H), 7.19-7.24 (m, 2H), 7.58 (d, J = 5.5 Hz, 1H), 7.72 (d, J = 5.2 Hz, 1H), 7.87 (dd, J = 7.9, 2.1 Hz, 1H), 8.26 (d, J = 7.9 Hz, 1H), 8.55 (d, J = 2.1 Hz, 1H), 10.16 (s, 1H) |
| N-(2-t-Butoxycarbonylaminophenyl)-5-[3-cyclopentyl-1-[3-(morpholin-4-yl)propyl]ureidomethyl]pyridine-2-carboxylic acid amide (Reference Compound No.6-106)<br>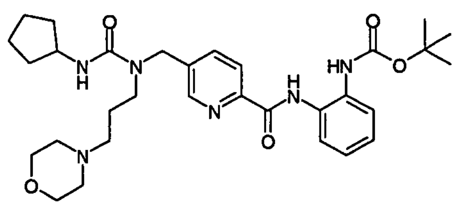 | $^1$H-NMR (400 MHz, $CDCl_3$)<br>δ 1.35 (m, 2H), 1.52 (s, 9H), 1.62 (m, 2H), 1.69 (m, 4H), 2.06 (m, 2H), 2.37 (t, J = 6.1 Hz, 2H), 2.44 (t, J = 4.6 Hz, 4H), 3.21 (t, J = 6.0 Hz, 2H), 3.74 (t, J = 4.6 Hz, 4H), 4.11 (m, 1H), 4.58 (s, 2H), 5.92 (d, J = 7.3 Hz, 1H), 7.05 (br s, 1H), 7.18-7.24 (m, 2H), 7.59 (d, J = 7.1 Hz, 1H), 7.70 (d, J = 6.6 Hz, 1H), 7.85 (dd, J = 8.1, 2.2 Hz, 1H), 8.25 (dd, J = 8.1, 0.7 Hz, 1H), 8.52 (dd, J = 2.2, 0.7 Hz, 1H), 10.14 (s, 1H) |
| 5-[3-[2-(Benzo[1,3]dioxol-5-yl)ethyl]-1-[3-(morpholin-4-yl)propyl]ureidomethyl]-N-(2-t-butoxycarbonylaminophenyl)pyridine-2-carboxylic acid amide (Reference Compound No.6-107)<br>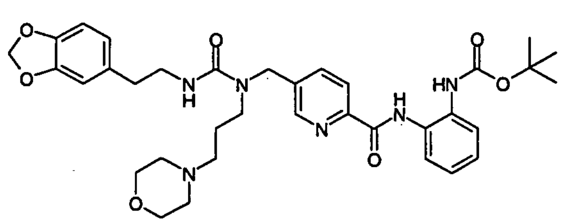 | $^1$H-NMR (500 MHz, $CDCl_3$)<br>δ 1.51 (s, 9H), 1.63 (m, 2H), 2.33 (t, J = 5.8 Hz, 2H), 2.33 (m, 4H), 2.77 (t, J = 6.7 Hz, 2H), 3.17 (t, J = 5.7 Hz, 2H), 3.43 (q, J = 6.7 Hz, 2H), 3.60 (s, 4H), 4.57 (s, 2H), 5.92 (s, 2H), 6.64 (dd, J = 7.7, 1.5 Hz, 1H), 6.70 (d, J = 1.5 Hz, 1H), 6.75 (d, J = 7.7 Hz, 1H), 7.07 (m, 1H), 7.18-7.24 (m, 2H), 7.61 (d, J = 6.7 Hz, 1H), 7.69 (d, J = 6.4 Hz, 1H), 7.80 (dd, J = 8.0, 1.9 Hz, 1H), 8.25 (d, J = 8.0 Hz, 1H), 8.47 (d, J = 1.9 Hz, 1H), 10.15 (s, 1H) |

(continued)

| | |
|---|---|
| N-(2-t-Butoxycarbonylaminophenyl)-5-[3-isopropyl-1-[3-(morpholin-4-yl)propyl]ureidomethyl]pyridine-2-carboxylic acid amide (Reference Compound No.6-108) 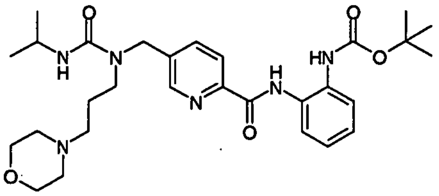 | $^1$H-NMR (400 MHz, $CDCl_3$) δ 1.20 (d, J = 6.6 Hz, 6H), 1.52 (s, 9H), 1.69 (m, 2H), 2.37 (t, J = 6.1 Hz, 2H), 2.45 (t, J = 4.5 Hz, 4H), 3.21 (t, J = 6.0 Hz, 2H), 3.76 (t, J = 4.5 Hz, 4H), 4.02 (m, 1H), 4.57 (s, 2H), 5.90 (d, J = 7.8 Hz, 1H), 7.06 (br s, 1H), 7.17-7.25 (m, 2H), 7.56 (d, J = 6.6 Hz, 1H), 7.70 (d, J = 6.1 Hz, 1H), 7.85 (dd, J = 8.1, 2.0 Hz, 1H), 8.24 (dd, J = 8.1, 0.7 Hz, 1H), 8.52 (dd, J = 2.0, 0.7 Hz, 1H), 10.15 (s, 1H) |
| N-(2-t-Butoxycarbonylaminophenyl)-5-[1-[3-(morpholin-4-yl)propyl]-3-propylureidomethyl]pyridine-2-carboxylic acid amide (Reference Compound No.6-109) 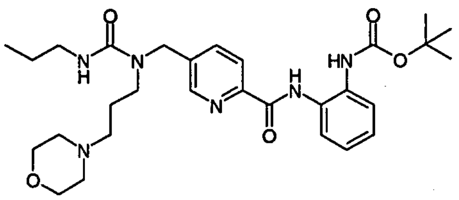 | $^1$H-NMR (500 MHz, $CDCl_3$) δ 0.95 (t, J = 7.5 Hz, 3H), 1.52 (s, 9H), 1.52 (m, 2H), 1.68 (m, 2H), 2.39 (t, J = 6.1 Hz, 2H), 2.45 (s, 4H), 3.19 (t, J = 6.3 Hz, 2H), 3.23 (t, J = 5.9 Hz, 2H), 3.74 (t, J = 4.4 Hz, 4H), 4.58 (s, 2H), 7.03 (t, J = 6.3 Hz, 1H), 7.05 (br s, 1H), 7.18-7.24 (m, 2H), 7.60 (d, J = 5.9 Hz, 1H), 7.68 (d, J = 7.6 Hz, 1H), 7.85 (dd, J = 7.9, 2.1 Hz, 1H), 8.24 (d, J = 7.9, 0.6 Hz, 1H), 8.52 (d, J = 2.1, 0.6 Hz, 1H), 10.14 (s, 1H) |
| N-(2-t-Butoxycarbonylaminophenyl)-5-[1-[4-(morpholin-4-yl)butyl]-3-propylureidomethyl]pyridine-2-carboxylic acid amide (Reference Compound No.6-110) 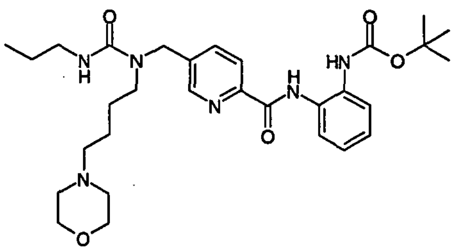 | $^1$H-NMR (500 MHz, $CDCl_3$) δ 0.92 (t, J = 7.3 Hz, 3H), 1.46-1.64 (m, 6H), 1.52 (s, 9H), 2.35 (t, J = 7.0 Hz, 2H), 2.41 (br s, 4H), 3.17 (t, J = 7.9 Hz, 2H), 3.25 (q, J = 5.7 Hz, 2H), 3.71 (t, J = 4.6 Hz, 4H), 4.61 (s, 2H), 4.78 (t, J = 5.7 Hz, 1H), 7.04 (br s, 1H), 7.18-7.24 (m, 2H), 7.59 (d, J = 6.7 Hz, 1H), 7.71 (d, J = 7.0 Hz, 1H), 7.82 (dd, J = 7.9, 2.1 Hz, 1H), 8.25 (d, J = 7.9 Hz, 1H), 8.51 (d, J = 2.1 Hz, 1H), 10.15 (s, 1H) |
| N-(2-t-Butoxycarbonylaminophenyl)-5-[3-isopropyl-1-[4-(morpholin-4-yl)butyl]ureidomethyl]pyridine-2-carboxylic acid amide (Reference Compound No.6-111) 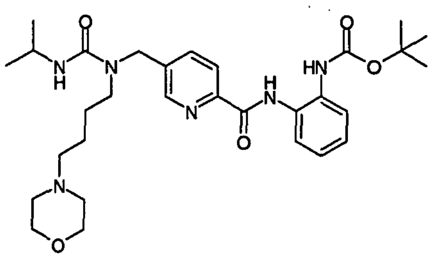 | $^1$H-NMR (500 MHz, $CDCl_3$) δ 1.17 (d, J = 6.4 Hz, 6H), 1.49 (m, 2H), 1.52 (s, 9H), 1.58 (m, 2H), 2.34 (t, J = 7.2 Hz, 2H), 2.41 (br s, 4H), 3.17 (t, J = 7.6 Hz, 2H), 3.71 (t, J = 4.6 Hz, 4H), 4.03 (m, 1H), 4.24 (d, J = 7.3 Hz, 1H), 4.59 (s, 2H), 7.04 (br s, 1H), 7.18-7.24 (m, 2H), 7.58 (d, J = 6.7 Hz, 1H), 7.72 (d, J = 6.7 Hz, 1H), 7.81 (dd, J = 7.9, 2.1 Hz, 1H), 8.26 (d, J = 7.9 Hz, 1H), 8.50 (d, J = 2.1 Hz, 1H), 10.15 (s, 1H) |

(continued)

| | |
|---|---|
| N-(2-t-Butoxycarbonylaminophenyl)-5-[3-cyclopentyl-1-[4-(morpholin-4-yl)butyl]ureidomethyl]pyridine-2-carboxylic acid amide (Reference Compound No.6-112)<br>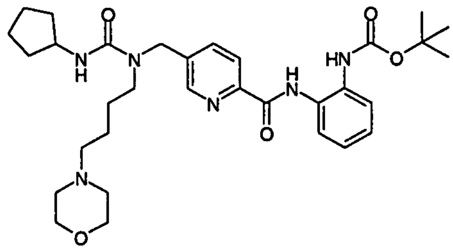 | $^1$H-NMR (500 MHz, $CDCl_3$)<br>δ 1.34 (m, 2H), 1.45-1.66 (m, 8H), 1.52 (s, 9H), 2.01 (m, 2H), 2.33 (t, J = 7.2 Hz, 2H), 2.41 (br s, 4H), 3.17 (t, J = 7.8 Hz, 2H), 3.70 (t, J = 4.7 Hz, 4H), 4.15 (m, 1H), 4.35 (d, J = 7.0 Hz, 1H), 4.59 (s, 2H), 7.04 (br s, 1H), 7.18-7.24 (m, 2H), 7.59 (d, J = 6.7 Hz, 1H), 7.72 (d, J = 7.0 Hz, 1H), 7.82 (dd, J = 8.2, 2.1 Hz, 1H), 8.26 (d, J = 8.2 Hz, 1H), 8.50 (d, J = 2.1 Hz, 1H), 10.15 (s, 1H) |
| N-(2-t-Butoxycarbonylaminophenyl)-5-[1-[5-(morpholin-4-yl)pentyl]-3-propylureidomethyl]pyridine-2-carboxylic acid amide (Reference Compound No.6-113)<br>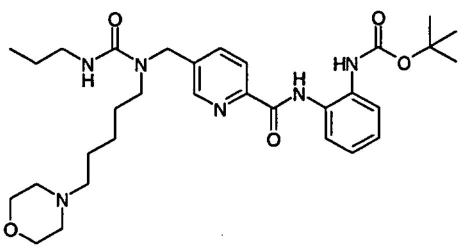 | $^1$H-NMR (400 MHz, $CDCl_3$)<br>δ 0.92 (t, J = 7.3 Hz, 3H), 1.32 (m, 2H), 1.46-1.62 (m, 6H), 1.52 (s, 9H), 2.30 (t, J = 7.6 Hz, 2H), 2.41 (br s, 4H), 3.17 (t, J = 7.7 Hz, 2H), 3.24 (m, 2H), 3.70 (t, J = 4.6 Hz, 4H), 4.42 (t, J = 5.6 Hz, 1H), 4.60 (s, 2H), 7.05 (br s, 1H), 7.18-7.25 (m, 2H), 7.59 (d, J = 6.6 Hz, 1H), 7.72 (d, J = 7.3 Hz, 1H), 7.81 (dd, J = 8.0, 2.2 Hz, 1H), 8.26 (d, J = 8.0 Hz, 1H), 8.50 (d, J = 2.2 Hz, 1H), 10.15 (s, 1H) |
| N-(2-t-Butoxycarbonylaminophenyl)-5-[3-isopropyl-1-[5-(morpholin-4-yl)pentyl]ureidomethyl]pyridine-2-carboxylic acid amide (Reference Compound No.6-114)<br>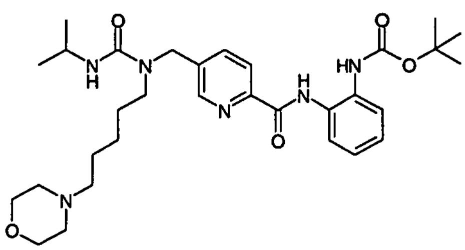 | $^1$H-NMR (400 MHz, $CDCl_3$)<br>δ 1.17 (d, J. = 6.3 Hz, 6H), 1.31 (m, 2H), 1.46-1.61 (m, 4H), 1.52 (s, 9H), 2.30 (t, J = 7.6 Hz, 2H), 2.41 (br s, 4H), 3.15 (t, J = 7.6 Hz, 2H), 3.70 (t, J = 4.6 Hz, 4H), 4.02 (m, 1H), 4.16 (d, J = 7.6 Hz, 1H), 4.59 (s, 2H), 7.05 (br s, 1H), 7.18-7.25 (m, 2H), 7.58 (d, J = 7.1 Hz, 1H), 7.72 (d, J = 6.8 Hz, 1H), 7.81 (dd, J = 8.0, 2.2 Hz, 1H), 8.26 (d, J = 8.0 Hz, 1H), 8.50 (d, J = 2.2 Hz, 1H), 10.16 (s, 1H) |
| N-(2-t-Butoxycarbonylaminophenyl)-5-[3-cyclopentyl-1-[5-(morpholin-4-yl)pentyl]ureidomethyl ]pyridine-2-carboxylic acid amide (Reference Compound No.6-115)<br>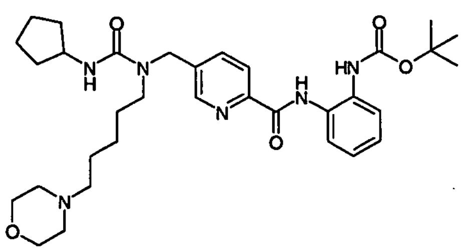 | $^1$H-NMR (400 MHz, $CDCl_3$)<br>δ 1.27-1.38 (m, 4H), 1.46-1.65 (m, 8H), 1.52 (s, 9H), 2.01 (m, 2H), 2.30 (t, J = 7.6 Hz, 2H), 2.41 (br s, 4H), 3.15 (t, J = 7.7 Hz, 2H), 3.70 (t, J = 4.6 Hz, 4H), 4.14 (m, 1H), 4.29 (d, J = 6.8 Hz, 1H), 4.59 (s, 2H), 7.05 (br s, 1H), 7.18-7.25 (m, 2H), 7.58 (d, J = 7.1 Hz, 1H), 7.72 (d, J = 6.8 Hz, 1H), 7.81 (dd, J = 8.0, 2.2 Hz, 1H), 8.26 (d, J = 8.0 Hz, 1H), 8.50 (d, J = 2.2 Hz, 1H), 10.16 (s, 1H) |

(continued)

| | |
|---|---|
| N-(2-t-Butoxycarbonylaminophenyl)-5-[1-(2-dimethylaminoethyl)-3-propylureidomethyl]pyridine-2-carboxylic acid amide (Reference Compound No.6-116)<br>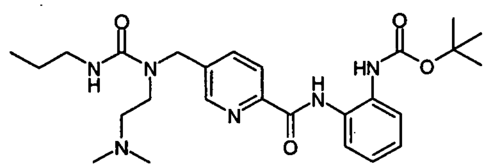 | $^1$H-NMR (400 MHz, $CDCl_3$)<br>δ 0.95 (t, J = 7.5 Hz, 3H), 1.52 (s, 9H), 1.53 (m, 2H), 2.26 (s, 6H), 2.41 (t, J = 4.6 Hz, 2H), 3.15-3.21 (m, 4H), 4.60 (s, 2H), 7.05 (s, 1H), 7.18-7.24 (m, 2H), 7.60 (d, J = 6.4 Hz, 1H), 7.69 (d, J = 7.0 Hz, 1H), 7.79 (t, J = 4.9 Hz, 1H), 7.86 (dd, J = 7.9, 2.1 Hz, 1H), 8.24 (d, J = 7.9 Hz, 1H), 8.51 (d, J = 2.1 Hz, 1H), 10.14 (s, 1H) |
| N-(2-t-Butoxycarbonylaminophenyl)-5-[1-(2-dimethylaminoethyl)-3-isopropylureidomethyl]pyridine-2-carboxylic acid amide (Reference Compound No.<br>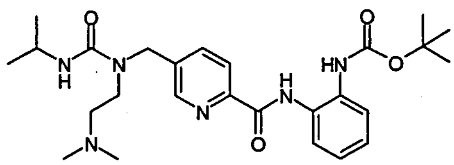<br>6-117) | $^1$H-NMR (500 MHz, $CDCl_3$)<br>δ 1.15 (d, J = 6.7 Hz, 6H), 1.52 (s, 9H), 2.26 (s, 6H), 2.40 (t, J = 4.5 Hz, 2H), 3.18 (t, J = 4.5 Hz, 2H), 3.91 (m, 1H), 4.59 (s, 2H), 7.05 (br s, 1H), 7.18-7.24 (m, 2H), 7.59 (d, J = 6.7 Hz, 1H), 7.70 (d, J = 6.7 Hz, 1H), 7.85 (dd, J = 7.9, 2.0 Hz, 1H), 7.93 (d, J = 6.7 Hz, 1H), 8.24 (d, J = 7.9 Hz, 1H), 8.51 (d, J = 2.0 Hz, 1H), 10.15 (s, 1H) |
| N-(2-t-Butoxycarbonylaminophenyl)-5-[3-sec-butyl-1-(2-dimethylaminoethyl)ureidomethyl]pyridine-2-carboxylic acid amide (Reference Compound No.6-118)<br>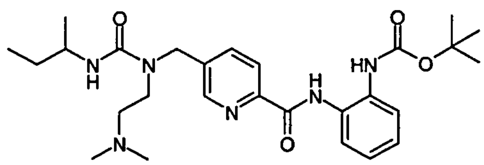 | $^1$H-NMR (500 MHz, $CDCl_3$)<br>δ 0.93 (t, J = 7.3 Hz, 3H), 1.12 (d, J = 6.4 Hz, 3H), 1.47 (m, 2H), 1.52 (s, 9H), 2.25 (s, 6H), 2.40 (t, J = 4.6 Hz, 2H), 3.18 (t, J = 4.6 Hz, 2H), 3.74 (m, 1H), 4.58 (d, J = 16.9 Hz, 1H), 4.62 (d, J = 16.9 Hz, 1H), 7.07 (br s, 1H), 7.17-7.24 (m, 2H), 7.59 (d, J = 6.4 Hz, 1H), 7.71 (d, J = 6.7 Hz, 1H), 7.83 (d, J = 7.3 Hz, 1H), 7.85 (dd, J = 7.9, 2.0 Hz, 1H), 8.24 (d, J = 7.9 Hz, 1H), 8.51 (d, J = 2.0 Hz, 1H), 10.15 (s, 1H) |
| N-(2-t-Butoxycarbonylaminophenyl)-5-[3-cyclopentyl-1-(2-dimethylaminoethyl)ureidomethyl]pyridine-2-carboxylic acid amide (Reference Compound No.6-119)<br>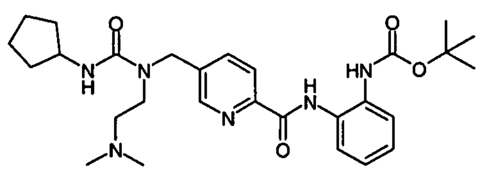 | $^1$H-NMR (500 MHz, $CDCl_3$)<br>δ 1.36-1.41 (m, 2H), 1.52 (s, 9H), 1.61-1.68 (m, 4H), 1.93-1.99 (m, 2H), 2.25 (s, 6H), 2.40 (t, J = 4.4 Hz, 2H), 3.18 (t, J = 4.4 Hz, 2H), 4.08 (m, 1H), 4.59 (s, 2H), 7.06 (br s, 1H), 7.18-7.24 (m, 2H), 7.60 (d, J = 7.0 Hz, 1H), 7.70 (d, J = 6.7 Hz, 1H), 7.86 (dd, J = 7.9, 2.1 Hz, 1H), 8.03 (d, J = 6.4 Hz, 1H), 8.24 (dd, J = 7.9, 0.6 Hz, 1H), 8.51 (dd, J = 2.1, 0.6 Hz, 1H), 10.15 (s, 1H) |
| N-(2-t-Butoxycarbonylaminophenyl)-5-[1-(2-dimethylaminoethyl)-3-hexylureidomethyl)pyridine-2-carboxylic acid amide (Reference Compound No.6-120)<br>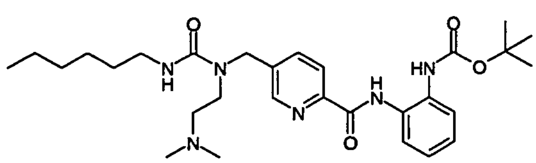 | $^1$H-NMR (500 MHz, $CDCl_3$)<br>δ 0.89 (t, J = 7.0 Hz, 3H), 1.29-1.38 (m, 6H), 1.48 (m, 2H), 1.52 (s, 9H), 2.26 (s, 6H), 2.41 (t, J = 4.6 Hz, 2H), 3.19 (t, J = 4.6 Hz, 2H), 3.21 (m, 2H), 4.60 (s, 2H), 7.06 (br s, 1H), 7.17-7.24 (m, 2H), 7.60 (d, J = 7.0 Hz, 1H), 7.68 (d, J = 6.7 Hz, 1H), 7.75 (t, J = 4.9 Hz, 1H), 7.86 (dd, J = 8.0, 1.9 Hz, 1H), 8.24 (d, J = 8.0 Hz, 1H), 8.51 (d, J = 1.9 Hz, 1H), 10.14 (s, 1H) |

(continued)

| | |
|---|---|
| N-(2-t-Butoxycarbonylaminophenyl)-5-[3-cyclohexyl-1-(2-dimethylaminoethyl)ureidomethyl]pyridine-2-carboxylic acid amide (Reference Compound No.6-121)<br>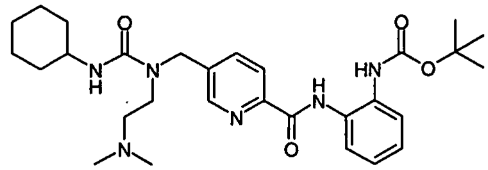 | $^1$H-NMR (500 MHz, $CDCl_3$)<br>δ 1.06-1.19 (m, 4H), 1.38 (m, 2H), 1.52 (s, 9H), 1.70 (m, 2H), 1.94 (m, 2H), 2.25 (s, 6H), 2.40 (t, J = 4.7 Hz, 2H), 3.19 (t, J = 4.7 Hz, 2H), 3.59 (m, 1H), 4.59 (s, 2H), 7.05 (br s, 1H), 7.17-7.24 (m, 2H), 7.60 (d, J = 7.3 Hz, 1H), 7.69 (d, J = 7.3 Hz, 1H), 7.86 (dd, J = 8.2, 1.8 Hz, 1H), 7.89 (d, J = 6.7 Hz, 1H), 8.24 (d, J = 8.2 Hz, 1H), 8.51 (d, J = 1.8 Hz, 1H), 10.14 (s, 1H) |
| N-(2-t-Butoxycarbonylaminophenyl)-5-[1-(2-dimethylaminoethyl)-3-phenethylureidomethyl]pyridine-2-carboxylic acid amide (Reference Compound No. 6-122)<br>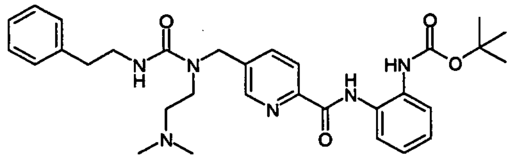 | $^1$H-NMR (500 MHz, $CDCl_3$)<br>δ 1.52 (s, 9H), 2.04 (s, 6H), 2.31 (t, J = 4.7 Hz, 2H), 2.84 (t, J = 6.9 Hz, 2H), 3.12 (t, J = 4.7 Hz, 2H), 3.53 (td, J = 6.9, 5.5 Hz, 2H), 4.59 (s, 2H), 7.06 (br s, 1H), 7.17-7.24 (m, 4H), 7.29 (d, J = 7.6 Hz, 2H), 7.31 (m, 1H), 7.60 (d, J = 7.0 Hz, 1H), 7.69 (d, J = 5.6 Hz, 1H), 7.69 (m, 1H), 7.82 (dd, J = 7.9, 2.0 Hz, 1H), 8.24 (dd, J = 7.9, 0.6 Hz, 1H), 8.50 (dd, J = 2.0, 0.6 Hz, 1H), 10.14 (s, 1H) |
| N-(2-t-Butoxycarbonylaminophenyl)-5-[1-(2-dimethylaminoethyl)-3-[2-(thiophen-2-yl)ethyl]ureidomethyl]pyridine-2-carboxylic acid amide (Reference Compound No.6-123)<br>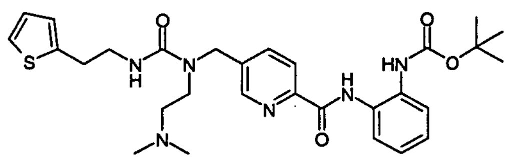 | $^1$H-NMR (500 MHz, $CDCl_3$)<br>δ 1.51 (s, 9H), 2.09 (s, 6H), 2.33 (t, J = 4.7 Hz, 2H), 3.05 (t, J = 6.5 Hz, 2H), 3.16 (t, J = 4.7 Hz, 2H), 3.53 (q, J = 6.5 Hz, 2H), 4.59 (s, 2H), 6.81 (dd, J = 3.4, 1.3 Hz, 1H), 6.93 (dd, J = 5.2, 3.4 Hz, 1H), 7.09 (br s, 1H), 7.14 (dd, J = 5.2, 1.3 Hz, 1H), 7.17-7.24 (m, 2H), 7.59 (d, J = 6.4 Hz, 1H), 7.71 (d, J = 6.4 Hz, 1H), 7.83 (dd, J = 7.9, 2.1 Hz, 1H), 7.90 (br s, 1H), 8.23 (dd, J = 7.9, 0.6 Hz, 1H), 8.50 (dd, J = 2.1, 0.6 Hz, 1H), 10.15 (s, 1H) |
| N-(2-t-Butoxycarbonylaminophenyl)-5-[3-isopropyl-1-[2-(4-methylpiperazin-1-yl)ethyl]ureidomethyl]pyridine-2-carboxylic acid amide (Reference Compound No.6-124)<br>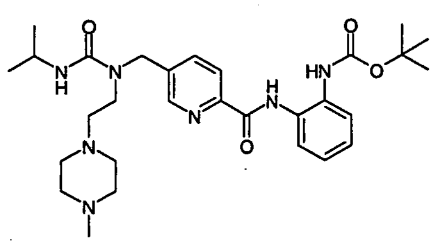 | $^1$H-NMR (400 MHz, $CDCl_3$)<br>δ 1.22 (d, J = 6.6 Hz, 6H), 1.52 (s, 9H), 2.30 (s, 3H), 2.46 (t, J = 4.5 Hz, 2H), 2.54 (br s, 8H), 3.21 (t, J = 4.5 Hz, 2H), 3.99 (m, 1H), 4.59 (s, 2H), 7.02-7.07 (m, 2H), 7.17-7.25 (m, 2H), 7.60 (d, J = 6.6 Hz, 1H), 7.70 (d, J = 6.6 Hz, 1H), 7.85 (dd, J = 8.0, 2.2 Hz, 1H), 8.24 (d, J = 8.0 Hz, 1H), 8.51 (d, J = 2.2 Hz, 1H), 10.14 (s, 1H) |

(continued)

| | |
|---|---|
| N-(2-t-Butoxycarbonylaminophenyl)-5-[1-[2-(4-methylpiperazin-1-yl)ethyl]-3-phenethylureidomethyl]pyridine-2-carboxylic acid amide (Reference Compound No.6-125)<br>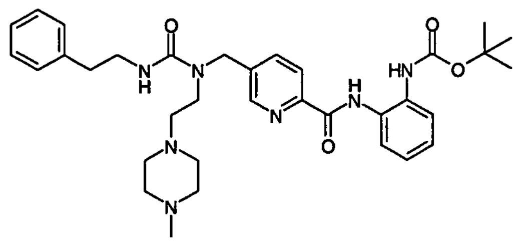 | $^1$H-NMR (400 MHz, $CDCl_3$)<br>δ 1.51 (s, 9H), 2.24 (s, 3H), 2.32 (br s, 8H), 2.39 (t, J = 4.5 Hz, 2H), 2.87 (t, J = 7.0 Hz, 2H), 3.16 (t, J = 4.5 Hz, 2H), 3.49 (m, 2H), 4.60 (s, 2H), 7.06 (br s, 1H), 7.17-7.25 (m, 5H), 7.26-7.32 (m, 2H), 7.60 (d, J = 7.1 Hz, 1H), 7.69-7.75 (m, 2H), 7.82 (dd, J = 7.9, 2.1 Hz, 1H), 8.25 (d, J = 7.9 Hz, 1H), 8.50 (d, J = 2.1 Hz, 1H), 10.15 (s, 1H) |
| N-(2-t-Butoxycarbonylaminophenyl)-5-[3-(4-methylphenyl)-1-[2-(4-methylpiperazin-1-yl)ethyl]ureidomethyl]pyridine-2-carboxylic acid amide (Reference Compound No.6-126)<br>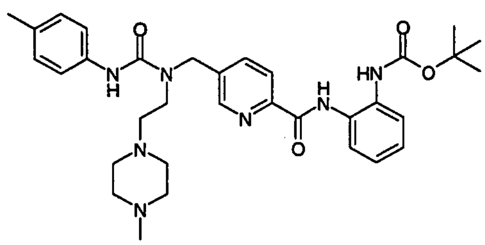 | $^1$H-NMR (400 MHz, $CDCl_3$)<br>δ 1.52 (s, 9H), 2.31 (s, 3H), 2.32 (s, 3H), 2.53 (br s, 4H), 2.58 (t, J = 4.3 Hz, 2H), 2.65 (br s, 4H), 3.36 (t, J = 4.3 Hz, 2H), 4.65 (s, 2H), 7.04 (br s, 1H), 7.12 (d, J = 8.3 Hz, 2H), 7.18-7.25 (m, 2H), 7.34 (d, J = 8.3 Hz, 2H), 7.60 (d, J = 6.6 Hz, 1H), 7.71 (d, J = 6.6 Hz, 1H), 7.93 (dd, J = 7.9, 2.1 Hz, 1H), 8.27 (d, J = 7.9 Hz, 1H), 8.57 (d, J = 2.1 Hz, 1H), 9.88 (s, 1H), 10.16 (s, 1H) |
| N-(2-t-Butoxycarbonylaminophenyl)-5-[3-isopropyl-1-[3-(4-methylpiperazin-1-yl)propyl]ureidomethyl]pyridine-2-carboxylic acid amide (Reference Compound No.6-127)<br>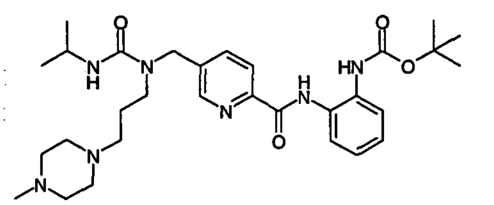 | $^1$H-NMR (400 MHz, $CDCl_3$)<br>δ 1.21 (d, J = 6.6 Hz, 6H), 1.52 (s, 9H), 1.68 (m, 2H), 2.32 (s, 3H), 2.36 (t, J = 6.1 Hz, 2H), 2.48 (br s, 8H), 3.19 (t, J = 6.0 Hz, 2H), 4.01 (m, 1H), 4.57 (s, 2H), 6.04 (d, J = 8.3 Hz, 1H), 7.07 (br s, 1H), 7.17-7.25 (m, 2H), 7.61 (d, J = 7.1 Hz, 1H), 7.69 (d, J = 6.6 Hz, 1H), 7.85 (dd, J = 8.0, 2.2 Hz, 1H), 8.24 (d, J = 8.0 Hz, 1H), 8.52 (d, J = 2.2 Hz, 1H), 10.14 (s, 1H) |
| N-(2-t-Butoxycarbonylaminophenyl)-5-[1-[3-(4-methylpiperazin-1-yl)propyl]-3-phenethylureidomethyl]pyridine-2-carboxylic acid amide (Reference Compound No.6-128)<br>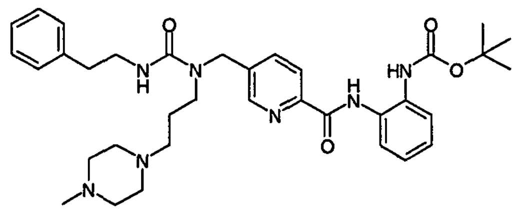 | $^1$H-NMR (400 MHz, $CDCl_3$)<br>δ 1.51 (s, 9H), 1.62 (m, 2H), 2.27 (s, 3H), 2.30 (br s, 8H), 2.32 (t, J = 6.1 Hz, 2H), 2.87 (t, J = 7.0 Hz, 2H), 3.15 (t, J = 5.5 Hz, 2H), 3.49 (m, 2H), 4.58 (s, 2H), 7.07 (br s, 1H), 7.17-7.35 (m, 8H), 7.61 (d, J = 6.3 Hz, 1H), 7.69 (d, J = 6.6 Hz, 1H), 7.80 (dd, J = 8.0, 2.2 Hz, 1H), 8.25 (d, J = 8.0 Hz, 1H), 8.51 (d, J = 2.2 Hz, 1H), 10.15 (s, 1H) |

(continued)

| | |
|---|---|
| N-(2-t-Butoxycarbonylaminophenyl)-5-[3-(4-methylphenyl)-1-[3-(4-methylpiperazin-1-yl)propyl] ureidomethyl]pyridine-2-carboxylic acid amide (Reference Compound No.6-129) 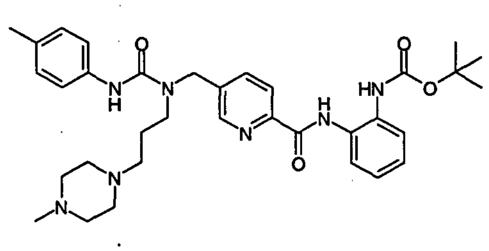 | $^1$H-NMR (400 MHz, $CDCl_3$) δ 1.51 (s, 9H), 1.77 (m, 2H), 2.25 (s, 3H), 2.32 (s, 3H), 2.41 (br s, 4H), 2.48 (t, J = 5.9 Hz, 2H), 2.49 (br s, 4H), 3.37 (t, J = 5.6 Hz, 2H), 4.64 (s, 2H), 7.06 (br s, 1H), 7.12 (d, J = 8.3 Hz, 2H), 7.17-7.24 (m, 2H), 7.33 (d, J = 8.3 Hz, 2H), 7.60 (d, J = 7.3 Hz, 1H), 7.68 (d, J = 7.1 Hz, 1H), 7.93 (dd, J = 8.0, 2.2 Hz, 1H), 8.24 (d, J = 8.0 Hz, 1H), 8.57 (d, J = 2.2 Hz, 1H), 8.99 (s, 1H), 10.14 (s, 1H) |
| N-(2-t-Butoxycarbonylaminophenyl)-5-[1-[3-(4-methylpiperidin-1-yl)propyl]-3-phenethylureidomethyl] pyridine-2-carboxylic acid amide (Reference Compound No.6-130) 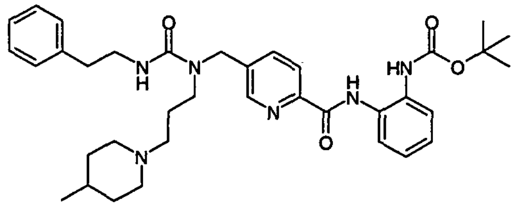 | $^1$H-NMR (500 MHz, $CDCl_3$) δ 0.92 (d, J = 6.7 Hz, 3H), 1.00 (m, 2H), 1.36 (m, 1H), 1.51 (s, 9H), 1.58-1.63 (m, 4H), 1.84 (t, J = 11.6 Hz, 2H), 2.28 (t, J = 6.1 Hz, 2H), 2.71 (d, J = 11.6 Hz, 2H), 2.85 (t, J = 7.0 Hz, 2H), 3.15 (t, J = 5.5 Hz, 2H), 3.47 (m, 2H), 4.57 (s, 2H), 7.08 (br s, 1H), 7.18-7.24 (m, 5H), 7.27-7.31 (m, 2H), 7.62 (d, J = 6.7 Hz, 1H), 7.66-7.70 (m, 2H), 7.79 (dd, J = 8.1, 2.0 Hz, 1H), 8.24 (d, J = 8.1 Hz, 1H), 8.50 (d, J = 2.0 Hz, 1H), 10.15 (s, 1H) |
| N-(2-t-Butoxycarbonylaminophenyl)-5-[1-[5-(morpholin-4-yl)pentyl]-3-phenethylureidomethyl]pyridine-2-carboxylic acid amide (Reference Compound No.6-131) 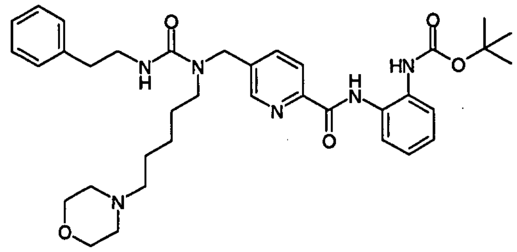 | $^1$H-NMR (500 MHz, $CDCl_3$) δ 1.20 (m, 2H), 1.39-1.48 (m, 4H), 1.52 (s, 9H), 2.27 (t, J = 7.5 Hz, 2H), 2.40 (br s, 4H), 2.84 (t, J = 6.6 Hz, 2H), 3.07 (t, J = 7.6 Hz, 2H), 3.54 (m, 2H), 3.70 (t, J = 4.6 Hz, 4H), 4.34 (t, J = 5.5 Hz, 1H), 4.55 (s, 2H), 7.08 (br s, 1H), 7.15-7.17 (m, 2H), 7.19-7.25 (m, 3H), 7.26-7.30 (m, 2H), 7.60 (d, J = 7.0 Hz, 1H), 7.71 (m, 1H), 7.73 (dd, J = 8.1, 2.0 Hz, 1H), 8.22 (d, J = 8.1 Hz, 1H), 8.45 (d, J = 2.0 Hz, 1H), 10.15 (s, 1H) |
| N-(2-t-Butoxycarbonylaminophenyl)-5-[3-(4-methoxyphenyl)-1-[5-(morpholin-4-yl)pentyl] ureidomethyl]pyridine-2-carboxylic acid amide (Reference Compound No.6-132) 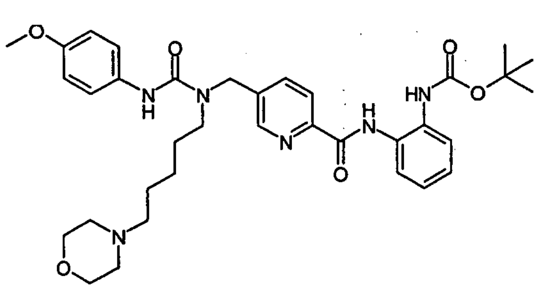 | $^1$H-NMR (400 MHz, $CDCl_3$) δ 1.38 (m, 2H), 1.52 (s, 9H), 1.55 (m, 2H), 1.68 (m, 2H), 2.32 (t, J = 7.4 Hz, 2H), 2.42 (t, J = 4.6 Hz, 4H), 3.30 (t, J = 7.7 Hz, 2H), 3.70 (t, J = 4.6 Hz, 4H), 3.79 (s, 3H), 4.68 (s, 2H), 6.24 (s, 1H), 6.85 (d, J = 9.0 Hz, 2H), 7.05 (br s, 1H), 7.19-7.22 (m, 2H), 7.25 (d, J = 9.0 Hz, 2H), 7.57 (d, J = 6.0 Hz, 1H), 7.72 (d, J = 6.6 Hz, 1H), 7.88 (dd, J = 8.0, 1.9 Hz, 1H), 8.28 (d, J = 8.0 Hz, 1H), 8.56 (d, J = 1.9 Hz, 1H), 10.17 (s, 1H) |

(continued)

| | |
|---|---|
| 5-[3-(4-Benzyloxyphenyl)-1-[5-(morpholin-4-yl)pentyl] ureidomethyl]-N-(2-t-butoxycarbonylaminophenyl) pyridine-2-carboxylic acid amide (Reference Compound No.6-133) 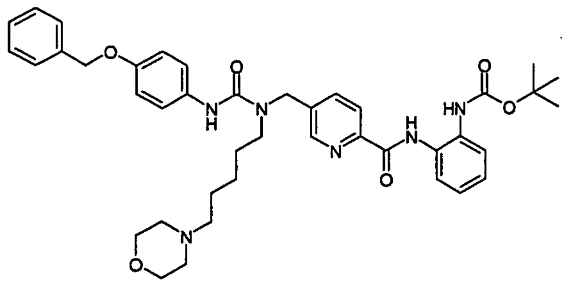 | $^1$H-NMR (500 MHz, $CDCl_3$) δ 1.38 (m, 2H), 1.52 (s, 9H), 1.57 (m, 2H), 1.68 (m, 2H), 2.32 (t, J = 7.5 Hz, 2H), 2.41 (s, 4H), 3.30 (t, J = 7.8 Hz, 2H), 3.70 (t, J = 4.6 Hz, 4H), 4.68 (s, 2H), 5.05 (s, 2H), 6.23 (s, 1H), 6.93 (d, J = 8.9 Hz, 2H), 7.05 (br s, 1H), 7.19-7.24 (m, 2H), 7.26 (m, 2H), 7.32 (d, J = 7.3 Hz, 1H), 7.36-7.40 (m, 2H), 7.42 (d, J = 7.3 Hz, 2H), 7.58 (d, J = 6.1 Hz, 1H), 7.73 (d, J = 7.9 Hz, 1H), 7.88 (d, J = 8.0, 1.9 Hz, 1H), 8.28 (d, J = 8.0 Hz, 1H), 8.56 (d, J = 1.9 Hz, 1H), 10.16 (s, 1H) |
| N-(2-t-Butoxycarbonylaminophenyl)-5-[3-(2-methoxyphenyl)-1-[5-(morpholin-4-yl)pentyl] ureidomethyl]pyridine-2-carboxylic acid amide (Reference Compound No.6-134) 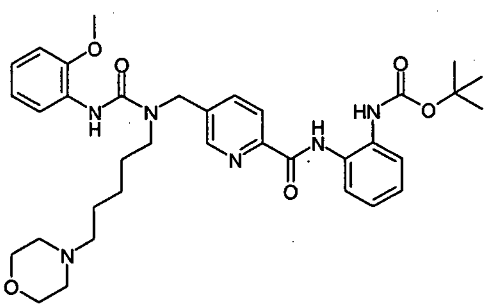 | $^1$H-NMR (500 MHz, $CDCl_3$) δ 1.39 (m, 2H), 1.51 (s, 9H), 1.54 (m, 2H), 1.71 (m, 2H), 2.33 (t, J = 7.6 Hz, 2H), 2.42 (m, 4H), 3.36 (t, J = 7.6 Hz, 2H), 3.70 (t, J = 4.7 Hz, 4H), 3.83 (s, 3H), 4.70 (s, 2H), 6.85 (m, 1H), 6.96-6.99 (m, 2H), 7.15 (s, 1H), 7.20-7.23 (m, 2H), 7.57 (d, J = 6.7 Hz, 1H), 7.73 (d, J = 6.4 Hz, 1H), 7.89 (dd, J = 7.9, 1.8 Hz, 1H), 8.17 (m, 1H), 8.28 (d, J = 7.9 Hz, 1H), 8.57 (d, J = 1.8 Hz, 1H), 10.17 (s, 1H) |
| N-(2-t-Butoxycarbonylaminophenyl)-5-[1-[3-(morpholin-4-yl)propyl]-3-(pyridin-3-yl)ureidomethyl]pyridine-2-carboxylic acid amide (Reference Compound No.6-135) 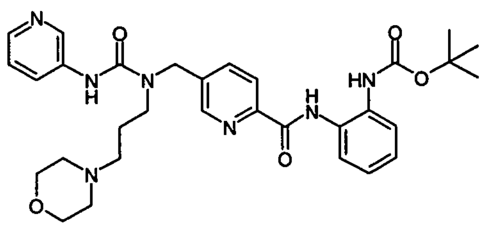 | $^1$H-NMR (500 MHz, $CDCl_3$) δ 1.51 (s, 9H), 1.82 (m, 2H), 2.48 (t, J = 4.5 Hz, 4H), 2.51 (t, J = 6.0 Hz, 2H), 3.42 (t, J = 5.5 Hz, 2H), 3.69 (t, J = 4.5 Hz, 4H), 4.65 (s, 2H), 7.04 (br s, 1H), 7.17-7.24 (m, 2H), 7.29 (ddd, J = 8.0, 4.8, 0.9 Hz, 1H), 7.58 (d, J = 6.4 Hz, 1H), 7.71 (d, J = 6.1 Hz, 1H), 7.92 (dd, J = 7.9, 2.1 Hz, 1H), 8.04 (ddd, J = 8.0, 2.4, 1.2 Hz, 1H), 8.26 (dd, J = 7.9, 0.9 Hz, 1H), 8.34 (dd, J = 4.8, 1.2 Hz, 1H), 8.58 (dd, J = 2.1, 0.9 Hz, 1H), 8.58 (dd, J = 2.4, 0.9 Hz, 1H), 9.24 (s, 1H), 10.15 (s, 1H) |

(continued)

| | |
|---|---|
| N-(2-t-Butoxycarbonylaminophenyl)-6-[1-(2-dimethylaminoethyl)-3-(3-methylphenyl)ureidomethyl] pyridine-3-carboxylic acid amide (Reference Compound No.6-136) 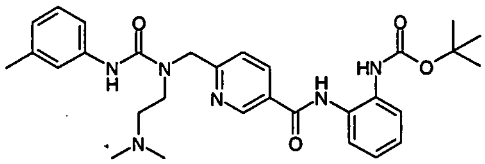 | $^1$H-NMR (400 MHz, $CDCl_3$) δ 1.51 (s, 9H), 2.32 (s, 3H), 2.39 (s, 6H), 2.58 (t, J = 4.4 Hz, 2H), 3.46 (t, J = 4.4 Hz, 2H), 4.73 (s, 2H), 6.78 (s, 1H), 6.80 (d, J = 7.8 Hz, 1H), 6.85 (d, J = 7.8 Hz, 1H), 7.06 (m, 1H), 7.10-7.25 (m, 4H), 7.54 (d, J = 7.8 Hz, 1H), 7.84 (d, J = 8.1 Hz, 1H), 8.21 (dd, J = 8.1, 2.0 Hz, 1H), 9.11 (d, J = 2.0 Hz, 1H), 9.48 (s, 1H), 10.91 (s, 1H) |
| N-(2-t-Butoxycarbonylaminophenyl)-6-[1-(2-dimethylaminoethyl)-3-(3-fluorophenyl)ureidomethyl] pyridine-3-carboxylic acid amide (Reference Compound No.6-137) 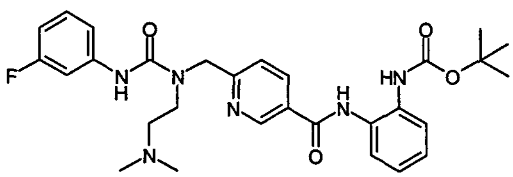 | $^1$H-NMR (400 MHz, $CDCl_3$) δ 1.51 (s, 9H), 2.40 (s, 6H), 2.61 (t, J = 4.3 Hz, 2H), 3.47 (t, J = 4.3 Hz, 2H), 4.72 (s, 2H), 6.64-6.71 (m, 2H), 6.77 (s, 1H), 6.97-7.00 (m, 2H), 7.17-7.22 (m, 3H), 7.53 (d, J = 7.6 Hz, 1H), 7.85 (d, J = 8.0 Hz, 1H), 8.22 (dd, J = 8.0, 1.9 Hz, 1H), 9.11 (d, J = 1.9 Hz, 1H), 9.50 (s, 1H), 11.30 (s, 1H) |
| N-(2-t-Butoxycarbonylaminophenyl)-6-[1-(2-dimethylaminoethyl)-3-(thiophen-3-yl)ureidomethyl] pyridine-3-carboxylic acid amide (Reference Compound No.6-138) 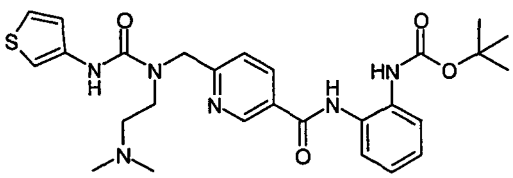 | $^1$H-NMR (400 MHz, $CDCl_3$) δ 1.51 (s, 9H), 2.39 (s, 6H), 2.58 (t, J = 4.3 Hz, 2H), 3.45 (t, J = 4.3 Hz, 2H), 4.74 (s, 2H), 6.80 (s, 1H), 6.86-6.89 (m, 2H), 7.19-7.24 (m, 3H), 7.28 (dd, J = 3.3, 1.3 Hz, 1H), 7.51 (d, J = 8.2 Hz, 1H), 7.84 (d, J = 8.2 Hz, 1H), 8.18 (dd, J = 8.2, 2.1 Hz, 1H), 9.09 (d, J = 2.1 Hz, 1H), 9.48 (s, 1H), 11.46 (s, 1H) |
| N-(2-t-Butoxycarbonylaminophenyl)-6-[3-(2,3-dihydrobenzo[1,4]dioxin-6-yl)-1-(2-dimethylaminoethyl) ureidomethyl]pyridine-3-carboxylic acid amide (Reference Compound No.6-139) 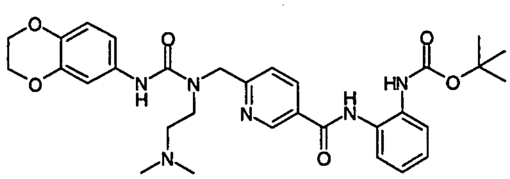 | $^1$H-NMR (400 MHz, $CDCl_3$) δ 1.51 (s, 9H), 2.37 (s, 6H), 2.56 (t, J = 4.2 Hz, 2H), 3.44 (t, J = 4.2 Hz, 2H), 4.20-4.24 (m, 4H), 4.71 (s, 2H), 6.72-6.83 (m, 4H), 6.92 (d, J = 2.2 Hz, 1H), 7.16-7.25 (m, 2H), 7.53 (d, J = 8.1 Hz, 1H), 7.83 (d, J = 7.6 Hz, 1H), 8.20 (dd, J = 8.1, 2.1 Hz, 1H), 9.10 (d, J = 2.1 Hz, 1H), 9.49 (s, 1H), 10.81 (s, 1H) |

(continued)

| | |
|---|---|
| N-(2-t-Butoxycarbonylaminophenyl)-6-[1-(2-dimethylaminoethyl)-3-(3-methoxyphenyl)ureidomethyl] pyridine-3-carboxylic acid amide (Reference Compound No.6-140) 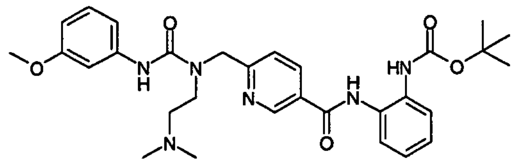 | $^1$H-NMR (400 MHz, $CDCl_3$) δ 1.51, (s, 9H), 2.39 (s, 6H), 2.58 (t, J = 4.4 Hz, 2H), 3.46 (t, J = 4.4 Hz, 2H), 3.79 (s, 3H), 4.73 (s, 2H), 6.54 (ddd, J = 8.2, 2.2, 0.8 Hz, 1H), 6.79 (m, 1H), 6.80 (ddd, J = 8.2, 2.2, 1.0 Hz, 1H), 7.07 (t, J = 2.2 Hz, 1H), 7.13-7.24 (m, 3H), 7.16 (t, J = 8.3 Hz, 1H), 7.53 (d, J = 8.2 Hz, 1H), 7.84 (d, J = 8.2 Hz, 1H), 8.21 (dd, J = 8.2, 2.2 Hz, 1H), 9.11 (d, J = 2.2 Hz, 1H), 9.49 (s, 1H), 11.08 (s, 1H) |
| N-(2-t-Butoxycarbonylaminophenyl)-5-[1-(4-dimethylaminobutyl)-3-(4-dimethylaminophenyl) ureidomethyl]pyridine-2-carboxylic acid amide (Reference Compound No.6-141) 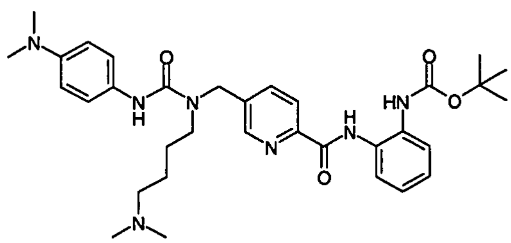 | $^1$H-NMR (400 MHz, $CDCl_3$) δ 1.52 (s, 9H), 1.53 (m, 2H), 1.71 (m, 2H), 2.19 (s, 6H), 2.34 (t, J = 6.5 Hz, 2H), 2.91 (s, 6H), 3.24 (t, J = 8.2 Hz, 2H), 4.69 (s, 2H), 6.72 (dd, J = 9.0 Hz, 2H), 7.11 (br s, 1H), 7.17-7.24 (m, 2H), 7.18 (d, J = 9.0 Hz, 2H), 7.60 (d, J = 6.4 Hz, 1H), 7.70 (d, J = 6.1 Hz, 1H), 7.84 (s, 1H), 7.91 (dd, J = 8.1, 2.1 Hz, 1H), 8.25 (dd, J = 8.1, 0.7 Hz, 1H), 8.57 (dd, J = 2.1, 0.7 Hz, 1H), 10.16 (s, 1H) |

Reference Example 7

5-[3-(1,3-Benzothiazol-2-yl)-1-(3-dimethylaminopropyl)ureidomethyl]-N-(2-t-butoxycarbonyl aminophenyl)pyridine-2-carboxylic acid amide (Reference Compound No.7-1)

Under ice cooling, 2-amino-1,3-benzothiazole (100 mg, 0.60 mmol) was added to a solution of N,N'-carbonyldiimidazole (98 mg, 0.60 mmol) in THF (3.0 mL), and then the reaction mixture was stirred for 2 hours. N-(2-t-butoxycarbonylaminophenyl)-5-(3-dimethylaminopropylaminomethyl)pyridine-2-carbo xylic acid amide (Reference Compound No.5-1, 90 mg, 0.20 mmol) was added thereto, and then the reaction mixture was stirred at 60°C for 3 hours. Water (30 mL) was added thereto, the whole was extracted with ethyl acetate (30 mL) twice, and then the organic layer was washed with water (30 mL) and brine (30 mL). After the organic layer was dried over anhydrous magnesium sulfate, the solvent was evaporated under reduced pressure to give 220 mg of a mixture containing the title reference compound as a white amorphous product.

| | |
|---|---|
| 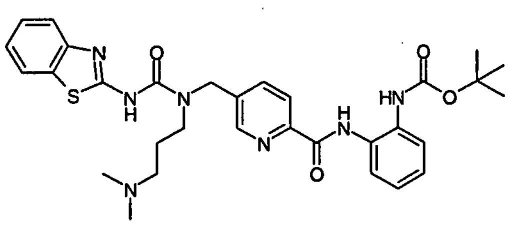 | $^1$H-NMR (400 MHz, DMSO-$d_6$) δ 1.45 (s, 9H), 1.74 (br s, 2H), 2.30 (br s, 6H), 2.38 (br s, 2H), 3.43 (br s, 2H), 4.70 (s, 2H), 7.13-7.27 (m, 4H), 7.34 (m, 1H), 7.44 (br s, 1H), 7.58 (br s, 1H), 7.84 (br s, 1H), 8.00-8.01 (m, 2H), 8.15 (d, J = 8.1 Hz, 1H), 8.60 (br s, 1H), 9.11 (br s, 1H), 10.47 (br s, 1H) |

By using any compounds selected from Reference Compounds No.5-1~5-20 and commercially available compounds, the following Reference Compounds No.7-2~7-49 were obtained by a method similar to that of Reference Compound No.7-1.

| | |
|---|---|
| 5-[3-(1H-Benzimidazol-2-yl)-1-(3-dimethylaminopropyl) ureidomethyl]-N-(2-t-butoxycarbonylaminophenyl) pyridine-2-carboxylic acid amide (Reference Compound No.7-2) 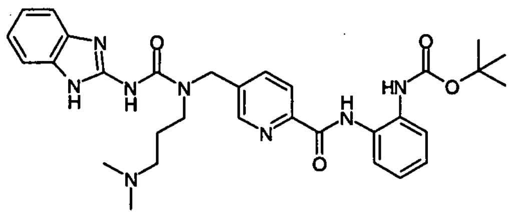 | $^{1}$H-NMR (400 MHz, DMSO-$d_6$) δ 1.41 (s, 9H), 1.71 (br s, 2H), 2.20 (br s, 8H), 3.42 (br s, 2H), 4.72 (s, 2H), 7.01-7.03 (m, 2H), 7.14 (td, J = 7.6, 1.5 Hz, 1H), 7.22-7.29 (m, 4H), 8.00-8.02 (m, 2H), 8.14 (d, J = 8.1 Hz, 1H), 8.59 (d, J = 1.2 Hz, 1H), 9.11 (br s, 1H), 10.47 (br s, 1H), 11.81 (br s, 2H) |
| N-(2-t-Butoxycarbonylaminophenyl)-5-[1-(3-dimethylaminopropyl)-3-(3-fluoro-4-methoxyphenyl) ureidomethyl]pyridine-2-carboxylic acid amide (Reference Compound No.7-3) 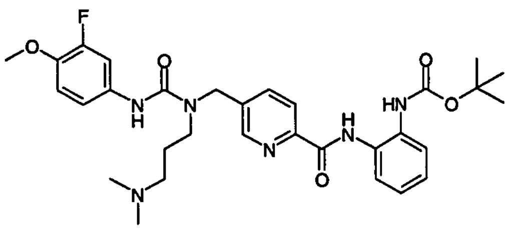 | $^{1}$H-NMR (400 MHz, $CDCl_3$) δ 1.51 (s, 9H), 1.73 (m, 2H), 2.30 (s, 6H), 2.40 (t, J = 6.0 Hz, 2H), 3.37 (t, J = 5.6 Hz, 2H), 3.86 (s, 3H), 4.61 (s, 2H), 6.88 (t, J = 9.1 Hz, 1H), 7.06-7.09 (m, 2H), 7.17-7.24 (m, 2H), 7.32 (dd, J = 13.4, 2.4 Hz, 1H), 7.58 (m, 1H), 7.69 (m, 1H), 7.92 (dd, J = 7.9, 2.2 Hz, 1H), 8.25 (dd, J = 7.9, 0.6 Hz, 1H), 8.58 (d, J = 2.2 Hz, 1H), 10.16 (s, 1H), 10.17 (s, 1H) |
| N-(2-t-Butoxycarbonylaminophenyl)-5-[1-(3-dimethylaminopropyl)-3-(phenylcarbonylmethyl) ureidomethyl]pyridine-2-carboxylic acid amide (Reference Compound No.7-4) 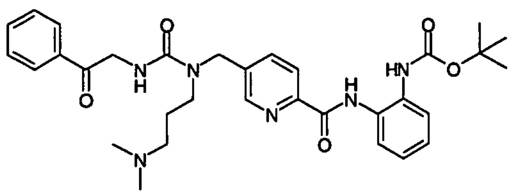 | $^{1}$H-NMR (400 MHz, $CDCl_3$) δ 1.51 (s, 9H), 1.71 (m, 2H), 2.22 (s, 6H), 2.40 (t, J = 6.1 Hz, 2H), 3.38 (t, J = 5.7 Hz, 2H), 4.61 (s, 2H), 4.71 (d, J = 4.4 Hz, 2H), 7.17-7.24 (m, 3H), 7.45-7.49 (m, 2H), 7.59 (t, J = 8.0 Hz, 2H), 7.62 (s, 1H), 7.87 (dd, J = 8.1, 2.2 Hz, 1H), 7.99 (dd, J = 8.0, 1.2 Hz, 2H), 8.24 (d, J = 8.1 Hz, 1H), 8.34 (s, 1H), 8.53 (d, J = 2.2 Hz, 1H), 10.15 (s, 1H) |
| N-(2-t-Butoxycarbonylaminophenyl)-5-[1-(3-dimethylaminopropyl)-3-(1,3-thiazol-2-yl)ureidomethyl] pyridine-2-carboxylic acid amide (Reference Compound No.7-5) 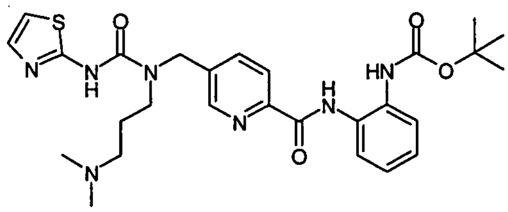 | $^{1}$H-NMR (400 MHz, $CDCl_3$) δ 1.51 (s, 9H), 1.73 (m, 2H), 2.35 (s, 6H), 2.40 (t, J = 6.0 Hz, 2H), 3.40 (m, 2H), 4.66 (s, 2H), 6.85 (d, J = 3.7 Hz, 1H), 7.08 (br s, 1H), 7.17-7.24 (m, 2H), 7.37 (d, J = 3.7 Hz, 1H), 7.58 (br s, 1H), 7.72 (br s, 1H), 7.91 (dd, J = 8.1, 2.1 Hz, 1H), 8.26 (dd, J = 8.1, 0.5 Hz, 1H), 8.57 (dd, J = 2.1, 0.5 Hz, 1H), 10.17 (br s, 1H), 13.17 (br s, 1H) |

(continued)

| | |
|---|---|
| N-(2-t-Butoxycarbonylaminophenyl)-5-[1-(3-dimethylaminopropyl)-3-(6-methoxy-1,3-benzothiazol-2-yl)ureidomethyl]pyridine-2-carboxylic acid amide (Reference Compound No.7-6) 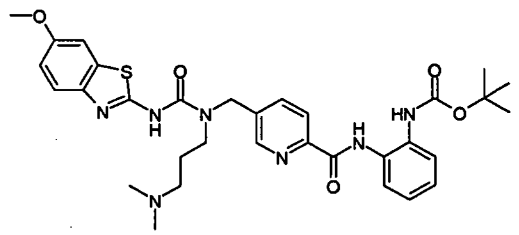 | $^1$H-NMR (400 MHz, DMSO-$d_6$) δ 1.46 (s, 9H), 1.73 (m, 2H), 2.28 (s, 6H), 2.33 (m, 2H), 3.41 (t, J = 5.1 Hz, 2H), 3.79 (s, 3H), 4.71 (s, 2H), 6.94 (dd, J = 8.8, 2.3 Hz, 1H), 7.15 (td, J = 7.6, 1.2 Hz, 1H), 7.21-7.29 (m, 3H), 7.45 (d, J = 2.3 Hz, 1H), 7.99-8.02 (m, 2H), 8.16 (d, J = 8.1 Hz, 1H), 8.61 (br s, 1H), 9.11 (br s, 1H), 10.47 (br s, 1H) |
| N-(2-t-Butoxycarbonylaminophenyl)-5-[3-(6-chloro-1,3-benzothiazol-2-yl)-1-(3-dimethylaminopropyl) ureidomethyl]pyridine-2-carboxylic acid amide (Reference Compound No.7-7) 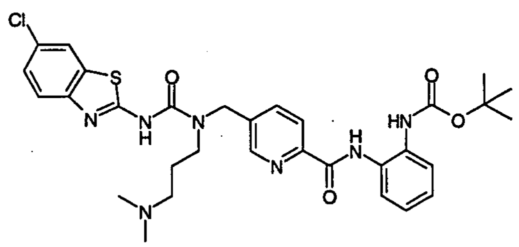 | $^1$H-NMR (400 MHz, DMSO-$d_6$) δ 1.45 (s, 9H), 1.75 (br s, 2H), 2.35 (br s, 8H), 3.42 (t, J = 6.3 Hz, 2H), 4.70 (s, 2H), 7.15 (td, J = 7.6, 1.5 Hz, 1H), 7.22-7.27 (m, 2H), 7.29 (d, J = 8.5 Hz, 1H), 7.34 (dd, J = 8.5, 2.1 Hz, 1H), 7.77 (d, J = 2.1 Hz, 1H), 7.96-8.02 (m, 2H), 8.15 (d, J = 8.1 Hz, 1H), 8.61 (br s, 1H), 9.11 (br s, 1H), 10.47 (br s, 1H) |
| N-(2-t-Butoxycarbonylaminophenyl)-5-[1-(3-dimethylaminopropyl)-3-(6-fluoro-1,3-benzothiazol-2-yl) ureidomethyl]pyridine-2-carboxylic acid amide (Reference Compound No.7-8) 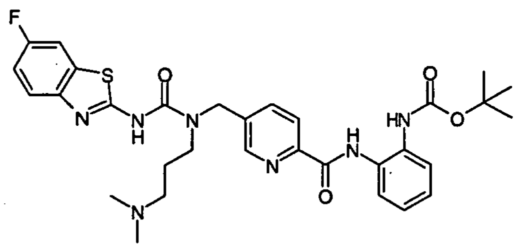 | $^1$H-NMR (400 MHz, DMSO-$d_6$) δ 1.45 (s, 9H), 1.74 (m, 2H), 2.33 (br s, 6H), 2.40 (br s, 2H), 3.42 (t, J = 5.5 Hz, 2H), 4.70 (s, 2H), 7.13-7.20 (m, 2H), 7.23-7.27 (m, 2H), 7.58 (m, 1H), 7.77 (dd, J = 8.9, 2.1 Hz, 1H), 7.99-8.02 (m, 2H), 8.15 (d, J = 8.1 Hz, 1H), 8.60 (br s, 1H), 9.12 (br s, 1H), 10.47 (br s, 1H) |
| N-(2-t-Butoxycarbonylaminophenyl)-5-[1-[3-(morpholin-4-yl)propyl]-3-(phenylcarbonylmethyl)ureidomethyl] pyridine-2-carboxylic acid amide (Reference Compound No.7-9) 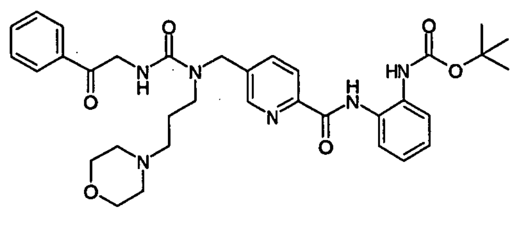 | $^1$H-NMR (400 MHz, $CDCl_3$) δ 1.52 (s, 9H), 1.77 (m, 2H), 2.44-2.52 (m, 6H), 3.39 (t, J = 6.0 Hz, 2H), 3.67 (t, J = 4.4 Hz, 4H), 4.63 (s, 2H), 4.72 (d, J = 5.1 Hz, 2H), 7.16-7.24 (m, 2H), 7.30 (m, 1H), 7.39 (t, J = 7.9 Hz, 1H), 7.48-7.52 (m, 2H), 7.61-7.63 (m, 2H), 7.68 (s, 1H), 7.88 (dd, J = 8.1, 2.2 Hz, 1H), 8.00 (dd, J = 8.5, 1.5 Hz, 2H), 8.26 (d, J = 8.1 Hz, 1H), 8.54 (d, J = 2.2 Hz, 1H), 10.14 (s, 1H) |

(continued)

| | |
|---|---|
| 5-[3-(1,3-Benzothiazol-2-yl)-1-[3-(morpholin-4-yl)propyl] ureidomethyl]-N-(2-t-butoxycarbonylaminophenyl) pyridine-2-carboxylic acid amide (Reference Compound No.7-10) 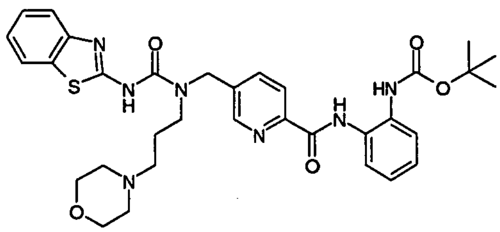 | $^1$H-NMR (500 MHz, DMSO-$d_6$) δ 1.44 (s, 9H), 1.75 (br s, 2H), 2.38 (br s, 6H), 3.40-3.49 (m, 2H), 3.76 (br s, 4H), 4.75 (br s, 2H), 7.15 (td, J = 7.6, 1.5 Hz, 1H), 7.23-7.27 (m, 3H), 7.36 (t, J = 7.3 Hz, 1H), 7.60 (br s, 1H), 7.85 (br s, 1H), 7.98-8.01 (m, 2H), 8.15 (d, J = 8.2 Hz, 1H), 8.59 (s, 1H), 9.10 (br s, 1H), 10.47 (br s, 1H) |
| N-(2-t-Butoxycarbonylaminophenyl)-5-[3-(6-fluoro-1,3-benzothiazol-2-yl)-1-[3-(morpholin-4-yl)propyl] ureidomethyl]pyridine-2-carboxylic acid amide (Reference Compound No.7-11) 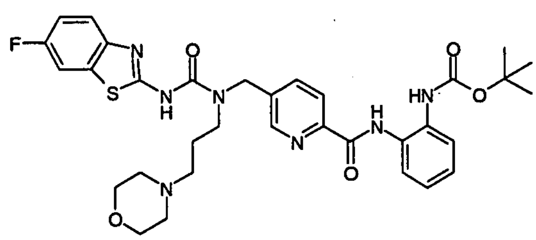 | $^1$H-NMR (400 MHz, $CDCl_3$) δ 1.51 (s, 9H), 1.83 (m, 2H), 2.49 (t, J = 6.0 Hz, 2H), 2.55 (br s, 4H), 3.43 (m, 2H), 4.11 (t, J = 7.2 Hz, 4H), 4.69 (s, 2H), 7.03 (m, 1H), 7.10 (td, J = 9.0, 2.4 Hz, 1H), 7.18-7.24 (m, 2H), 7.46 (dd, J = 8.1, 2.4 Hz, 1H), 7.57 (d, J = 5.6 Hz, 1H), 7.64 (dd, J = 9.0, 4.6 Hz, 1H), 7.72 (d, J = 4.9 Hz, 1H), 7.92 (dd, J = 8.1, 2.2 Hz, 1H), 8.27 (dd, J = 8.1, 0.7 Hz, 1H), 8.59 (dd, J = 2.2, 0.7 Hz, 1H), 10.17 (br s, 1H) |
| N-(2-t-Butoxycarbonylaminophenyl)-5-[1-[3-(morpholin-4-yl)propyl]-3-(pyridin-2-yl)ureidomethyl]pyridine-2-carboxylic acid amide (Reference Compound No.7-12) 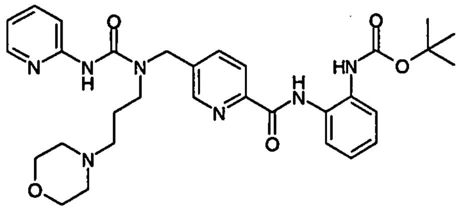 | $^1$H-NMR (400MHz, Solv. $CDCl_3$) δ 1.51 (s, 9H), 1.81 (m, 2H), 2.46 (t, J = 6.1 Hz, 2H), 2.51 (br s, 4H), 3.44 (t, J = 5.6 Hz, 2H), 3.99 (t, J = 4.3 Hz, 4H), 4.66 (s, 2H), 6.93 (ddd, J = 7.2, 4.9, 1.0 Hz, 1H), 7.05 (br s, 1H), 7.17-7.25 (m, 2H), 7.59 (d, J = 7.3 Hz, 1H), 7.63 (ddd, J = 8.6, 7.2, 2.0 Hz, 1H), 7.70 (d, J = 6.8 Hz, 1H), 7.92 (dd, J = 8.1, 2.2 Hz, 1H), 8.00 (dt, J = 8.6, 1.0 Hz, 1H), 8.22 (ddd, J = 4.9, 2.0, 1.0 Hz, 1H), 8.27 (dd, J = 8.1, 0.6 Hz, 1H), 8.59 (dd, J = 2.2, 0.6 Hz, 1H), 9.83 (s, 1H), 10.16 (s, 1H) |
| N-(2-t-Butoxycarbonylaminophenyl)-5-[1-[3-(morpholin-4-yl)propyl]-3-(pyridin-4-yl)ureidomethyl]pyridine-2-carboxylic acid amide (Reference Compound No.7-13) 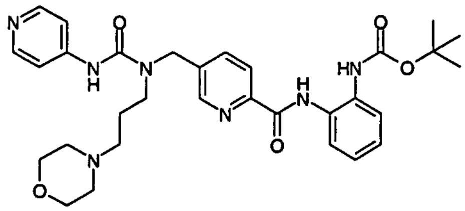 | $^1$H-NMR (500 MHz, $CDCl_3$) δ 1.51 (s, 9H), 1.83 (m, 2H), 2.44-2.56 (m, 6H), 3.39 (t, J = 5.6 Hz, 2H), 3.80 (t, J = 4.6 Hz, 4H), 4.65 (s, 2H), 7.02 (br s, 1H), 7.18-7.25 (m, 2H), 7.49 (d, J = 6.3 Hz, 2H), 7.57 (d, J = 6.3 Hz, 1H), 7.71 (d, J = 6.3 Hz, 1H), 7.91 (dd, J = 8.0, 2.2 Hz, 1H), 8.27 (dd, J = 8.0, 0.7 Hz, 1H), 8.47 (d, J = 6.3 Hz, 2H), 8.58 (dd, J = 2.2, 0.7 Hz, 1H), 9.10 (s, 1H), 10.17 (s, 1H) |

(continued)

| | |
|---|---|
| 5-[3-(1,3-Benzothiazol-2-yl)-1-(2-dimethylaminoethyl) ureidomethyl]-N-(2-t-butoxycarbonylaminophenyl) pyridine-2-carboxylic acid amide (Reference Compound No.7-144) 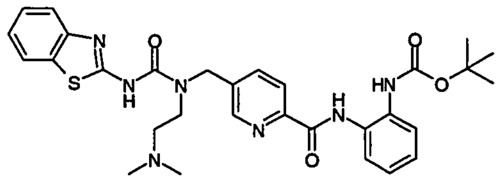 | $^1$H-NMR (500 MHz, $CDCl_3$) δ 1.51 (s, 9H), 2.50 (s, 6H), 2.60 (t, J = 4.3 Hz, 2H), 3.39 (t, J = 4.3 Hz, 2H), 4.73 (s, 2H), 7.02 (br s, 1H), 7.19-7.24 (m, 3H), 7.36 (m, 1H), 7.57 (d, J = 5.5 Hz, 1H), 7.70 (d, J = 8.7 Hz, 1H), 7.73 (m,1H), 7.77 (d, J = 7.6 Hz, 1H), 7.92 (dd, J = 7.9, 2.1 Hz, 1H), 8.29 (d, J = 7.9 Hz, 1H), 8.58 (d, J = 2.1 Hz, 1H), 10.17 (s, 1H) |
| N-(2-t-Butoxycarbonylaminophenyl)-5-[1-(2-dimethylaminoethyl)-3-(quinolin-6-yl)ureidomethyl] pyridine-2-carboxylic acid amide (Reference Compound No.7-15) 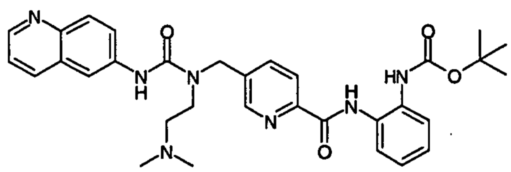 | $^1$H-NMR (500 MHz, $CDCl_3$) δ 1.51 (s, 9H), 2.48 (s, 6H), 2.61 (t, J = 4.3 Hz, 2H), 3.40 (t, J = 4.3 Hz, 2H), 4.71 (s, 2H), 7.05 (br s, 1H), 7.19-7.24 (m, 2H), 7.35 (dd, J = 8.2, 4.3 Hz, 1H), 7.39 (dd, J = 9.0, 2.4 Hz, 1H), 7.59 (d, J = 6.1 Hz, 1H), 7.72 (d, J = 6.1 Hz, 1H), 7.95 (dd, J = 7.9, 2.1 Hz, 1H), 8.01 (d, J = 9.0 Hz, 1H), 8.09 (d, J = 8.2 Hz, 1H), 8.18 (d, J = 2.4 Hz, 1H), 8.29 (d, J = 7.9 Hz, 1H), 8.61 (d, J = 2.1 Hz, 1H), 8.77 (d, J = 4.3 Hz, 1H), 10.17 (s, 1H), 11.50 (s, 1H) |
| N-(2-t-Butoxycarbonylaminophenyl)-5-[1-(2-dimethylaminoethyl)-3-(1,3-thiazol-2-yl)ureidomethyl] pyridine-2-carboxylic acid amide (Reference Compound No.7-16) 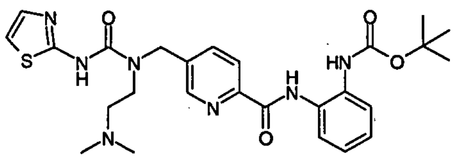 | $^1$H-NMR (400 MHz, $CDCl_3$) δ 1.51 (s, 9H), 2.45 (s, 6H), 2.57 (t, J = 4.3 Hz, 2H), 3.35 (t, J = 4.3 Hz, 2H), 4.71 (s, 2H), 6.86 (d, J = 3.7 Hz, 1H), 7.02 (br s, 1H), 7.18-7.25 (m, 2H), 7.39 (d, J = 3.7 Hz, 1H), 7.58 (d, J = 6.3 Hz, 1H), 7.72 (d, J = 6.1 Hz, 1H), 7.90 (dd, J = 8.1, 2.2 Hz, 1H), 8.28 (d, J = 8.1 Hz, 1H), 8.56 (d, J = 2.2 Hz, 1H), 10.16 (s, 1H) |
| N-(2-t-Butoxycarbonylaminophenyl)-5-[1-(2-dimethylaminoethyl)-3-phenylcarbonylmethylureidomethyl]pyridine-2-carboxylic acid amide (Reference Compound No.7-17) 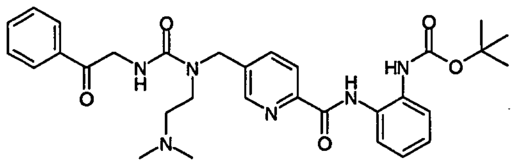 | $^1$H-NMR (400 MHz, $CDCl_3$) δ 1.51 (s, 9H), 2.35 (s, 6H), 2.51 (t, J = 5.0 Hz, 2H), 3.34 (t, J = 5.0 Hz, 2H), 4.66 (s, 2H), 4.79 (d, J = 3.7 Hz, 2H), 7.07 (br s, 1H), 7.17-7.25 (m, 2H), 7.47-7.52 (m, 2H), 7.58-7.63 (m, 2H), 7.69 (d, J = 8.1 Hz, 1H), 7.89 (dd, J = 8.1, 2.0 Hz, 1H), 7.99-8.01 (m, 2H), 8.27 (d, J = 8.1 Hz, 1H), 8.41 (br s, 1H, 8.54 (d, J = 2.0 Hz, 1H), 10.15 (s, 1H) |

(continued)

| | |
|---|---|
| N-(2-t-Butoxycarbonylaminophenyl)-5-[1-(3-dimethylaminopropyl)-3-(4,5,6,7-tetrahydro-1,3-benzothiazol-2-yl)ureidomethyl]pyridine-2-carboxylic acid amide (Reference Compound No.7-18)<br>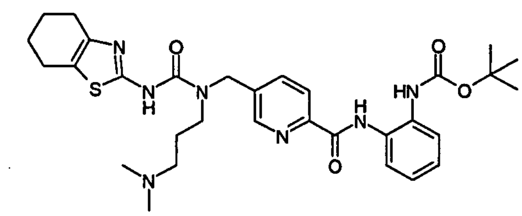 | $^1$H-NMR (400 MHz, DMSO-$d_6$)<br>δ 1.46 (s, 9H), 1.67 (m, 2H), 1.74 (br s, 4H), 2.18 (s, 6H), 2.25 (br s, 2H), 2.49-2.55 (m, 4H), 3.34 (m, 2H), 4.66 (s, 2H), 7.15 (t, J = 7.4 Hz, 1H), 7.24 (d, J = 7.4 Hz, 1H), 7.25 (m, 1H), 7.94 (d, J = 8.1 Hz, 1H), 8.00 (d, J = 7.4 Hz, 1H), 8.14 (d, J = 8.1 Hz, 1H), 8.56 (s, 1H), 9.12 (br s, 1H), 10.47 (br s, 1H), 12.39 (br s, 1H) |
| N-(2-t-Butoxycarbonylaminophenyl)-5-[1-[3-(morpholin-4-yl)propyl]-3-[2-(morpholin-4-yl)pyridin-5-yl] ureidomethyl]pyridine-2-carboxylic acid amide (Reference Compound No.7-19)<br>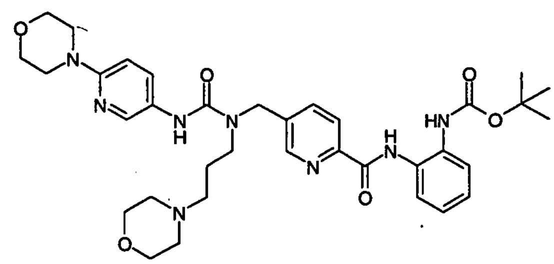 | $^1$H-NMR (400 MHz, $CDCl_3$)<br>δ 1.51 (s, 9H), 1.79 (m, 2H), 2.45 (br s, 4H), 2.50 (t, J = 6.0 Hz, 2H), 3.39 (t, J = 5.4 Hz, 2H), 3.46 (t, J = 4.9 Hz, 4H), 3.61 (t, J = 4.4 Hz, 4H), 3.83 (t, J = 4.9 Hz, 4H), 4.63 (s, 2H), 6.66 (d, J = 9.0 Hz, 1H), 7.05 (br s, 1H), 7.17-7.25 (m, 2H), 7.60 (d, J = 6.8 Hz, 1H), 7.70 (m, 1H), 7.70 (dd, J = 9.0, 2.8 Hz, 1H), 7.92 (dd, J = 7.9, 2.1 Hz, 1H), 8.11 (d, J = 2.8 Hz, 1H), 8.25 (dd, J = 7.9, 0.7 Hz, 1H), 8.57 (dd, J = 2.1, 0.7 Hz, 1H), 9.20 (s, 1H), 10.14 (s, 1H) |
| N-(2-t-Butoxycarbonylaminophenyl)-5-[1-(3-dimethylaminopropyl)-3-(4-phenyl-1,3-thiazol-2-yl) ureiomethyl]pyridine-2-carboxylic acid amide (Reference Compound No.7-20)<br>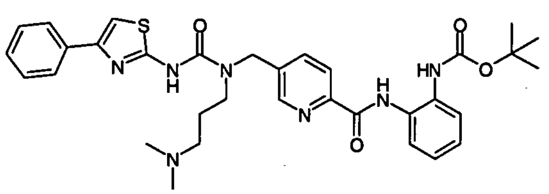 | $^1$H-NMR (400 MHz, DMSO-$d_6$)<br>δ 1.47 (s, 9H), 1.74 (m, 2H), 2.33 (s, 6H), 2.37 (m, 2H), 3.41 (t, J = 5.6 Hz, 2H), 4.68 (s, 2H), 7.15 (t, J = 8.1 Hz, 1H), 7.23-7.27 (m, 2H), 7.29 (t, J = 7.7 Hz, 1H), 7.41 (t, J = 7.7 Hz, 2H), 7.48 (s, 1H), 7.89-7.91 (m, 2H), 8.01 (d, J = 7.7 Hz, 2H), 8.16 (d, J = 8.1 Hz,1H), 8.61 (s, 1H), 9.12 (br s, 1H), 10.48 (br s, 1H), 13.44 (br s, 1H) |
| N-(2-t-Butoxycarbonylaminophenyl)-5-[3-(2-methoxypyridin-5-yl)-1-[3-(morpholin-4-yl)propyl] ureidomethyl]pyridine-2-carboxylic acid amide (Reference Compound No.7-21)<br>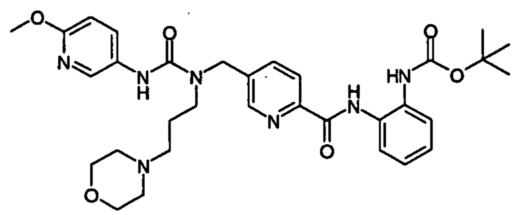 | $^1$H-NMR (400 MHz, $CDCl_3$)<br>δ 1.51 (s, 9H), 1.79 (m, 2H), 2.45 (br s, 4H), 2.51 (t, J = 5.9 Hz, 2H), 3.40 (t, J = 5.2 Hz, 2H), 3.62 (t, J = 4.3 Hz, 4H), 3.92 (s, 3H), 4.64 (s, 2H), 6.75 (dd, J = 8.9, 0.6 Hz, 1H), 7.05 (br s, 1H), 7.17-7.25 (m, 2H), 7.60 (d, J = 7.3 Hz, 1H), 7.69 (d, J = 7.3 Hz, 1H), 7.78 (dt, J = 8.9, 2.6 Hz, 1H), 7.92 (dd, J = 8.1, 2.1 Hz, 1H), 8.06 (m, 1H), 8.26 (dd, J = 8.1, 0.7 Hz, 1H), 8.57 (dd, J = 2.1, 0.7 Hz, 1H), 9.27 (s, 1H), 10.15 (s, 1H) |

(continued)

| | |
|---|---|
| N-(2-t-Butoxycarbinylaminophenyl)-5-[3-(4-methylpyridin-2-yl)-1[[3-(morpholin-4-yl)propyl] ureidomethyl]pyridine-2-carboxylic acid amide (Reference Compound No.7-22)<br>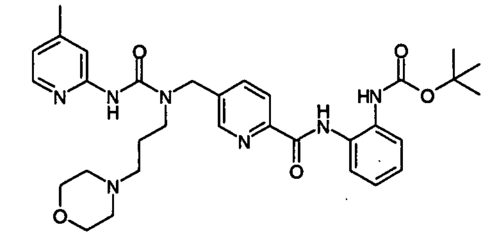 | $^1$H-NMR (500 MHz, $CDCl_3$)<br>δ 1.51 (s, 9H), 1.80 (m, 2H), 2.34 (s, 3H), 2.45 (t, J = 6.0 Hz, 2H), 2.50 (br s, 4H), 3.44 (t, J = 5.7 Hz, 2H), 3.97 (br s, 4H), 4.66 (s, 2H), 6.76 (d, J = 5.0 Hz, 1H), 7.06 (br s, 1H), 7.17-7.25 (m, 2H), 7.59 (d, J = 5.8 Hz, 1H), 7.70 (d, J = 5.5 Hz, 1H), 7.86 (s, 1H), 7.92 (dd, J = 7.9, 1.8 Hz, 1H), 8.08 (d, J = 5.0 Hz, 1H), 8.27 (d, J = 7.9 Hz, 1H), 8.59 (d, J = 1.8 Hz, 1H), 9.70 (s, 1H), 10.16 (s, 1H) |
| N-(2-t-Butoxycarbonylaminophenyl)-5-[1-(2-dimethylaminoethyl)-3-[2-(piperidin-1-y))ethyl] ureidomethyl]pyridine-2-carboxylic acid amide (Reference Compound No.7-23)<br>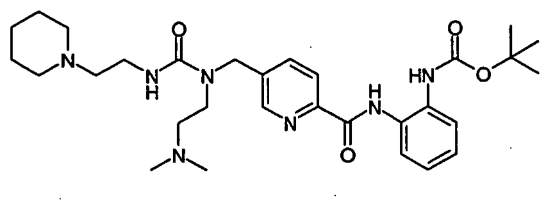 | $^1$H-NMR (400 MHz, $CDCl_3$)<br>δ 1.51 (s, 9H), 1.55 (m, 4H), 1.70 (m, 4H), 2.26 (s, 6H), 2.39 (s, 2H), 2.42 (t, J = 5.6 Hz, 2H), 2.44 (t, J = 6.8 Hz, 2H), 3.26 (t, J = 5.6 Hz, 2H), 3.33 (q, J = 6.8 Hz, 2H), 4.61 (s, 2H), 7.08 (br s, 1H), 7.13 (br s, 1H), 7.18-7.24 (m, 2H), 7.61 (d, J = 7.3 Hz, 1H), 7.68 (d, J = 7.6 Hz, 1H), 7.86 (dd, J = 7.9, 2.1 Hz, 1H), 8.25 (dd, J = 8.0, 0.7 Hz, 1H), 8.52 (dd, J = 2.1, 0.7 Hz, 1H), 10.14 (s, 1H) |
| N-(2-t-Butoxycarbonylaminophenyl)-5-[3-(2-diisopropylaminoethyl)-1-(2-dimethylaminoethyl) ureidomethyl]pyridine-2-carboxylic acid amide (Reference Compound No.7-24)<br>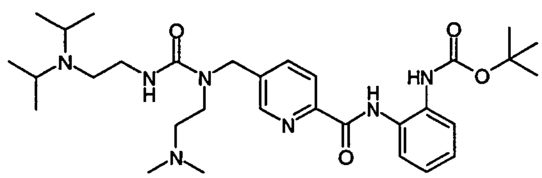 | $^1$H-NMR (400 MHz, $CDCl_3$)<br>δ 1.00 (d, J = 4.9 Hz,, 12H), 1.52 (s, 9H), 2.25 (s, 6H), 2.43 (t, J = 5.9 Hz, 2H), 2.58 (s, 2H), 3.00 (s, 2H), 3.22 (s, 2H), 3.27 (s, 2H), 4.63 (s, 2H), 6.62 (s, 1H), 7.06 (br s, 1H), 7.17-7.25 (m, 2H), 7.60 (d, J = 7.6 Hz, 1H), 7.69 (d, J = 6.8 Hz, 1H), 7.85 (dd, J = 8.0, 2.1 Hz, 1H), 8.25 (dd, J = 8.0, 0.7 Hz, 1H), 8.51 (dd, J = 2.1, 0.7 Hz, 1H), 10.14 (s, 1H) |
| N-(2-t-Butoxycarbonylaminophenyl)-5-[3-[2-(cyclohexen-1-yl)ethyl]-1-(2-dimethylaminoethyl) ureidomethyl]pyridine-2-carboxylic acid amide (Reference Compound No.7-25)<br>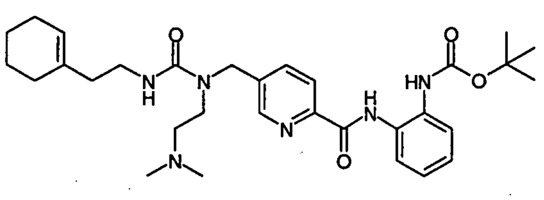 | $^1$H-NMR (400 MHz, $CDCl_3$)<br>δ 1.51 (s, 9H), 1.55 (m, 2H), 1.62 (m, 2H), 1.92-1.97 (m, 4H), 2.14 (t, J = 7.0 Hz, 2H), 2.24 (s, 6H), 2.40 (t, J = 5.0 Hz, 2H), 3.20 (t, J = 5.0 Hz, 2H), 3.30 (td, J = 7.0, 5.1 Hz, 2H), 4.59 (s, 2H), 5.45 (m, 1H), 7.09 (br s, 1H), 7.17-7.24 (m, 2H), 7.33 (m, 1H), 7.60 (d, J = 6.8 Hz, 1H), 7.69 (d, J = 7.3 Hz, 1H), 7.84 (dd, J = 8.0, 2.2 Hz, 1H), 8.24 (dd, J = 8.0, 0.7 Hz, 1H), 8.50 (dd, J = 2.2, 0.7 Hz, 1H), 10.15 (s, 1H) |

(continued)

| | |
|---|---|
| N-(2-t-Butoxycarbonylaminophenyl)-5-[1-(2-dimethylaminoethyl)-3-(2,2,2-trifluoroethyl) ureidomethyl]pyridine-2-carboxylic acid amide (Reference Compound No.7-26) 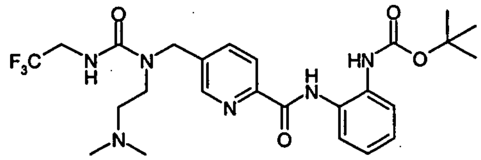 | $^1$H-NMR (400 MHz, $CDCl_3$) δ 1.52 (s, 9H), 2.28 (s, 6H), 2.45 (t, J = 4.3 Hz, 2H), 3.24 (t, J = 4.3 Hz, 2H), 3.87 (m, 2H), 4.61 (s, 2H), 7.09 (br s, 1H), 7.19-7.21 (m, 2H), 7.56 (d, J = 5.8 Hz, 1H), 7.73 (d, J = 5.5 Hz, 1H), 7.83 (dd, J = 7.9, 2.1 Hz, 1H), 8.25 (dd, J = 7.9, 0.6 Hz, 1H), 8.51 (dd, J = 2.1, 0.6 Hz, 1H), 9.48 (s, 1H), 10.18 (s, 1H) |
| N-(2-t-Butoxycarbonylaminophenyl)-5-[3-(2-cyanoethyl)-1-(2-dimethylaminoethyl)ureidomethyl] pyridine-2-carboxylic acid amide (Reference Compound No.7-27) 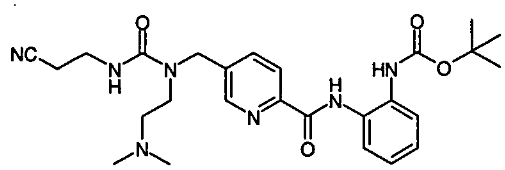 | $^1$H-NMR (500 MHz, $CDCl_3$) δ 1.52 (s, 9H), 2.32 (s, 6H), 2.45 (t, J = 4.4 Hz, 2H), 2.59 (t, J = 6.3 Hz, 2H), 3.24 (t, J = 4.4 Hz, 2H), 3.45 (m, 2H), 4.60 (s, 2H), 7.10 (m, 1H), 7.19-7.25 (m, 2H), 7.58 (d, J = 6.7 Hz, 1H), 7.71 (d, J = 6.7 Hz, 1H), 7.84 (dd, J = 7.9, 2.1 Hz, 1H), 8.25 (dd, J = 7.9, 0.6 Hz, 1H), 8.51 (dd, J = 2.1, 0.6 Hz, 1H), 9.06 (s, 1H), 10.17 (s, 1H) |
| N-(2-t-Butoxycarbonylaminophenyl)-5-[1-(2-dimethylaminoethyl)-3-[3-(pyrrolidin-2-on-1-yl)propyl] ureidomethyl]pyridine-2-carboxylic acid amide (Reference Compound No.7-28) 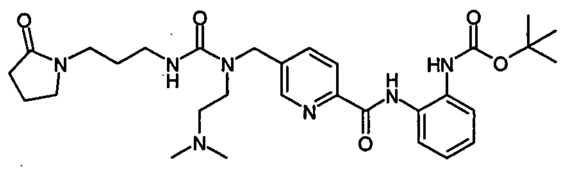 | $^1$H-NMR (500 MHz, $CDCl_3$) δ 1. 52 (s, 9H), 1.71 (m, 2H), 2.04 (m, 2H), 2.27 (s, 6H), 2.39 (t, J = 8.2 Hz, 2H), 2.46 (t, J = 5.5 Hz, 2H), 3.20 (m, 2H), 3.30-3.34 (m, 4H), 3.40 (t, J = 7.2 Hz, 2H), 4.63 (s, 2H), 7.10 (br s, 1H), 7.17-7.24 (m, 2H), 7.38 (s, 1H), 7.60 (d, J = 7.0 Hz, 1H), 7.68 (m, 1H), 7.85 (dd, J = 7.9, 2.0 Hz, 1H), 8.24 (d, J = 7.9 Hz, 1H), 8.52 (d, J = 2.0 Hz, 1H), 10.14 (s, 1H) |
| N-(2-t-Butoxycarbonylaminophenyl)-5-[3-propargyl-1-(2-dimethylaminoethyl)ureidomethyl]pyridine-2-carboxylic acid amide (Reference Compound No.7-29) 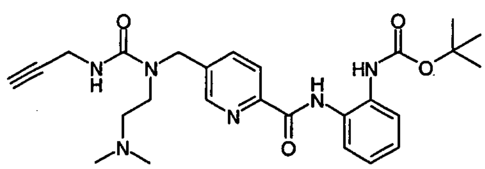 | $^1$H-NMR (500 MHz, $CDCl_3$) δ 1.52 (s, 9H), 2.20 (t, J = 2.5 Hz, 1H), 2.29 (s, 6H), 2.44 (t, J = 4.4 Hz, 2H), 3.22 (t, J = 4.4 Hz, 2H), 3.99 (dd, J = 4.9, 2.5 Hz, 2H), 4.61 (s, 2H), 7.09 (m, 1H), 7.18-7.24 (m, 2H), 7.59 (d, J = 6.7 Hz, 1H), 7.70 (d, J = 6.4 Hz, 1H), 7.85 (dd, J = 7.8, 2.1 Hz, 1H), 8.24 (dd, J = 7.8, 0.6 Hz, 1H), 8.51 (dd, J = 2.1, 0.6 Hz, 1H), 8.80 (s, 1H), 10.15 (s, 1H) |
| N-(2-t-Butoxycarbonylaminophenyl)-5-[1-(2-dimethylaminoethyl)-3-(2-methoxyethyl)ureidomethyl] pyridine-2-carboxylic acid amide (Reference Compound No.7-30) 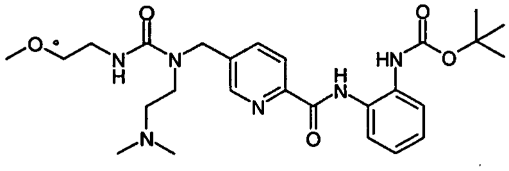 | $^1$H-NMR (400 MHz, $CDCl_3$) δ 1.52 (s, 9H), 2.26 (s, 6H), 2.42 (t, J = 4.7 Hz, 2H), 3.22 (t, J = 4.7 Hz, 2H), 3.36 (s, 3H), 3.41 (m, 2H), 3.49 (t, J = 4.9 Hz, 2H), 4.61 (s, 2H), 7.10 (br s, 1H), 7.17-7.25 (m, 2H), 7.60 (d, J = 7.3 Hz, 1H), 7.69 (m, 1H), 7.85 (dd, J = 8.0, 2.0 Hz, 1H), 8:10 (br s, 1H), 8.24 (d, J = 8.0 Hz, 1H), 8.51 (d, J = 2.0 Hz, 1H), 10.16 (s, 1H) |

(continued)

| | |
|---|---|
| N-(2-t-Butoxycarbonylaminophenyl)-5-[1-(2-dimethylaminoethyl)-3-(2-methylthioethyl)ureidomethyl] pyridine-2-carboxylic acid amide (Reference Compound No.7-31) 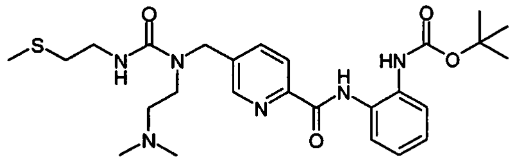 | $^1$H-NMR (400 MHz, $CDCl_3$) δ 1.52 (s, 9H), 2.13 (s, 3H), 2.28 (s, 6H), 2.43 (t, J = 4.7 Hz, 2H), 2.66 (t, J = 6.5 Hz, 2H), 3.23 (t, J = 4.7 Hz, 2H), 3.45 (m, 2H), 4.61 (s, 2H), 7.10 (br s, 1H), 7.17-7.24 (m, 2H), 7.59 (d, J = 7.1 Hz, 1H), 7.69 (m, 1H), 7.85 (dd, J = 8.0, 2.2 Hz, 1H), 8.11 (m, 1H), 8.24 (dd, J = 8.0, 0.6 Hz, 1H), 8.51 (dd, J = 2.2, 0.6 Hz, 1H), 10.16 (s, 1H) |
| N-(2-t-Butoxycarbonylaminophenyl)-5-[1-(2-dimethylaminoethyl)-3-methylaminocarbonylmethylureidomethyl]pyridine-2-carboxylic acid amide (Reference Compound No.7-32) 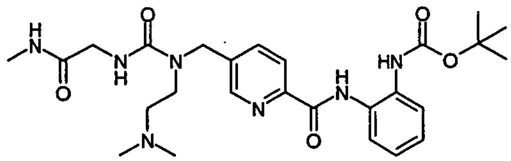 | $^1$H-NMR (400 MHz, $CDCl_3$) δ 1.52 (s, 9H), 2.46 (s, 6H), 2.67 (t, J = 4.2 Hz, 2H), 2.83 (d, J = 4.9 Hz, 3H), 3.44 (t, J = 4.2 Hz, 2H), 3.86 (d, J = 5.1 Hz, 2H), 4.65 (s, 2H), 6.76 (br s, 1H), 7.06 (br s, 1H), 7.18-7.23 (m, 2H), 7.54 (d, J = 6.8 Hz, 1H), 7.72 (m, 1H), 7.82 (dd, J = 7.9, 1.9 Hz, 1H), 7.97 (br s, 1H), 8.25 (d, J = 7.9 Hz, 1H), 8.50 (d, J = 1.9 Hz, 1H), 10.16 (s, 1H) |
| N-(2-t-Butoxycarbonylaminophenyl)-5-[3-cyclopropyl-1-(2-dimethylaminoethyl)ureidomethyl]pyridine-2-carboxylic acid amide (Reference Compound No.7-33) 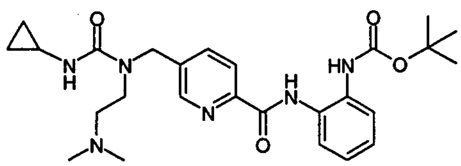 | $^1$H-NMR (400 MHz, $CDCl_3$) δ 0.38 (m, 2H), 0.70 (td, J = 6.8, 5.2 Hz, 2H), 1.52 (s, 9H), 2.24 (s, 6H), 2.38 (t, J = 4.5 Hz, 2H), 2.69 (m, 1H), 3.15 (t, J = 4.5 Hz, 2H), 4.59 (s, 2H), 7.07 (br s, 1H), 7.17-7.24 (m, 2H), 7.56 (d, J = 7.1 Hz, 1H), 7.69 (m, 1H), 7.86 (dd, J = 8.0, 2.2 Hz, 1H), 8.24 (dd, J = 8.0, 0.7 Hz, 1H), 8.28 (s, 1H), 8.51 (dd, J = 2.2, 0.7 Hz, 1H), 10.15 (s, 1H) |
| N-(2-t-Butoxycarbonylaminophenyl)-5-[1-(2-dimethylaminoethyl)-3-[2-(pyridin-4-yl)ethyl] ureidomethyl]pyridine-2-carboxylic acid amide (Reference Compound No.7-34) 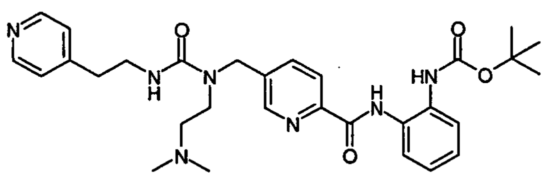 | $^1$H-NMR (400 MHz, $CDCl_3$) δ 1.51 (s, 9H), 2.06 (s, 6H), 2.33 (t, J = 4.5 Hz, 2H), 2.85 (t, J = 6.8 Hz, 2H), 3.14 (t, J = 4.5 Hz, 2H), 3.53 (m, 2H), 4.57 (s, 2H), 7.06 (br s, 1H), 7.16 (d, J = 6.1 Hz, 2H), 7.18-7.23 (m, 2H), 7.60 (d, J = 6.8 Hz, 1H), 7.73 (d, J = 6.6 Hz, 1H), 7.81 (dd, J = 7.9, 2.2 Hz, 1H), 7.92 (s, 1H), 8.24 (dd, J = 7.9, 0.6 Hz, 1H), 8.46 (dd, J = 2.2, 0.6 Hz, 1H), 8.52 (d, J = 6.1 Hz, 2H), 10.16 (s, 1H) |

(continued)

| | |
|---|---|
| N-(2-t-Butoxycarbonylaminophenyl)-5-[1-(2-dimethylaminoethyl)-3-[2-(indol-3-yl)ethyl]ureidomethyl] pyridine-2-carboxylic acid amide (Reference Compound No.7-35) 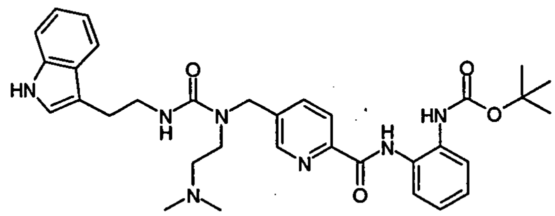 | $^1$H-NMR (400 MHz, $CDCl_3$) δ 1.52 (s, 9H), 1.97 (s, 6H), 2.28 (t, J = 5.0 Hz, 2H), 2.99 (t, J = 6.7 Hz, 2H), 3.13 (t, J = 5.0 Hz, 2H), 3.60 (m, 2H), 4.58 (s, 2H), 7.02 (d, J = 2.2 Hz, 1H), 7.07 (br s, 1H), 7.08 (m, 1H), 7.18 (dd, J = 8.1, 1.1 Hz, 1H), 7.20-7.24 (m, 2H), 7.35 (dt, J = 8.1, 1.1 Hz, 1H), 7.52 (m, 1H), 7.57 (m, 1H), 7.62 (d, J = 8.1 Hz, 1H), 7.72 (m, 1H), 7.77 (dd, J = 8.0, 2.2 Hz, 1H), 8.20 (dd, J = 8.0, 0.6 Hz, 1H), 8.27 (s, 1H), 8.46 (dd, J = 2.2, 0.6 Hz, 1H), 10.19 (s, 1H) |
| N-(2-t-Butoxycarbonylaminophenyl)-5-[1-[2-(4-methylpiperazin-1-yl)ethyl]-3-(1,3-thiazol-2-yl) ureidomethyl]pyridine-2-carboxylic acid amide (Reference Compound No.7-36) 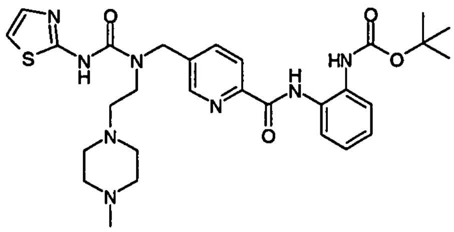 | $^1$H-NMR (400 MHz, $CDCl_3$) δ 1.51 (s, 9H), 2.38 (s, 3H), 2.60 (t, J = 4.1 Hz, 2H), 2.71 (br s, 4H), 2.76 (br s, 4H), 3.38 (t, J = 4.1 Hz, 2H), 4.71 (s, 2H), 6.88 (d, J = 3.6 Hz, 1H), 7.10 (br s, 1H), 7.18-7.25 (m, 2H), 7.39 (d, J = 3.6 Hz, 1H), 7.56 (m, 1H), 7.73 (m, 1H), 7.89 (dd, J = 8.0, 2.2 Hz, 1H), 8.27 (d, J = 8.0 Hz, 1H), 8.56 (d, J = 2.2 Hz, 1H), 10.18 (s, 1H) |
| N-(2-t-Butoxycarbonylaminophenyl)-5-[1-[3-(4-methylpiperazin-1-yl)propyl]-3-(1,3-thiazol-2-yl) ureidomethyl]pyridine-2-carboxylic acid amide (Reference Compound No.7-37) 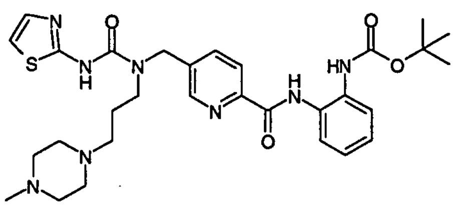 | $^1$H-NMR (400 MHz, $CDCl_3$) δ 1.51 (s, 9H), 1.80 (m, 2H), 2.40 (s, 3H), 2.46 (t, J = 5.8 Hz, 2H), 2.56 (br s, 4H), 2.77 (br s, 4H), 3.39 (t, J = 5.8 Hz, 2H), 4.67 (s, 2H), 6.87 (d, J = 3.7 Hz, 1H), 7.11 (m, 1H), 7.18-7.25 (m, 2H), 7.37 (d, J = 3.7 Hz, 1H), 7.57 (d, J = 8.8 Hz, 1H), 7.72 (m, 1H), 7.90 (dd, J = 8.0, 2.2 Hz, 1H), 8.26 (d, J = 8.0 Hz, 1H), 8.57 (d, J = 2.2 Hz, 1H), 10.18 (s, 1H) |
| N-(2-t-Butoxycarbonylaminophenyl)-5-[1-[4-(morpholin-4-yl)butyl]-3-(1,3-thiazol-2-yl)ureidomethyl]pyridine-2-carboxylic acid amide (Reference Compound No.7-38) 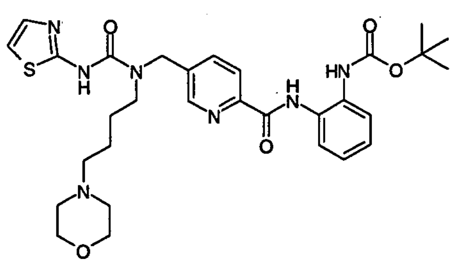 | $^1$H-NMR (400 MHz, $CDCl_3$) δ 1.51 (s, 9H), 1.52 (m, 2H), 1.70 (m, 2H), 2.39 (t, J = 6.8 Hz, 2H), 2.47 (t, J = 4.8 Hz, 4H), 3.36 (t, J = 8.0 Hz, 2H), 3.77 (t, J = 4.8 Hz, 4H), 4.73 (s, 2H), 6.88 (d, J = 3.6 Hz, 1H), 7.08 (br s, 1H), 7.18-7.25 (m, 2H), 7.33 (d, J = 3.6 Hz, 1H), 7.58 (m, 1H), 7.73 (m, 1H), 7.86 (dd, J = 8.0, 2.2 Hz, 1H), 8.28 (d, J = 8.0 Hz, 1H), 8.54 (d, J = 2.2 Hz, 1H), 10.16 (s, 1H) |

(continued)

| | |
|---|---|
| N-(2-t-Butoxycarbonylaminophenyl)-5-[1-[3-(4-methylpiperidin-1-yl)propyl]-3-(1,3-thiazol-2-yl)ureidomethyl]pyridine-2-carboxylic acid amide (Reference Compound No.7-39) 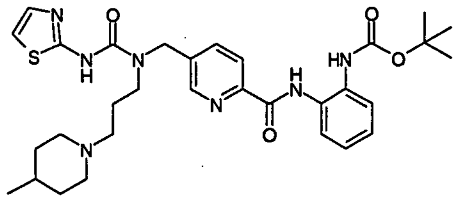 | $^1$H-NMR (400 MHz, $CDCl_3$) δ 1.01 (d, J = 6.6 Hz, 3H), 1.44 (m, 1H), 1.51 (s, 9H), 1.57 (m, 2H), 1.74-1.86 (m, 4H), 2.01 (t, J = 11.7 Hz, 2H), 2.40 (t, J = 6.0 Hz, 2H), 2.90 (d, J = 11.7 Hz, 2H), 3.39 (t, J = 5.6 Hz, 2H), 4.66 (s, 2H), 6.86 (d, J = 3.7 Hz, 1H), 7.03 (br s, 1H), 7.18-7.25 (m, 2H), 7.36 (d, J = 3.7 Hz, 1H), 7.59 (d, J = 7.3 Hz, 1H), 7.71 (d, J = 6.6 Hz, 1H), 7.91 (dd, J = 8.0, 2.0 Hz, 1H), 8.27 (d, J = 8.0 Hz, 1H), 8.57 (d, J = 2.0 Hz, 1H), 10.16 (s, 1H) |
| N-(2-t-Butoxycarbonylaminophenyl)-5-[1-[3-(morpholin-4-yl)propyl]-3-(1,3-thiazol-2-yl)ureidomethyl]pyridine-2-carboxylic acid amide (Reference Compound No.7-40) 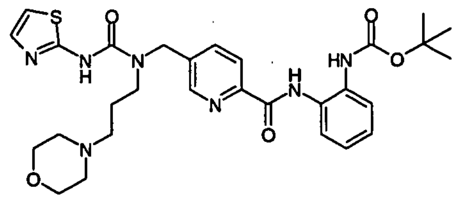 | $^1$H-NMR (500 MHz, $CDCl_3$) δ 1.51 (s, 9H), 1.80 (m, 2H), 2.46 (t, J = 6.1 Hz, 2H), 2.51 (br s, 4H), 3.40 (t, J = 5.7 Hz, 2H), 4.04 (br s, 4H), 4.68 (s, 2H), 6.87 (d, J = 3.7 Hz, 1H), 7.01 (br s, 1H), 7.18-7.24 (m, 2H), 7.37 (d, J = 3.7 Hz, 1H), 7.58 (d, J = 6.1 Hz, 1H), 7.71 (d, J = 6.1 Hz, 1H), 7.91 (dd, J = 8.1, 2.2 Hz, 1H), 8.27 (dd, J = 8.1, 0.8 Hz, 1H), 8.58 (dd, J = 2.2, 0.8 Hz, 1H), 10.16 (s, 1H), 11.67 (br s, 1H) |
| N-(2-t-Butoxycarbonylaminophenyl)-5-[1-[3-(morpholin-4-yl)propyl]-3-(5-nitro-1,3-thiazol-2-yl)ureidomethyl]pyridine-2-carboxylic acid amide (Reference Compound No.7-41) 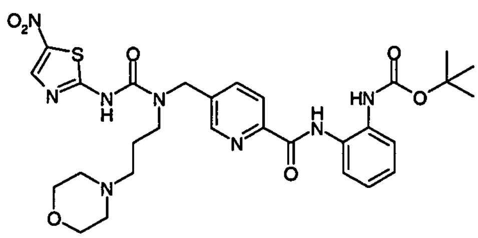 | $^1$H-NMR (500 MHz, $CDCl_3$) δ 1.52 (s, 9H), 1.85 (m, 2H), 2.49 (t, J = 6.1 Hz, 2H), 2.54 (br s, 4H), 3.40 (t, J = 5.7 Hz, 2H), 4.01 (br s, 4H), 4.66 (s, 2H), 6.96 (br s, 1H), 7.19-7.25 (m, 2H), 7.55 (br s, 1H), 7.75 (br s, 1H), 7.90 (dd, J = 7.9, 2.0 Hz, 1H), 8.26 (s, 1H), 8.29 (d, J = 7.9 Hz, 1H), 8.60 (d, J = 2.0 Hz, 1H), 10.19 (s, 1H) |
| N-(2-t-Butoxycarbonylaminophenyl)-5-[3-(1-methylpiperidin-4-yl)-1-[3-(morpholin-4-yl)propyl]ureidomethyl]pyridine-2-carboxylic acid amide (Reference Compound No.7-42) 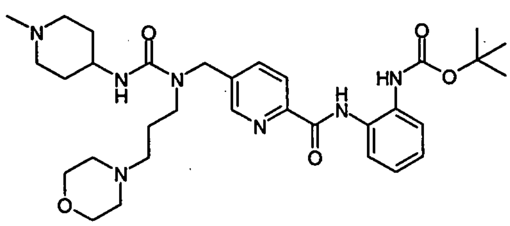 | $^1$H-NMR (400 MHz, $CDCl_3$) δ 1.42-1.55 (m, 2H), 1.52 (s, 9H), 1.70 (m, 2H), 1.96 (d, J = 12.0 Hz, 2H), 2.03-2.12 (m, 2H), 2.27 (s, 3H), 2.37 (t, J = 6.2 Hz, 2H), 2.44 (t, J = 4.5 Hz, 4H), 2.84 (d, J = 12.0 Hz, 2H), 3.22 (t, J = 6.1 Hz, 2H), 3.66 (m, 1H), 3.75 (t, J = 4.5 Hz, 4H), 4.57 (s, 2H), 5.84 (d, J = 7.6 Hz, 1H), 7.06 (br s, 1H), 7.17-7.26 (m, 2H), 7.60 (d, J = 7.3 Hz, 1H), 7.69 (d, J = 7.1 Hz, 1H), 7.85 (dd, J = 8.0, 2.1 Hz, 1H), 8.25 (dd, J = 8.0, 0.7 Hz, 1H), 8.52 (dd, J = 2.1, 0.7 Hz, 1H), 10.14 (s, 1H) |

(continued)

| | |
|---|---|
| N-(2-t-Butoxycarbonylaminophenyl)-5-[1-(2-dimethylaminoethyl)-3-(5,5-dimethyl-4,5-dihydro-1,3-thiazol-4-on-2-yl)ureidomethyl]pyridine-2-carboxylic acid amide (Reference Compound No.7-43) 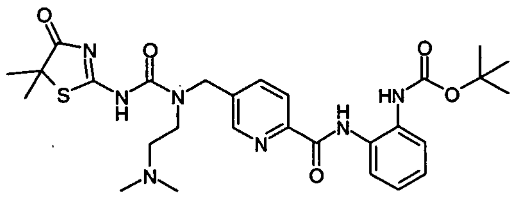 | $^1$H-NMR (400 MHz, $CDCl_3$) δ 1.52 (s, 9H), 2.25 (s, 6H), 2.29 (s, 6H), 2.52 (t, J = 5.7 Hz, 2H), 3.49 (t, J = 5.7 Hz, 2H), 4.71 (s, 2H), 7.09 (br s, 1H), 7.19-7.24 (m, 2H), 7.57 (d, J = 7.1 Hz, 1H), 7.77 (m, 1H), 7.86 (dd, J = 8.0, 2.0 Hz, 1H), 8.19 (d, J = 8.0 Hz, 1H), 8.44 (s, 1H), 8.54 (d, J = 2.0 Hz, 1H), 10.19 (s, 1H) |
| N-(2-t-Butoxycarbonylaminophenyl)-5-[3-(4,5-dihydro-1,3-thiazol-2-yl)-1-(2-dimethylaminoethyl)ureidomethyl] pyridine-2-carboxylic acid amide (Reference Compound No.7-44) 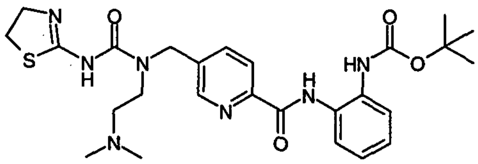 | $^1$H-NMR (400 MHz, $CDCl_3$) δ 1.52 (s, 9H), 2.24 (s, 6H), 2.46 (m, 2H), 3.23 (t, J = 7.6 Hz, 2H), 3.44 (m, 1H), 3.58 (m, 1H), 3.75 (br s, 2H), 4.70 (br s, 1H), 4.88 (br s, 1H), 7.04 (br s, 1H), 7.17-7.25 (m, 2H), 7.59 (m, 1H), 7.69 (m, 1H), 7.81 (m, 1H), 8.22 (d, J = 7.8 Hz, 1H), 8.50 (m, 1H), 10.17 (m, 1H) |
| 5-[3-(Bicyclo[2,2,1]heptan-2-yl)-1-(2-dimethylaminoethyl)ureidomethyl]-N-(2-t-butoxycarbonylaminophenyl)pyridine-2-carboxylic acid amide (Reference Compound No.7-45) 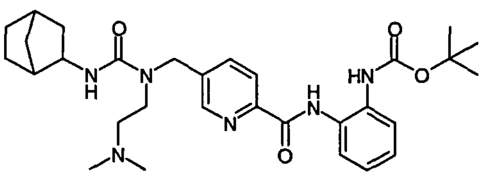 | $^1$H-NMR (400 MHz, $CDCl_3$) δ 0.69 (m, 1H), 1.16 (m, 1H), 1.32 (m, 1H), 1.44 (m, 2H), 1.52 (s, 9H), 1.58 (m, 2H), 2.08 (m, 1H), 2.18 (m, 1H), 2.28 (s, 6H), 2.41 (m, 1H), 2.45 (m, 2H), 3.20 (m, 2H), 4.02 (m, 1H), 4.53 (d, J = 15.6 Hz, 1H), 4.64 (d, J = 15.6 Hz, 1H), 7.06 (br s, 1H), 7.17-7.24 (m, 2H), 7.60 (d, J = 7.3 Hz, 1H), 7.68 (d, J = 6.9 Hz, 1H), 7.86 (dd, J = 8.0, 2.2 Hz, 1H), 8.05 (d, J = 7.1 Hz, 1H), 8.24 (dd, J = 8.0, 0.7 Hz, 1H), 8.51 (dd, J = 2.2, 0.7 Hz, 1H), 10.14 (s, 1H) |
| N-(2-t-Butoxycarbonylaminophenyl)-5-[1-(2-dimethylaminoethyl)-3-(5-nitro-1,3-thiazol-2-yl) ureidomethyl]pyridine-2-carboxylic acid amide (Reference Compound No.7-46) 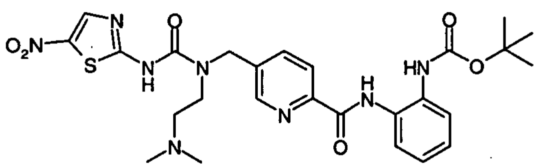 | $^1$H-NMR (400 MHz, $CDCl_3$) δ 1.52 (s, 9H), 2.48 (s, 6H), 2.67 (t, J = 4.4 Hz, 2H), 3.39 (t, J = 4.4 Hz, 2H), 4.70 (s, 2H), 7.04 (s, 1H), 7.18-7.24 (m, 2H), 7.54 (m, 1H), 7.75 (m, 1H), 7.87 (dd, J = 8.0, 1.8 Hz, 1H), 8.26 (s, 1H), 8.28 (d, J = 8.0 Hz, 1H), 8.56 (d, J = 1.8 Hz, 1H), 10.19 (s, 1H) |

(continued)

| | |
|---|---|
| N-(2-t-Butoxycarbonylaminophenyl)-5-[1-[5-(morpholin-4-yl)pentyl]-3-(pyrrolidin-1-yl)ureidomethyl]pyridine-2-carboxylic acid amide (Reference Compound No.7-47) 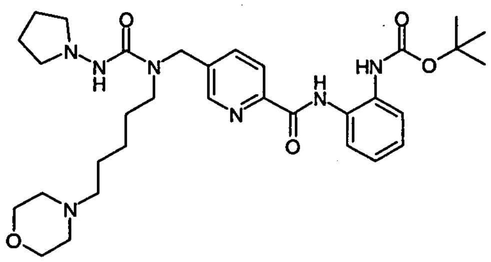 | 1H-NMR (400 MHz; $CDCl_3$) δ 1.30 (m, 2H), 1.48 (m, 2H), 1.52 (s, 9H), 1.67 (m, 2H), 1.76-1.81 (m, 4H), 2.30 (t, J = 7.7 Hz, 2H), 2.41 (t, J = 4.4 Hz, 4H), 2.80-2.85 (m, 4H), 3.20 (t, J = 7.7 Hz, 2H), 3.71 (t, J = 4.4 Hz, 4H), 4.61 (s, 2H), 7.06 (br s, 1H), 7.20-7.23 (m, 2H), 7.57 (d, J = 6.8 Hz, 1H), 7.73 (m, 1H), 7.83 (dd, J = 8.0, 2.1 Hz, 1H), 8.26 (dd, J = 8.0, 0.5 Hz, 1H), 8.51 (dd, J = 2.1, 0.5 Hz, 1H), 10.17 (s, 1H) |
| N-(2-t-Butoxycarbonylaminophenyl)-5-[1-[3-(morpholin-4-yl)propyl]-3-(pyrrolidin-1-yl)ureidomethyl]pyridine-2-carboxylic acid amide (Reference Compound No.7-48) 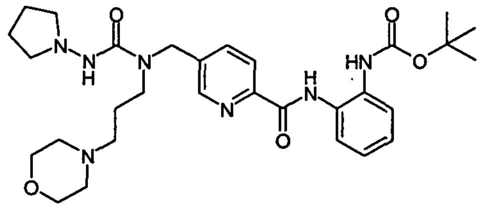 | $^1$H-NMR (400 MHz, $CDCl_3$) δ 1.52 (s, 9H), 1.71 (m, 2H), 1.86 (br s, 4H), 2.38 (t, J = 6.1 Hz, 2H), 2.48 (br s, 4H), 2.99 (br s, 4H), 3.20 (t, J = 6.0 Hz, 2H), 3.78 (t, J = 4.6 Hz, 4H), 4.56 (s, 2H), 7.06 (br s, 1H), 7.17-7.26 (m, 2H), 7.60 (d, J = 6.8 Hz, 1H), 7.69 (d, J = 7.3 Hz, 1H), 7.73 (br s, 1H), 7.92 (dd, J = 8.0, 2.2 Hz, 1H), 8.25 (dd, J = 8.0, 0.7 Hz, 1H), 8.54 (dd, J = 2.2, 0.7 Hz, 1H), 10.14 (s, 1H) |
| N-(2-t-Butoxycarbonylaminophenyl)-5-[3-(morpholin-4-yl)-1-[3-(morpholin-4-yl)propyl]ureidomethyl]pyridine-2-carboxylic acid amide (Reference Compound No.7-49) 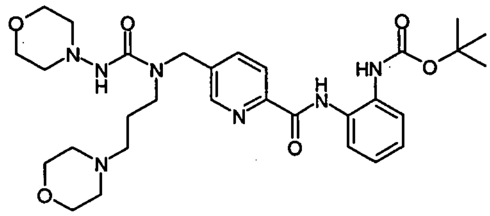 | $^1$H-NMR (400 MHz, $CDCl_3$) δ 1.52 (s, 9H), 1.72 (m, 2H), 2.39 (t, J = 5.9 Hz, 2H), 2.49 (br s, 4H), 2.97 (t, J = 4.8 Hz, 4H), 3.21 (t, J = 5.7 Hz, 2H), 3.80 (t, J = 4.8 Hz, 4H), 3.82 (t, J = 4.8 Hz, 4H), 4.55 (s, 2H), 7.05 (br s, 1H), 7.17-7.25 (m, 2H), 7.60 (d, J = 7.3 Hz, 1H), 7.69 (d, J = 7.3 Hz, 1H), 7.91 (dd, J = 8.0, 2.0 Hz, 1H), 7.97 (s, 1H), 8.25 (dd, J = 8.0, 0.5 Hz, 1H), 8.54 (dd, J = 2.0, 0.5 Hz, 1H), 10.14 (s, 1H) |

Reference Example 8

6-Methylpyridine-3-carboxylic acid methyl ester N-oxide (Reference Compound No.8-1)

30% Hydrogen peroxide in water (5.7 mL, 50 mmol) and sodium tungstate dihydrate (270 mg, 0.80 mmol) were added to 6-methylpyridine-3-carboxylic acid methyl ester (3.0 g, 20 mmol). The reaction mixture was stirred at 60°C for 70 minutes and at 80°C for 4.5 hours. Under ice cooling, methanol (10 mL) and manganese oxide (0.51 g, 6.0 mmol) were added thereto, and then the reaction mixture was stirred at room temperature for 16 hours. After the insoluble was filtered off with celite, the filtrate was concentrated to give 3.2 g of the title reference compound as a pale brown solid. (Yield 98%)

| | |
|---|---|
| 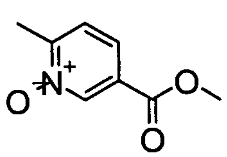 | $^1$H-NMR (400 MHz, DMSO-$d_6$) δ 2.42 (s, 3H), 3.88 (s, 3H), 7.64 (d, J = 8.2 Hz, 1H), 7.72 (dd, J = 8.2, 1.5 Hz, 1H), 8.58 (d, J = 1.5 Hz, 1H) |

Reference Example 9

6-Acetoxymethylpyridine-3-carboxylic acid methyl ester (Reference Compound No.9-1)

N,N-Diisopropylethylamine (2.2 mL, 13 mmol) and acetic anhydride (3.5 mL, 38 mmol) were added to a solution of 6-methylpyridine-3-carboxylic acid methyl ester N-oxide (Reference Compound No.8-1, 2.1 g, 13 mmol) in acetic acid (14 mL), and then the reaction mixture was stirred at 120˚C for 135 minutes. After cooling, saturated aqueous sodium hydrogen carbonate solution (100 mL) was added thereto, and then sodium hydrogen carbonate was added until foam formation was ended. The whole was extracted with ethyl acetate (100 mL, 50 mL), the organic layer was dried over anhydrous magnesium sulfate, and then the solvent was evaporated under reduced pressure. The residue was purified by silica gel column chromatography (hexane-ethyl acetate), and then the resulting solid was collected by filtration with hexane and ethyl acetate to give 1.2 g of the title reference compound as a pale yellow solid. (Yield 48%)

| | |
|---|---|
| 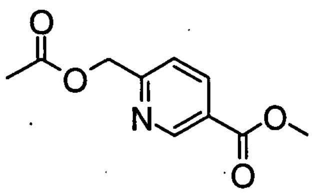 | $^1$H-NMR (400 MHz, DMSO-$d_6$)<br>δ 2.15 (s, 3H), 3.89 (s, 3H), 5.23 (s, 2H), 7.57 (dd, J = 8.1, 0.7 Hz, 1H), 8.32 (dd, J = 8.1, 2.1 Hz, 1H), 9.05 (dd, J = 2.1, 0.7 Hz, 1H) |

Reference Example 10

6-Hydroxymethylpyridine-3-carboxylic acid (Reference Compound No.10-1)

Under ice cooling, 2 M aqueous sodium hydroxide solution (3.0 mL) was added to a solution of 6-acetoxymethylpyridine-3-carboxylic acid methyl ester (Reference Compound No.9-1, 0.60 g, 2.9 mmol) in methanol (6.0 mL). The reaction mixture was stirred under ice cooling for 40 minutes and at room temperature for 4 hours. Under ice cooling, 2 M hydrochloric acid (3.1 mL) was added thereto, and then the solvent was evaporated under reduced pressure to give the title reference compound as an orange solid.

| | |
|---|---|
| 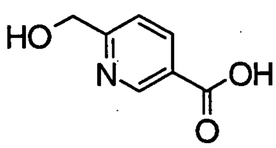 | $^1$H-NMR (400 MHz, DMSO-$d_6$)<br>δ 4.64 (s, 2H), 5.31 (br s, 1H), 7.63 (d, J = 8.2 Hz, 1H), 8.30 (dd, J = 8.2, 2.2 Hz, 1H), 8.97 (d, J = 2.2 Hz, 1H), 13.34 (s, 1H) |

Reference Example 11

N-(2-t-Butoxycarbonylaminophenyl)-6-hydroxymethylpyridine-3-carboxylic acid amide (Reference Compound No.11-1)

2-Aminophenyl carbamic acid t-butyl ester (Reference Compound No.1-1, 0.60 g, 2.9 mmol), N,N-diisopropylethylamine (1.5 mL, 8.6 mmol), and HATU (1.1 g, 2.9 mmol) were added to a suspension of 6-hydroxymethylpyridine-3-carboxylic acid (Reference Compound No. 10-1) in DMF (10 mL), and then the reaction mixture was stirred at room temperature for 2 hours. Water (200 mL) was added thereto, the whole was extracted with ethyl acetate (100 mL, 50 mL), and then the organic layer was washed with brine (100 mL) twice. After the organic layer was dried over anhydrous magnesium sulfate, the solvent was evaporated under reduced pressure. The residue was purified by silica gel column chromatography (hexane-ethyl acetate) to give 0.66 g of the title reference compound as a brown oil. (Yield 67% in 2 steps)

| | |
|---|---|
| 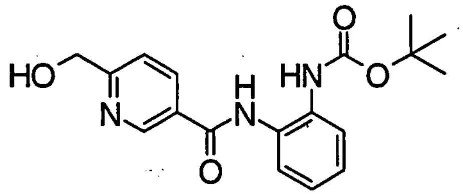 | $^1$H-NMR (400 MHz, DMSO-$d_6$)<br>δ 1.44 (s, 9H), 4.65 (d, J = 5.9 Hz, 2H), 5.59 (t, J = 5.9 Hz, 1H), 7.14 (td, J = 7.7, 1.5 Hz, 1H), 7.21 (td, J = 7.7, 1.5 Hz, 1H), 7.52 (dd, J = 7.7, 1.5 Hz, 1H), 7.59 (dd, J = 7.7, 1.5 Hz, 1H), 7.63 (d, J = 8.3 Hz, 1H), 8.31 (dd, J = 8.3, 2.1 Hz, 1H), 8.69 (s, 1H), 9.03 (d, J = 2.1 Hz, 1H), 9.94 (s, 1H) |

Example 1

N-(2-Aminophenyl)-5-[1-(3-dimethylaminopropyl)-3-phenethylureidomethyl]pyridine-2-carbo xylic acid amide (Compound No.1-1)

4.0 M hydrogen chloride-ethyl acetate solution (1.0 mL) was added to a solution of N-(2-t-butoxycarbonylaminophenyl)-5-[1-(3-dimethylaminopropyl)-3-phenethylureidomethyl] pyridine-2-carboxylic acid amide (Reference Compound No.6-1, 31 mg, 0.060 mmol) in methanol (2.0 mL), and then the reaction mixture was stirred at room temperature for 4 hours. Saturated aqueous sodium hydrogen carbonate solution (30 mL) was added thereto, and then the whole was extracted with ethyl acetate (30 mL) three times. After the organic layer was dried over anhydrous magnesium sulfate, the solvent was evaporated under reduced pressure. The residue was purified by silica gel column chromatography (chloroform-methanol) to give 24 mg of the title compound as a yellow amorphous product. (Yield 84%)

| | |
|---|---|
| 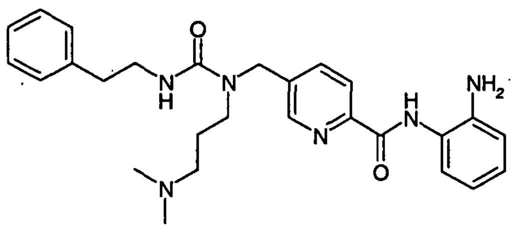 | $^1$H-NMR (500 MHz, DMSO-$d_6$)<br>δ 1.56 (m, 2H), 2.08 (s, 6H), 2.17 (br s, 2H), 2.74 (t, J = 7.3 Hz, 2H), 3.14 (t, J = 6.6 Hz, 2H), 3.31 (m, 2H), 4.50 (s, 2H), 4.90 (br s, 2H), 6.66 (td, J = 7.8, 1.2 Hz, 1H), 6.83 (dd, J = 7.8, 1.2 Hz, 1H), 6.95 (td, J = 7.8, 1.2 Hz, 1H), 7.12 (br s, 1H), 7.18-7.22 (m, 3H), 7.27-7.31 (m, 2H), 7.51 (dd, J = 7.8, 1.2 Hz, 1H), 7.82 (dd, J = 7.9, 2.0 Hz, 1H), 8.09 (d, J = 7.9 Hz, 1H), 8.56 (d, J = 7.9 Hz, 1H), 10.03 (br s, 1H) |

By using any compounds selected from Reference Compounds No.6-2~6-141 and Neo.7-1~7-49, the following Compounds No.1-2~1-190 were obtained by a method similar to that of Compound No.1-1.

| | |
|---|---|
| N-(2-Aminophenyl)-5-[3-(2,3-dihydrobenzo[1,4]dioxin-6-yl)-1-(3-dimethylaminopropyl)ureidomethyl]pyridine-2-carboxylic acid amide (Compound No.1-2)<br>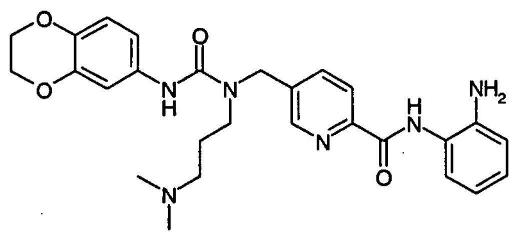 | $^1$H-NMR (500 MHz, DMSO-$d_6$)<br>δ 1.69 (m, 2H), 2.18 (s, 6H), 2.26 (t, J = 6.3 Hz, 2H), 3.34 (m, 2H), 4.16-4.21 (m, 4H), 4.61 (s, 2H), 4.89 (br s, 2H), 6.65 (td, J = 7.8, 1.2 Hz, 1H), 6.73 (d, J = 8.9 Hz, 1H), 6.78 (dd, J = 8.9, 2.4 Hz, 1H), 6.82 (dd, J = 7.8, 1.2 Hz, 1H), 6.95 (td, J = 7.8, 1.2 Hz, 1H), 7.07 (d, J = 2.4 Hz, 1H), 7.51 (dd, J = 7.8, 1.2 Hz, 1H), 7.94 (dd, J = 7.9, 2.1 Hz, 1H), 8.11 (d, J = 7.9 Hz, 1H), 8.65 (d, J = 2.1 Hz, 1H), 9.43 (br s, 1H), 10.02 (br s, 1H) |
| N-(2-Aminophenyl)-5-[1-[3-(morpholin-4-yl)propyl]-3-phenethylureidomethyl]pyridine-2-carboxylic acid amide (Compound No.1-3) | $^1$H-NMR (400 MHz, DMSO-$d_6$)<br>δ 1.59 (m, 2H), 2.21 (t, J = 6.6 Hz, 2H), 2.25 (br s, 4H), 2.75 (t, J = 7.2 Hz, 2H), 3.14 |

| | |
|---|---|
| 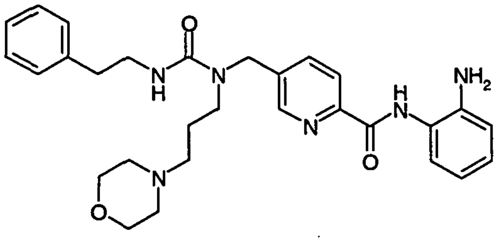 | (t, J = 6.6 Hz, 2H), 3.34 (m, 2H), 3.50-3.52 (m, 4H), 4.56 (s, 2H), 4.89 (br s, 2H), 6.66 (td, J = 7.8, 1.2 Hz, 1H), 6.83 (dd, J = 7.8, 1.2 Hz, 1H), 6.90 (br s, 1H), 6.95 (td, J = 7.8, 1.2 Hz, 1H), 7.18-7.22 (m, 3H), 7.27-7.31 (m, 2H), 7.51 (dd, J = 7.8, 1.2 Hz, 1H), 7.82 (dd, J = 8.1, 2.0 Hz, 1H), 8.10 (d, J = 8.1 Hz, 1H), 8.57 (br s, 1H), 10.05 (br s, 1H) |
| N-(2-Aminophenyl)-5-[3-(3,4-difluorophenyl)-1-(3-dimethylaminopropyl)ureidomethyl]pyridine-2-carboxylic acid amide (Compound No.1-4) 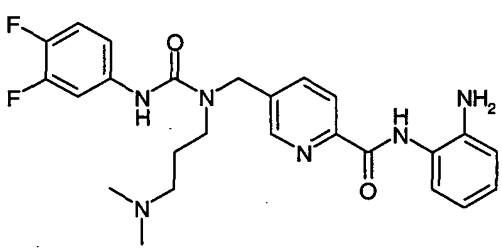 | $^1$H-NMR (500 MHz, DMSO-$d_6$) δ 1.71 (m, 2H), 2.19 (s, 6H), 2.27 (t, J = 6.4 Hz, 2H), 3.36 (t, J = 6.4 Hz, 2H), 4.63 (s, 2H), 4.89 (br s, 2H), 6.65 (td, J = 7.6, 1.4 Hz, 1H), 6.82 (d, J = 7.6 Hz, 1H), 6.95 (td, J = 7.6, 1.4 Hz, 1H), 7.09 (m, 1H), 7.31 (dd, J = 19.9, 9.2 Hz, 1H), 7.51 (d, J = 7.6 Hz, 1H), 7.68 (ddd, J = 13.7, 7.6, 2.4 Hz, 1H), 7.96 (dd, J = 7.9, 1.8 Hz, 1H), 8.10 (d, J = 7.9 Hz, |

| | |
|---|---|
| | 1H), 8.66 (d, J = 1.8 Hz, 1H), 9.93 (br s, 1H), 10.02 (br s, 1H) |
| N-(2-Aminophenyl)-5-[3-(benzo[1,3]dioxol-5-yl)-1-(3-dimethylaminopropyl)ureidomethyl]pyridine-2-carboxylic acid amide (Compound No.1-5) 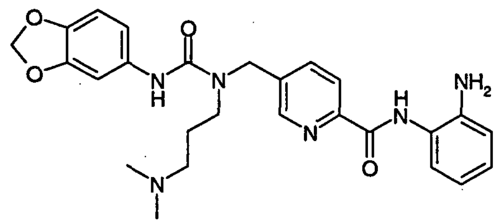 | $^1$H-NMR (500 MHz, DMSO-d$_6$) δ 1.70 (m, 2H), 2.18 (s, 6H), 2.27 (t, J = 6.3 Hz, 2H), 3.33 (m, 2H), 4.62 (s, 2H), 4.89 (br s, 2H), 5.95 (s, 2H), 6.65 (t, J = 7.6, Hz, 1H), 6.73 (dd, J = 8.6, 2.0 Hz, 1H), 6.80 (d, J = 8.6 Hz, 1H), 6.82 (d, J = 7.6 Hz, 1H), 6.95 (td, J = 7.6, 1.2 Hz, 1H), 7.19 (d, J = 2.0 Hz, 1H), 7.51 (d, J = 7.6 Hz, 1H), 7.95 (dd, J = 7.9, 2.1 Hz, 1H), 8.12 (d, J = 7.9 Hz, 1H), 8.65 (d, J = 2.1 Hz, 1H), 9.51 (br s, 1H), 10.02 (br s, 1H) |
| N-(2-Aminophenyl)-5-[1-(3-dimethylaminopropyl)-3-(4-methoxyphenyl)ureidomethyl]pyridine-2-carboxylic acid amide (Compound No.1-6) | $^1$H-NMR (400 MHz, DMSO-d$_6$) δ 1.70 (m, 2H), 2.18 (s, 6H), 2.27 (t, J = 6.3 Hz, 2H), 3.34 (m, 2H), 3.70 (s, 3H), 4.62 (s, 2H), 4.90 (br s, 2H), 6.65 (td, J = 7.6, 1.2 Hz, 1H), 6.83 (m, 1H), 6.84 (d, J = 9.0 Hz, |

| | |
|---|---|
| 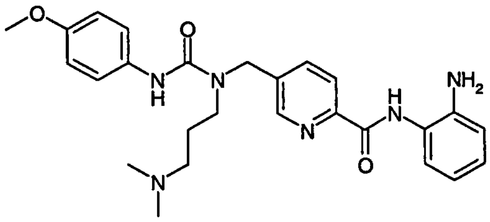 | 2H), 6.95 (td, J = 7.6, 1.2 Hz, 1H), 7.33 (d, J = 9.0 Hz, 2H), 7.51 (dd, J = 7.6, 1.2 Hz, 1H), 7.95 (dd, J = 8.1, 1.8 Hz, 1H), 8.11 (d, J = 8.1 Hz, 1H), 8.66 (d, J = 1.8 Hz, 1H), 9.43 (br s, 1H), 10.03 (br s, 1H) |
| N-(2-Aminophenyl)-5-[3-(benzo[1,3]dioxol-5-yl)-1-(3-diethylaminopropyl)ureidomethyl]pyridine-2-carboxylic acid amide (Compound No.1-7) 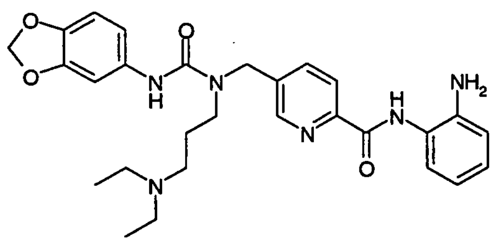 | $^1$H-NMR (500 MHz, DMSO-$d_6$) δ 0.93 (t, J = 7.2 Hz, 6H), 1.68 (m, 2H), 2.38 (m, 2H), 2.49 (m, 4H), 3.34 (m, 2H), 4.64 (s, 2H), 4.89 (br s, 2H), 5.95 (s, 2H), 6.65 (td, J = 7.6, 1.2 Hz, 1H), 6.75 (dd, J = 8.2, 2.1 Hz, 1H), 6.80-6.83 (m, 2H), 6.95 (td, J = 7.6, 1.2 Hz, 1H), 7.14 (d, J = 2.1 Hz, 1H), 7.50 (dd, J = 7.6, 1.2 Hz, 1H), 7.94 (dd, J = 8.1, 2.0 Hz, 1H), 8.11 (d, J = 8.1 Hz, 1H), 8.64 (d, J = 2.0 Hz, 1H), 8.98 (br s, 1H), 10.03 (br s, 1H) |
| N-(2-Aminophenyl)-5-[1-[3-(morpholin-4-yl)propyl]-3-(pyridin-3-yl)ureidomethyl]pyridine-2- | $^1$H-NMR (500 MHz, DMSO-$d_6$) δ 1.72 (m, 2H), 2.29-2.32 (m, 6H), 3.41 (t, J |

| | |
|---|---|
| carboxylic acid amide (Compound No.1-8)<br>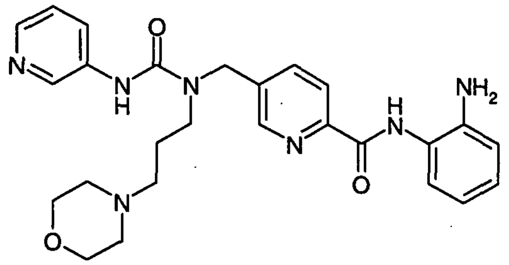 | = 7.0 Hz, 2H), 3.54-3.55 (m, 4H), 4.72 (s, 2H), 4.89 (br s, 2H), 6.65 (td, J = 7.9, 1.2 Hz, 1H), 6.82 (dd, J = 7.9, 1.2 Hz, 1H), 6.95 (td, J = 7.9, 1.2 Hz, 1H), 7.30 (ddd, J = 8.2, 4.6, 0.6 Hz, 1H), 7.50 (dd, J = 7.9, 1.2 Hz, 1H), 7.91 (ddd, J = 8.2, 2.7, 1.5 Hz, 1H), 7.95 (dd, J = 7.9, 1.8 Hz, 1H), 8.13 (d, J = 7.9 Hz, 1H), 8.19 (dd, J = 4.6, 1.5 Hz, 1H), 8.65 (d, J = 1.8 Hz, 1H), 8.66 (d, J = 2.7 Hz, 1H), 8.81 (br s, 1H), 10.03 (br s, 1H) |
| N-(2-Aminophenyl)-5-[1-(3-dimethylaminopropyl)-3-(2-fluorophenyl)ureidomethyl]pyridine-2-carboxylic acid amide (Compound No.1-9)<br>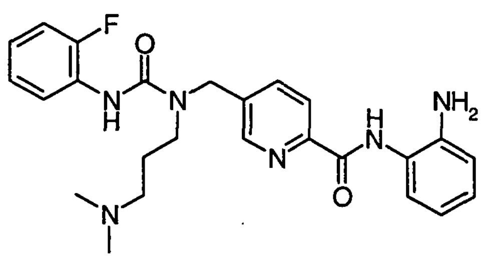 | $^1$H-NMR (500 MHz, DMSO-$d_6$)<br>δ 1.75 (m, 2H), 2.16 (s, 6H), 2.28 (t, J = 6.1 Hz, 2H), 3.37 (t, J = 5.7 Hz, 2H), 4.62 (s, 2H), 4.90 (s, 2H), 6.65 (m, 1H), 6.82 (dd, J = 7.9, 1.2 Hz, 1H), 6.95 (m, 1H), 7.04 (ddd, J = 11.3, 5.6, 1.5 Hz, 1H), 7.11 (t, J = 7.9 Hz, 1H), 7.20 (ddd, J = 11.3, 7.9, 1.5 Hz, 1H), 7.51 (d, J = 7.9 Hz, 1H), 7.90 (t, J = 7.9 Hz, |

| | |
|---|---|
| | 1H), 7.97 (dd, J = 7.9, 2.1 Hz, 1H), 8.12 (d, J = 7.9 Hz, 1H), 8.68 (d, J = 2.1 Hz, 1H), 9.90 (s, 1H), 10.03 (s, 1H) |
| N-(2-Aminophenyl)-5-[1-(3-dimethylaminopropyl)-3-(3-fluorophenyl)ureidomethyl]pyridine-2-carboxylic acid amide (Compound No.1-10) 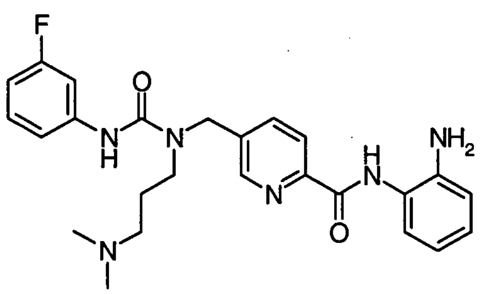 | $^1$H-NMR (500 MHz, DMSO-d$_6$) δ 1.72 (m, 2H), 2.21 (s, 6H), 2.29 (t, J = 6.3 Hz, 2H), 3.37 (t, J = 6.0 Hz, 2H), 4.63 (s, 2H), 4.89 (s, 2H), 6.65 (td, J = 7.9, 1.2 Hz, 1H), 6.75 (td, J = 8.3, 2.5 Hz, 1H), 6.82 (dd, J = 7.9, 1.2 Hz, 1H), 6.95 (td, J = 7.9, 1.2 Hz, 1H), 7.08 (dd, J = 8.3, 1.2 Hz, 1H), 7.27 (dd, J = 15.3, 8.3 Hz, 1H), 7.47-7.52 (m, 2H), 7.97 (dd, J = 7.9, 1.8 Hz, 1H), 8.12 (d, J = 7.9 Hz, 1H), 8.67 (d, J = 1.8 Hz, 1H), 10.03 (s, 2H) |
| N-(2-Aminophenyl)-5-[3-(2,5-difluorophenyl)-1-(3-dimethylaminopropyl)ureidomethyl]pyridine-2-carboxylic acid amide (Compound No.1-11) | $^1$H-NMR (500 MHz, DMSO-d$_6$) δ 1.75 (m, 2H), 2.17 (s, 6H), 2.28 (t, J = 6.1 Hz, 2H), 3.36 (t, J = 6.1 Hz, 2H), 4.62 (s, 2H), 4.89 (s, 2H), 6.65 (td, J = 8.2, 1.2 Hz, |

| | |
|---|---|
| 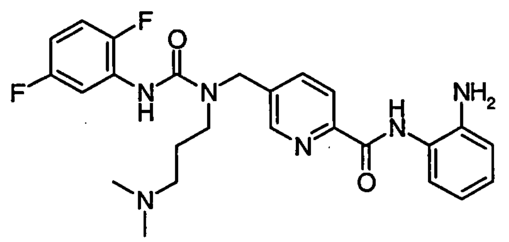 | 1H), 6.79-6.83 (m, 2H), 6.95 (td, J = 8.2, 1.2 Hz, 1H), 7.26 (m, 1H), 7.51 (dd, J = 8.2, 1.2 Hz, 1H), 7.95 (m, 1H), 7.98 (dd, J = 7.9, 2.4 Hz, 1H), 8.12 (d, J = 7.9 Hz, 1H), 8.69 (d, J = 2.4 Hz, 1H), 10.03 (s, 1H), 10.44 (s, 1H) |
| N-(2-Aminophenyl)-5-[3-(3,5-difluorophenyl)-1-(3-dimethylaminopropyl)ureidomethyl]pyridine-2-carboxylic acid amide (Compound No.1-12) 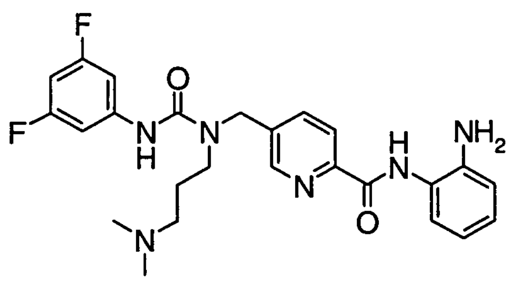 | $^1$H-NMR (500 MHz, DMSO-$d_6$) δ 1.72 (m, 2H), 2.20 (s, 6H), 2.28 (t, J = 6.3 Hz, 2H), 3.37 (t, J = 6.3 Hz, 2H), 4.64 (s, 2H), 4.88 (s, 2H), 6.65 (td, J = 8.1, 1.2 Hz, 1H), 6.76 (tt, J = 9.3, 2.4 Hz, 1H), 6.82 (dd, J = 8.1, 1.2 Hz, 1H), 6.95 (td, J = 8.1, 1.2 Hz, 1H), 7.16 (dd, J = 10.2, 2.4 Hz, 2H), 7.51 (dd, J = 8.1, 1.2 Hz, 1H), 7.96 (dd, J = 7.9, 2.1 Hz, 1H), 8.12 (d, J = 7.9 Hz, 1H), 8.67 (d, J = 2.1 Hz, 1H), 10.03 (s, 1H), 10.15 (s, 1H) |
| N-(2-Aminophenyl)-5-[1-(3-dimethylaminopropyl)-3-(3-fluoro-4-methylphenyl)ureidomethyl] | $^1$H-NMR (500 MHz, DMSO-$d_6$) δ 1.71 (m, 2H), 2.15 (s, 3H), 2.20 (s, 6H), |

| | |
|---|---|
| pyridine-2-carboxylic acid amide (Compound No.1-13) 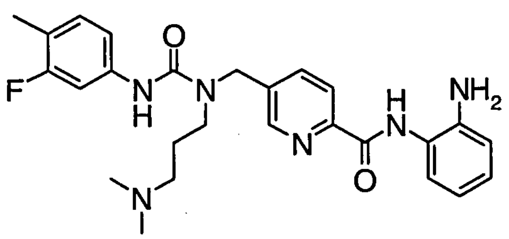 | 2.28 (t, J = 6.3 Hz, 2H), 3.35 (t, J = 6.3 Hz, 2H), 4.62 (s, 2H), 4.89 (s, 2H), 6.65 (td, J = 7.9, 1.5 Hz, 1H), 6.82 (dd, J = 7.9, 1.5 Hz, 1H), 6.95 (td, J = 7.9, 1.5 Hz, 1H), 7.00 (dd, J = 8.4, 2.1 Hz, 1H), 7.13 (t, J = 8.4 Hz, 1H), 7.43 (dd, J = 12.8, 2.1 Hz, 1H), 7.50 (d, J = 7.9, 1.5 Hz, 1H), 7.96 (dd, J = 8.0, 2.1 Hz, 1H), 8.12 (d, J = 8.0 Hz, 1H), 8.66 (d, J = 2.1 Hz, 1H), 9.84 (s, 1H), 10.03 (s, 1H) |
| N-(2-Aminophenyl)-5-[1-(3-dimethylaminopropyl)-3-(4-fluoro-3-methylphenyl)ureidomethyl] pyridine-2-carboxylic acid amide (Compound No.1-14) 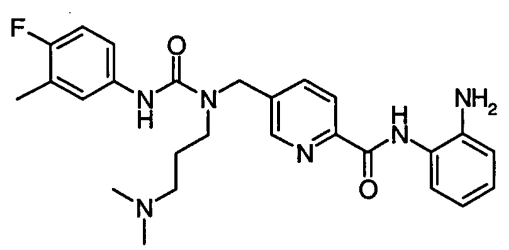 | $^1$H-NMR (500 MHz, DMSO-$d_6$) δ 1.71 (m, 2H), 2.20 (s, 6H), 2.21 (s, 3H), 2.28 (t, J = 6.1 Hz, 2H), 3.35 (t, J = 6.1 Hz, 2H), 4.62 (s, 2H), 4.88 (s, 2H), 6.65 (td, J = 7.9, 1.5 Hz, 1H), 6.82 (dd, J = 7.9, 1.5 Hz, 1H), 6.95 (td, J = 7.9, 1.5 Hz, 1H), 7.01 (t, J = 9.2 Hz, 1H), 7.23 (m, 1H), 7.34 (dd, J = 7.0, 2.4 Hz, 1H), 7.51 (dd, J = 7.9, 1.5 Hz, 1H), 7.95 (dd, J = 8.1, 2.1 Hz, 1H), 8.12 (d, |

| | |
|---|---|
| | J = 8.1 Hz, 1H), 8.66 (d, J = 2.1 Hz, 1H), 9.57 (s, 1H), 10.03 (s, 1H) |
| N-(2-Aminophenyl)-5-[1-carboxymethyl-3-(4-dimethylaminophenyl)ureidomethyl]pyridine-2-carboxylic acid amide (Compound No.1-15) 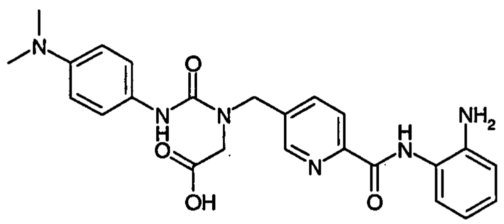 | $^1$H-NMR (400 MHz, DMSO-$d_6$) δ 2.95 (s, 6H), 4.11 (s, 2H), 4.74 (s, 2H), 6.86 (br s, 2H), 7.15-7.27 (m, 5H), 7.59 (m, 2H), 8.08 (dd, J = 8.1, 1.8 Hz, 1H), 8.17 (dd, J = 8.1, 0.7 Hz, 1H), 8.76 (d, J = 1.8 Hz, 1H), 10.55 (br s, 1H) |
| N-(2-Aminophenyl)-5-[3-(benzo[1,3]dioxol-5-yl)-1-(2-carboxyethyl)ureidomethyl]pyridine-2-carboxylic acid amide (Compound No.1-16) 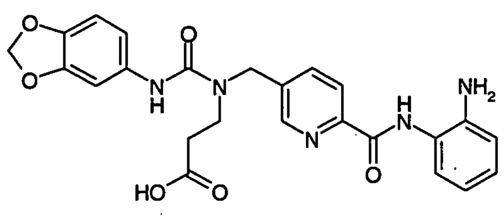 | $^1$H-NMR (500 MHz, DMSO-$d_6$) δ 2.57 (t, J = 7.0 Hz, 2H), 3.55 (t, J = 7.0 Hz, 2H), 4.69 (s, 2H), 4.89 (br s, 2H), 5.95 (s, 2H), 6.65 (td, J = 7.6, 1.2 Hz, 1H), 6.79-6.84 (m, 3H), 6.95 (td, J = 7.6, 1.2 Hz, 1H), 7.13 (d, J = 1.8 Hz, 1H), 7.51 (dd, J = 7.6, 1.2 Hz, 1H), 7.92 (dd, J = 8.1, 1.8 Hz, 1H), 8.12 (d, J = 8.1 Hz, 1H), 8.54 (br s, 1H), 8.62 (d, J = 1.8 Hz, 1H), 10.03 (br s, 1H) |

| | |
|---|---|
| N-(2-Aminophenyl)-5-[1-(3-dimethylaminopropyl)-3-(indan-5-yl)ureidomethyl]pyridine-2-carboxylic acid amide (Compound No.1-17) 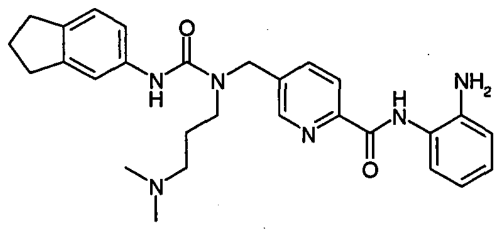 | $^1$H-NMR (500 MHz, DMSO-$d_6$) δ 1.71 (m, 2H), 1.96-2.02 (m, 2H), 2.19 (s, 6H), 2.28 (t, J = 6.3 Hz, 2H), 2.76-2.83 (m, 4H), 3.35 (m, 2H), 4.62 (s, 2H), 4.89 (br s, 2H), 6.65 (t, J = 7.9 Hz, 1H), 6.82 (d, J = 7.9 Hz, 1H), 6.95 (td, J = 7.9, 1.4 Hz, 1H), 7.08 (d, J = 8.2 Hz, 1H), 7.10 (d, J = 8.2 Hz, 1H), 7.38 (s, 1H), 7.51 (d, J = 7.9 Hz, 1H), 7.96 (dd, J = 7.9, 2.1 Hz, 1H), 8.12 (d, J = 7.9 Hz, 1H), 8.66 (d, J = 2.1 Hz, 1H), 9.52 (br s, 1H), 10.03 (br s, 1H) |
| N-(2-Aminophenyl)-5-[3-(biphenyl-4-yl)-1-(3-dimethylaminopropyl)ureidomethyl]pyridine-2-carboxylic acid amide (Compound No.1-18) 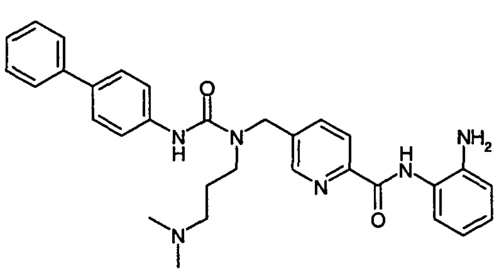 | $^1$H-NMR (400 MHz, DMSO-$d_6$) δ 1.74 (m, 2H), 2.23 (s, 6H), 2.31 (t, J = 6.3 Hz, 2H), 3.39 (t, J = 6.3 Hz, 2H), 4.65 (s, 2H), 4.90 (br s, 2H), 6.65 (t, J = 7.6 Hz, 1H), 6.82 (d, J = 7.6 Hz, 1H), 6.95 (td, J = 7.6, 1.3 Hz, 1H), 7.31 (t, J = 7.6 Hz, 1H), 7.43 (t, J = 7.6 Hz, 2H), 7.50-7.64 (m, 7H), 7.98 (dd, |

| | |
|---|---|
| | J = 8.1, 2.0 Hz, 1H), 8.13 (dd, J = 8.1, 0.7 Hz, 1H), 8.69 (d, J = 2.0 Hz, 1H), 9.88 (br s, 1H), 10.04 (br s, 1H) |
| N-(2-Aminophenyl)-5-[1-(3-dimethylaminopropyl)-3-(4-trifluoromethylphenyl)ureidomethyl] pyridine-2-carboxylic acid amide (Compound No.1-19) 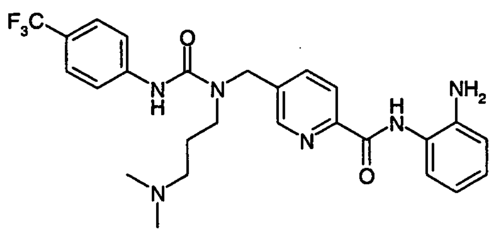 | $^1$H-NMR (500 MHz, DMSO-$d_6$) δ 1.72 (m, 2H), 2.06 (s, 6H), 2.28 (t, J = 6.3 Hz, 2H), 3.36 (t, J = 6.3 Hz, 2H), 4.64 (s, 2H), 4.89 (br s, 2H), 6.65 (t, J = 7.6 Hz, 1H), 6.82 (d, J = 7.6 Hz, 1H), 6.95 (td, J = 7.6, 1.4 Hz, 1H), 7.39 (m, 1H), 7.50 (m, 1H), 7.65 (m, 2H), 7.69 (d, J = 7.6 Hz, 1H), 7.93 (dd, J = 7.9, 1.8 Hz, 1H), 8.11 (d, J = 7.9 Hz, 1H), 8.63 (d, J = 1.8 Hz, 1H), 9.61 (br s, 1H), 10.04 (br s, 1H) |
| N-(2-Aminophenyl)-5-[3-(4-cyanophenyl)-1-(3-dimethylaminopropyl)ureidomethyl]pyridine-2-carboxylic acid amide (Compound No.1-20) | $^1$H-NMR (400 MHz, DMSO-$d_6$) δ 1.74 (m, 2H), 2.20 (s, 6H), 2.29 (t, J = 6.0 Hz, 2H), 3.38 (t, J = 6.0 Hz, 2H), 4.64 (s, 2H), 4.90 (br s, 2H), 6.65 (td, J = 7.6, 1.5 Hz, 1H), 6.82 (dd, J = 7.6, 1.5 Hz, 1H), 6.95 |

| | |
|---|---|
| 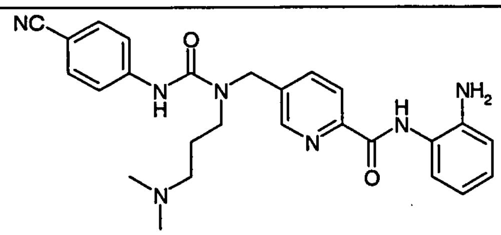 | (td, J = 7.6, 1.5 Hz, 1H), 7.50 (dd, J = 7.6, 1.5 Hz, 1H), 7.60 (d, J = 9.0 Hz, 2H), 7.70 (d, J = 9.0 Hz, 2H), 7.98 (dd, J = 8.1, 2.0 Hz, 1H), 8.12 (dd, J = 8.1, 0.5 Hz, 1H), 8.68 (d, J = 2.0 Hz, 1H), 10.03 (br s, 1H), 10.46 (br s, 1H) |
| N-(2-Aminophenyl)-5-[1-(3-dimethylaminopropyl)-3-(4-trifluoromethoxyphenyl)ureidomethyl]pyridine-2-carboxylic acid amide (Compound No.1-21) 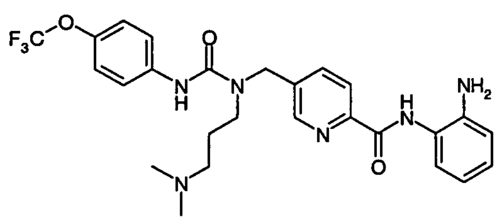 | $^1$H-NMR (500 MHz, DMSO-$d_6$) δ 1.72 (m, 2H), 2.21 (s, 6H), 2.28 (t, J = 6.3 Hz, 2H), 3.37 (t, J = 6.3 Hz, 2H), 4.63 (s, 2H), 4.89 (br s, 2H), 6.65 (td, J = 7.6, 1.2 Hz, 1H), 6.82 (dd, J = 7.6, 1.2 Hz, 1H), 6.95 (td, J = 7.6, 1.2 Hz, 1H), 7.25 (d, J = 8,7 Hz, 2H), 7.52 (m, 1H), 7.53 (d, J = 8.7 Hz, 2H), 7.96 (dd, J = 7.9, 2.0 Hz, 1H), 8.12 (d, J = 7.9 Hz, 1H), 8.67 (d, J = 2.0 Hz, 1H), 9.96 (br s, 1H), 10.03 (br s, 1H) |
| N-(2-Aminophenyl)-5-[3-(4-dimethylaminophenyl)-1-(3-hydroxypropyl)ureidomethyl]pyridi | $^1$H-NMR (400 MHz, DMSO-$d_6$) δ 1.69 (m, 2H), 2.82 (s, 6H), 3.40 (t, J = 6.7 |

| | |
|---|---|
| ne-2-carboxylic acid amide (Compound No.1-22) 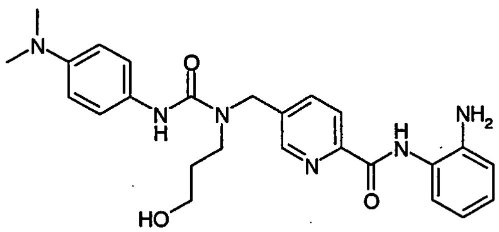 | Hz, 2H), 3.47 (m, 2H), 4.66 (s, 2H), 4.85 (br s, 1H), 4.90 (br s, 2H), 6.65 (m, 1H), 6.67 (d, J = 9.2 Hz, 2H), 6.83 (dd, J = 7.9, 1.5 Hz, 1H), 6.95 (td, J = 7.9, 1.5 Hz, 1H), 7.23 (d, J = 9.2 Hz, 2H), 7.51 (dd, J = 7.9, 1.5 Hz, 1H), 7.93 (dd, J = 8.1, 1.8 Hz, 1H), 8.12 (d, J = 8.1 Hz, 1H), 8.31 (br s, 1H), 8.64 (d, J = 1.8 Hz, 1H), 10.03 (br s, 1H) |
| N-(2-Aminophenyl)-5-[3-(2,3-dihydrobenzo[1,4]dioxin-6-yl)-1-(3-hydroxypropyl)ureidomethyl]pyridine-2-carboxylic acid amide (Compound No.1-23) 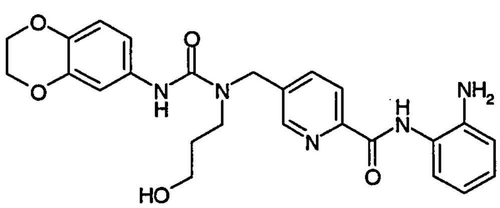 | $^1$H-NMR (500 MHz, DMSO-$d_6$) δ 1.68 (m, 2H), 3.40 (t, J = 6.6 Hz, 2H), 3.47 (m, 2H), 4.17-4.21 (m, 4H), 4.65 (s, 2H), 4.91 (br s, 3H), 6.65 (td, J = 7.8, 1.4 Hz, 1H), 6.72 (d, J = 8.7 Hz, 1H), 6.82 (dd, J = 7.8, 1.4 Hz, 1H), 6.85 (dd, J = 8.7, 2.6 Hz, 1H), 6.95 (td, J = 7.8, 1.4 Hz, 1H), 7.03 (d, J = 2.6 Hz, 1H), 7.51 (dd, J = 7.8, 1.4 Hz, 1H), 7.92 (dd, J = 8.1, 2.0 Hz, 1H), 8.12 (d, J = 8.1 Hz, 1H), 8.44 (br s, 1H), 8.63 (d, J = 2.0 |

| | |
|---|---|
| | Hz, 1H), 10.03 (br s, 1H) |
| N-(2-Aminophenyl)-5-[3-(4-cyanophenyl)-1-(3-hydroxypropyl)ureidomethyl]pyridine-2-carboxylic acid amide (Compound No.1-24) 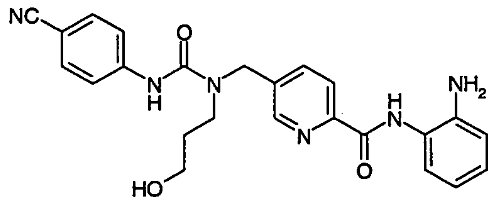 | $^1$H-NMR (500 MHz, DMSO-d$_6$) δ 1.71 (m, 2H), 3.45-3.49 (m, 4H), 4.70 (s, 2H), 4.89 (br s, 2H), 5.03 (br s, 1H), 6.65 (td, J = 7.8, 1.2 Hz, 1H), 6.82 (dd, J = 7.8, 1.2 Hz, 1H), 6.95 (td, J = 7.8, 1.2 Hz, 1H), 7.50 (dd, J = 7.8, 1.2 Hz, 1H), 7.65 (d, J = 9.0 Hz, 2H), 7.71 (d, J = 9.0 Hz, 2H), 7.92 (dd, J = 8.1, 1.8 Hz, 1H), 8.12 (d, J = 8.1 Hz, 1H), 8.66 (d, J = 1.8 Hz, 1H), 9.15 (br s, 1H), 10.03 (br s, 1H) |
| N-(2-Aminophenyl)-5-[3-(benzo[1,3]dioxol-5-yl)-1-(3-hydroxypropyl)ureidomethyl]pyridine-2-carboxylic acid amide (Compound No.1-25) 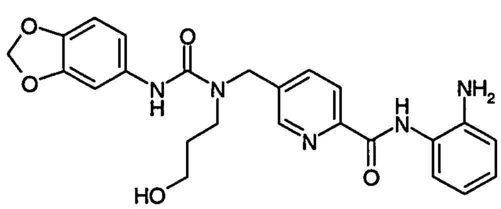 | $^1$H-NMR (500 MHz, DMSO-d$_6$) δ 1.69 (m, 2H), 3.40 (t, J = 6.9 Hz, 2H), 3.47 (m, 2H), 4.66 (s, 2H), 4.90 (br s, 2H), 4.92 (br s, 1H), 5.95 (s, 2H), 6.65 (td, J = 7.6, 1.2 Hz, 1H), 6.80 (m, 2H), 6.82 (dd, J = 7.6, 1.2 Hz, 1H), 6.95 (td, J = 7.6, 1.2 Hz, 1H), 7.13 (t, J = 1.2 Hz, 1H), 7.51 (d, J = 7.6 |

| | |
|---|---|
| | Hz, 1H), 7.93 (dd, J = 8.1, 2.0 Hz, 1H), 8.12 (d, J = 8.1 Hz, 1H), 8.53 (br s, 1H), 8.66 (d, J = 2.0 Hz, 1H), 10.03 (br s, 1H) |
| N-(2-Aminophenyl)-5-[1-(3-hydroxypropyl)-3-(4-methoxyphenyl)ureidomethyl]pyridine-2-carboxylic acid amide (Compound No.1-26) 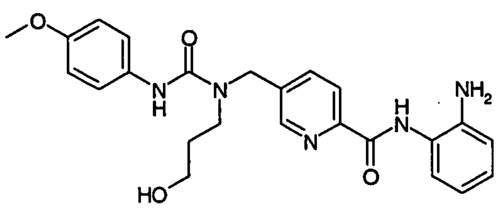 | $^1$H-NMR (500 MHz, DMSO-d$_6$) δ 1.69 (m, 2H), 3.41 (t, J = 6.6 Hz, 2H), 3.48 (m, 2H), 3.71 (s, 3H), 4.66 (s, 2H), 4.87-4.89 (m, 3H), 6.63 (td, J = 7.8, 1.4 Hz, 1H), 6.82 (m, 1H), 6.84 (d, J = 9.2 Hz, 2H), 6.95 (td, J = 7.8, 1.4 Hz, 1H), 7.33 (d, J = 9.2 Hz, 2H), 7.51 (dd, J = 7.8, 1.4 Hz, 1H), 7.94 (dd, J = 8.1, 1.8 Hz, 1H), 8.12 (dd, J = 8.1, 0.6 Hz, 1H), 8.46 (br s, 1H), 8.64 (d, J = 1.8 Hz, 1H), 10.03 (br s, 1H) |
| N-(2-Aminophenyl)-5-[3-(4-dimethylaminophenyl)-1-(2-ethoxycarbonylethyl)ureidomethyl] pyridine-2-carboxylic acid amide (Compound No.1-27) | $^1$H-NMR (400 MHz, DMSO-d$_6$) δ 1.17 (t, J = 7.1 Hz, 3H), 2.62 (t, J = 7.1 Hz, 2H), 2.82 (s, 6H), 3.59 (t, J = 7.1 Hz, 2H), 4.04 (q, J = 7.1 Hz, 2H), 4.70 (s, 2H), 4.90 (br s, 2H), 6.66 (d, J = 9.0 Hz, 2H), |

| | |
|---|---|
| 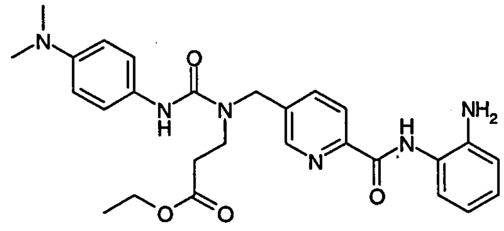 | 6.67 (m, 1H), 6.82 (dd, J = 7.9, 1.2 Hz, 1H), 6.95 (m, 1H), 7.23 (d, J = 9.0 Hz, 2H), 7.50 (dd, J = 7.9, 1.2 Hz, 1H), 7.91 (dd, J = 8.1, 1.8 Hz, 1H), 8.12 (d, J = 8.1 Hz, 1H), 8.30 (br s, 1H), 8.62 (d, J = 1.8 Hz, 1H), 10.04 (br s, 1H) |
| N-(2-Aminophenyl)-5-[3-(benzo[1,3]dioxol-5-yl)-1-(2-ethoxycarbonylethyl)ureidomethyl]pyridine-2-carboxylic acid amide (Compound No.1-28) 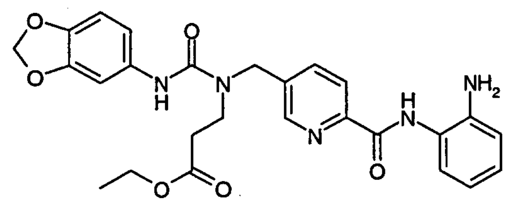 | $^1$H-NMR (400 MHz, DMSO-d$_6$) δ 1.17 (t, J = 7.1 Hz, 3H), 2.62 (t, J = 7.1 Hz, 2H), 3.59 (t, J = 7.1 Hz, 2H), 4.04 (q, J = 7.1 Hz, 2H), 4.70 (s, 2H), 4.90 (br s, 2H), 5.95 (s, 2H), 6.65 (t, J = 7.6 Hz, 1H), 6.79-6.84 (m, 3H), 6.95 (td, J = 7.6, 1.3 Hz, 1H), 7.13 (d, J = 2.0 Hz, 1H), 7.50 (dd, J = 7.6, 1.3 Hz, 1H), 7.91 (dd, J = 8.1, 1.8 Hz, 1H), 8.12 (d, J = 8.1 Hz, 1H), 8.48 (br s, 1H), 8.62 (d, J = 1.8 Hz, 1H), 10.04 (br s, 1H) |
| N-(2-Aminophenyl)-5-[1-(2-ethoxycarbonylet | $^1$H-NMR (400 MHz, DMSO-d$_6$) |

| | |
|---|---|
| hyl)-3-(4-methoxyphenyl)ureidomethyl]pyridine-2-carboxylic acid amide (Compound No.1-29) 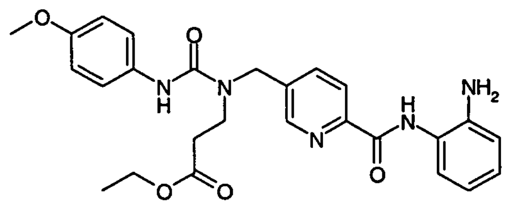 | δ 1.17 (t, J = 7.1 Hz, 3H), 2.63 (t, J = 7.1 Hz, 2H), 3.60 (t, J = 7.1 Hz, 2H), 3.71 (s, 3H), 4.04 (q, J = 7.1 Hz, 2H), 4.71 (s, 2H), 4.90 (br s, 2H), 6.65 (td, J = 7.8, 1.2 Hz, 1H), 6.83 (m, 1H), 6.84 (d, J = 9.2 Hz, 2H), 6.95 (m, 1H), 7.34 (d, J = 9.2 Hz, 2H), 7.50 (d, J = 7.8 Hz, 1H), 7.91 (dd, J = 8.1, 1.8 Hz, 1H), 8.12 (d, J = 8.1 Hz, 1H), 8.44 (br s, 1H), 8.62 (d, J = 1.8 Hz, 1H), 10.04 (br s, 1H) |
| N-(2-Aminophenyl)-5-[3-(1,3-benzothiazol-2-yl)-1-(3-dimethylaminopropyl)ureidomethyl]pyridine-2-carboxylic acid amide (Compound No.1-30) 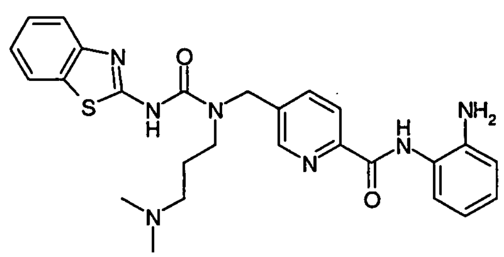 | $^1$H-NMR (500 MHz, DMSO-$d_6$) δ 1.76 (br s, 2H), 2.31 (br s, 6H), 2.38 (br s, 2H), 3.44 (br s, 2H), 4.71 (s, 2H), 4.90 (br s, 2H), 6.65 (td, J = 7.6, 1.4 Hz, 1H), 6.82 (dd, J = 7.6, 1.4 Hz, 1H), 6.95 (td, J = 7.6, 1.4 Hz, 1H), 7.19 (td, J = 8.1, 0.9 Hz, 1H), 7.34 (td, J = 8.1, 0.9 Hz, 1H), 7.51 (dd, J = 8.1, 0.9 Hz, 1H), 7.57 (br s, 1H), 7.83 (d, J = 7.6 |

| | |
|---|---|
| | Hz, 1H), 7.99 (dd, J = 7.9, 1.5 Hz, 1H), 8.13 (d, J = 7.9 Hz, 1H), 8.70 (d, J = 1.5 Hz, 1H), 10.03 (br s, 1H) |
| N-(2-Aminophenyl)-5-[3-(1H-benzimidazol-2-yl)-1-(3-dimethylaminopropyl)ureidomethyl]pyridine-2-carboxylic acid amide (Compound No.1-31) 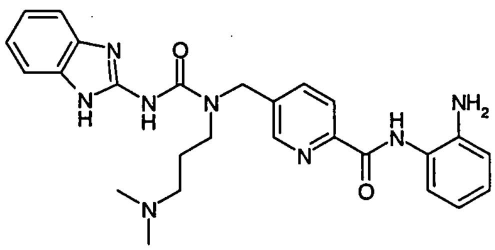 | $^1$H-NMR (500 MHz, DMSO-d$_6$) δ 1.74 (br s, 2H), 2.22 (br s, 8H), 3.44 (br s, 2H), 4.70 (s, 2H), 4.89 (br s, 2H), 6.65 (td, J = 7.6, 1.2 Hz, 1H), 6.82 (dd, J = 7.6, 1.2 Hz, 1H), 6.95 (td, J = 7.6, 1.2 Hz, 1H), 7.01-7.03 (m, 2H), 7.30 (br s, 2H), 7.51 (dd, J = 7.6, 1.2, Hz, 1H), 7.99 (d, J = 7.9 Hz, 1H), 8.12 (d, J = 7.9 Hz, 1H), 8.70 (br s, 1H), 10.03 (br s, 1H), 11.80 (br s, 1H) |
| N-(2-Aminophenyl)-5-[1-(3-dimethylaminopropyl)-3-(thiophen-2-yl)ureidomethyl]pyridine-2-carboxylic acid amide (Compound No.1-32) 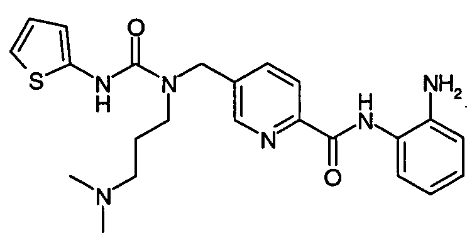 | $^1$H-NMR (400 MHz, DMSO-d$_6$) δ 1.71 (m, 2H), 2.22 (s, 6H), 2.29 (t, J = 6.1 Hz, 2H), 3.33 (m, 2H), 4.64 (s, 2H), 4.90 (br s, 2H), 6.53 (dd, J = 3.5, 1.6 Hz, 1H), 6.65 (td, J = 7.6, 1.2 Hz, 1H), 6.78-6.83 (m, 3H), 6.95 (td, J = 7.6, 1.2 Hz, 1H), 7.51 (dd, J = |

| | |
|---|---|
| | 7.6, 1.2 Hz, 1H), 7.94 (dd, J = 8.1, 1.8 Hz, 1H), 8.12 (d, J = 8.1 Hz, 1H), 8.66 (d, J = 1.8 Hz, 1H), 10.03 (br s, 1H), 11.10 (br s, 1H) |
| N-(2-Aminophenyl)-5-[1-(3-dimethylaminopropyl)-3-(thiophen-3-yl)ureidomethyl]pyridine-2-carboxylic acid amide (Compound No.1-33) 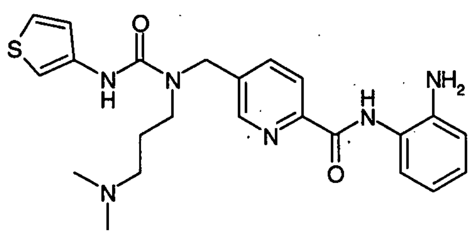 | $^1$H-NMR (500 MHz, DMSO-$d_6$) δ 1.69 (m, 2H), 2.19 (s, 6H), 2.27 (t, J = 6.4 Hz, 2H), 3.34 (m, 2H), 4.63 (s, 2H), 4.89 (br s, 2H), 6.65 (td, J = 7.6, 1.2 Hz, 1H), 6.82 (dd, J = 7.6, 1.2 Hz, 1H), 6.94 (td, J = 7.6, 1.2 Hz, 1H), 7.04 (dd, J = 5.0, 1.4 Hz, 1H), 7.28 (dd, J = 3.2, 1.4 Hz, 1H), 7.39 (dd, J = 5.0, 3.2 Hz, 1H), 7.51 (dd, J = 7.6, 1.2 Hz, 1H), 7.95 (dd, J = 8.1, 2.0 Hz, 1H), 8.11 (d, J = 8.1 Hz, 1H), 8.66 (d, J = 2.0 Hz, 1H), 10.03 (br s, 1H), 10.06 (br s, 1H) |
| N-(2-Aminophenyl)-5-[3-(2,3-dihydrobenzo[1,4]dioxin-6-yl)-1-[3-(morpholin-4-yl)propyl]ureidomethyl]pyridine-2-carboxylic acid amide | $^1$H-NMR (500 MHz, DMSO-$d_6$) δ 1.69 (m, 2H), 2.27-2.31 (m, 6H), 3.35 (m, 2H), 3.53-3.55 (m, 4H), 4.17-4.21 (m, 4H), |

| | |
|---|---|
| (Compound No.1-34)<br>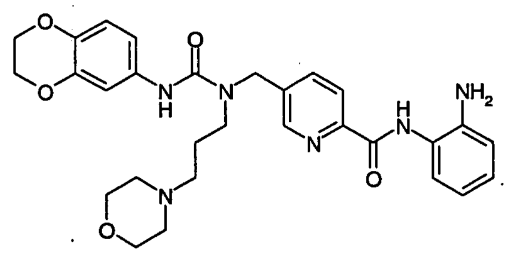 | 4.67 (s, 2H), 4.89 (br s, 2H), 6.65 (td, J = 7.6, 1.2 Hz, 1H), 6.74 (d, J = 8.6 Hz, 1H), 6.82 (d, J = 7.6 Hz, 1H), 6.88 (dd, J = 8.6, 2.4 Hz, 1H), 6.95 (td, J = 7.6, 1.2 Hz, 1H), 7.05 (d, J = 2.4 Hz, 1H), 7.50 (d, J = 7.6 Hz, 1H), 7.92 (dd, J = 8.1, 2.0 Hz, 1H), 8.12 (d, J = 8.1 Hz, 1H), 8.48 (br s, 1H), 8.63 (d, J = 2.0 Hz, 1H), 10.03 (br s, 1H) |
| N-(2-Aminophenyl)-5-[3-(benzo[1,3]dioxol-5-yl)-1-[3-(morpholin-4-yl)propyl)]ureidomethyl] pyridine-2-carboxylic acid amide (Compound No.1-35)<br>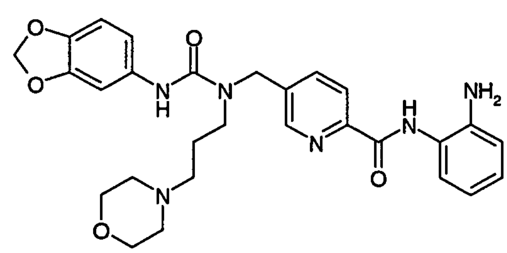 | $^1$H-NMR (400 MHz, DMSO-$d_6$)<br>δ 1.70 (m, 2H), 2.27-2.31 (m, 6H), 3.36 (t, J = 6.6 Hz, 2H), 3.52-3.55 (m, 4H), 4.67 (s, 2H), 4.89 (br s, 2H), 5.96 (s, 2H), 6.65 (td, J = 7.6, 1.2 Hz, 1H), 6.80-6.83 (m, 3H), 6.93 (td, J = 7.6, 1.2 Hz, 1H), 7.15 (m, 1H), 7.50 (dd, J = 7.6, 1.2 Hz, 1H), 7.93 (dd, J = 8.1, 1.7 Hz, 1H), 8.12 (d, J = 8.1 Hz, 1H), 8.58 (br s, 1H), 8.64 (d, J = 1.7 Hz, 1H), 10.04 (br s, 1H) |

| | |
|---|---|
| N-(2-Aminophenyl)-5-[3-(4-methoxyphenyl)-1-[3-(morpholin-4-yl)propyl]ureidomethyl]pyridine-2-carboxylic acid amide (Compound No.1-36)<br>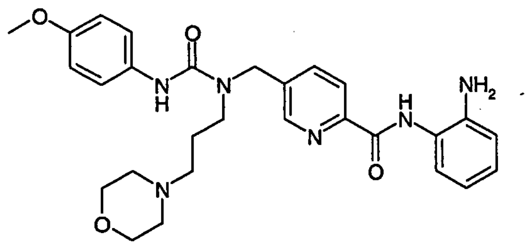 | $^1$H-NMR (400 MHz, DMSO-$d_6$)<br>δ 1.71 (m, 2H), 2.27-2.32 (m, 6H), 3.36 (m, 2H), 3.52-3.54 (m, 4H), 3.71 (s, 3H), 4.67 (s, 2H), 4.90 (br s, 2H), 6.65 (td, J = 7.7, 1.2 Hz, 1H), 6.83 (m, 1H), 6.85 (d, J = 9.2 Hz, 2H), 6.95 (td, J = 7.7, 1.2 Hz, 1H), 7.34 (d, J = 9.2 Hz, 2H), 7.51 (dd, J = 7.7, 1.2 Hz, 1H), 7.93 (dd, J = 8.1, 1.8 Hz, 1H), 8.12 (d, J = 8.1 Hz, 1H), 8.55 (br s, 1H), 8.64 (d, J = 1.8 Hz, 1H), 10.04 (br s, 1H) |
| N-(2-Aminophenyl)-5-[3-(2,3-dihydro-1-benzofuran-5-yl)-1-(3-dimethylaminopropyl)ureidomethyl]pyridine-2-carboxylic acid amide (Compound No.1-37)<br>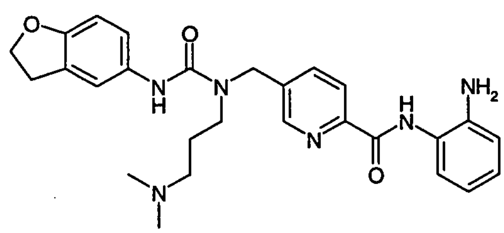 | $^1$H-NMR (400 MHz, DMSO-$d_6$)<br>δ 1.70 (m, 2H), 2.17 (s, 6H), 2.27 (t, J = 6.3 Hz, 2H), 3.14 (t, J = 8.7 Hz, 2H), 3.33 (br s, 2H), 4.47 (t, J = 8.7 Hz, 2H), 4.62 (s, 2H), 4.90 (br s, 2H), 6.65 (d, J = 8.4 Hz, 1H), 6.65 (m, 1H), 6.82 (dd, J = 7.7, 1.2 Hz, 1H), 6.95 (td, J = 7.7, 1.2 Hz, 1H), 7.02 (dd, J = 8.4, 2.1 Hz, 1H), 7.36 (d, J = 2.1 Hz, 1H), |

| | |
|---|---|
| | 7.51 (dd, J = 7.7, 1.2 Hz, 1H), 7.95 (dd, J = 8.1, 2.0 Hz, 1H), 8.12 (d, J = 8.1 Hz, 1H), 8.66 (d, J = 2.0 Hz, 1H), 9.34 (br s, 1H), 10.03 (br s, 1H) |
| N-(2-Aminophenyl)-5-[3-(2,3-dihydro-1-benzofuran-5-yl)-1-[3-(morpholin-4-yl)propyl]ureidomethyl]pyridine-2-carboxylic acid amide (Compound No.1-38) 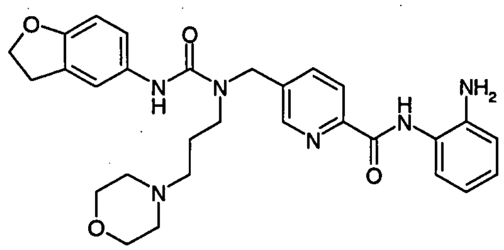 | $^1$H-NMR (400 MHz, DMSO-$d_6$) δ 1.71 (m, 2H), 2.27-2.32 (m, 6H), 3.14 (t, J = 8.5 Hz, 2H), 3.36 (m, 2H), 3.52-3.54 (m, 4H), 4.48 (t, J = 8.5 Hz, 2H), 4.67 (s, 2H), 4.90 (br s, 2H), 6.65 (d, J = 8.4 Hz, 1H), 6.65 (m, 1H), 6.82 (dd, J = 7.8, 1.2 Hz, 1H), 6.95 (td, J = 7.8, 1.2 Hz, 1H), 7.05 (dd, J = 8.4, 2.1 Hz, 1H), 7.33 (d, J = 2.1 Hz ,1H), 7.50 (dd, J = 7.8, 1.2 Hz, 1H), 7.93 (dd, J = 8.1, 1.7 Hz, 1H), 8.12 (dd, J = 8.1, 0.5 Hz, 1H), 8.51 (br s, 1H), 8.64 (d, J = 1.7 Hz, 1H), 10.04 (br s, 1H) |
| N-(2-Aminophenyl)-5-[1-(3-dimethylaminopropyl)-3-(3-fluoro-4-methoxyphenyl)ureidometh | $^1$H-NMR (500 MHz, $CDCl_3$) δ 1.74 (br s, 2H), 2.31 (s, 6H), 2.40 (br s, |

| | |
|---|---|
| yl]pyridine-2-carboxylic acid amide (Compound No.1-39) 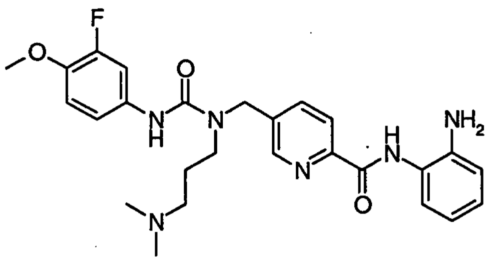 | 2H), 3.38 (br s, 2H), 3.86 (s, 3H), 3.96 (s, 2H), 4.62 (s, 2H), 6.83-6.90 (m, 3H), 7.05-7.09 (m, 2H), 7.31 (dd, J = 13.6, 2.4 Hz, 1H), 7.49 (dd, J = 7.8, 1.4 Hz, 1H), 7.92 (dd, J = 7.9, 2.1 Hz, 1H), 8.24 (dd, J = 7.9, 0.6 Hz, 1H), 8.60 (d, J = 2.1 Hz, 1H), 9.83 (s, 1H), 10.16 (s, 1H) |
| N-(2-Aminophenyl)-5-[1-(3-dimethylaminopropyl)-3-(phenylcarbonylmethyl)ureidomethyl]pyridine-2-carboxylic acid amide (Compound No.1-40) 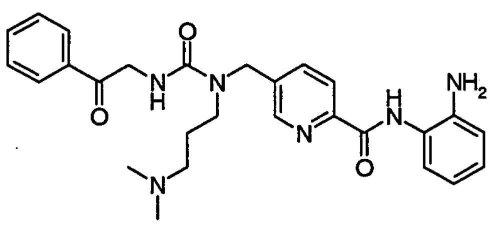 | $^1$H-NMR (400 MHz, $CDCl_3$) δ 1.72 (m, 2H), 2.30 (s, 6H), 2.43 (br s, 2H), 3.40 (t, J = 5.7 Hz, 2H), 3.97 (s, 2H), 4.63 (s, 2H), 4.73 (d, J = 4.2 Hz, 2H), 6.82-6.88 (m, 2H), 7.08 (td, J = 7.7, 1.5 Hz, 1H), 7.47-7.52 (m, 3H), 7.60 (tt, J = 7.7, 1.5 Hz, 1H), 7.87 (dd, J = 7.7, 2.1 Hz, 1H), 7.99-8.02 (m, 2H), 8.22 (s, 1H), 8.24 (dd, J = 8.1, 0.7 Hz, 1H), 8.56 (d, J = 1.5 Hz, 1H), 9.84 (s, 1H) |
| N-(2-Aminophenyl)-5-[1-(3-dimethylaminopro | $^1$H-NMR (500 MHz, DMSO-$d_6$) |

| | |
|---|---|
| pyl)-3-(1,3-thiazol-2-yl)ureidomethyl]pyridine-2-carboxylic acid amide (Compound No.1-41) 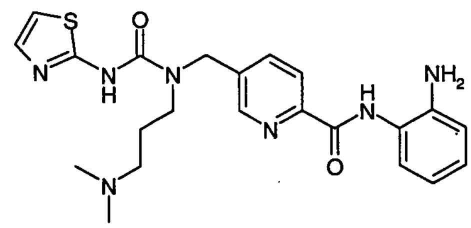 | δ 1.73 (m, 2H), 2.24 (s, 6H), 2.32 (t, J = 6.0 Hz, 2H), 3.38 (t, J = 6.0 Hz, 2H), 4.66 (s, 2H), 4.90 (br s, 2H), 6.65 (td, J = 7.6, 1.5 Hz, 1H), 6.82 (dd, J = 7.6, 1.5 Hz, 1H), 6.95 (td, J = 7.6, 1.5 Hz, 1H), 7.02 (br s, 1H), 7.34 (d, J = 3.4 Hz, 1H), 7.50 (dd, J = 7.6, 1.5 Hz, 1H), 7.96 (dd, J = 7.9, 1.8 Hz, 1H), 8.11 (dd, J = 7.9, 0.6 Hz, 1H), 8.68 (d, J = 1.8 Hz, 1H), 10.03 (br s, 1H) |
| N-(2-Aminophenyl)-5-[1-(3-dimethylaminopropyl)-3-(6-methoxy-1,3-benzothiazol-2-yl)ureidomethyl]pyridine-2-carboxylic acid amide (Compound No.1-42) 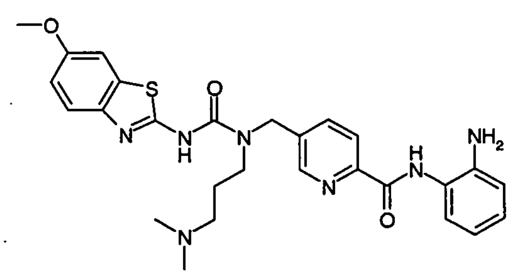 | $^1$H-NMR (500 MHz, DMSO-$d_6$) δ 1.75 (br s, 2H), 2.29 (s, 6H), 2.36 (br s, 2H), 3.42 (t, J = 5.7 Hz, 2H), 3.78 (s, 3H), 4.70 (s, 2H), 4.90 (br s, 2H), 6.65 (td, J = 7.8, 1.2 Hz, 1H), 6.82 (d, J = 7.8 Hz, 1H), 6.93-6.97 (m, 2H), 7.45 (d, J = 1.8 Hz, 1H), 7.48 (m, 1H), 7.50 (dd, J = 7.8, 1.2 Hz, 1H), 7.98 (dd, J = 7.9, 1.5 Hz, 1H), 8.13 (d, J = 7.9 Hz, 1H), 8.68 (d, J = 1.5 Hz, 1H), 10.03 |

| | |
|---|---|
| | (br s, 1H) |
| N-(2-Aminophenyl)-5-[3-(6-chloro-1,3-benzothiazol-2-yl)-1-(3-dimethylaminopropyl)ureidomethyl]pyridine-2-carboxylic acid amide (Compound No.1-43) 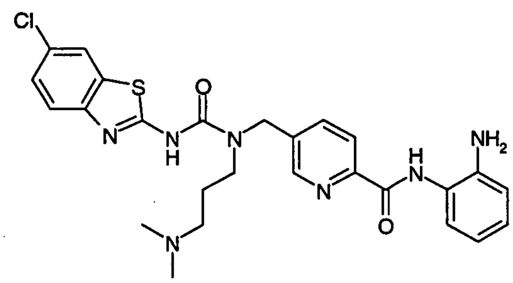 | $^1$H-NMR (500 MHz, DMSO-$d_6$) δ 1.77 (br s, 2H), 2.36 (s, 6H), 2.45 (br s, 2H), 3.43 (t, J = 5.2 Hz, 2H), 4.69 (s, 2H), 4.90 (br s, 2H), 6.65 (td, J = 7.9, 1.2 Hz, 1H), 6.82 (d, J = 7.9 Hz, 1H), 6.95 (td, J = 7.9, 1.2 Hz, 1H), 7.34 (dd, J = 8.6, 2.1 Hz, 1H), 7.51 (dd, J = 7.9, 1.2 Hz, 1H), 7.53 (br s, 1H), 7.95 (br s, 1H), 7.99 (dd, J = 8.1, 1.7 Hz, 1H), 8.13 (d, J = 8.1 Hz, 1H), 8.70 (d, J = 1.7 Hz, 1H), 10.03 (br s, 1H) |
| N-(2-Aminophenyl)-5-[1-(3-dimethylaminopropyl)-3-(6-fluoro-1,3-benzothiazol-2-yl)ureidomethyl]pyridine-2-carboxylic acid amide (Compound No.1-44) | $^1$H-NMR (500 MHz, DMSO-$d_6$) δ 1.76 (br s, 2H), 2.33 (s, 6H), 2.41 (br s, 2H), 3.43 (br s, 2H), 4.69 (s, 2H), 4.90 (br s, 2H), 6.65 (td, J = 7.8, 1.2 Hz, 1H), 6.82 (d, J = 7.8 Hz, 1H), 6.95 (td, J = 7.8, 1.2 Hz, 1H), 7.17 (ddd, J = 9.2, 8.9, 2.3 Hz, 1H), 7.51 (dd, J = 7.8, 1.2 Hz, 1H), 7.57 (br s, 1H), |

| | |
|---|---|
| 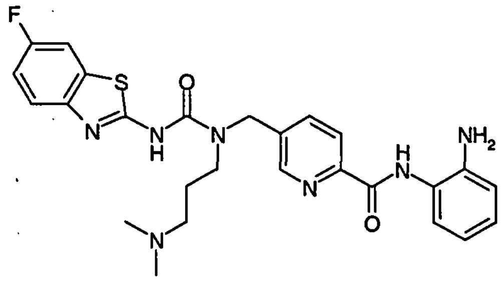 | 7.75 (dd, J = 8.1, 2.3 Hz, 1H), 7.98 (dd, J = 7.9, 1.5 Hz, 1H), 8.13 (d, J = 7.9 Hz, 1H), 8.70 (d, J = 1.5 Hz, 1H), 10.03 (br s, 1H) |
| N-(2-Aminophenyl)-5-[1-(3-diethylaminopropyl)-3-(4-fluorophenyl)ureidomethyl]pyridine-2-carboxylic acid amide (Compound No.1-45) 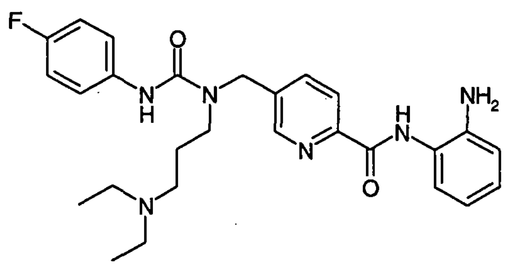 | 1H-NMR (500 MHz, DMSO-$d_6$) δ 0.93 (t, J = 7.0 Hz, 6H), 1.69 (m, 2H), 2.39 (t, J = 6.7 Hz, 2H), 2.48 (m, 4H), 3.36 (t, J = 6.7 Hz, 2H), 4.66 (s, 2H), 4.89 (br s, 2H), 6.65 (td, J = 7.6, 1.4 Hz, 1H), 6.82 (dd, J = 7.6, 1.4 Hz, 1H), 6.95 (td, J = 7.6, 1.4 Hz, 1H), 7.10 (t, J = 8.9 Hz, 2H), 7.43 (m, 2H), 7.50 (dd, J = 7.6, 1.4 Hz, 1H), 7.95 (dd, J = 8.2, 2.0 Hz, 1H), 8.12 (d, J = 8.2 Hz, 1H), 8.65 (d, J = 2.0 Hz, 1H), 9.13 (br s, 1H), 10.03 (br s, 1H) |
| N-(2-Aminophenyl)-5-[1-(3-diethylaminopropyl)-3-(3,4-difluorophenyl)ureidomethyl]pyridine-2-carboxylic acid amide (Compound | 1H-NMR (500 MHz, DMSO-$d_6$) δ 0.93 (t, J = 7.2 Hz, 6H), 1.69 (m, 2H), 2.38 (t, J = 6.7 Hz, 2H), 2.48 (m, 4H), 3.36 |

| | |
|---|---|
| No.1-46)<br>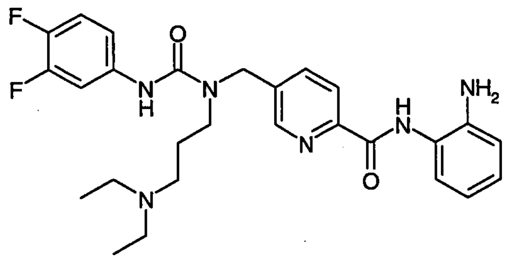 | (t, J = 6.7 Hz, 2H), 4.67 (s, 2H), 4.89 (br s, 2H), 6.65 (td, J = 7.6, 1.4 Hz, 1H), 6.82 (dd, J = 7.6, 1.4 Hz, 1H), 6.95 (td, J = 7.6, 1.4 Hz, 1H), 7.13 (m, 1H), 7.32 (dd, J = 19.7, 9.3 Hz, 1H), 7.50 (dd, J = 7.6, 1.4 Hz, 1H), 7.65 (ddd, J = 13.7, 7.6, 2.6 Hz, 1H), 7.95 (dd, J = 8.1, 1.8 Hz, 1H), 8.12 (d, J = 8.1 Hz, 1H), 8.65 (d, J = 1.8 Hz, 1H), 9.31 (br s, 1H), 10.03 (br s, 1H) |
| N-(2-Aminophenyl)-5-[1-(3-diethylaminopropyl)-3-(3,5-difluorophenyl)ureidomethyl]pyridine-2-carboxylic acid amide (Compound No.1-47)<br>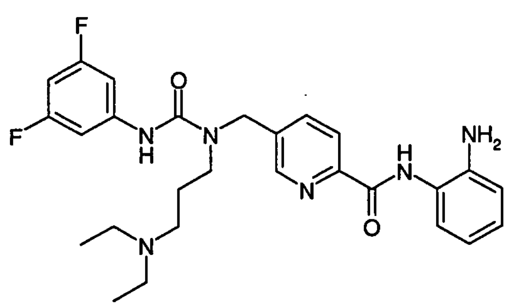 | $^1$H-NMR (500 MHz, DMSO-$d_6$) δ 0.94 (t, J = 7.2 Hz, 6H), 1.70 (m, 2H), 2.38 (t, J = 6.4 Hz, 2H), 2.49 (m, 4H), 3.38 (t, J = 6.4 Hz, 2H), 4.68 (s, 2H), 4.89 (br s, 2H), 6.65 (td, J = 7.8, 1.4 Hz, 1H), 6.77 (tt, J = 9.3, 2.4 Hz, 1H), 6.82 (dd, J = 7.8, 1.4 Hz, 1H), 6.95 (td, J = 7.8, 1.4 Hz, 1H), 7.20 (dd, J = 10.2, 2.4 Hz, 2H), 7.50 (dd, J = 7.8, 1.4 Hz, 1H), 7.95 (dd, J = 7.9, 1.8 Hz, 1H), 8.12 |

| | |
|---|---|
| | (d, J = 7.9 Hz, 1H), 8.66 (d, J = 1.8 Hz, 1H), 9.47 (br s, 1H), 10.03 (br s, 1H) |
| N-(2-Aminophenyl)-5-[3-(3-chloro-4-fluorophenyl)-1-(3-diethylaminopropyl)ureidomethyl]pyridine-2-carboxylic acid amide (Compound No.1-48) 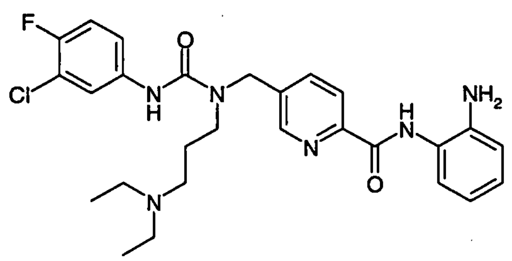 | $^1$H-NMR (500 MHz, DMSO-$d_6$) δ 0.93 (t, J = 7.0 Hz, 6H), 1.69 (m, 2H), 2.39 (t, J = 6.7 Hz, 2H), 2.48 (m, 4H), 3.37 (t, J = 6.7 Hz, 2H), 4.67 (s, 2H), 4.89 (br s, 2H), 6.65 (td, J = 7.8, 1.4 Hz, 1H), 6.82 (dd, J = 7.8, 1.4 Hz, 1H), 6.95 (td, J = 7.8, 1.4 Hz, 1H), 7.30-7.35 (m, 2H), 7.50 (dd, J = 7.8, 1.4 Hz, 1H), 7.75 (dd, J = 6.9, 2.3 Hz, 1H), 7.95 (dd, J = 7.9, 2.0 Hz, 1H), 8.12 (d, J = 7.9 Hz, 1H), 8.65 (d, J = 2.0 Hz, 1H), 9.26 (br s, 1H), 10.03 (br s, 1H) |
| N-(2-Aminophenyl)-5-[1-(3-diethylaminopropyl)-3-(3-fluorophenyl)ureidomethyl]pyridine-2-carboxylic acid amide (Compound No.1-49) | $^1$H-NMR (500 MHz, DMSO-$d_6$) δ 0.95 (t, J = 7.2 Hz, 6H), 1.71 (m, 2H), 2.39 (t, J = 6.6 Hz, 2H), 2.50 (m, 4H), 3.38 (t, J = 6.6 Hz, 2H), 4.67 (s, 2H), 4.89 (br s, 2H), 6.65 (td, J = 7.6, 1.2 Hz, 1H), 6.76 (td, |

| | |
|---|---|
| 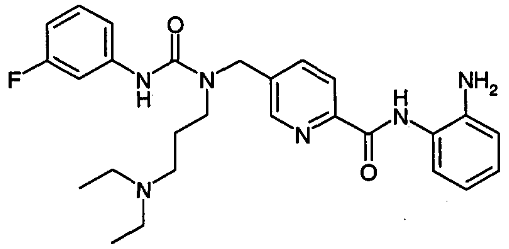 | J = 8.2, 2.3 Hz, 1H), 6.82 (dd, J = 7.6, 1.2 Hz, 1H), 6.95 (td, J = 7.6, 1.2 Hz, 1H), 7.15 (dd, J = 8.2, 2.3 Hz, 1H), 7.28 (dd, J = 15.3, 8.2 Hz, 1H), 7.46 (dt, J = 12.2, 2.3 Hz, 1H), 7.50 (dd, J = 7.6, 1.2 Hz, 1H), 7.95 (dd, J = 7.9, 1.8 Hz, 1H), 8.12 (d, J = 7.9 Hz, 1H), 8.66 (d, J = 1.8 Hz, 1H), 9.36 (br s, 1H), 10.03 (br s, 1H) |
| N-(2-Aminophenyl)-5-[1-(3-diethylaminopropyl)-3-(4-fluoro-3-methylphenyl)ureidomethyl]pyridine-2-carboxylic acid amide (Compound No.1-50) 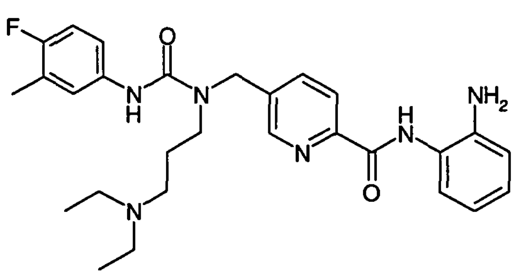 | $^1$H-NMR (500 MHz, DMSO-$d_6$) δ 0.93 (t, J = 7.0 Hz, 6H), 1.69 (m, 2H), 2.19 (d, J = 1.5 Hz, 3H), 2.39 (t, J = 6.6 Hz, 2H), 2.49 (q, J = 7.0 Hz, 4H), 3.36 (t, J = 6.6 Hz, 2H), 4.65 (s, 2H), 4.89 (s, 2H), 6.65 (td, J = 7.7, 1.2 Hz, 1H), 6.82 (dd, J = 7.7, 1.2 Hz, 1H), 6.95 (td, J = 7.7, 1.2 Hz, 1H), 7.02 (t, J = 9.3 Hz, 1H), 7.23 (m, 1H), 7.34 (dd, J = 7.0, 2.4 Hz, 1H), 7.51 (dd, J = 7.7, 1.2 Hz, 1H), 7.94 (dd, J = 7.9, 2.1 Hz, 1H), 8.12 (d, |

| | |
|---|---|
| | J = 7.9 Hz, 1H), 8.65 (d, J = 2.1 Hz, 1H), 9.04 (s, 1H), 10.03 (s, 1H) |
| N-(2-Aminophenyl)-5-[1-(3-diethylaminopropyl)-3-(4-fluoro-3-nitrophenyl)ureidomethyl]pyridine-2-carboxylic acid amide (Compound No.1-51) 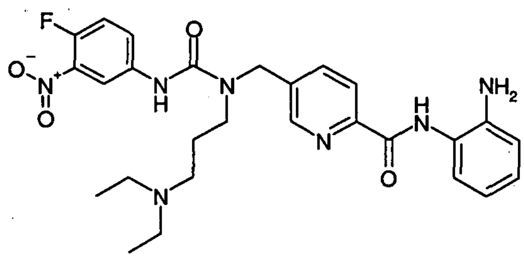 | $^1$H-NMR (400 MHz, DMSO-$d_6$) δ 0.94 (t, J = 7.1 Hz, 6H), 1.71 (m, 2H), 2.40 (t, J = 6.7 Hz, 2H), 2.49 (q, J = 7.1 Hz, 4H), 3.40 (t, J = 6.7 Hz, 2H), 4.70 (s, 2H), 4.90 (s, 2H), 6.65 (td, J = 7.7, 1.1 Hz, 1H), 6.83 (dd, J = 7.7, 1.1 Hz, 1H), 6.95 (td, J = 7.7, 1.1 Hz, 1H), 7.48-7.53 (m, 2H), 7.81 (m, 1H), 7.96 (dd, J = 8.2, 2.1 Hz, 1H), 8.13 (d, J = 8.2 Hz, 1H), 8.37 (dd, J = 6.8, 2.7 Hz, 1H), 8.67 (d, J = 2.1 Hz, 1H), 9.53 (s, 1H), 10.04 (s, 1H) |
| N-(2-Aminophenyl)-5-[1-(3-diethylaminopropyl)-3-(3-ethoxyphenyl)ureidomethyl]pyridine-2-carboxylic acid amide (Compound No.1-52) | $^1$H-NMR (400 MHz, DMSO-$d_6$) δ 0.95 (t, J = 7.1 Hz, 6H), 1.31 (t, J = 6.9 Hz, 3H), 1.71 (m, 2H), 2.39 (t, J = 6.5 Hz, 2H), 2.50 (m, 4H), 3.36 (t, J = 6.5 Hz, 2H), 3.97 (q, J = 6.9 Hz, 2H), 4.66 (s, 2H), 4.90 |

| | |
|---|---|
| 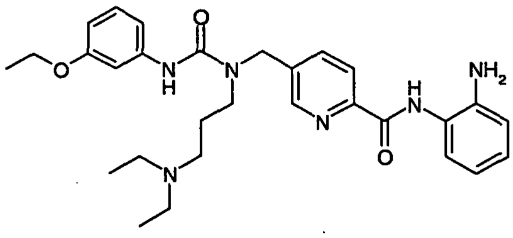 | (s, 2H), 6.51 (dd, J = 7.9, 2.1 Hz, 1H), 6.65 (td, J = 7.7, 1.1 Hz, 1H), 6.82 (dd, J = 7.7, 1.1 Hz, 1H), 6.93-6.97 (m, 2H), 7.11-7.15 (m, 2H), 7.51 (dd, J = 7.7, 1.1 Hz, 1H), 7.95 (dd, J = 7.9, 2.1 Hz, 1H), 8.13 (d, J = 7.9 Hz, 1H), 8.66 (d, J = 2.1 Hz, 1H), 9.15 (s, 1H), 10.04 (s, 1H) |
| N-(2-Aminophenyl)-5-[3-(2,3-dihydrobenzo[1,4]dioxin-6-yl)-1-(2-dimethylaminoethyl)ureidomethyl]pyridine-2-carboxylic acid amide (Compound No.1-53) 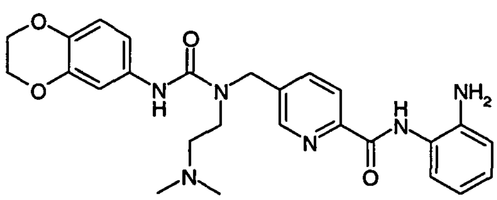 | $^1$H-NMR (400 MHz, $CDCl_3$) δ 2.38 (s, 6H), 2.51 (t, J = 4.3 Hz, 2H), 3.31 (t, J = 4.3 Hz, 2H), 3.96 (s, 2H), 4.21-4.24 (m, 4H), 4.65 (s, 2H), 6.77-6.88 (m, 4H), 6.93 (d, J = 2.2 Hz, 1H), 7.08 (td, J = 7.7, 1.5 Hz, 1H), 7.48 (d, J = 7.7 Hz, 1H), 7.90 (dd, J = 8.1, 2.2 Hz, 1H), 8.24 (d, J = 8.1 Hz, 1H), 8.57 (d, J = 2.2 Hz, 1H), 9.83 (s, 1H), 10.84 (s, 1H) |
| N-(2-Aminophenyl)-5-[1-(2-dimethylaminoethyl)-3-(3-methoxyphenyl)ureidomethyl]pyridin | $^1$H-NMR (400 MHz, $CDCl_3$) δ 2.40 (s, 6H), 2.53 (t, J = 4.3 Hz, 2H), 3.34 |

| | |
|---|---|
| e-2-carboxylic acid amide (Compound No.1-54)<br>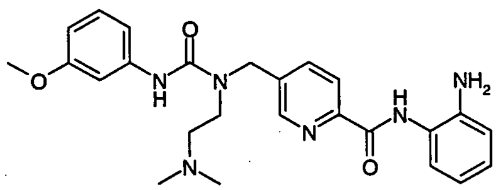 | (t, J = 4.3 Hz, 2H), 3.82 (s, 3H), 3.96 (s, 2H), 4.67 (s, 2H), 6.57 (ddd, J = 7.8, 2.4, 0.7 Hz, 1H), 6.81-6.87 (m, 3H), 7.09 (td, J = 7.6, 1.5 Hz, 1H), 7.15 (t, J = 2.4 Hz, 1H), 7.17 (t, J = 7.8 Hz, 1H), 7.48 (d, J = 7.8 Hz, 1H), 7.91 (dd, J = 8.1, 2.2 Hz, 1H), 8.26 (d, J = 8.1 Hz, 1H), 8.59 (d, J = 2.2 Hz, 1H), 9.83 (s, 1H), 11.11 (s, 1H) |
| N-(2-Aminophenyl)-5-[1-(2-dimethylaminoethyl)-3-(3-fluorophenyl)ureidomethyl]pyridine-2-carboxylic acid amide (Compound No.1-55)<br>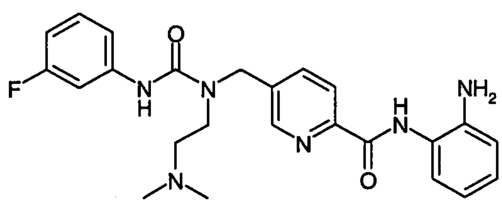 | $^1$H-NMR (500 MHz, $CDCl_3$)<br>δ 2.41 (s, 6H), 2.54 (t, J = 4.3 Hz, 2H), 3.34 (t, J = 4.3 Hz, 2H), 3.96 (s, 2H), 4.66 (s, 2H), 6.68 (td, J = 8.1, 2.3 Hz, 1H), 6.83-6.88 (m, 2H), 7.01 (dd, J = 8.1, 1.5 Hz, 1H), 7.08 (m, 1H), 7.20 (m, 1H), 7.28 (m, 1H), 7.48 (d, J = 8.1 Hz, 1H), 7.91 (dd, J = 7.9, 2.1 Hz, 1H), 8.26 (d, J = 7.9 Hz, 1H), 8.59 (d, J = 2.1 Hz, 1H), 9.83 (s, 1H), 11.31 (s, 1H) |
| N-(2-Aminophenyl)-5-[1-(2-dimethylaminoeth | $^1$H-NMR (400 MHz, $CDCl_3$) |

| | |
|---|---|
| yl)-3-(thiophen-3-yl)ureidomethyl]pyridine-2-carboxylic acid amide (Compound No.1-56) 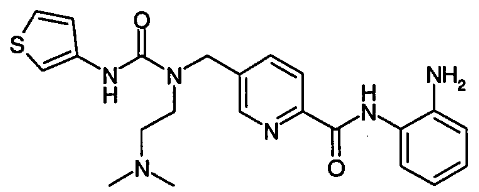 | δ 2.39 (s, 6H), 2.52 (t, J = 4.4 Hz, 2H), 3.31 (d, J = 4.4 Hz, 2H), 3.96 (s, 2H), 4.67 (s, 2H), 6.83-6.87 (m, 3H), 7.08 (td, J = 7.8, 1.5 Hz, 1H), 7.21 (dd, J = 5.1, 3.3 Hz, 1H), 7.30 (dd, J = 3.3, 1.2 Hz, 1H), 7.48 (d, J = 7.8 Hz, 1H), 7.90 (dd, J = 8.1, 2.2 Hz, 1H), 8.25 (dd, J = 8.1, 0.7 Hz, 1H), 8.57 (d, J = 2.2 Hz, 1H), 9.83 (s, 1H), 11.48 (s, 1H) |
| N-(2-Aminophenyl)-5-[3-(3,4-difluorophenyl)-1-[3-(pyrrolidin-1-yl)propyl]ureidomethyl]pyridine-2-carboxylic acid amide (Compound No.1-57) 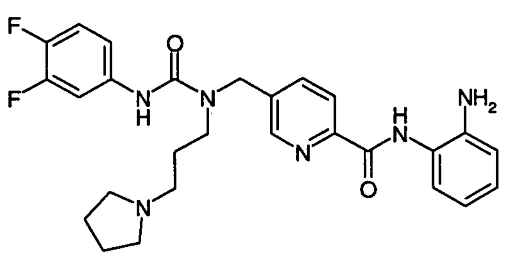 | $^1$H-NMR (500 MHz, DMSO-$d_6$) δ 1.70-1.73 (m, 6H), 2.41-2.45 (m, 6H), 3.40 (t, J = 6.3 Hz, 2H), 4.65 (s, 2H), 4.89 (br s, 2H), 6.65 (td, J = 7.6, 1.2 Hz, 1H), 6.82 (dd, J = 7.6, 1.2 Hz, 1H), 6.95 (td, J = 7.6, 1.2 Hz, 1H), 7.11 (m, 1H), 7.34 (dd, J = 19.9, 9.2 Hz, 1H), 7.51 (dd, J = 7.6, 1.2 Hz, 1H), 7.65 (ddd, J = 13.7, 7.6, 2.4 Hz, 1H), 7.95 (dd, J = 7.9, 1.8 Hz, 1H), 8.12 (d, J = 7.9 Hz, 1H), 8.66 (d, J = 1.8 Hz, 1H), 9.57 |

| | |
|---|---|
| | (br s, 1H), 10.03 (br s, 1H) |
| N-(2-Aminophenyl)-5-[3-(pyridin-3-yl)-1-[3-(pyrrolidin-1-yl)propyl]ureidomethyl]pyridine-2-carboxylic acid amide (Compound No.1-58)<br>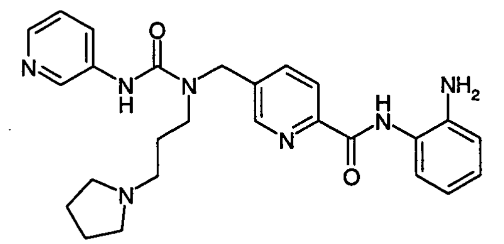 | $^1$H-NMR (500 MHz, DMSO-$d_6$)<br>δ 1.73-1.75 (m, 6H), 2.43-2.46 (m, 6H), 3.43 (t, J = 6.3 Hz, 2H), 4.66 (s, 2H), 4.89 (br s, 2H), 6.65 (t, J = 7.6 Hz, 1H), 6.82 (dd, J = 7.6, 1.2 Hz, 1H), 6.95 (td, J = 7.6, 1.2 Hz, 1H), 7.30 (dd, J = 8.2, 4.6 Hz, 1H), 7.51 (dd, J = 7.6, 1.2 Hz, 1H), 7.90 (ddd, J = 8.2, 2.6, 1.5 Hz, 1H), 7.96 (dd, J = 7.9, 1.8 Hz, 1H), 8.12 (d, J = 7.9 Hz, 1H), 8.17 (dd, J = 4.6, 1.5 Hz, 1H), 8.56 (d, J = 2.6 Hz, 1H), 8.66 (d, J = 1.8 Hz, 1H), 9.59 (br s, 1H), 10.03 (br s, 1H) |
| N-(2-Aminophenyl)-5-[1-[3-(morpholin-4-yl)propyl]-3-(phenylcarbonylmethyl)ureidomethyl]pyridine-2-carboxylic acid amide (Compound No.1-59) | $^1$H-NMR (400 MHz, $CDCl_3$)<br>δ 1.75 (m, 2H), 2.42-2.52 (m, 6H), 3.39 (t, J = 6.0 Hz, 2H), 3.67 (t, J = 4.4 Hz, 4H), 3.96 (s, 2H), 4.63 (s, 2H), 4.72 (br s, 2H), 6.82-6.84 (m, 2H), 7.07 (dd, J = 7.6, 1.5 Hz, |

| | |
|---|---|
| 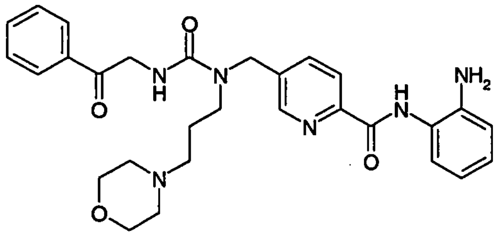 | 1H), 7.47-7.52 (m, 3H), 7.61 (d, J = 7.6 Hz, 2H), 7.86 (dd, J = 8.0, 2.2 Hz, 1H), 7.97-8.01 (m, 2H), 8.23 (dd, J = 8.0, 0.7 Hz, 1H), 8.56 (d, J = 2.2 Hz, 1H), 9.83 (s, 1H) |
| N-(2-Aminophenyl)-5-[3-(3-chlorophenyl)-1-[3-(morpholin-4-yl)propyl]ureidomethyl]pyridine-2-carboxylic acid amide (Compound No.1-60) 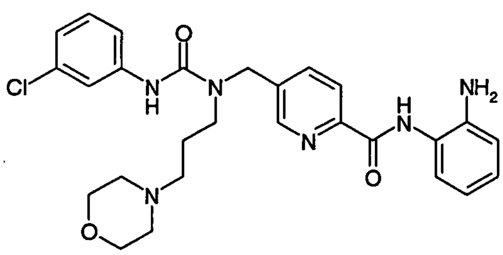 | $^1$H-NMR (500 MHz, $CDCl_3$) δ 1.80 (m, 2H), 2.43-2.49 (m, 6H), 3.39 (t, J = 5.7 Hz, 2H), 3.68-3.74 (m, 4H), 3.95 (s, 2H), 4.65 (s, 2H), 6.84-6.88 (m, 2H), 7.05-7.10 (m, 2H), 7.25 (m, 1H), 7.32 (dd, J = 8.3, 1.2 Hz, 1H), 7.48 (d, J = 7.9 Hz, 1H), 7.59 (m, 1H), 7.91 (dd, J = 7.9, 2.0 Hz, 1H), 8.25 (d, J = 7.9 Hz, 1H), 8.59 (d, J = 2.0 Hz, 1H), 9.05 (s, 1H), 9.82 (s, 1H) |
| N-(2-Aminophenyl)-5-[3-(2-fluorophenethyl)-1-[3-(morpholin-4-yl)propyl]ureidomethyl]pyridine-2-carboxylic acid amide (Compound No.1-61) | $^1$H-NMR (500 MHz, DMSO-$d_6$) δ 1.58 (m, 2H), 2.21 (t, J = 6.8 Hz, 2H), 2.25 (br s, 4H), 2.80 (t, J = 6.8 Hz, 2H), 3.13 (t, J = 6.8 Hz, 2H), 3.32 (m, 2H), 3.51 (br s, 4H), 4.55 (s, 2H), 4.90 (br s, 2H), 6.66 (t, J = |

| | |
|---|---|
| 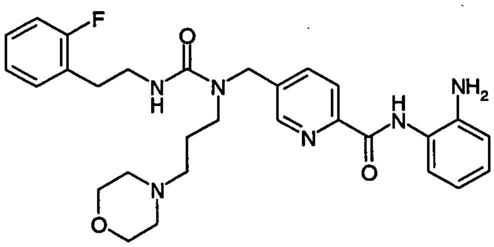 | 7.9 Hz, 1H), 6.83 (d, J = 7.9 Hz, 1H), 6.93-7.01 (m, 2H), 7.10-7.18 (m, 2H), 7.23-7.30 (m, 2H), 7.52 (m, 1H), 7.80 (dd, J = 8.1, 1.8 Hz, 1H), 8.09 (d, J = 8.1 Hz, 1H), 8.55 (d, J = 1.8 Hz, 1H), 10.04 (s, 1H) |
| N-(2-Aminophenyl)-5-[3-(4-fluorophenethyl)-1-[3-(morpholin-4-yl)propyl]ureidomethyl]pyridine-2-carboxylic acid amide (Compound No.1-62) 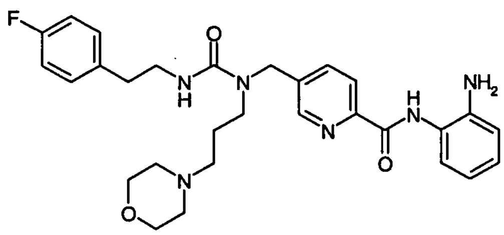 | $^1$H-NMR (500 MHz, DMSO-$d_6$) δ 1.58 (m, 2H), 2.20 (t, J = 6.8 Hz, 2H), 2.26 (br s, 4H), 2.74 (t, J = 6.8 Hz, 2H), 3.14 (t, J = 6.8 Hz, 2H), 3.29 (m, 2H), 3.51 (t, J = 4.3 Hz, 4H), 4.55 (s, 2H), 4.89 (s, 2H), 6.66 (t, J = 7.7 Hz, 1H), 6.83 (d, J = 7.7 Hz, 1H), 6.86 (t, J = 5.6 Hz, 1H), 6.96 (td, J = 7.7, 1.4 Hz, 1H), 7.10 (t, J = 8.9 Hz, 2H), 7.22 (dd, J = 8.9, 5.6 Hz, 2H), 7.51 (m, 1H), 7.80 (dd, J = 8.1, 1.8 Hz, 1H), 8.10 (d, J = 8.1 Hz, 1H), 8.55 (d, J = 1.8 Hz, 1H), 10.04 (s, 1H) |
| N-(2-Aminophenyl)-5-[3-(3-fluorophenyl)-1-[2-(4-methylpiperazin-1-yl)ethyl]ureidomethyl]p | $^1$H-NMR (400 MHz, DMSO-$d_6$) δ 2.13 (s, 3H), 2.30 (s, 4H), 2.46 (br s, 4H), |

| | |
|---|---|
| yridine-2-carboxylic acid amide (Compound No.1-63) 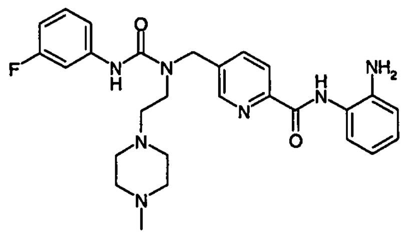 | 2.48 (m, 2H), 3.49 (t, J = 5.4 Hz, 2H), 4.70 (s, 2H), 4.89 (s, 2H), 6.65 (t, J = 7.6 Hz, 1H), 6.77 (m, 1H), 6.83 (d, J = 8.1 Hz, 1H), 6.95 (td, J = 7.6, 1.3 Hz, 1H), 7.22-7.32 (m, 2H), 7.45 (d, J = 12.2 Hz, 1H), 7.51 (d, J = 7.6 Hz, 1H), 7.96 (dd, J = 8.1, 1.8 Hz, 1H), 8.12 (d, J = 8.1 Hz, 1H), 8.65 (d, J = 1.8 Hz, 1H), 9.37 (s, 1H), 10.04 (s, 1H) |
| N-(2-Aminophenyl)-5-[3-(3,4-difluorophenyl)-1-[2-(4-methylpiperazin-1-yl)ethyl]ureidomethyl]pyridine-2-carboxylic acid amide (Compound No.1-64) 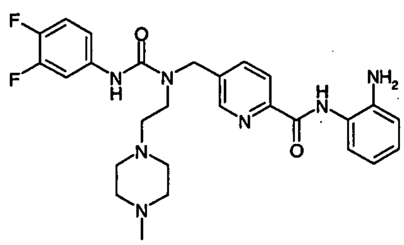 | $^1$H-NMR (500 MHz, DMSO-$d_6$) δ 2.12 (s, 3H), 2.28 (br s, 4H), 2.44 (br s, 4H), 2.48 (t, J = 5.7 Hz, 2H), 3.48 (t, J = 5.7 Hz, 2H), 4.70 (s, 2H), 4.89 (s, 2H), 6.65 (t, J = 7.9 Hz, 1H), 6.83 (d, J = 7.9 Hz, 1H), 6.95 (t, J = 7.9 Hz, 1H), 7.21 (d, J = 9.2 Hz, 1H), 7.33 (m, 9.2 Hz, 1H), 7.51 (d, J = 7.9 Hz, 1H), 7.63 (m, 1H), 7.95 (dd, J = 7.9, 1.5 Hz, 1H), 8.12 (d, J = 7.9 Hz, 1H), 8.64 (d, J = 1.5 Hz, 1H), 9.31 (s, 1H), 10.03 (s, 1H) |

| | |
|---|---|
| N-(2-Aminophenyl)-5-[3-(2,3-dihydrobenzo[1,4]dioxin-6-yl)-1-[2-(4-methylpiperazin-1-yl)ethyl]ureidomethyl]pyridine-2-carboxylic acid amide (Compound No.1-65) 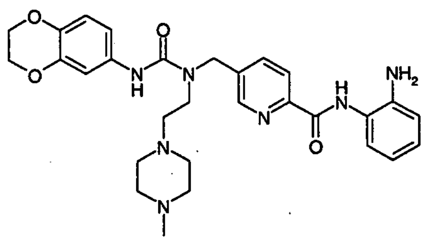 | $^1$H-NMR (400 MHz, DMSO-d$_6$) δ 2.13 (s, 3H), 2.30 (br s, 4H), 2.46 (br s, 4H), 2.47 (t, J = 4.6 Hz, 2H), 3.44 (t, J = 4.6 Hz, 2H), 4.17-4.21 (m, 4H), 4.67 (s, 2H), 4.89 (s, 2H), 6.65 (t, J = 7.4 Hz, 1H), 6.74 (d, J = 8.8 Hz, 1H), 6.81-6.87 (m, 2H), 6.95 (t, J = 7.4 Hz, 1H), 7.03 (d, J = 2.0 Hz, 1H), 7.51 (d, J = 7.8 Hz, 1H), 7.94 (d, J = 7.8 Hz, 1H), 8.11 (d, J = 7.8 Hz, 1H), 8.63 (s, 1H), 9.02 (s, 1H), 10.04 (s, 1H) |
| N-(2-Aminophenyl)-5-[3-(3-fluorophenyl)-1-[3-(4-methylpiperazin-1-yl)propyl]ureidomethyl]pyridine-2-carboxylic acid amide (Compound No.1-66) 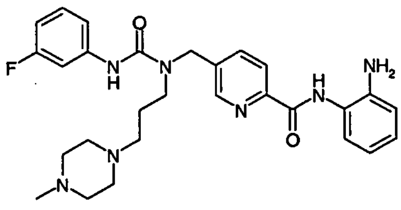 | $^1$H-NMR (400 MHz, DMSO-d$_6$) δ 1.71 (m, 2H), 2.13 (s, 3H), 2.28 (t, J = 6.4 Hz, 2H), 2.31 (br s, 8H), 3.38 (t, J = 6.4 Hz, 2H), 4.70 (s, 2H), 4.89 (s, 2H), 6.65 (t, J = 7.6 Hz, 1H), 6.76-6.83 (m, 2H), 6.95 (t, J = 7.6 Hz, 1H), 7.27-7.29 (m, 2H), 7.46-7.51 (m, 2H), 7.94 (d, J = 8.1 Hz, 1H), 8.12 (d, J = 8.1 Hz, 1H), 8.65 (s, 1H), 8.84 (s, 1H), |

| | |
|---|---|
| | 10.03 (s, 1H) |
| N-(2-Aminophenyl)-5-[3-(3,4-difluorophenyl)-1-[3-(4-methylpiperazin-1-yl)propyl]ureidomethyl]pyridine-2-carboxylic acid amide (Compound No.1-67) 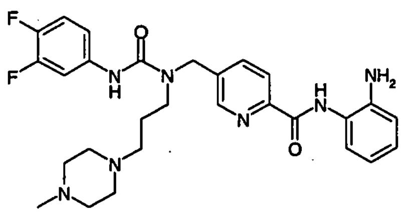 | $^1$H-NMR (400 MHz, DMSO-d$_6$) δ 1.70 (m, 2H), 2.12 (s, 3H), 2.28 (t, J = 6.7 Hz, 2H), 2.32 (br s, 8H), 3.37 (t, J = 6.7 Hz, 2H), 4.69 (s, 2H), 4.89 (s, 2H), 6.65 (t, J = 7.6 Hz, 1H), 6.82 (d, J = 7.6 Hz, 1H), 6.95 (t, J = 7.6 Hz, 1H), 7.25-7.33 (m, 2H), 7.50 (d, J = 7.6 Hz, 1H), 7.65 (m, 1H), 7.93 (dd, J = 8.1, 1.7 Hz, 1H), 8.12 (d, J = 8.1 Hz, 1H), 8.64 (d, J = 1.7 Hz, 1H), 8.86 (s, 1H), 10.03 (s, 1H) |
| N-(2-Aminophenyl)-5-[3-(2,3-dihydrobenzo[1,4]dioxin-6-yl)-1-[3-(4-methylpiperazin-1-yl)propyl]ureidomethyl]pyridine-2-carboxylic acid amide (Compound No.1-68) 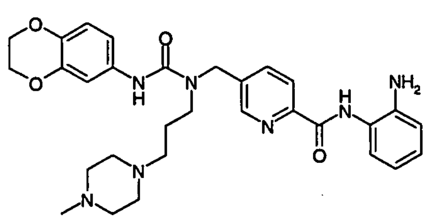 | $^1$H-NMR (400 MHz, DMSO-d$_6$) δ 1.69 (m, 2H), 2.12 (s, 3H), 2.28 (t, J = 6.6 Hz, 2H), 2.32 (br s, 8H), 3.33 (m, 2H), 4.17-4.21 (m, 4H), 4.65 (s, 2H), 4.89 (s, 2H), 6.65 (t, J = 7.6 Hz, 1H), 6.73 (d, J = 8.7 Hz, 1H), 6.82 (dd, J = 7.6, 1.4 Hz, 1H), 6.87 (dd, J = 8.7, 2.4 Hz, 1H), 6.95 (td, J = 7.6, |

| | |
|---|---|
| | 1.4 Hz, 1H), 7.05 (d, J = 2.4 Hz, 1H), 7.50 (d, J = 7.6 Hz, 1H), 7.92 (dd, J = 8.1, 2.0 Hz, 1H), 8.12 (d, J = 8.1 Hz, 1H), 8.55 (s, 1H), 8.63 (d, J = 2.0 Hz, 1H), 10.03 (s, 1H) |
| N-(2-Aminophenyl)-5-[3-(3-fluorophenyl)-1-[4-(pyrrolidin-1-yl)butyl]ureidomethyl]pyridine-2-carboxylic acid amide (Compound No.1-69) 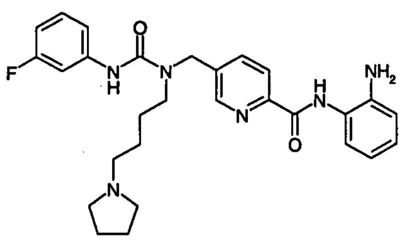 | $^1$H-NMR (400 MHz, DMSO-$d_6$) δ 1.41 (m, 2H), 1.55 (m, 2H), 1.63 (m, 4H), 2.32-2.36 (m, 6H), 3.40 (t, J = 7.3 Hz, 2H), 4.71 (s, 2H), 4.89 (s, 2H), 6.65 (t, J = 7.8 Hz, 1H), 6.76 (m, 1H), 6.82 (d, J = 7.8 Hz, 1H), 6.95 (t, J = 7.8 Hz, 1H), 7.23-7.29 (m, 2H), 7.44-7.53 (m, 2H), 7.93 (dd, J = 8.1, 1.8 Hz, 1H), 8.12 (d, J = 8.1 Hz, 1H), 8.62 (s, 1H), 8.64 (d, J = 1.8 Hz, 1H), 10.03 (s, 1H) |
| N-(2-Aminophenyl)-5-[3-(3,4-difluorophenyl)-1-[4-(pyrrolidin-1-yl)butyl]ureidomethyl]pyridine-2-carboxylic acid amide (Compound No.1-70) | $^1$H-NMR (500 MHz, DMSO-$d_6$) δ 1.41 (m, 2H), 1.54 (m, 2H), 1.63 (m, 4H), 2.33-2.36 (m, 6H), 3.39 (t, J = 7.3 Hz, 2H), 4.70 (s, 2H), 4.89 (s, 2H), 6.65 (t, J = 7.9 |

| | |
|---|---|
| 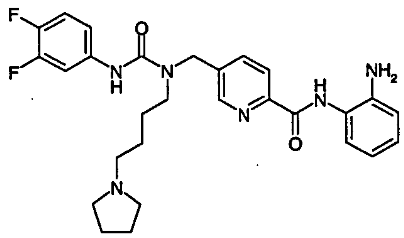 | Hz, 1H), 6.83 (d, J = 7.9 Hz, 1H), 6.95 (t, J = 7.9 Hz, 1H), 7.26 (m, 1H), 7.31 (m, 1H), 7.50 (d, J = 7.9 Hz, 1H), 7.65 (m, 1H), 7.93 (dd, J = 8.1, 2.0 Hz, 1H), 8.12 (d, J = 8.1 Hz, 1H), 8.63 (s, 1H), 8.64 (d, J = 2.0 Hz, 1H), 10.03 (s, 1H) |
| N-(2-Aminophenyl)-5-[3-(2,3-dihydrobenzo[1,4]dioxin-6-yl)-1-[4-(pyrrolidin-1-yl)butyl]ureido methyl]pyridine-2-carboxylic acid amide (Compound No.1-71) 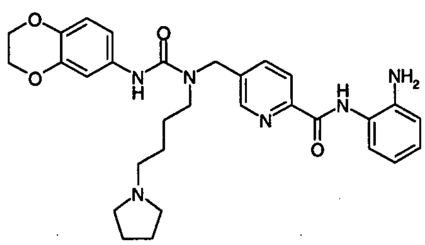 | $^1$H-NMR (500 MHz, DMSO-d$_6$) δ 1.40 (m, 2H), 1.53 (m, 2H), 1.63 (m, 4H), 2.32-2.36 (m, 6H), 3.35 (t, J = 7.5 Hz, 2H), 4.17-4.21 (m, 4H), 4.67 (s, 2H), 4.89 (s, 2H), 6.65 (t, J = 7.8 Hz, 1H), 6.72 (d, J = 8.6 Hz, 1H), 6.82 (d, J = 7.8 Hz, 1H), 6.87 (dd, J = 8.6, 2.4 Hz, 1H), 6.95 (t, J = 7.8 Hz, 1H), 7.04 (d, J = 2.4 Hz, 1H), 7.50 (d, J = 7.8 Hz, 1H), 7.91 (dd, J = 8.1, 2.0 Hz, 1H), 8.11 (d, J = 8.1 Hz, 1H), 8.23 (s, 1H), 8.62 (d, J = 2.0 Hz, 1H), 10.03 (s, 1H) |
| N-(2-Aminophenyl)-5-[1-(2-diethylaminoethyl | $^1$H-NMR (400 MHz, DMSO-d$_6$) |

| | |
|---|---|
| )-3-(3-fluorophenyl)ureidomethyl]pyridine-2-carboxylic acid amide (Compound No.1-72) 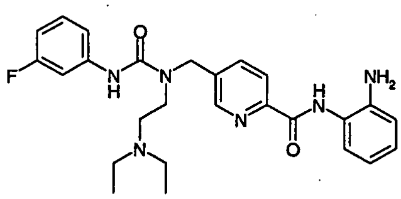 | δ 0.99 (t, J = 7.1 Hz, 6H), 2.57-2.62 (m, 6H), 3.41 (t, J = 4.6 Hz, 2H), 4.67 (s, 2H), 4.90 (s, 2H), 6.66 (td, J = 7.7, 1.1 Hz, 1H), 6.76 (td, J = 8.1, 2.2 Hz, 1H), 6.83 (dd, J = 7.7, 1.1 Hz, 1H), 6.96 (td, J = 7.7, 1.1 Hz, 1H), 7.03 (dd, J = 8.1, 1.1 Hz, 1H), 7.29 (dd, J = 15.4, 8.1 Hz, 1H), 7.39 (dt, J = 12.1, 2.2 Hz, 1H), 7.52 (dd, J = 7.7, 1.1 Hz, 1H), 7.98 (dd, J = 8.1, 1.8 Hz, 1H), 8.13 (d, J = 8.1 Hz, 1H), 8.67 (d, J = 1.8 Hz, 1H), 10.05 (s, 1H), 10.72 (s, 1H) |
| N-(2-Aminophenyl)-5-[1-(2-diethylaminoethyl)-3-(3,4-difluorophenyl)ureidomethyl]pyridine-2-carboxylic acid amide (Compound No.1-73) 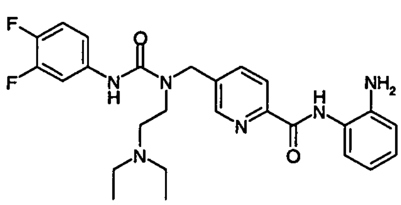 | $^1H$-NMR (400 MHz, DMSO-$d_6$) δ 0.98 (t, J = 7.1 Hz, 6H), 2.56-2.61 (m, 6H), 3.40 (t, J = 4.8 Hz, 2H), 4.67 (s, 2H), 4.90 (s, 2H), 6.66 (td, J = 7.7, 1.3 Hz, 1H), 6.83 (dd, J = 7.7, 1.3 Hz, 1H), 6.96 (td, J = 7.7, 1.3 Hz, 1H), 7.02 (d, J = 9.0 Hz, 1H), 7.32 (m, 1H), 7.52 (dd, J = 7.7, 1.3 Hz, 1H), |

| | |
|---|---|
| | 7.58 (m, 1H), 7.97 (dd, J = 8.1, 2.0 Hz, 1H), 8.13 (d, J = 8.1 Hz, 1H), 8.67 (d, J = 2.0 Hz, 1H), 10.04 (s, 1H), 10.61 (s, 1H) |
| N-(2-Aminophenyl)-5-[1-(2-diethylaminoethyl)-3-(2,3-dihydrobenzo[1,4]dioxin-6-yl)ureidomethyl]pyridine-2-carboxylic acid amide (Compound No.1-74) 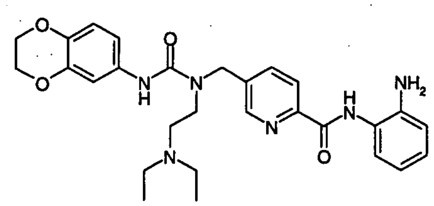 | $^1$H-NMR (500 MHz, DMSO-$d_6$) δ 0.98 (t, J = 7.2 Hz, 6H), 2.54-2.58 (m, 6H), 3.36 (t, J = 4.7 Hz, 2H), 4.17-4.21 (m, 4H), 4.64 (s, 2H), 4.90 (s, 2H), 6.65 (td, J = 7.9, 1.2 Hz, 1H), 6.73-6.74 (m, 2H), 6.83 (dd, J = 7.9, 1.2 Hz, 1H), 6.94-6.97 (m, 2H), 7.51 (dd, J = 7.9, 1.2 Hz, 1H), 7.95 (dd, J = 8.1, 2.0 Hz, 1H), 8.12 (d, J = 8.1 Hz, 1H), 8.65 (d, J = 2.0 Hz, 1H), 10.04 (s, 1H), 10.15 (s, 1H) |
| N-(2-Aminophenyl)-5-[3-(2,3-dihydrobenzo[1,4]dioxin-6-yl)-1-(4-dimethylaminobutyl)ureidomethyl]pyridine-2-carboxylic acid amide (Compound No.1-75) | $^1$H-NMR (500 MHz, $CDCl_3$) δ 1.53 (m, 2H), 1.69 (m, 2H), 2.21 (s, 6H), 2.36 (t, J = 6.4 Hz, 2H), 3.24 (t, J = 8.4 Hz, 2H), 3.96 (s, 2H), 4.23 (s, 4H), 4.68 (s, 2H), 6.78-6.80 (m, 2H), 6.85 (dd, J = 7.8, 1.5 Hz, |

| | |
|---|---|
| 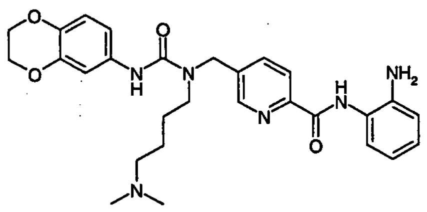 | 1H), 6.86 (td, J = 7.8, 1.5 Hz, 1H), 6.91 (dd, J = 1.8, 0.9 Hz, 1H), 7.09 (td, J = 7.8, 1.5 Hz, 1H), 7.49 (dd, J = 7.8, 1.5 Hz, 1H), 7.87 (s, 1H), 7.89 (dd, J = 8.0, 2.1 Hz, 1H), 8.24 (d, J = 8.0 Hz, 1H), 8.58 (d, J = 2.1 Hz, 1H), 9.83 (s, 1H) |
| N-(2-Aminophenyl)-5-[1-(4-dimethylaminobutyl)-3-(4-dimethylaminophenyl)ureidomethyl]pyridine-2-carboxylic acid amide (Compound No.1-76) 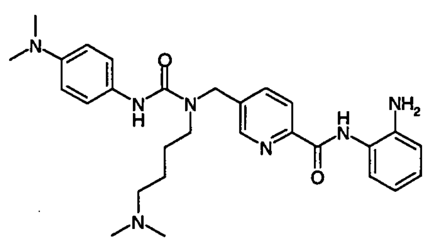 | $^1$H-NMR (400 MHz, $CDCl_3$) δ 1.53 (m, 2H), 1.68 (m, 2H), 2.22 (s, 6H), 2.37 (t, J = 6.6 Hz, 2H), 2.91 (s, 6H), 3.26 (t, J = 8.2 Hz, 2H), 3.96 (s, 2H), 4.69 (s, 2H), 6.72 (d, J = 9.0 Hz, 2H), 6.85 (dd, J = 7.8, 1.5 Hz, 1H), 6.86 (td, J = 7.8, 1.5 Hz, 1H), 7.09 (td, J = 7.8, 1.5 Hz, 1H), 7.18 (d, J = 9.0 Hz, 2H), 7.48 (dd, J = 7.8, 1.5 Hz, 1H), 7.79 (s, 1H), 7.90 (dd, J = 8.0, 2.1 Hz, 1H), 8.24 (dd, J = 8.0, 0.7 Hz, 1H), 8.58 (d, J = 2.1 Hz, 1H), 9.84 (s, 1H) |
| N-(2-Aminophenyl)-5-[1-(4-dimethylaminobut | $^1$H-NMR (500 MHz, $CDCl_3$) |

| | |
|---|---|
| yl)-3-(3-fluorophenyl)ureidomethyl]pyridine-2-carboxylic acid amide (Compound No.1-77) 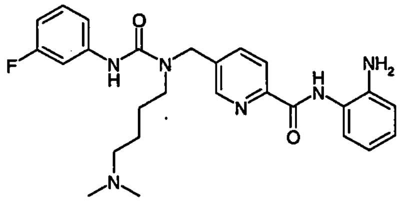 | δ 1.56 (m, 2H), 1.72 (m, 2H), 2.25 (s, 6H), 2.38 (t, J = 6.4 Hz, 2H), 3.27 (t, J = 8.4 Hz, 2H), 3.96 (s, 2H), 4.70 (s, 2H), 6.76 (tdd, J = 8.2, 2.4, 0.9 Hz, 1H), 6.85 (dd, J = 7.8 Hz, 1H), 6.86 (td, J = 7.8, 1.5 Hz, 1H), 7.06-7.10 (m, 2H), 7.22-7.27 (m, 2H), 7.49 (dd, J = 7.8, 1.5 Hz, 1H), 7.89 (dd, J = 7.9, 2.3 Hz, 1H), 7.96 (s, 1H), 8.26 (d, J = 7.9 Hz, 1H), 8.59 (d, J = 2.3 Hz, 1H), 9.83 (s, 1H) |
| N-(2-Aminophenyl)-5-[3-(1,3-benzothiazol-2-yl)-1-[3-(morpholin-4-yl)propyl]ureidomethyl]pyridine-2-carboxylic acid amide (Compound No.1-78) 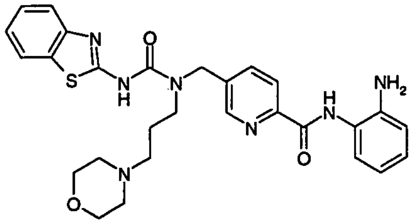 | $^1$H-NMR (500 MHz, DMSO-d$_6$) δ 1.77 (br s, 2H), 2.39 (br s, 6H), 3.46 (br s, 2H), 3.79 (br s, 4H), 4.74 (br s, 2H), 4.89 (br s, 2H), 6.65 (t, J = 7.7 Hz, 1H), 6.82 (d, J = 7.7 Hz, 1H), 6.95 (t, J = 7.7 Hz, 1H), 7.22 (t, J = 7.0 Hz, 1H), 7.36 (t, J = 7.0 Hz, 1H), 7.50 (d, J = 7.7 Hz, 1H), 7.60 (br s, 1H), 7.87 (br s, 1H), 7.97 (d, J = 7.8 Hz, 1H), 8.13 (d, J = 7.8 Hz, 1H), 8.69 (s, 1H), 10.03 |

| | |
|---|---|
| | (br s, 1H) |
| N-(2-Aminophenyl)-5-[1-(2-dimethylaminoethyl)-3-(3-methylphenyl)ureidomethyl]pyridine-2-carboxylic acid amide (Compound No.1-79) 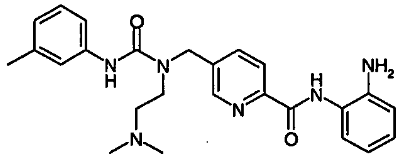 | $^1$H-NMR (500 MHz, DMSO-$d_6$) δ 2.26 (s, 3H), 2.28 (s, 6H), 2.49 (m, 2H), 3.42 (t, J = 4.9 Hz, 2H), 4.66 (s, 2H), 4.90 (s, 2H), 6.65 (t, J = 7.6 Hz, 1H), 6.75 (d, J = 6.4 Hz, 1H), 6.83 (d, J = 7.6 Hz, 1H), 6.95 (t, J = 7.6 Hz, 1H), 7.11-7.15 (m, 2H), 7.24 (s, 1H), 7.52 (d, J = 7.6 Hz, 1H), 7.96 (dd, J = 7.9, 1.8 Hz, 1H), 8.12 (d, J = 7.9 Hz, 1H), 8.66 (d, J = 1.8 Hz, 1H), 10.04 (s, 1H), 10.17 (s, 1H) |
| N-(2-Aminophenyl)-5-[3-(3-chlorophenyl)-1-(2-dimethylaminoethyl)ureidomethyl]pyridine-2-carboxylic acid amide (Compound No.1-80) 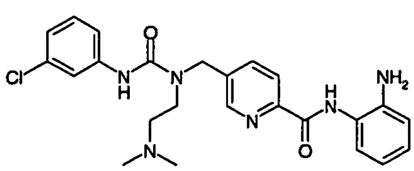 | $^1$H-NMR (500 MHz, DMSO-$d_6$) δ 2.28 (s, 6H), 2.50 (m, 2H), 3.44 (m, 2H), 4.67 (s, 2H), 4.90 (s, 2H), 6.65 (t, J = 7.6 Hz, 1H), 6.83 (d, J = 7.9 Hz, 1H), 6.94-6.99 (m, 2H), 7.19 (d, J = 7.6 Hz, 1H), 7.27 (t, J = 7.6 Hz, 1H), 7.51 (d, J = 7.6 Hz, 1H), 7.66 (s, 1H), 7.97 (d, J = 7.9 Hz, 1H), 8.12 (d, J = |

| | |
|---|---|
| | 7.9 Hz, 1H), 8.66 (s, 1H), 10.04 (s, 1H), 10.52 (s, 1H) |
| N-(2-Aminophenyl)-5-[1-(2-dimethylaminoethyl)-3-(pyridin-3-yl)ureidomethyl]pyridine-2-carboxylic acid amide (Compound No.1-81)<br>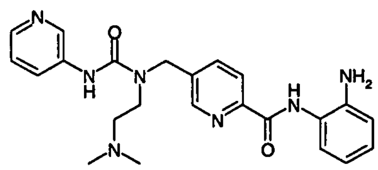 | $^1$H-NMR (400 MHz, DMSO-$d_6$)<br>δ 2.29 (s, 6H), 2.51 (m, 2H), 3.46 (t, J = 4.9 Hz, 2H), 4.69 (s, 2H), 4.90 (s, 2H), 6.65 (t, J = 7.6 Hz, 1H), 6.83 (d, J = 7.6 Hz, 1H), 6.95 (t, J = 7.6 Hz, 1H), 7.29 (dd, J = 8.3, 4.6 Hz, 1H), 7.51 (d, J = 7.6 Hz, 1H), 7.86 (d, J = 8.3 Hz, 1H), 7.98 (dd, J = 8.1, 2.0 Hz, 1H), 8.13 (d, J = 8.1 Hz, 1H), 8.16 (dd, J = 4.6, 1.2 Hz, 1H), 8.53 (d, J = 2.0 Hz, 1H), 8.67 (d, J = 1.2 Hz, 1H), 10.04 (s, 1H), 10.56 (s, 1H) |
| N-(2-Aminophenyl)-5-[3-(6-fluoro-1,3-benzothiazol-2-yl)-1-[3-(morpholin-4-yl)propyl]ureidomethyl]pyridine-2-carboxylic acid amide (Compound No.1-82) | $^1$H-NMR (500 MHz, DMSO-$d_6$)<br>δ 1.77 (br s, 2H), 2.18 (t, J = 7.5 Hz, 2H), 2.40 (br s, 4H), 3.45 (br s, 2H), 3.78 (br s, 4H), 4.74 (s, 2H), 4.89 (br s, 2H), 6.65 (t, J = 7.8 Hz, 1H), 6.82 (dd, J = 7.8, 1.2 Hz, 1H), |

| | |
|---|---|
| 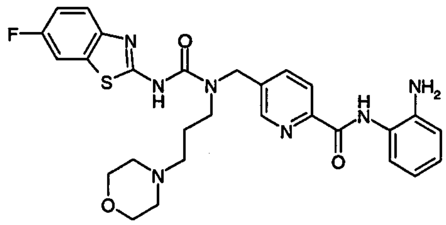 | 6.95 (td, J = 7.8, 1.2 Hz, 1H), 7.21 (t, J = 7.9 Hz, 1H), 7.50 (d, J = 7.8 Hz, 1H), 7.60 (br s, 1H), 7.79 (br s, 1H), 7.97 (d, J = 8.1 Hz, 1H), 8.13 (d, J = 8.1 Hz, 1H), 8.68 (s, 1H), 10.03 (br s, 1H), 11.94 (br s, 1H) |
| N-(2-Aminophenyl)-5-[3-(3-chlorophenyl)-1-(3-dimethylaminopropyl)ureidomethyl]pyridine-2-carboxylic acid amide (Compound No.1-83) 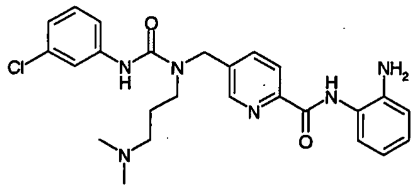 | $^1$H-NMR (400 MHz, DMSO-$d_6$) δ 1.72 (m, 2H), 2.20 (s, 6H), 2.28 (t, J = 6.2 Hz, 2H), 3.37 (t, J = 6.1 Hz, 2H), 4.63 (s, 2H), 4.90 (br s, 2H), 6.65 (t, J = 7.8 Hz, 1H), 6.82 (d, J = 7.8 Hz, 1H), 6.95 (t, J = 7.8 Hz, 1H), 6.98 (m, 1H), 7.22 (d, J = 8.4 Hz, 1H), 7.28 (t, J = 8.4 Hz, 1H), 7.51 (d, J = 7.8 Hz, 1H), 7.72 (t, J = 2.0 Hz, 1H), 7.97 (dd, J = 8.1, 1.7 Hz, 1H), 8.12 (d, J = 8.1 Hz, 1H), 8.67 (d, J = 1.7 Hz, 1H), 9.96 (br s, 1H), 10.03 (br s, 1H) |
| N-(2-Aminophenyl)-5-[1-(3-dimethylaminopropyl)-3-(3-methylphenyl)ureidomethyl]pyridine | $^1$H-NMR (400 MHz, DMSO-$d_6$) δ 1.72 (m, 2H), 2.21 (s, 6H), 2.26 (s, 3H), |

| | |
|---|---|
| -2-carboxylic acid amide (Compound No.1-84)<br>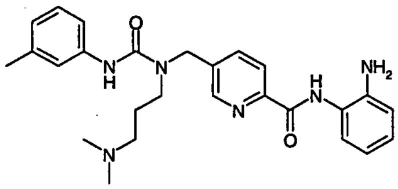 | 2.29 (m, 2H), 3.36 (t, J = 6.2 Hz, 2H), 4.62 (s, 2H), 4.90 (br s, 2H), 6.65 (t, J = 7.7 Hz, 1H), 6.75 (d, J = 7.7 Hz, 1H), 6.82 (d, J = 7.7 Hz, 1H), 6.95 (t, J = 7.7 Hz, 1H), 7.13 (t, J = 7.7 Hz, 1H), 7.20 (d, J = 7.7 Hz, 1H), 7.28 (s, 1H), 7.51 (d, J = 7.7 Hz, 1H), 7.96 (dd, J = 7.9, 2.0 Hz, 1H), 8.12 (d, J = 7.9 Hz, 1H), 8.67 (d, J = 2.0 Hz, 1H), 9.62 (br s, 1H), 10.03 (br s, 1H) |
| N-(2-Aminophenyl)-5-[1-[3-(morpholin-4-yl)propyl]-3-(pyridin-2-yl)ureidomethyl]pyridine-2-carboxylic acid amide (Compound No.1-85)<br>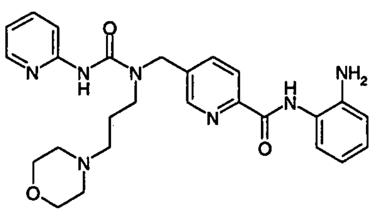 | $^1$H-NMR (400 MHz, $CDCl_3$)<br>δ 1.80 (m, 2H), 2.46 (t, J = 6.0 Hz, 2H), 2.51 (br s, 4H), 3.44 (t, J = 5.7 Hz, 2H), 3.59 (br s, 2H), 3.98 (t, J = 4.5 Hz, 4H), 4.66 (s, 2H), 6.83-6.88 (m, 2H), 6.93 (ddd, J = 7.3, 4.9, 1.0 Hz, 1H), 7.09 (td, J = 7.9, 1.5 Hz, 1H), 7.48 (d, J = 7.9 Hz, 1H), 7.63 (ddd, J = 8.5, 7.3, 2.0 Hz, 1H), 7.92 (dd, J = 8.1, 2.2 Hz, 1H), 8.00 (dt, J = 8.5, 1.0 Hz, 1H), 8.22 |

| | |
|---|---|
| | (ddd, J = 4.9, 2.0, 1.0 Hz, 1H), 8.26 (dd, J = 8.1, 0.5 Hz, 1H), 8.61 (dd, J = 2.2, 0.5 Hz, 1H), 9.83 (s, 2H) |
| N-(2-Aminophenyl)-5-[1-[3-(morpholin-4-yl)propyl]-3-(pyridin-4-yl)ureidomethyl]pyridine-2-carboxylic acid amide (Compound No.1-86) 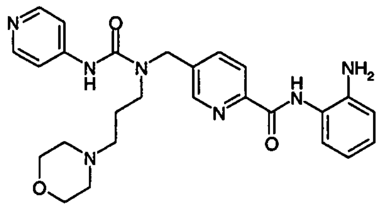 | $^1$H-NMR (400 MHz, $CDCl_3$) δ 1.82 (m, 2H), 2.45-2.55 (m, 6H), 3.40 (t, J = 5.7 Hz, 2H), 3.79 (t, J = 4.8 Hz, 4H), 3.95 (br s, 2H), 4.65 (s, 2H), 6.83-6.89 (m, 2H), 7.09 (td, J = 7.7, 1.5 Hz, 1H), 7.48 (m, 1H), 7.49 (d, J = 6.3 Hz, 2H), 7.90 (dd, J = 8.0, 2.2 Hz, 1H), 8.26 (dd, J = 8.0, 0.7 Hz, 1H), 8.47 (d, J = 6.3 Hz, 2H), 8.60 (dd, J = 2.2, 0.7 Hz, 1H), 9.10 (s, 1H), 9.82 (s, 1H) |
| N-(2-Aminophenyl)-5-[3-(4-dimethylaminophenyl)-1-[4-(morpholin-4-yl)butyl]ureidomethyl] pyridine-2-carboxylic acid amide (Compound No.1-87) | $^1$H-NMR (500 MHz, $CDCl_3$) δ 1.54 (m, 2H), 1.71 (m, 2H), 2.39 (t, J = 7.0 Hz, 2H), 2.41 (m, 4H), 2.92 (s, 6H), 3.30 (t, J = 7.9 Hz, 2H), 3.63 (t, J = 4.6 Hz, 4H), 3.96 (s, 2H), 4.68 (s, 2H), 6.60 (s, 1H), 6.71 (d, J = 8.9 Hz, 2H), 6.85 (dd, J = 7.9, 1.5 Hz, |

| | |
|---|---|
| 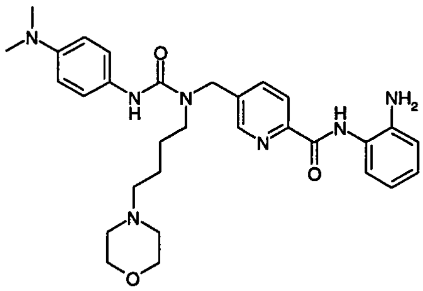 | 1H), 6.86 (td, J = 7.9, 1.5 Hz, 1H), 7.09 (td, J = 7.9, 1.5 Hz, 1H), 7.17 (d, J = 8.9 Hz, 2H), 7.49 (dd, J = 7.9, 1.5 Hz, 1H), 7.89 (dd, J = 8.1, 1.8 Hz, 1H), 8.25 (d, J = 8.1 Hz, 1H), 8.58 (d, J = 1.8 Hz, 1H), 9.83 (s, 1H) |
| N-(2-Aminophenyl)-5-[3-(3-fluorophenyl)-1-[4-(morpholin-4-yl)butyl]ureidomethyl]pyridine-2-carboxylic acid amide (Compound No.1-88) 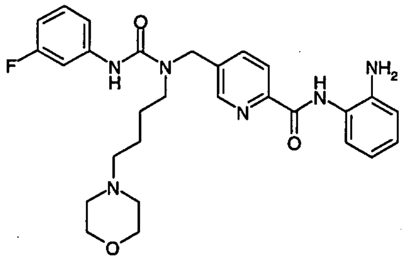 | $^1$H-NMR (400 MHz, $CDCl_3$) δ 1.54 (m, 2H), 1.72 (m, 2H), 2.39 (t, J = 7.0 Hz, 2H), 2.43 (t, J = 4.5 Hz, 4H), 3.34 (t, J = 7.9 Hz, 2H), 3.68 (t, J = 4.5 Hz, 4H), 3.95 (s, 2H), 4.69 (s, 2H), 6.65 (s, 1H), 6.78 (tdd, J = 8.3, 2.3, 0.9 Hz, 1H), 6.85 (dd, J = 7.8, 1.5 Hz, 1H), 6.86 (td, J = 7.8, 1.5 Hz, 1H), 7.01 (ddd, J = 8.3, 2.3, 0.9 Hz, 1H), 7.09 (td, J = 7.8, 1.5 Hz, 1H), 7.23 (td, J = 8.3, 6.6 Hz, 1H), 7.32 (dt, J = 11.0, 2.3 Hz, 1H), 7.50 (dd, J = 7.8, 1.5 Hz, 1H), 7.88 (dd, J = 8.0, 1.8 Hz, 1H), 8.27 (d, J = 8.0 Hz, 1H), 8.59 (d, J = 1.8 Hz, 1H), 9.82 (s, 1H) |

| | |
|---|---|
| N-(2-Aminophenyl)-5-[1-[4-(morpholin-4-yl)butyl]-3-(thiophen-3-yl)ureidomethyl]pyridine-2-carboxylic acid amide (Compound No.1-89) 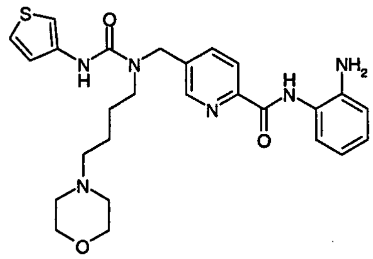 | $^1$H-NMR (400 MHz, $CDCl_3$) δ 1.54 (m, 2H), 1.70 (m, 2H), 2.38 (t, J = 7.0 Hz, 2H), 2.42 (t, J = 4.5 Hz, 4H), 3.31 (t, J = 7.9 Hz, 2H), 3.67 (t, J = 4.5 Hz, 4H), 3.95 (s, 2H), 4.69 (s, 2H), 6.84 (s, 1H), 6.85 (dd, J = 7.8, 1.4 Hz, 1H), 6.86 (td, J = 7.8, 1.4 Hz, 1H), 6.97 (dd, J = 5.1, 1.4 Hz, 1H), 7.09 (td, J = 7.8, 1.4 Hz, 1H), 7.24 (dd, J = 5.1, 3.3 Hz, 1H), 7.28 (dd, J = 3.3, 1.4 Hz, 1H), 7.49 (dd, J = 7.8, 1.4 Hz, 1H), 7.87 (dd, J = 8.0, 1.8 Hz, 1H), 8.26 (d, J = 8.0 Hz, 1H), 8.58 (d, J = 1.8 Hz, 1H), 9.82 (s, 1H) |
| N-(2-Aminophenyl)-5-[3-(2,3-dihydrobenzo[1,4]dioxin-6-yl)-1-[4-(morpholin-4-yl)butyl]ureidomethyl]pyridine-2-carboxylic acid amide (Compound No.1-90) | $^1$H-NMR (400 MHz, $CDCl_3$) δ 1.54 (m, 2H), 1.70 (m, 2H), 2.39 (t, J = 6.8 Hz, 2H), 2.42 (t, J = 4.5 Hz, 4H), 3.30 (t, J = 7.9 Hz, 2H), 3.64 (t, J = 4.5 Hz, 4H), 3.96 (s, 2H), 4.24 (s, 4H), 4.68 (s, 2H), 6.60 (s, 1H), 6.75 (dd, J = 8.5, 2.3 Hz, 1H), 6.80 |

| | |
|---|---|
| 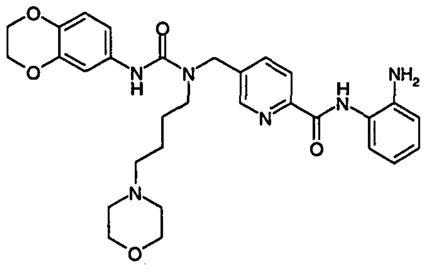 | (d, J = 8.5 Hz, 1H), 6.85 (dd, J = 7.8, 1.5 Hz, 1H), 6.86 (td, J = 7.8, 1.5 Hz, 1H), 6.92 (d, J = 2.3 Hz, 1H), 7.09 (td, J = 7.8, 1.5 Hz, 1H), 7.50 (dd, J = 7.8, 1.5 Hz, 1H), 7.88 (dd, J = 8.0, 1.8 Hz, 1H), 8.26 (d, J = 8.0 Hz, 1H), 8.58 (d, J = 1.8 Hz, 1H), 9.83 (s, 1H) |
| N-(2-Aminophenyl)-5-[1-[4-(morpholin-4-yl)butyl]-3-(pyridin-3-yl)ureidomethyl]pyridine-2-carboxylic acid amide (Compound No.1-91) 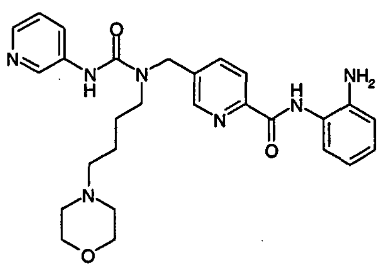 | $^1$H-NMR (500 MHz, $CDCl_3$) δ 1.55 (m, 2H), 1.70 (m, 2H), 2.41 (t, J = 6.9 Hz, 2H), 2.43 (t, J = 4.5 Hz, 4H), 3.36 (t, J = 8.1 Hz, 2H), 3.66 (t, J = 4.5 Hz, 4H), 3.95 (s, 2H), 4.71 (s, 2H), 6.75 (s, 1H), 6.85 (dd, J = 7.8, 1.5 Hz, 1H), 6.86 (td, J = 7.8, 1.5 Hz, 1H), 7.09 (td, J = 7.8, 1.5 Hz, 1H), 7.28 (dd, J = 8.2, 4.2 Hz, 1H), 7.49 (dd, J = 7.8, 1.5 Hz, 1H), 7.88 (dd, J = 7.9, 1.8 Hz, 1H), 7.99 (ddd, J = 8.2, 2.7, 1.5 Hz, 1H), 8.27 (dd, J = 7.9, 0.6 Hz, 1H), 8.34 (dd, J = 4.2, 1.5 Hz, 1H), 8.43 (d, J = 2.7 Hz, 1H), |

| | |
|---|---|
| | 8.59 (dd, J = 1.8, 0.6 Hz, 1H), 9.82 (s, 1H) |
| N-(2-Aminophenyl)-5-[1-[4-(morpholin-4-yl)butyl]-3-phenethylureidomethyl]pyridine-2-carboxylic acid amide (Compound No.1-92) 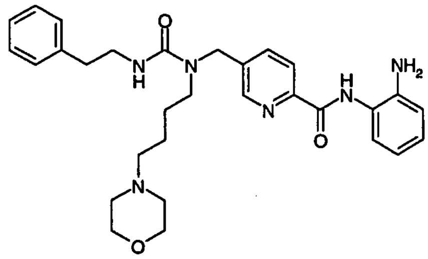 | $^1$H-NMR (400 MHz, $CDCl_3$) δ 1.39 (m, 2H), 1.48 (m, 2H), 2.25 (t, J = 7.0 Hz, 2H), 2.32 (t, J = 4.5 Hz, 4H), 2.86 (t, J = 6.6 Hz, 2H), 3.09 (t, J = 7.8 Hz, 2H), 3.55 (td, J = 6.6, 5.6 Hz, 2H), 3.65 (t, J = 4.5 Hz, 4H), 3.96 (s, 2H), 4.58 (s, 2H), 4.76 (t, J = 5.6 Hz, 1H), 6.86 (dd, J = 7.8, 1.5 Hz, 1H), 6.87 (td, J = 7.8, 1.5 Hz, 1H), 7.09 (td, J = 7.8, 1.5 Hz, 1H), 7.15-7.25 (m, 3H), 7.27-7.32 (m, 2H), 7.50 (dd, J = 7.8, 1.5 Hz, 1H), 7.76 (dd, J = 8.0, 2.0 Hz, 1H), 8.23 (dd, J = 8.0, 0.7 Hz, 1H), 8.49 (dd, J = 2.0, 0.7 Hz, 1H), 9.83 (s, 1H) |
| N-(2-Aminophenyl)-5-[3-(3-methoxyphenyl)-1-[4-(morpholin-4-yl)butyl]ureidomethyl]pyridine-2-carboxylic acid amide (Compound No.1-93) | $^1$H-NMR (400 MHz, $CDCl_3$) δ 1.55 (m, 2H), 1.71 (m, 2H), 2.38 (t, J = 7.1 Hz, 2H), 2.42 (t, J = 4.5 Hz, 4H), 3.33 (t, J = 7.8 Hz, 2H), 3.67 (t, J = 4.5 Hz, 4H), |

| | |
|---|---|
| 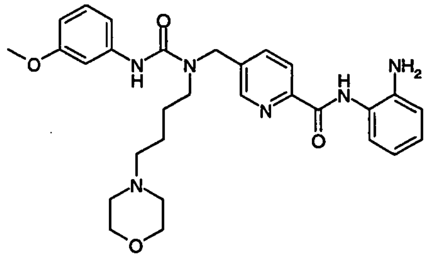 | 3.81 (s, 3H), 3.95 (s, 2H), 4.70 (s, 2H), 6.59 (s, 1H), 6.64 (ddd, J = 8.3, 2.6, 0.9 Hz, 1H), 6.84 (m, 1H), 6.85 (d, J = 7.8 Hz, 1H), 6.86 (t, J = 7.8 Hz, 1H), 7.08 (t, J = 7.8 Hz, 1H ), 7.11 (m, 1H), 7.20 (t, J = 8.2 Hz, 1H), 7.50 (d, J = 7.8 Hz, 1H), 7.88 (dd, J = 8.0, 2.2 Hz, 1H), 8.27 (dd, J = 8.0, 0.7 Hz, 1H), 8.58 (dd, J = 2.2, 0.7 Hz, 1H), 9.82 (s, 1H) |
| N-(2-Aminophenyl)-5-[3-(1,3-benzothiazol-2-yl)-1-(2-dimethylaminoethyl)ureidomethyl]pyridine-2-carboxylic acid amide (Compound No.1-94) 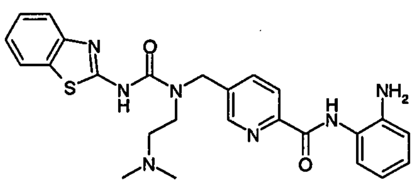 | $^1$H-NMR (500 MHz, $CDCl_3$) δ 2.48 (s, 6H), 2.58 (t, J = 3.8 Hz, 2H), 3.38 (t, J = 3.8 Hz, 2H), 3.97 (br s, 2H), 4.72 (s, 2H), 6.83-6.87 (m, 2H), 7.08 (t, J = 7.5 Hz, 1H), 7.22 (t, J = 7.5 Hz, 1H), 7.36 (t, J = 7.5 Hz, 1H), 7.49 (d, J = 7.5 Hz, 1H), 7.70 (d, J = 7.5 Hz, 1H), 7.76 (d, J = 7.5 Hz, 1H), 7.90 (d, J = 7.9 Hz, 1H), 8.26 (d, J = 7.9 Hz, 1H), 8.58 (s, 1H), 9.82 (s, 1H) |
| N-(2-Aminophenyl)-5-[1-(2-dimethylaminoeth | $^1$H-NMR (500 MHz, $CDCl_3$) |

| | |
|---|---|
| yl)-3-(quinolin-6-yl)ureidomethyl]pyridine-2-carboxylic acid amide (Compound No.1-95) 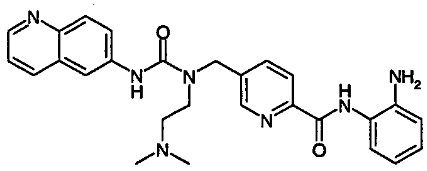 | δ 2.46 (s, 6H), 2.59 (t, J = 4.1 Hz, 2H), 3.39 (t, J = 4.1 Hz, 2H), 3.97 (br s, 2H), 4.70 (s, 2H), 6.83-6.87 (m, 2H), 7.08 (m, 1H), 7.34 (dd, J = 7.9, 4.3 Hz, 1H), 7.39 (dd, J = 9.0, 2.4 Hz, 1H), 7.50 (d, J = 7.0 Hz, 1H), 7.93 (dd, J = 7.9, 1.8 Hz, 1H), 8.01 (d, J = 9.0 Hz, 1H), 8.09 (d, J = 7.9 Hz, 1H), 8.18 (d, J = 2.4 Hz, 1H), 8.27 (d, J = 7.9 Hz, 1H), 8.61 (d, J = 1.8 Hz, 1H), 8.77 (d, J = 4.3 Hz, 1H), 9.84 (s, 1H), 11.50 (s, 1H) |
| N-(2-Aminophenyl)-5-[1-(2-dimethylaminoethyl)-3-(1,3-thiazol-2-yl)ureidomethyl]pyridine-2-carboxylic acid amide (Compound No.1-96) 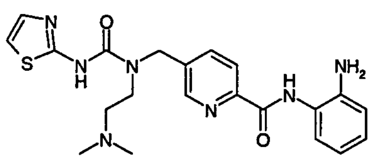 | $^1$H-NMR (500 MHz, $CDCl_3$) δ 2.45 (s, 6H), 2.56 (t, J = 4.3 Hz, 2H), 3.36 (t, J = 4.3 Hz, 2H), 3.96 (s, 2H), 4.71 (s, 2H), 6.84-6.88 (m, 3H), 7.09 (m, 1H), 7.39 (d, J = 3.7 Hz, 1H), 7.49 (d, J = 7.9 Hz, 1H), 7.89 (dd, J = 8.1, 1.8 Hz, 1H), 8.26 (d, J = 8.1 Hz, 1H), 8.58 (d, J = 1.8 Hz, 1H), 9.82 (s, 1H) |
| N-(2-Aminophenyl)-5-[1-(2-dimethylaminoeth | $^1$H-NMR (500 MHz, $CDCl_3$) |

| | |
|---|---|
| yl)-3-phenylcarbonylmethylureidomethyl]pyridine-2-carboxylic acid amide (Compound No.1-97) 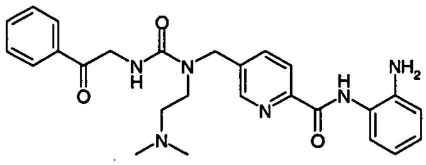 | δ 2.34 (s, 6H), 2.50 (t, J = 5.0 Hz, 2H), 3.35 (t, J = 5.0 Hz, 2H), 3.96 (br s, 2H), 4.66 (s, 2H), 4.79 (s, 2H), 6.84-6.87 (m, 2H), 7.08 (td, J = 7.6, 1.2 Hz, 1H), 7.47-7.51 (m, 3H), 7.60 (t, J = 7.6 Hz, 1H), 7.87 (dd, J = 8.1, 2.0 Hz, 1H), 7.99-8.01 (m, 2H), 8.25 (d, J = 8.1 Hz, 1H), 8.36 (br s, 1H), 8.56 (d, J = 2.0 Hz, 1H), 9.83 (s, 1H) |
| N-(2-Aminophenyl)-5-[3-(2,3-dihydrobenzo[1,4]dioxin-6-yl)-1-(5-dimethylaminopentyl)ureidomethyl]pyridine-2-carboxylic acid amide (Compound No.1-98) 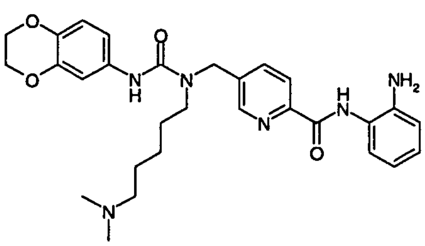 | $^1$H-NMR (500 MHz, DMSO-$d_6$) δ 1.26 (m, 2H), 1.49-1.58 (m, 4H), 2.09 (s, 6H), 2.54 (m, 2H), 3.35 (t, J = 7.3 Hz, 2H), 4.17-4.20 (m, 4H), 4.69 (s, 2H), 4.89 (s, 2H), 6.65 (t, J = 7.8 Hz, 1H), 6.72 (d, J = 8.8 Hz, 1H), 6.83 (d, J = 7.8 Hz, 1H), 6.89 (dd, J = 8.8, 2.4 Hz, 1H), 6.95 (t, J = 7.8 Hz, 1H), 7.06 (d, J = 2.4 Hz, 1H), 7.50 (d, J = 7.8 Hz, 1H), 7.91 (dd, J = 8.1, 1.8 Hz, 1H), 8.12 (d, J = 8.1 Hz, 1H), 8.30 (s, 1H), 8.63 (d, J = 1.8 |

| | |
|---|---|
| | Hz, 1H), 10.03 (s, 1H) |
| N-(2-Aminophenyl)-5-[1-(5-dimethylaminopentyl)-3-(3-fluorophenyl)ureidomethyl]pyridine-2-carboxylic acid amide (Compound No.1-99) 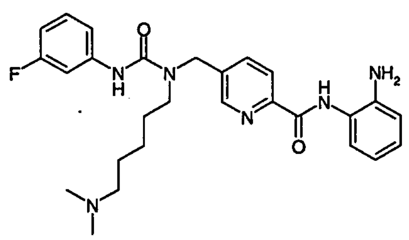 | $^1$H-NMR (400 MHz, $CDCl_3$) δ 1.39 (m, 2H), 1.52 (m, 2H), 1.68 (m, 2H), 2.21 (s, 6H), 2.27 (t, J = 7.1 Hz, 2H), 3.31 (t, J = 7.8 Hz, 2H), 3.96 (s, 2H), 4.69 (s, 2H), 6.73 (s, 1H), 6.76 (tdd, J = 8.3, 2.4, 0.9 Hz, 1H), 6.85 (dd, J = 7.7, 1.5 Hz, 1H), 6.86 (td, J = 7.7, 1.5 Hz, 1H), 7.04 (ddd, J = 8.3, 2.4, 0.9 Hz, 1H), 7.09 (td, J = 7.7, 1.5 Hz, 1H), 7.23 (td, J = 8.3, 6.5 Hz, 1H), 7.35 (dt, J = 11.1, 2.4 Hz, 1H), 7.50 (dd, J = 7.7, 1.5 Hz, 1H), 7.87 (dd, J = 8.1, 2.2 Hz, 1H), 8.26 (dd, J = 8.1, 0.7 Hz, 1H), 8.58 (dd, J = 2.2, 0.7 Hz, 1H), 9.82 (s, 1H) |
| N-(2-Aminophenyl)-5-[1-(3-dimethylaminopropyl)-3-(4,5,6,7-tetrahydro-1,3-benzothiazol-2-yl)ureidomethyl]pyridine-2-carboxylic acid amide (Compound No.1-100) | $^1$H-NMR (400 MHz, DMSO-$d_6$) δ 1.70 (m, 2H), 1.74 (br s, 4H), 2.21 (s, 6H), 2.29 (br s, 4H), 2.55 (br s, 2H), 3.35 (m, 2H), 4.66 (s, 2H), 4.90 (br s, 2H), 6.65 (td, J |

| | |
|---|---|
| 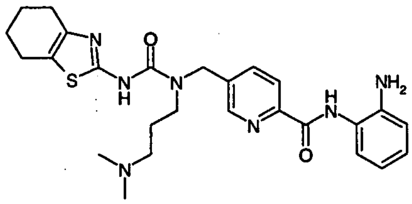 | = 7.7, 1.3 Hz, 1H), 6.82 (dd, J = 7.7, 1.3 Hz, 1H), 6.95 (td, J = 7.7, 1.3 Hz, 1H), 7.50 (dd, J = 7.7, 1.3 Hz, 1H), 7.93 (dd, J = 8.0, 2.1 Hz, 1H), 8.11 (d, J = 8.0 Hz, 1H), 8.65 (d, J = 2.1 Hz, 1H), 10.03 (br s, 1H), 12.36 (br s, 1H) |
| N-(2-Aminophenyl)-5-[1-(3-dimethylaminopropyl)-3-(pyridin-3-yl)ureidomethyl]pyridine-2-carboxylic acid amide (Compound No.1-101) 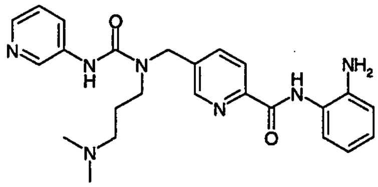 | $^1$H-NMR (500 MHz, DMSO-$d_6$) δ 1.75 (m, 2H), 2.26 (s, 6H), 2.36 (m, 2H), 3.39 (t, J = 6.3 Hz, 2H), 4.66 (s, 2H), 4.89 (br s, 2H), 6.65 (td, J = 7.8, 1.2 Hz, 1H), 6.82 (dd, J = 7.8, 1.2 Hz, 1H), 6.95 (td, J = 7.8, 1.2 Hz, 1H), 7.29 (dd, J = 8.4, 4.6 Hz, 1H), 7.51 (dd, J = 7.8, 1.2 Hz, 1H), 7.91 (ddd, J = 8.4, 2.4, 1.5 Hz,1H), 7.98 (dd, J = 7.9, 2.0 Hz, 1H), 8.13 (d, J = 7.9 Hz, 1H), 8.16 (dd, J = 4.6, 1.5 Hz,1H), 8.59 (d, J = 2.4 Hz, 1H), 8.67 (d, J = 2.0 Hz, 1H), 9.86 (br s, 1H), 10.03 (br s, 1H) |

| | |
|---|---|
| N-(2-Aminophenyl)-5-[1-(3-dimethylaminopropyl)-3-(3-nitrophenyl)ureidomethyl]pyridine-2-carboxylic acid amide (Compound No.1-102) 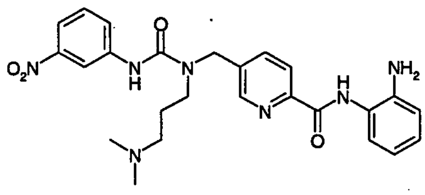 | $^1$H-NMR (400 MHz, DMSO-$d_6$) δ 1.74 (m, 2H), 2.22 (s, 6H), 2.30 (t, J = 6.2 Hz, 2H), 3.41 (t, J = 6.2 Hz, 2H), 4.67 (s, 2H), 4.90 (br s, 2H), 6.65 (t, J = 7.7 Hz, 1H), 6.82 (d, J = 7.7 Hz,1H), 6.95 (t, J = 7.7 Hz, 1H), 7.50 (d, J = 7.7 Hz,1H), 7.55 (t, J = 8.2 Hz,1H), 7.72 (m, 1H), 7.80 (m, 1H), 7.98 (dd, J = 8.1, 1.8 Hz, 1H), 8.13 (d, J = 8.1 Hz, 1H), 8.55 (t, J = 2.2 Hz, 1H), 8.69 (d, J = 1.8 Hz, 1H), 10.04 (br s, 1H), 10.28 (br s, 1H) |
| 5-[3-(3-Acetylphenyl)-1-(3-dimethylaminopropyl)ureidomethyl]-N-(2-aminophenyl)pyridine-2-carboxylic acid amide (Compound No.1-103) 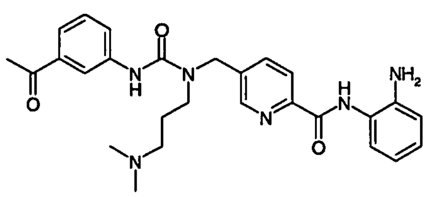 | $^1$H-NMR (500 MHz, DMSO-$d_6$) δ 1.73 (m, 2H), 2.21 (s, 6H), 2.30 (t, J = 6.3 Hz, 2H), 2.56 (s, 3H), 3.39 (t, J = 6.3 Hz, 2H), 4.66 (s, 2H), 4.89 (br s, 2H), 6.65 (t, J = 7.8 Hz, 1H), 6.82 (d, J = 7.8 Hz, 1H), 6.95 (t, J = 7.8 Hz, 1H), 7.41 (t, J = 7.7 Hz, 1H), 7.51 (d, J = 7.8 Hz, 1H), 7.56 (d, J = 7.7 Hz, 1H), 7.70 (d, J = 7.7 Hz, 1H), 7.97 (dd, J = |

| | |
|---|---|
| | 8.0, 2.0 Hz, 1H), 8.03 (s, 1H), 8.12 (d, J = 8.0 Hz, 1H), 8.68 (d, J = 2.0 Hz, 1H), 9.90 (br s, 1H), 10.03 (br s, 1H) |
| N-(2-Aminophenyl)-5-[1-(3-dimethylaminopropyl)-3-(3-methylthiophenyl)ureidomethyl]pyridine-2-carboxylic acid amide (Compound No.1-104) 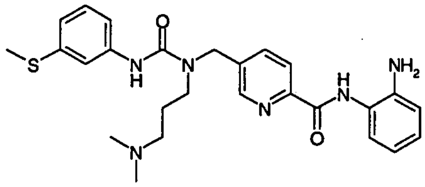 | $^1$H-NMR (400 MHz, DMSO-$d_6$) δ 1.72 (m, 2H), 2.20 (s, 6H), 2.28 (t, J = 6.1 Hz, 2H), 2.45 (s, 3H), 3.36 (t, J = 6.0 Hz, 2H), 4.63 (s, 2H), 4.90 (br s, 2H), 6.65 (t, J = 7.9 Hz, 1H), 6.81-6.84 (m, 2H), 6.95 (t, J = 7.9 Hz, 1H), 7.15 (d, J = 8.1 Hz, 1H), 7.20 (t, J = 8.1 Hz, 1H), 7.45 (s, 1H), 7.51 (d, J = 7.9 Hz, 1H), 7.96 (dd, J = 8.1, 1.7 Hz, 1H), 8.12 (d, J = 8.1 Hz, 1H), 8.67 (s, 1H), 9.77 (br s, 1H), 10.03 (br s, 1H) |
| N-(2-Aminophenyl)-5-[1-[3-(morpholin-4-yl)propyl]-3-[6-(morpholin-4-yl)pyridin-3-yl]ureidomethyl]pyridine-2-carboxylic acid amide (Compound No.1-105) | $^1$H-NMR (500 MHz, $CDCl_3$) δ 1.77 (m, 2H), 2.44 (br s, 4H), 2.49 (t, J = 6.0 Hz, 2H), 3.39 (t, J = 5.5 Hz, 2H), 3.45 (t, J = 4.9 Hz, 4H), 3.60 (t, J = 4.3 Hz, 4H), 3.83 (t, J = 4.9 Hz, 4H), 3.92 (br s, 2H), 4.62 |

| | |
|---|---|
| 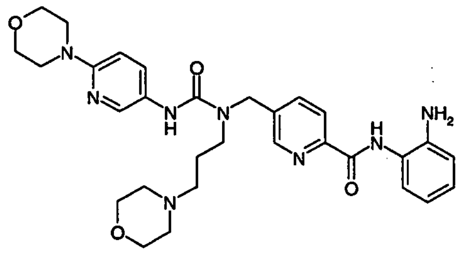 | (s, 2H), 6.65 (d, J = 8.9 Hz, 1H), 6.82-6.87 (m, 2H), 7.07 (td, J = 7.7, 1.3 Hz, 1H), 7.48 (dd, J = 7.7, 1.3 Hz, 1H), 7.70 (dd, J = 8.9, 2.6 Hz, 1H), 7.90 (dd, J = 7.9, 2.1 Hz, 1H), 8.11 (d, J = 2.6 Hz, 1H), 8.22 (dd, J = 7.9, 0.6 Hz, 1H), 8.57 (dd, J = 2.1, 0.6 Hz, 1H), 9.18 (s, 1H), 9.82 (s, 1H) |
| N-(2-Aminophenyl)-5-[3-(3-fluorophenyl)-1-[3-(morpholin-4-yl)propyl]ureidomethyl]pyridine-2-carboxylic acid amide (Compound No.1-106) 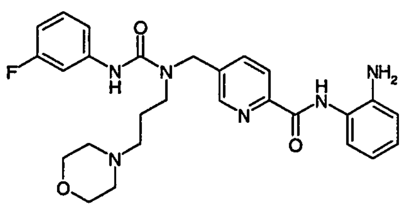 | $^1H$-NMR (400 MHz, $CDCl_3$) δ 1.80 (m, 2H), 2.44-2.52 (m, 6H), 3.39 (t, J = 5.7 Hz, 2H), 3.71 (t, J = 4.8 Hz, 4H), 3.96 (br s, 2H), 4.65 (s, 2H), 6.78 (tdd, J = 8.3, 2.6, 0.9 Hz, 1H), 6.83-6.88 (m, 2H), 7.09 (td, J = 7.8, 1.5 Hz, 1H), 7.13 (ddd, J = 8.3, 2.2, 1.0 Hz, 1H), 7.26 (m, 1H), 7.41 (dt, J = 11.1, 2.2 Hz, 1H), 7.48 (d, J = 7.8 Hz, 1H), 7.91 (dd, J = 8.1, 2.2 Hz, 1H), 8.24 (dd, J = 8.1, 0.6 Hz, 1H), 8.59 (dd, J = 2.2, 0.6 Hz, 1H), 9.04 (s, 1H), 9.82 (s, 1H) |

| | |
|---|---|
| N-(2-Aminophenyl)-5-[3-(3-fluoro-4-methylphenyl)-1-[3-(morpholin-4-yl)propyl]ureidomethyl]pyridine-2-carboxylic acid amide (Compound No.1-107) 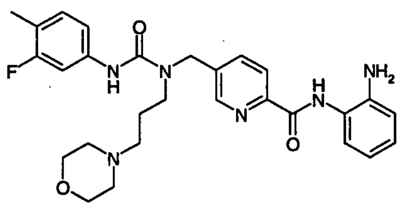 | $^1$H-NMR (400 MHz, $CDCl_3$) δ 1.78 (m, 2H), 2.23 (d, J = 1.7 Hz, 3H), 2.43-2.50 (m, 6H), 3.38 (t, J = 5.6 Hz, 2H), 3.69 (t, J = 4.6 Hz, 4H), 3.95 (br s, 2H), 4.64 (s, 2H), 6.83-6.89 (m, 2H), 7.02 (dd, J = 8.2, 2.1 Hz, 1H), 7.06-7.13 (m, 2H), 7.31 (dd, J = 11.5, 2.1 Hz, 1H), 7.48 (d, J = 7.8 Hz, 1H), 7.91 (dd, J = 8.0, 2.1 Hz, 1H), 8.24 (dd, J = 8.0, 0.7 Hz, 1H), 8.59 (dd, J = 2.1, 0.7 Hz, 1H), 9.01 (s, 1H), 9.83 (s, 1H) |
| N-(2-Aminophenyl)-5-[1-[3-(morpholin-4-yl)propyl]-3-(thiophen-3-yl)ureidomethyl]pyridine-2-carboxylic acid amide (Compound No.1-108) 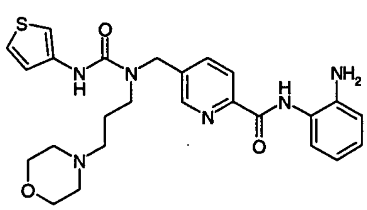 | $^1$H-NMR (400 MHz, $CDCl_3$) δ 1.78 (m, 2H), 2.42-2.51 (m, 6H), 3.37 (t, J = 5.6 Hz, 2H), 3.72 (t, J = 4.6 Hz, 4H), 3.95 (br s, 2H), 4.65 (s, 2H), 6.83-6.88 (m, 2H), 7.09 (td, J = 7.8, 1.5 Hz, 1H), 7.10 (dd, J = 5.2, 1.5 Hz, 1H), 7.25 (dd, J = 5.2, 3.2 Hz, 1H), 7.31 (dd, J = 3.2, 1.5 Hz, 1H), 7.48 (d, J = 7.8 Hz, 1H), 7.91 (dd, J = 8.0, 2.1 Hz, |

| | |
|---|---|
| | 1H), 8.24 (dd, J = 8.0, 0.5 Hz, 1H), 8.58 (dd, J = 2.1, 0.5 Hz, 1H), 9.25 (s, 1H), 9.83 (s, 1H) |
| N-(2-Aminophenyl)-5-[3-(4-methoxyphenyl)-1-[4-(morpholin-4-yl)butyl]ureidomethyl]pyridine-2-carboxylic acid amide (Compound No.1-109) 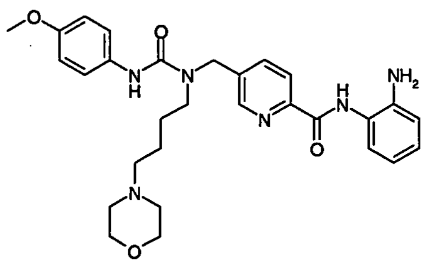 | $^1$H-NMR (400 MHz, $CDCl_3$) δ 1.55 (m, 2H), 1.71 (m, 2H), 2.39 (t, J = 6.8 Hz, 2H), 2.41 (t, J = 4.6 Hz, 4H), 3.32 (t, J = 8.1 Hz, 2H), 3.63 (t, J = 4.6 Hz, 4H), 3.80 (s, 3H), 3.96 (s, 2H), 4.69 (s, 2H), 6.67 (s, 1H), 6.86 (t, J = 7.6 Hz, 1H), 6.87 (m, 1H), 6.87 (d, J = 9.0 Hz, 2H), 7.09 (td, J = 7.6, 1.3 Hz, 1H), 7.24 (d, J = 9.0 Hz, 2H), 7.49 (dd, J = 7.6, 1.3 Hz, 1H), 7.89 (dd, J = 8.0, 2.1 Hz, 1H), 8.26 (dd, J = 8.0, 0.7 Hz, 1H), 8.58 (dd, J = 2.1, 0.7 Hz, 1H), 9.83 (s, 1H) |
| N-(2-Aminophenyl)-5-[3-(4-methylphenyl)-1-[4-(morpholin-4-yl)butyl]ureidomethyl]pyridine-2-carboxylic acid amide (Compound | $^1$H-NMR (400 MHz, $CDCl_3$) δ 1.55 (m, 2H), 1.71 (m, 2H), 2.31 (s, 3H), 2.39 (t, J = 6.0 Hz, 2H), 2.42 (t, J = 4.6 Hz, |

| | |
|---|---|
| No.1-110)<br>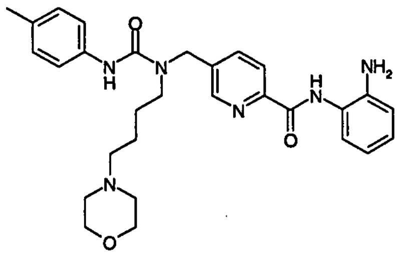 | 4H), 3.32 (t, J = 7.9 Hz, 2H), 3.65 (t, J = 4.6 Hz, 4H), 3.96 (s, 2H), 4.69 (s, 2H), 6.60 (s, 1H), 6.86 (d, J = 7.7 Hz, 1H), 6.87 (t, J = 7.7 Hz, 1H), 7.09 (t, J = 7.7 Hz, 1H), 7.12 (d, J = 8.4 Hz, 2H), 7.23 (d, J = 8.4 Hz, 2H), 7.50 (d, J = 7.7 Hz, 1H), 7.89 (dd, J = 8.1, 2.2 Hz, 1H), 8.26 (dd, J = 8.1, 0.7 Hz, 1H), 8.58 (dd, J = 2.2, 0.7 Hz, 1H), 9.83 (s, 1H) |
| N-(2-Aminophenyl)-5-[3-(3,4-dimethoxyphenyl)-1-[4-(morpholin-4-yl)butyl]ureidomethyl]pyridine-2-carboxylic acid amide (Compound No.1-111)<br>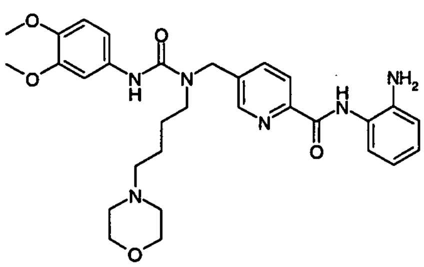 | $^1$H-NMR (500 MHz, $CDCl_3$)<br>δ 1.54 (m, 2H), 1.72 (m, 2H), 2.39 (t, J = 7.0 Hz, 2H), 2.42 (t, J = 4.6 Hz, 4H), 3.32 (t, J = 8.1 Hz, 2H), 3.64 (t, J = 4.6 Hz, 4H), 3.86 (s, 3H), 3.90 (s, 3H), 3.95 (s, 2H), 4.70 (s, 2H), 6.64 (s, 1H), 6.71 (dd, J = 8.6, 2.4 Hz, 1H), 6.80 (d, J = 8.6 Hz, 1H), 6.85 (dd, J = 7.8, 1.2 Hz, 1H), 6.88 (td, J = 7.8, 1.2 Hz, 1H), 7.09 (td, J = 7.8, 1.2 Hz, 1H), 7.14 (d, J = 2.4 Hz, 1H), 7.48 (dd, J = 7.8, 1.2 Hz, 1H), |

| | |
|---|---|
| | 7.89 (dd, J = 8.1, 1.9 Hz, 1H), 8.27 (d, J = 8.1 Hz, 1H), 8.59 (d, J = 1.9 Hz, 1H), 9.82 (s, 1H) |
| N-(2-Aminophenyl)-5-[3-(3-ethoxyphenyl)-1-[4-(morpholin-4-yl)butyl]ureidomethyl]pyridine-2-carboxylic acid amide (Compound No.1-112) 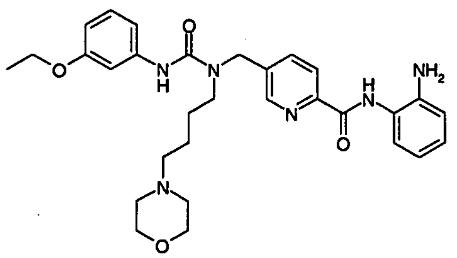 | $^1$H-NMR (500 MHz, $CDCl_3$) δ 1.39 (t, J = 7.0 Hz, 3H), 1.54 (m, 2H), 1.71 (m, 2H), 2.38 (t, J = 7.0 Hz, 2H), 2.42 (m, 4H), 3.33 (t, J = 7.8 Hz, 2H), 3.68 (t, J = 4.6 Hz, 4H), 3.95 (s, 2H), 4.04 (q, J = 7.0 Hz, 2H), 4.70 (s, 2H), 6.57 (s, 1H), 6.63 (dd, J = 8.2, 2.4 Hz, 1H), 6.81-6.88 (m, 3H), 7.07-7.10 (m, 2H), 7.19 (t, J = 7.8 Hz, 1H), 7.49 (d, J = 7.8 Hz, 1H), 7.88 (dd, J = 7.9, 2.1 Hz, 1H), 8.27 (d, J = 7.9 Hz, 1H), 8.59 (d, J = 2.1 Hz, 1H), 9.82 (s, 1H) |
| N-(2-Aminophenyl)-5-[3-(3-methylphenyl)-1-[4-(morpholin-4-yl)butyl]ureidomethyl]pyridine-2-carboxylic acid amide (Compound No.1-113) | $^1$H-NMR (500 MHz, $CDCl_3$) δ 1.55 (m, 2H), 1.72 (m, 2H), 2.34 (s, 3H), 2.39 (t, J = 7.2 Hz, 2H), 2.53 (m, 4H), 3.33 (t, J = 7.9 Hz, 2H), 3.67 (t, J = 4.6 Hz, 4H), |

| | |
|---|---|
| 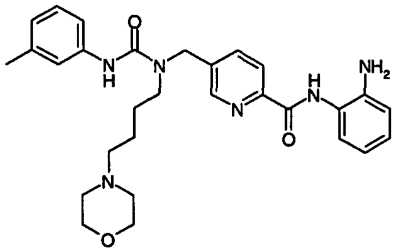 | 3.96 (s, 2H), 4.70 (s, 2H), 6.57 (s, 1H), 6.84-6.88 (m, 2H), 6.91 (d, J = 7.0 Hz, 1H), 7.09 (td, J = 7.7, 1.3 Hz, 1H), 7.11 (d, J = 7.0 Hz, 1H), 7.20 (t, J = 7.7 Hz, 1H), 7.23 (s, 1H), 7.48 (d, J = 7.7 Hz, 1H), 7.89 (dd, J = 8.0, 2.1 Hz, 1H), 8.27 (d, J = 8.0 Hz, 1H), 8.59 (d, J = 2.1 Hz, 1H), 9.82 (s, 1H) |
| 5-[3-(3-Acetylphenyl)-1-[4-(morpholin-4-yl)butyl]ureidomethyl]-N-(2-aminophenyl)pyridine-2-carboxylic acid amide (Compound No.1-114) 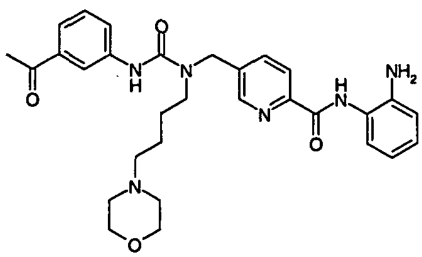 | $^1H$-NMR (500 MHz, $CDCl_3$) δ 1.56 (m, 2H), 1.74 (m, 2H), 2.40 (t, J = 7.0 Hz, 2H), 2.44 (t, J = 4.7 Hz, 4H), 2.61 (s, 3H), 3.35 (t, J = 7.9 Hz, 2H), 3.67 (t, J = 4.7 Hz, 4H), 3.95 (s, 2H), 4.71 (s, 2H), 6.81 (s, 1H), 6.85 (dd, J = 7.8, 1.3 Hz, 1H), 6.86 (td, J = 7.8, 1.3 Hz, 1H), 7.09 (td, J = 7.8, 1.3 Hz, 1H), 7.43 (t, J = 7.9 Hz, 1H), 7.49 (dd, J = 7.8, 1.3 Hz, 1H), 7.66 (ddd, J = 7.9, 2.2, 1.1 Hz, 1H), 7.78 (ddd, J = 7.9, 2.2, 1.1 Hz, 1H), 7.82 (t, J = 2.2 Hz, 1H), 7.89 (dd, J = |

| | |
|---|---|
| | 7.9, 2.1 Hz, 1H), 8.27 (d, J = 7.9 Hz, 1H), 8.59 (d, J = 2.1 Hz, 1H), 9.82 (s, 1H) |
| N-(2-Aminophenyl)-5-[3-(2-methoxyphenyl)-1-[4-(morpholin-4-yl)butyl]ureidomethyl]pyridine-2-carboxylic acid amide (Compound No.1-115) 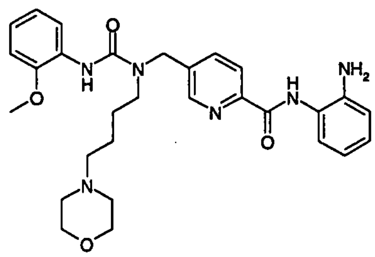 | $^{1}$H-NMR (400 MHz, $CDCl_3$) δ 1.56 (m, 2H), 1.73 (m, 2H), 2.37 (t, J = 7.3 Hz, 2H), 2.42 (t, J = 4.6 Hz, 4H), 3.39 (t, J = 7.5 Hz, 2H), 3.69 (t, J = 4.6 Hz, 4H), 3.83 (s, 3H), 3.95 (s, 2H), 4.70 (s, 2H), 6.85 (d, J = 7.7 Hz, 1H), 6.86 (m, 1H), 6.86 (td, J = 7.7, 1.4 Hz, 1H), 6.98-7.00 (m, 2H), 7.09 (td, J = 7.7, 1.4 Hz, 1H), 7.15 (s, 1H), 7.49 (dd, J = 7.7, 1.4 Hz, 1H), 7.89 (dd, J = 8.0, 2.2 Hz, 1H), 8.14 (m, 1H), 8.27 (dd, J = 8.0, 0.7 Hz, 1H), 8.60 (dd, J = 2.2, 0.7 Hz, 1H), 9.83 (s, 1H) |
| N-(2-Aminophenyl)-5-[3-(3-methylphenyl)-1-[3-(4-methylpiperidin-1-yl)propyl]ureidomethyl]pyridine-2-carboxylic acid amide (Compound No.1-116) | $^{1}$H-NMR (400 MHz, DMSO-$d_6$) δ 0.86 (d, J = 6.6 Hz, 3H), 1.11 (m, 2H), 1.30 (m, 1H), 1.54 (d, J = 11.5 Hz, 2H), 1.71 (m, 2H), 1.81 (t, J = 11.1 Hz, 2H), 2.26 (s, |

| | |
|---|---|
| 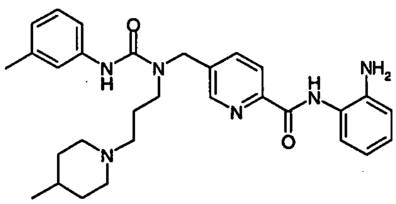 | 3H), 2.27 (t, J = 6.8 Hz, 2H), 2.79 (d, J = 11.1 Hz, 2H), 3.36 (t, J = 6.3 Hz, 2H), 4.66 (s, 2H), 4.89 (s, 2H), 6.65 (t, J = 7.7 Hz, 1H), 6.79-6.83 (m, 2H), 6.95 (m, 1H), 7.13 (t, J = 7.7 Hz, 1H), 7.28-7.32 (m, 2H), 7.50 (d, J = 7.7 Hz, 1H), 7.93 (dd, J = 8.2, 2.1 Hz, 1H), 8.12 (d, J = 8.2 Hz, 1H), 8.64 (d, J = 2.1 Hz, 1H), 8.79 (s, 1H), 10.03 (s, 1H) |
| N-(2-Aminophenyl)-5-[3-(3,4-difluorophenyl)-1-[3-(4-methylpiperidin-1-yl)propyl]ureidomethyl]pyridine-2-carboxylic acid amide (Compound No.1-117) 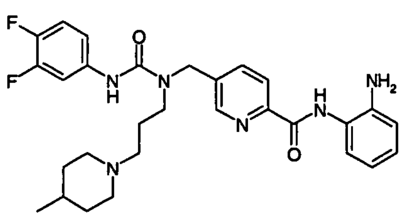 | $^1$H-NMR (400 MHz, DMSO-$d_6$) δ 0.86 (d, J = 6.3 Hz, 3H), 1.07 (m, 2H), 1.29 (m, 1H), 1.53 (d, J = 11.0 Hz, 2H), 1.70 (m, 2H), 1.80 (t, J = 11.0 Hz, 2H), 2.26 (t, J = 6.5 Hz, 2H), 2.77 (d, J = 11.0 Hz, 2H), 3.37 (t, J = 7.0 Hz, 2H), 4.68 (s, 2H), 4.89 (s, 2H), 6.65 (t, J = 7.8 Hz, 1H), 6.82 (d, J = 8.3 Hz, 1H), 6.95 (m, 1H), 7.24 (m, 1H), 7.33 (m, 1H), 7.50 (d, J = 7.8 Hz, 1H), 7.65 (m, 1H), 7.93 (dd, J = 8.1, 2.0 Hz, 1H), 8.12 |

| | |
|---|---|
| | (d, J = 8.1 Hz, 1H), 8.64 (d, J = 2.0 Hz, 1H), 9.00 (s, 1H), 10.03 (s, 1H) |
| N-(2-Aminophenyl)-5-[3-(2,3-dihydrobenzo[1,4]dioxin-6-yl)-1-[3-(4-methylpiperidin-1-yl)propyl]ureidomethyl]pyridine-2-carboxylic acid amide (Compound No.1-118) 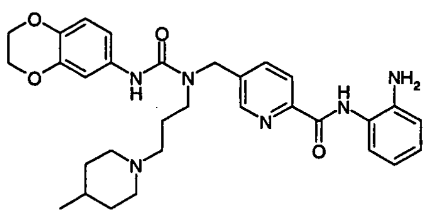 | $^1$H-NMR (400 MHz, DMSO-$d_6$) δ 0.85 (d, J = 6.3 Hz, 3H), 1.08 (m, 2H), 1.29 (m, 1H), 1.53 (d, J = 11.2 Hz, 2H), 1.69 (m, 2H), 1.80 (t, J = 11.2 Hz, 2H), 2.26 (t, J = 6.3 Hz, 2H), 2.78 (d, J = 11.2 Hz, 2H), 3.32 (m, 2H), 4.17-4.21 (m, 4H), 4.64 (s, 2H), 4.90 (s, 2H), 6.65 (m, 1H), 6.74 (d, J = 8.7 Hz, 1H), 6.82 (dd, J = 8.1, 1.3 Hz, 1H), 6.86 (dd, J = 8.7, 2.4 Hz, 1H), 6.95 (m, 1H), 7.04 (d, J = 2.4 Hz, 1H), 7.50 (d, J = 8.1 Hz, 1H), 7.92 (dd, J = 8.1, 2.0 Hz, 1H), 8.11 (d, J = 8.1 Hz, 1H), 8.63 (d, J = 2.0 Hz, 1H), 8.72 (s, 1H), 10.03 (s, 1H) |
| N-(2-Aminophenyl)-5-[3-(4-fluorophenyl)-1-[3-(morpholin-4-yl)propyl]ureidomethyl]pyridine-2-carboxylic acid amide (Compound | $^1$H-NMR (500 MHz, $CDCl_3$) δ 1.79 (m, 2H), 2.45 (br s, 4H), 2.49 (t, J = 6.0 Hz, 2H), 3.39 (t, J = 5.7 Hz, 2H), 3.63 (t, |

| | |
|---|---|
| No.1-119)<br>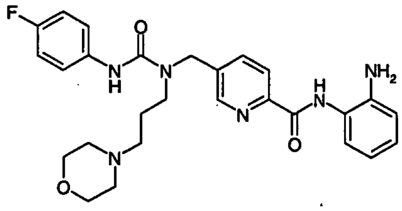 | J = 4.4 Hz, 4H), 3.96 (br s, 2H), 4.64 (s, 2H), 6.83-6.88 (m, 2H), 7.03 (t, J = 8.9 Hz, 2H), 7.09 (td, J = 7.7, 1.1 Hz, 1H), 7.38 (dd, J = 8.9, 4.9 Hz, 2H), 7.48 (dd, J = 7.7, 1.1 Hz, 1H), 7.91 (dd, J = 8.0, 1.9 Hz, 1H), 8.24 (d, J = 8.0 Hz, 1H), 8.59 (d, J = 1.9 Hz, 1H), 9.08 (s, 1H), 9.82 (s, 1H) |
| N-(2-Aminophenyl)-5-[3-(4-fluoro-3-methylphenyl)-1-[3-(morpholin-4-yl)propyl]ureidomethyl]pyridine-2-carboxylic acid amide (Compound No.1-120)<br>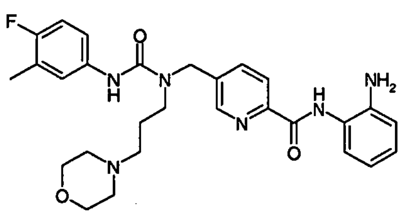 | $^{1}$H-NMR (500 MHz, $CDCl_3$)<br>δ 1.78 (m, 2H), 2.27 (d, J = 2.1 Hz, 3H), 2.45 (br s, 4H), 2.48 (t, J = 6.0 Hz, 2H), 3.38 (t, J = 5.7 Hz, 2H), 3.64 (t, J = 4.6 Hz, 4H), 3.95 (br s, 2H), 4.64 (s, 2H), 6.83-6.88 (m, 2H), 6.95 (t, J = 8.7 Hz, 1H), 7.09 (td, J = 7.7, 1.3 Hz, 1H), 7.13 (ddd, J = 8.7, 4.3, 2.7 Hz, 1H), 7.30 (dd, J = 6.9, 2.7 Hz, 1H), 7.48 (dd, J = 7.7, 1.3 Hz, 1H), 7.91 (dd, J = 7.9, 2.0 Hz, 1H), 8.24 (d, J = 7.9 Hz, 1H), 8.59 (d, J = 2.0 Hz, 1H), 8.99 (s, 1H), 9.82 (s, 1H) |

| | |
|---|---|
| N-(2-Aminophenyl)-5-[3-(3,4-difluorophenyl)-1-[3-(morpholin-4-yl)propyl]ureidomethyl]pyridine-2-carboxylic acid amide (Compound No.1-121) 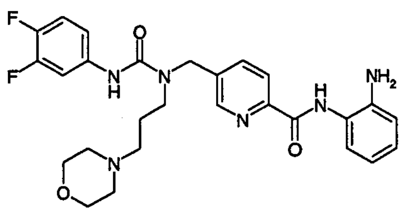 | $^1$H-NMR (400 MHz, $CDCl_3$) δ 1.79 (m, 2H), 2.43-2.52 (m, 6H), 3.38 (t, J = 5.6 Hz, 2H), 3.67 (t, J = 4.8 Hz, 4H), 3.96 (br s, 2H), 4.64 (s, 2H), 6.85 (d, J = 7.6 Hz, 1H), 6.86 (m, 1H), 7.01-7.14 (m, 3H), 7.46-7.53 (m, 2H), 7.90 (dd, J = 8.1, 2.2 Hz, 1H), 8.24 (dd, J = 8.1, 0.7 Hz, 1H), 8.58 (dd, J = 2.2, 0.7 Hz, 1H), 9.16 (s, 1H), 9.82 (s, 1H) |
| N-(2-Aminophenyl)-5-[3-(3-methylphenyl)-1-[3-(morpholin-4-yl)propyl]ureidomethyl]pyridine-2-carboxylic acid amide (Compound No.1-122) 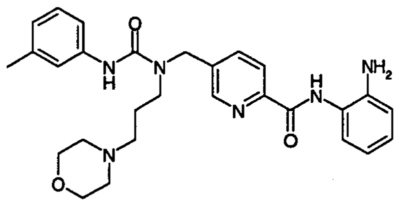 | $^1$H-NMR (400 MHz, $CDCl_3$) δ 1.79 (m, 2H), 2.35 (s, 3H), 2.43-2.52 (m, 6H), 3.39 (t, J = 5.7 Hz, 2H), 3.70 (t, J = 4.6 Hz, 4H), 3.96 (br s, 2H), 4.65 (s, 2H), 6.85 (d, J = 7.6 Hz, 1H), 6.86 (m, 1H), 6.91 (m, 1H), 7.09 (td, J = 7.6, 1.4 Hz, 1H), 7.19-7.22 (m, 2H), 7.34 (s, 1H), 7.48 (dd, J = 7.6, 1.4 Hz, 1H), 7.92 (dd, J = 8.1, 2.2 Hz, 1H), 8.24 (dd, J = 8.1, 0.7 Hz, 1H), 8.59 (dd, J = 2.2, |

| | |
|---|---|
| | 0.7 Hz, 1H), 8.87 (s, 1H), 9.83 (s, 1H) |
| 5-[3-(3-Acetylphenyl)-1-[3-(morpholin-4-yl)propyl]ureidomethyl]-N-(2-aminophenyl)pyridine-2-carboxylic acid amide (Compound No.1-123) 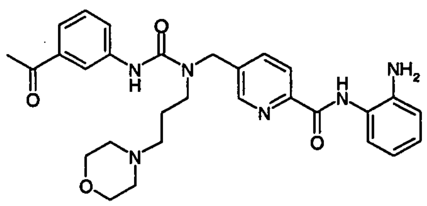 | $^1$H-NMR (400 MHz, $CDCl_3$) δ 1.81 (m, 2H), 2.45-2.53 (m, 6H), 2.62 (s, 3H), 3.42 (t, J = 5.6 Hz, 2H), 3.72 (t, J = 4.6 Hz, 4H), 3.96 (br s, 2H), 4.66 (s, 2H), 6.85 (d, J = 7.8 Hz, 1H), 6.86 (m, 1H), 7.09 (td, J = 7.8, 1.5 Hz, 1H), 7.44 (t, J = 7.9 Hz, 1H), 7.48 (d, J = 7.8 Hz, 1H), 7.67 (ddd, J = 7.9, 1.8, 1.0 Hz, 1H), 7.81 (ddd, J = 7.9, 1.8, 1.0 Hz, 1H), 7.92 (dd, J = 8.1, 2.2 Hz, 1H), 8.00 (t, J = 1.8 Hz, 1H), 8.25 (dd, J = 8.1, 0.7 Hz, 1H), 8.60 (dd, J = 2.2, 0.7 Hz, 1H), 9.22 (s, 1H), 9.83 (s, 1H) |
| N-(2-Aminophenyl)-5-[3-(3-methylphenyl)-1-[5-(morpholin-4-yl)pentyl]ureidomethyl]pyridine-2-carboxylic acid amide (Compound No.1-124) | $^1$H-NMR (400 MHz, $CDCl_3$) δ 1.37 (m, 2H), 1.53 (m, 2H), 1.67 (m, 2H), 2.32 (m, 2H), 2.33 (s, 3H), 2.42 (br s, 4H), 3.31 (t, J = 7.4 Hz, 2H), 3.70 (br s, 4H), 3.95 (br s, 2H), 4.68 (s, 2H), 6.37 (s, 1H), |

| | |
|---|---|
| 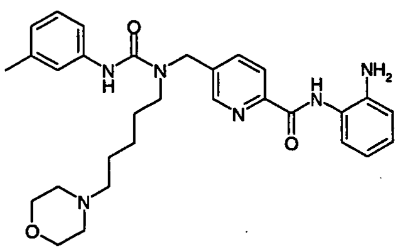 | 6.83-6.89 (m, 3H), 7.07-7.20 (m, 3H), 7.25 (s, 1H), 7.49 (d, J = 7.6 Hz, 1H), 7.86 (d, J = 8.1 Hz, 1H), 8.25 (d, J = 8.1 Hz, 1H), 8.57 (s, 1H), 9.82 (s, 1H) |
| N-(2-Aminophenyl)-5-[3-(3,4-difluorophenyl)-1-[5-(morpholin-4-yl)pentyl]ureidomethyl]pyridine-2-carboxylic acid amide (Compound No.1-125) 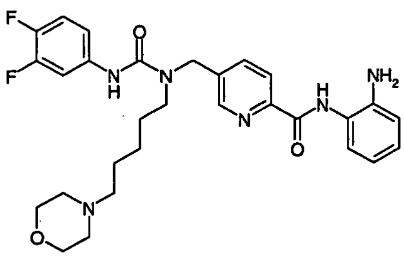 | $^1$H-NMR (500 MHz, $CDCl_3$) δ 1.35 (m, 2H), 1.52 (m, 2H), 1.65 (m, 2H), 2.32 (t, J = 7.5 Hz, 2H), 2.42 (br s, 4H), 3.29 (t, J = 7.8 Hz, 2H), 3.70 (t, J = 4.6 Hz, 4H), 3.94 (s, 2H), 4.65 (s, 2H), 6.69 (s, 1H), 6.83-6.87 (m, 2H), 6.97 (d, J = 8.9 Hz, 1H), 7.02-7.10 (m, 2H), 7.45 (m, 1H), 7.48 (d, J = 7.9 Hz, 1H), 7.80 (dd, J = 7.9, 1.8 Hz, 1H), 8.18 (d, J = 7.9 Hz, 1H), 8.53 (d, J = 1.8 Hz, 1H), 9.81 (s, 1H) |
| N-(2-Aminophenyl)-5-[3-(2,3-dihydrobenzo[1,4]dioxin-6-yl)-1-[5-(morpholin-4-yl)pentyl]ureidomethyl]pyridine-2-carboxylic acid amide (Compound No.1-126) | $^1$H-NMR (400 MHz, $CDCl_3$) δ 1.35 (m, 2H), 1.52 (m, 2H), 1.65 (m, 2H), 2.31 (t, J = 7.7 Hz, 2H), 2.41 (br s, 4H), 3.28 (t, J = 7.7 Hz, 2H), 3.70 (t, J = 4.5 Hz, 4H), |

| | |
|---|---|
| 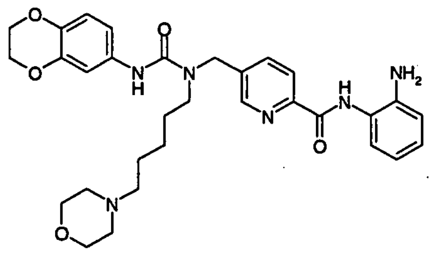 | 3.96 (s, 2H), 4.20-4.24 (m, 4H), 4.65 (s, 2H), 6.32 (s, 1H), 6.75-6.79 (m, 2H), 6.83-6.88 (m, 2H), 6.95 (m, 1H), 7.08 (td, J = 7.7, 1.4 Hz, 1H), 7.49 (d, J = 7.7 Hz, 1H), 7.84 (dd, J = 8.1, 2.0 Hz, 1H), 8.23 (d, J = 8.1 Hz, 1H), 8.55 (d, J = 2.0 Hz, 1H), 9.82 (s, 1H) |
| N-(2-Aminophenyl)-5-[1-(3-dimethylaminopropyl)-3-(4-phenyl-1,3-thiazol-2-yl)ureidomethyl]pyridine-2-carboxylic acid amide (Compound No.1-127) 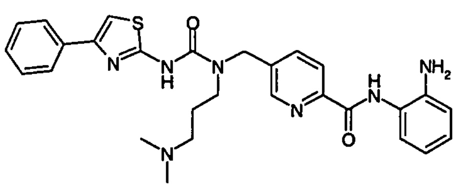 | $^1$H-NMR (400 MHz, DMSO-$d_6$) δ 1.76 (m, 2H), 2.34 (s, 6H), 2.37 (t, J = 5.7 Hz, 2H), 3.42 (t, J = 5.5 Hz, 2H), 4.68 (s, 2H), 4.91 (br s, 2H), 6.65 (t, J = 7.8 Hz, 1H), 6.82 (d, J = 7.8 Hz, 1H), 6.95 (t, J = 7.8 Hz, 1H), 7.29 (t, J = 7.8 Hz, 1H), 7.41 (t, J = 7.8 Hz, 2H), 7.48 (s, 1H), 7.51 (d, J = 7.8 Hz, 1H), 7.90 (d, J = 7.8 Hz, 2H), 7.99 (dd, J = 8.1, 1.7 Hz, 1H), 8.13 (d, J = 8.1 Hz, 1H), 8.70 (d, J = 1.7 Hz, 1H), 10.04 (br s, 1H), 13.45 (br s, 1H) |

| | |
|---|---|
| N-(2-Aminophenyl)-5-[3-(6-methoxypyridin-3-yl)-1-[3-(morpholin-4-yl)propyl]ureidomethyl]pyridine-2-carboxylic acid amide (Compound No.1-128) 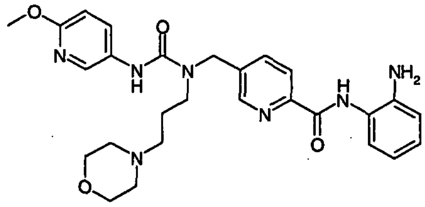 | $^1$H-NMR (500 MHz, $CDCl_3$) δ 1.79 (m, 2H), 2.45 (br s, 4H), 2.50 (t, J = 6.0 Hz, 2H), 3.41 (t, J = 5.5 Hz, 2H), 3.61 (t, J = 4.3 Hz, 4H), 3.92 (s, 3H), 3.98 (br s, 2H), 4.64 (s, 2H), 6.75 (d, J = 8.9 Hz, 1H), 6.84-6.88 (m, 2H), 7.09 (td, J = 7.7, 1.5 Hz, 1H), 7.48 (dd, J = 7.7, 1.5 Hz, 1H), 7.78 (dd, J = 8.9, 2.6 Hz, 1H), 7.91 (dd, J = 7.9, 2.1 Hz, 1H), 8.06 (d, J = 2.6 Hz, 1H), 8.24 (dd, J = 7.9, 0.6 Hz, 1H), 8.59 (dd, J = 2.1, 0.6 Hz, 1H), 9.26 (s, 1H), 9.82 (s, 1H) |
| N-(2-Aminophenyl)-5-[3-(4-methylpyridin-2-yl)-1-[3-(morpholin-4-yl)propyl]ureidomethyl]pyridine-2-carboxylic acid amide (Compound No.1-129) 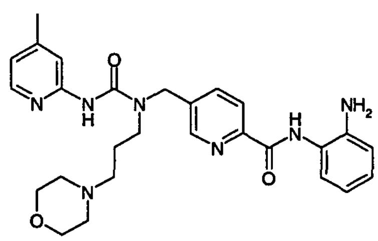 | $^1$H-NMR (400 MHz, $CDCl_3$) δ 1.80 (m, 2H), 2.34 (s, 3H), 2.45 (t, J = 5.9 Hz, 2H), 2.50 (br s, 4H), 3.44 (t, J = 5.6 Hz, 2H), 3.54 (br s, 2H), 3.97 (t, J = 4.3 Hz, 4H), 4.66 (s, 2H), 6.76 (d, J = 4.9 Hz, 1H), 6.83-6.89 (m, 2H), 7.09 (td, J = 7.8, 1.4 Hz, 1H), 7.48 (dd, J = 7.8, 1.4 Hz, 1H), 7.86 (s, |

| | |
|---|---|
| | 1H), 7.91 (dd, J = 8.0, 2.1 Hz, 1H), 8.08 (d, J = 4.9 Hz, 1H), 8.26 (dd, J = 8.0, 0.5 Hz, 1H), 8.60 (dd, J = 2.1, 0.5 Hz, 1H), 9.71 (s, 1H), 9.83 (s, 1H) |
| N-(2-Aminophenyl)-5-[1-[3-(morpholin-4-yl)propyl]-3-(1,3-thiazol-2-yl)ureidomethyl]pyridine-2-carboxylic acid amide (Compound No.1-130) 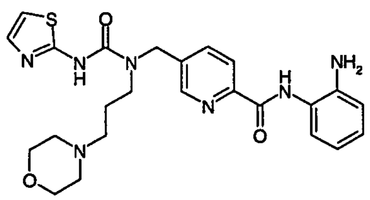 | $^1H$-NMR (500 MHz, $CDCl_3$) δ 1.79 (m, 2H), 2.46 (t, J = 6.0 Hz, 2H), 2.51 (br s, 4H), 3.40 (t, J = 5.7 Hz, 2H), 3.95 (br s, 2H), 4.03 (br s, 4H), 4.68 (s, 2H), 6.83-6.88 (m, 2H), 6.87 (d, J = 3.5 Hz, 1H), 7.09 (td, J = 7.7, 1.5 Hz, 1H), 7.37 (d, J = 3.5 Hz, 1H), 7.48 (dd, J = 7.7, 1.5 Hz, 1H), 7.91 (dd, J = 7.9, 2.1 Hz, 1H), 8.26 (dd, J = 7.9, 0.6 Hz, 1H), 8.60 (dd, J = 2.1, 0.6 Hz, 1H), 9.82 (s, 1H), 11.67 (br s, 1H) |
| N-(2-Aminophenyl)-5-[1-[3-(morpholin-4-yl)propyl]-3-(5-nitro-1,3-thiazol-2-yl)ureidomethyl]pyridine-2-carboxylic acid amide (Compound No.1-131) | $^1H$-NMR (500 MHz, $CDCl_3$) δ 1.84 (m, 2H), 2.48 (t, J = 6.0 Hz, 2H), 2.54 (br s, 4H), 3.41 (t, J = 5.7 Hz, 2H), 3.93 (br s, 2H), 4.01 (br s, 4H), 4.67 (s, 2H), |

| | |
|---|---|
| 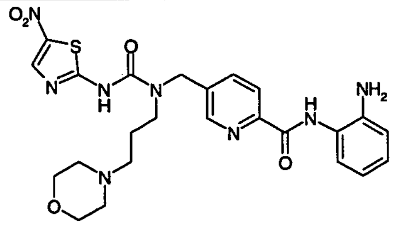 | 6.84-6.89 (m, 2H), 7.10 (td, J = 7.8, 1.2 Hz, 1H), 7.49 (dd, J = 7.8, 1.2 Hz, 1H), 7.90 (dd, J = 8.0, 2.1 Hz, 1H), 8.26 (s, 1H), 8.28 (d, J = 8.0 Hz, 1H), 8.61 (d, J = 2.1 Hz, 1H), 9.81 (s, 1H) |
| N-(2-Aminophenyl)-5-[3-cyclopentyl-1-[3-(morpholin-4-yl)propyl]ureidomethyl]pyridine-2-carboxylic acid amide (Compound No.1-132) 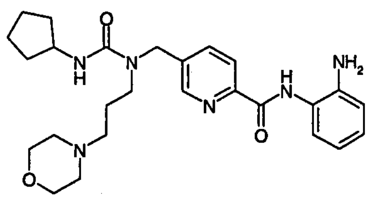 | $^1$H-NMR (400 MHz, $CDCl_3$) δ 1.35 (m, 2H), 1.58-1.70 (m, 6H), 2.07 (m, 2H), 2.37 (t, J = 6.1 Hz, 2H), 2.45 (t, J = 4.4 Hz, 4H), 3.21 (t, J = 6.1 Hz, 2H), 3.74 (t, J = 4.5 Hz, 4H), 3.96 (s, 2H), 4.11 (m, 1H), 4.58 (s, 2H), 5.91 (d, J = 6.8 Hz, 1H), 6.85 (dd, J = 7.7, 1.5 Hz, 1H), 6.87 (td, J = 7.7, 1.5 Hz, 1H), 7.08 (td, J = 7.7, 1.5 Hz, 1H), 7.48 (dd, J = 7.7, 1.5 Hz, 1H), 7.84 (dd, J = 8.0, 2.2 Hz, 1H), 8.23 (dd, J = 8.0, 0.6 Hz, 1H), 8.53 (dd, J = 2.2, 0.6 Hz, 1H), 9.82 (s, 1H) |
| N-(2-Aminophenyl)-5-[3-[2-(benzo[1,3]dioxol-5-yl)ethyl]-1-[3-(morpholin-4-yl)propyl]ureido | $^1$H-NMR (500 MHz, $CDCl_3$) δ 1.63 (m, 2H), 2.33 (t, J = 5.8 Hz, 2H), |

| | |
|---|---|
| methyl]pyridine-2-carboxylic acid amide (Compound No.1-133) 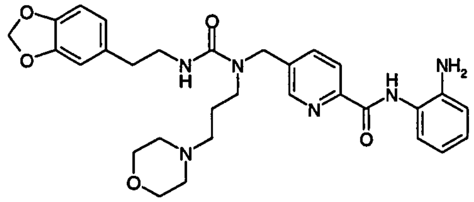 | 2.33 (s, 4H), 2.78 (t, J = 6.9 Hz, 2H), 3.17 (t, J = 5.8 Hz, 2H), 3.44 (q, J = 6.9 Hz, 2H), 3.60 (s, 4H), 3.97 (s, 2H), 4.57 (s, 2H), 5.92 (s, 2H), 6.64 (dd, J = 8.2, 1.7 Hz, 1H), 6.70 (d, J = 1.7 Hz, 1H), 6.75 (d, J = 8.2 Hz, 1H), 6.85 (dd, J = 7.8, 1.3 Hz, 1H), 6.86 (td, J = 7.8, 1.3 Hz, 1H), 7.06 (br s, 1H), 7.09 (td, J = 7.8, 1.3 Hz, 1H), 7.49 (dd, J = 7.8, 1.3 Hz, 1H), 7.80 (dd, J = 7.9, 2.1 Hz, 1H), 8.23 (dd, J = 7.9, 0.9 Hz, 1H), 8.48 (dd, J = 2.1, 0.9 Hz, 1H), 9.84 (s, 1H) |
| N-(2-Aminophenyl)-5-[3-isopropyl-1-[3-(morpholin-4-yl)propyl]ureidomethyl]pyridine-2-carboxylic acid amide (Compound No.1-134) 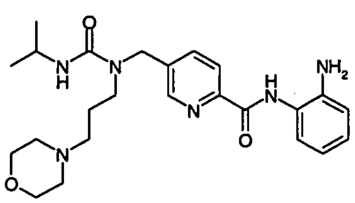 | $^1$H-NMR (400 MHz, $CDCl_3$) δ 1.20 (d, J = 6.6 Hz, 6H), 1.68 (m, 2H), 2.37 (t, J = 6.2 Hz, 2H), 2.45 (t, J = 4.5 Hz, 4H), 3.21 (t, J = 6.2 Hz, 2H), 3.76 (t, J = 4.5 Hz, 4H), 3.96 (s, 2H), 4.01 (m, 1H), 4.58 (s, 2H), 5.88 (d, J = 8.1 Hz, 1H), 6.85 (dd, J = 7.6, 1.5 Hz, 1H), 6.86 (td, J = 7.6, 1.5 Hz, |

| | |
|---|---|
| | 1H), 7.09 (td, J = 7.6, 1.5 Hz, 1H), 7.49 (dd, J = 7.6, 1.5 Hz, 1H), 7.84 (dd, J = 8.1, 2.2 Hz, 1H), 8.23 (dd, J = 8.1, 0.9 Hz, 1H), 8.53 (dd, J = 2.2, 0.9 Hz, 1H), 9.82 (s, 1H) |
| N-(2-Aminophenyl)-5-[1-[3-(morpholin-4-yl)propyl]-3-propylureidomethyl]pyridine-2-carboxylic acid amide (Compound No.1-135) 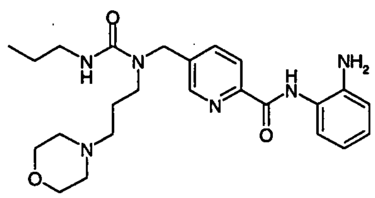 | $^1$H-NMR (500 MHz, $CDCl_3$) δ 0.95 (t, J = 7.5 Hz, 3H), 1.55 (m, 2H), 1.67 (m, 2H), 2.38 (t, J = 6.0 Hz, 2H), 2.44 (s, 4H), 3.20 (m, 2H), 3.24 (t, J = 5.8 Hz, 2H), 3.73 (t, J = 4.4 Hz, 4H), 3.96 (s, 2H), 4.58 (s, 2H), 6.85 (dd, J = 7.6, 1.2 Hz, 1H), 6,86 (td, J = 7.6, 1.2 Hz, 1H), 7.01 (t, J = 5.7 Hz, 1H), 7.08 (td, J = 7.6, 1.2 Hz, 1H), 7.48 (dd, J = 7.6, 1.2 Hz, 1H), 7.85 (dd, J = 7.9, 2.0 Hz, 1H), 8.23 (d, J = 7.9 Hz, 1H), 8.53 (d, J = 2.0 Hz, 1H), 9.82 (s, 1H) |
| N-(2-Aminophenyl)-5-[1-[4-(morpholin-4-yl)butyl]-3-propylureidomethyl]pyridine-2-carboxylic acid amide (Compound No.1-136) | $^1$H-NMR (500 MHz, $CDCl_3$) δ 0.92 (t, J = 7.5 Hz, 3H), 1.46-1.63 (m, 6H), 2.35 (t, J = 7.0 Hz, 2H), 2.41 (br s, 4H), |

| | |
|---|---|
| 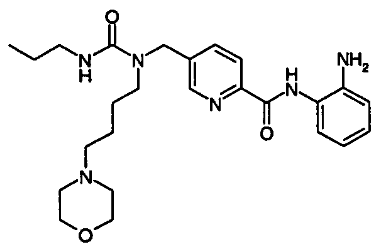 | 3.18 (t, J = 7.9 Hz, 2H), 3.25 (m, 2H), 3.71 (t, J = 4.6 Hz, 4H), 3.96 (br s, 2H), 4.61 (s, 2H), 4.78 (t, J = 5.5 Hz, 1H), 6.84-6.88 (m, 2H), 7.09 (td, J = 7.6, 1.5 Hz, 1H), 7.48 (d, J = 7.6 Hz, 1H), 7.81 (dd, J = 7.9, 2.0 Hz, 1H), 8.24 (d, J = 7.9 Hz, 1H), 8.52 (d, J = 2.0 Hz, 1H), 9.82 (s, 1H) |
| N-(2-Aminophenyl)-5-[3-isopropyl-1-[4-(morpholin-4-yl)butyl]ureidomethyl]pyridine-2-carboxylic acid amide (Compound No.1-137) 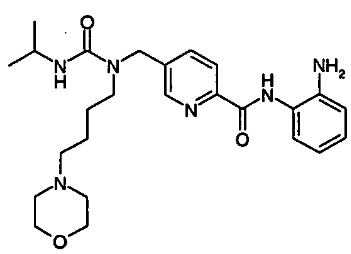 | ¹H-NMR (500 MHz, $CDCl_3$) δ 1.17 (d, J = 6.4 Hz, 6H), 1.48 (m, 2H), 1.59 (m, 2H), 2.33 (t, J = 7.2 Hz, 2H), 2.41 (br s, 4H), 3.17 (t, J = 7.6 Hz, 2H), 3.71 (t, J = 4.4 Hz, 4H), 3.96 (br s, 2H), 4.03 (m, 1H), 4.26 (d, J = 7.6 Hz, 1H), 4.59 (s, 2H), 6.84-6.89 (m, 2H), 7.08 (t, J = 7.6 Hz, 1H), 7.49 (d, J = 7.6 Hz, 1H), 7.80 (d, J = 7.9 Hz, 1H), 8.24 (d, J = 7.9 Hz, 1H), 8.51 (s, 1H), 9.82 (s, 1H) |
| N-(2-Aminophenyl)-5-[3-cyclopentyl-1-[4-(mo | ¹H-NMR (500 MHz, $CDCl_3$) |

| | |
|---|---|
| rpholin-4-yl)butyl]ureidomethyl]pyridine-2-carboxylic acid amide (Compound No.1-138) 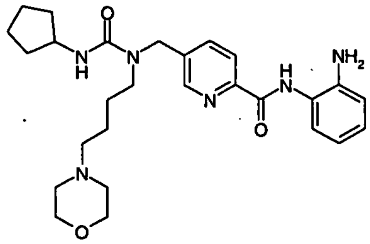 | δ 1.34 (m, 2H), 1.48 (m, 2H), 1.55-1.66 (m, 6H), 2.01 (m, 2H), 2.33 (t, J = 7.2 Hz, 2H), 2.40 (br s, 4H), 3.18 (t, J = 7.6 Hz, 2H), 3.70 (t, J = 4.6 Hz, 4H), 3.96 (br s, 2H), 4.15 (m, 1H), 4.37 (d, J = 6.7 Hz, 1H), 4.59 (s, 2H), 6.84-6.87 (m, 2H), 7.08 (td, J = 7.6, 1.2 Hz, 1H), 7.49 (d, J = 7.6 Hz, 1H), 7.81 (dd, J = 7.9, 2.1 Hz, 1H), 8.24 (d, J = 7.9 Hz, 1H), 8.51 (d, J = 2.1 Hz, 1H), 9.82 (s, 1H) |
| N-(2-Aminophenyl)-5-[1-[5-(morpholin-4-yl)pentyl]-3-propylureidomethyl]pyridine-2-carboxylic acid amide (Compound No.1-139) 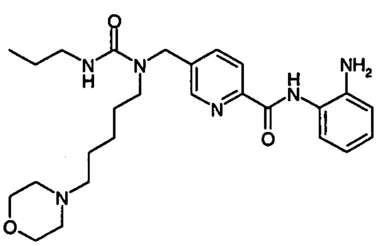 | $^1$H-NMR (400 MHz, $CDCl_3$) δ 0.91 (t, J = 7.4 Hz, 3H), 1.31 (m, 2H), 1.46-1.62 (m, 6H), 2.30 (t, J = 7.6 Hz, 2H), 2.41 (br s, 4H), 3.17 (t, J = 7.7 Hz, 2H), 3.23 (m, 2H), 3.70 (t, J = 4.6 Hz, 4H), 3.96 (br s, 2H), 4.46 (t, J = 5.5 Hz, 1H), 4.60 (s, 2H), 6.84-6.88 (m, 2H), 7.08 (td, J = 7.7, 1.4 Hz, 1H), 7.48 (d, J = 7.7 Hz, 1H), 7.80 (dd, J = 7.9, 2.1 Hz, 1H), 8.23 (d, J = 7.9 Hz, 1H), |

| | |
|---|---|
| | 8.51 (d, J = 2.1 Hz, 1H), 9.82 (s, 1H) |
| N-(2-Aminophenyl)-5-[3-isopropyl-1-[5-(morpholin-4-yl)pentyl]ureidomethyl]pyridine-2-carboxylic acid amide (Compound No.1-140) 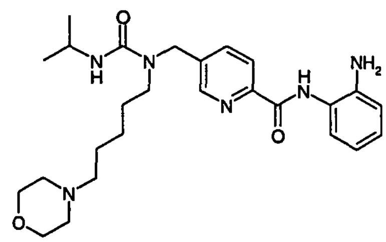 | $^1$H-NMR (500 MHz, $CDCl_3$) δ 1.16 (d, J = 6.7 Hz, 6H), 1.31 (m, 2H), 1.46-1.59 (m, 4H), 2.30 (t, J = 7.5 Hz, 2H), 2.41 (br s, 4H), 3.15 (t, J = 7.6 Hz, 2H), 3.70 (t, J = 4.7 Hz, 4H), 3.97 (br s, 2H), 4.01 (m, 1H), 4.20 (d, J = 7.3 Hz, 1H), 4.59 (s, 2H), 6.84-6.87 (m, 2H), 7.08 (td, J = 7.6, 1.5 Hz, 1H), 7.49 (d, J = 7.6 Hz, 1H), 7.79 (dd, J = 8.1, 2.0 Hz, 1H), 8.23 (d, J = 8.1 Hz, 1H), 8.51 (d, J = 2.0 Hz, 1H), 9.82 (s, 1H) |
| N-(2-Aminophenyl)-5-[3-cyclopentyl-1-[5-(morpholin-4-yl)pentyl]ureidomethyl]pyridine-2-carboxylic acid amide (Compound No.1-141) 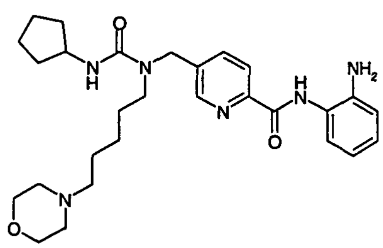 | $^1$H-NMR (500 MHz, $CDCl_3$) δ 1.27-1.37 (m, 4H), 1.46-1.66 (m, 8H), 2.00 (m, 2H), 2.30 (t, J = 7.5 Hz, 2H), 2.41 (br s, 4H), 3.15 (t, J = 7.6 Hz, 2H), 3.70 (t, J = 4.6 Hz, 4H), 3.97 (br s, 2H), 4.14 (m, 1H), 4.33 (d, J = 7.0 Hz, 1H), 4.58 (s, 2H), 6.84-6.87 (m, 2H), 7.08 (td, J = 7.7, 1.2 Hz, |

| | |
|---|---|
| | 1H), 7.49 (d, J = 7.7 Hz, 1H), 7.80 (dd, J = 8.2, 1.8 Hz, 1H), 8.23 (d, J = 8.2 Hz, 1H), 8.51 (d, J = 1.8 Hz, 1H), 9.82 (s, 1H) |
| N-(2-Aminophenyl)-5-[1-(2-dimethylaminoethyl)-3-[2-(piperidin-1-yl)ethyl]ureidomethyl]pyridine-2-carboxylic acid amide (Compound No.1-142) 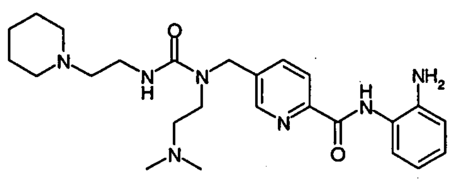 | $^1$H-NMR (500 MHz, $CDCl_3$) δ 1.44 (m, 2H), 1.54 (m, 4H), 2.26 (s, 6H), 2.39 (m, 4H), 2.41-2.46 (m, 4H), 3.26 (t, J = 5.5 Hz, 2H), 3.33 (m, 2H), 3.96 (s, 2H), 4.61 (s, 2H), 6.85 (dd, J = 7.8, 1.4 Hz, 1H), 6.86 (td, J = 7.8, 1.4 Hz, 1H), 7.09 (td, J = 7.8, 1.4 Hz, 1H), 7.10 (br s, 1H), 7.48 (dd, J = 7.8, 1.4 Hz, 1H), 7.85 (dd, J = 8.1, 2.1 Hz, 1H), 8.23 (dd, J = 8.1, 0.6 Hz, 1H), 8.54 (dd, J = 2.1, 0.6 Hz, 1H), 9.83 (s, 1H) |
| N-(2-Aminophenyl)-5-[3-(2-diisopropylaminoethyl)-1-(2-dimethylaminoethyl)ureidomethyl] pyridine-2-carboxylic acid amide (Compound No.1-143) | $^1$H-NMR (500 MHz, $CDCl_3$) δ 1.38 (m, 12H), 2.30 (s, 6H), 2.55 (m, 2H), 3.07 (m, 2H), 3.42-3.50 (m, 4H), 3.60 (m, 2H), 3.96 (s, 2H), 4.71 (s, 2H), 6.85 (dd, J = 7.6, 1.3 Hz, 1H), 6.86 (td, J = 7.6, 1.3 Hz, |

| | |
|---|---|
| 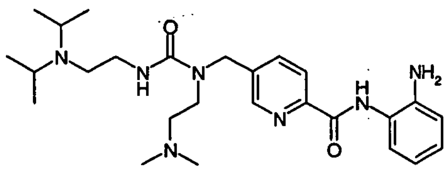 | 1H), 7.08 (td, J = 7.6, 1.3 Hz, 1H), 7.49 (dd, J = 7.6, 1.3 Hz, 1H), 7.85 (dd, J = 8.0, 2.0 Hz, 1H), 8.22 (d, J = 8.0 Hz, 1H), 8.57 (m, 1H), 9.82 (s, 1H) |
| N-(2-Aminophenyl)-5-[3-[2-(cyclohexen-1-yl) ethyl]-1-(2-dimethylaminoethyl)ureidomethyl] pyridine-2-carboxylic acid amide (Compound No.1-144) 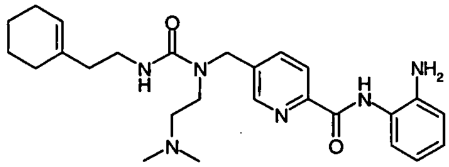 | $^1$H-NMR (500 MHz, $CDCl_3$) δ 1.54-1.62 (m, 4H), 1.95-2.02 (m, 4H), 2.14 (t, J = 7.0 Hz, 1H), 2.24 (m, 1H), 2.24 (s, 6H), 2.40 (m, 2H), 3.22 (m, 2H), 3.31 (m, 1H), 3.46 (m, 1H), 3.96 (s, 2H), 4.60 (s, 2H), 5.45 (m, 1H), 6,85 (dd, J = 7.8, 1.3 Hz, 1H), 6.86 (td, J = 7.8, 1.3 Hz, 1H), 7.08 (td, J = 7.8, 1.3 Hz, 1H ), 7.11 (m, 1H), 7.48 (dd, J = 7.8, 1.3 Hz, 1H), 7.84 (dd, J = 7.9, 2.1 Hz, 1H), 8.23 (dd, J = 7.9, 0.9 Hz, 1H), 8.52 (dd, J = 2.1, 0.9 Hz, 1H), 9.82 (s, 1H) |
| N-(2-Aminophenyl)-5-[1-(2-dimethylaminoethyl)-3-propylureidomethyl]pyridine-2-carboxylic acid amide (Compound No.1-145) | $^1$H-NMR (400 MHz, $CDCl_3$) δ 0.95 (t, J = 7.5 Hz, 3H), 1.53 (m, 2H), 2.26 (s, 6H), 2.39 (t, J = 4.6 Hz, 2H), 3.18 |

| | |
|---|---|
| 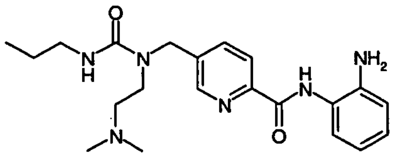 | (m, 2H), 3.21 (t, J = 4.6 Hz, 2H), 3.96 (s, 2H), 4.60 (s, 2H), 6.85 (dd, J = 7.6, 1.2 Hz, 1H), 6.86 (td, J = 7.6, 1.2 Hz, 1H), 7.08 (td, J = 7.6, 1.2 Hz, 1H), 7.48 (dd, J = 7.6, 1.2 Hz, 1H), 7.78 (t, J = 5.2 Hz, 1H), 7.85 (dd, J = 7.9, 1.8 Hz, 1H), 8.23 (d, J = 7.9 Hz, 1H), 8.53 (d, J = 1.8 Hz, 1H), 9.83 (s, 1H) |
| N-(2-Aminophenyl)-5-[1-(2-dimethylaminoethyl)-3-isopropylureidomethyl]pyridine-2-carboxylic acid amide (Compound No.1-146) 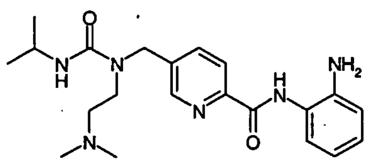 | $^1$H-NMR (400 MHz, $CDCl_3$) δ 1.15 (d, J = 6.4 Hz, 6H), 2.27 (s, 6H), 2.41 (s, 2H), 3.20 (s, 2H), 3.90 (m, 1H), 3.96 (s, 2H), 4.60 (s, 2H), 6.85 (dd, J =7.8, 1.4 Hz, 1H), 6.86 (td, J = 7.8, 1.4 Hz, 1H), 7.09 (td, J = 7.8, 1.4 Hz, 1H), 7.48 (dd, J = 7.8, 1.4 Hz, 1H), 7.84 (dd, J = 7.9, 2.1 Hz, 1H), 7.91 (s, 1H), 8.23 (dd, J = 7.9, 0.6 Hz, 1H), 8.53 (dd, J = 2.1, 0.6 Hz, 1H), 9.83 (s, 1H) |
| N-(2-Aminophenyl)-5-[3-sec-butyl-1-(2-dimethylaminoethyl)ureidomethyl]pyridine-2-carbo | $^1$H-NMR (400 MHz, $CDCl_3$) δ 0.93 (t, J = 7.3 Hz, 3H), 1.12 (d, J = 6.4 |

| | |
|---|---|
| xylic acid amide (Compound No.1-147) 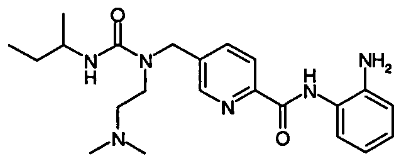 | Hz, 3H), 1.48 (m, 2H), 2.26 (s, 6H), 2.40 (t, J = 4.4 Hz, 2H), 3.20 (m, 2H), 3.75 (m, 1H), 3.96 (s, 2H), 4.60 (d, J = 15.9 Hz, 1H), 4.60 (d, J = 15.9 Hz, 1H), 6.84 (dd, J = 7.6, 0.8 Hz, 1H), 6.85 (td, J = 7.6, 0.8 Hz, 1H), 7.07 (td, J = 7.6, 0.8 Hz, 1H), 7.48 (dd, J = 7.6, 0.8 Hz, 1H), 7.80 (s, 1H), 7.84 (dd, J = 7.9, 1.8 Hz, 1H), 8.23 (d, J = 7.9 Hz, 1H), 8.53 (d, J = 1.8 Hz, 1H), 9.82 (s, 1H) |
| N-(2-Aminophenyl)-5-[3-cyclopentyl-1-(2-dimethylaminoethyl)ureidomethyl]pyridine-2-carboxylic acid amide (Compound No.1-148) 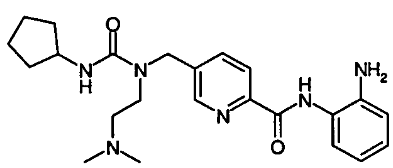 | $^1$H-NMR (400 MHz, $CDCl_3$) δ 1.40 (m, 2H), 1.62-1.69 (m, 4H), 1.97 (m, 2H), 2.26 (s, 6H), 2.40 (t, J = 4.4 Hz, 2H), 3.18 (t, J = 4.4 Hz, 2H), 3.96 (s, 2H), 4.07 (m, 1H), 4.59 (s, 2H), 6.85 (dd, J = 7.8, 1.5 Hz, 1H), 6.86 (td, J = 7.8, 1.5 Hz, 1H), 7.08 (td, J = 7.8, 1.5 Hz, 1H), 7.48 (dd, J = 7.8, 1.5 Hz, 1H), 7.85 (dd, J = 8.0, 1.9 Hz, 1H), 8.02 (s, 1H), 8.23 (d, J = 8.0 Hz, 1H), 8.53 |

| | |
|---|---|
| | (d, J = 1.9 Hz, 1H), 9.83 (s, 1H) |
| N-(2-Aminophenyl)-5-[1-(2-dimethylaminoethyl)-3-hexylureidomethyl]pyridine-2-carboxylic acid amide (Compound No.1-149) 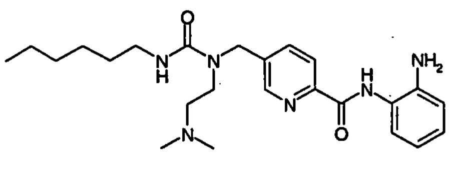 | $^1$H-NMR (500 MHz, $CDCl_3$) δ 0.89 (t, J =7.0 Hz, 3H), 1.28-1.36 (m, 6H), 1.50 (m, 2H), 2.38 (s, 6H), 2.56 (m, 2H), 3.21 (m, 2H), 3.35 (s, 2H), 3.96 (s, 2H), 4.62 (s, 2H), 6.85 (dd, J = 7.7, 1.3 Hz, 1H), 6.86 (td, J = 7.7, 1.3 Hz, 1H), 7.08 (td, J = 7.7, 1.3 Hz, 1H), 7.48 (dd, J = 7.7, 1.3 Hz, 1H), 7.84 (dd, J = 7.9, 2.1 Hz, 1H), 8.22 (dd, J = 7.9, 0.6 Hz, 1H), 8.52 (dd, J = 2.1, 0.6 Hz, 1H), 9.82 (s, 1H) |
| N-(2-Aminophenyl)-5-[3-cyclohexyl-1-(2-dimethylaminoethyl)ureidomethyl]pyridine-2-carboxylic acid amide (Compound No.1-150) 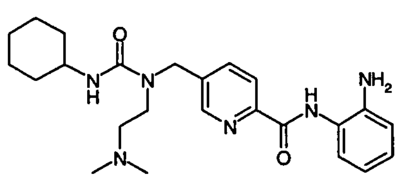 | $^1$H-NMR (400 MHz, $CDCl_3$) δ 1.11 (m, 2H), 1.39 (m, 2H), 1.60 (m, 2H), 1.71 (m, 2H), 1.96 (m, 2H), 2.25 (s, 6H), 2.40 (t, J = 4.5 Hz, 2H), 3.19 (t, J = 4.5 Hz, 2H), 3.59 (m, 1H), 3.96 (s, 2H), 4.59 (s, 2H), 6.85 (dd, J = 7.7, 1,5 Hz, 1H), 6.86 (td, J = 7.7, 1.5 Hz, 1H), 7.08 (td, J = 7.7, 1.5 Hz, |

| | |
|---|---|
| | 1H), 7.49 (dd, J = 7.7, 1.5 Hz, 1H), 7.85 (dd, J = 8.0, 2.2 Hz, 1H), 7.89 (d, J = 6.6 Hz, 1H), 8.23 (dd, J = 8.0, 0.7 Hz, 1H), 8.53 (dd, J = 2.2, 0.7 Hz, 1H), 9.83 (s, 1H) |
| N-(2-Aminophenyl)-5-[1-(2-dimethylaminoethyl)-3-phenethylureidomethyl]pyridine-2-carboxylic acid amide (Compound No.1-151) 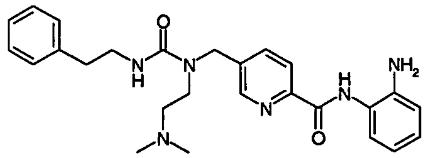 | $^1$H-NMR (400 MHz, $CDCl_3$) δ 2.04 (s, 6H), 2.30 (t, J = 4.8 Hz, 2H), 2.85 (t, J = 6.9 Hz, 2H), 3.12 (t, J = 4.8 Hz, 2H), 3.53 (m, 2H), 3.96 (s, 2H), 4.59 (s, 2H), 6.85 (dd, J = 7.8, 1.5 Hz, 1H), 6.86 (td, J = 7.8, 1.5 Hz, 1H), 7.09 (td, J = 7.8, 1.5 Hz, 1H), 7.19-7.24 (m, 3H), 7.28-7.32 (m, 2H), 7.48 (dd, J = 7.8, 1.5 Hz, 1H), 7.70 (t, J = 4.9 Hz, 1H), 7.81 (dd, J = 7.9, 2.1 Hz, 1H), 8.23 (dd, J = 7.9, 0.6 Hz, 1H), 8.51 (dd, J = 2.1, 0.6 Hz, 1H), 9.83 (s, 1H) |
| N-(2-Aminophenyl)-5-[1-(2-dimethylaminoethyl)-3-[2-(thiophen-2-yl)ethyl]ureidomethyl]pyridine-2-carboxylic acid amide (Compound | $^1$H-NMR (400 MHz, $CDCl_3$) δ 2.10 (s, 6H), 2.34 (t, J = 4.7 Hz, 2H), 3.06 (t, J = 6.3 Hz, 2H), 3.17 (t, J = 4.7 Hz, 2H), |

| | |
|---|---|
| No.1-152)<br>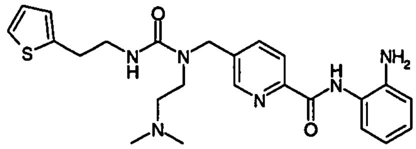 | 3.64 (m, 2H), 3.96 (s, 2H), 4.60 (s, 2H), 6.85 (dd, J = 3.4, 1.2 Hz, 1H), 6.85 (dd, J = 7.8, 1.5 Hz, 1H), 6.86 (td, J = 7.8, 1.5 Hz, 1H), 6.94 (dd, J = 5.1, 3.4 Hz, 1H), 7.09 (td, J = 7.7, 1.5 Hz, 1H), 7.14 (dd, J = 5.1, 1.2 Hz, 1H), 7.50 (dd, J = 7.7, 1.5 Hz, 1H), 7.82 (dd, J = 8.0, 2.1 Hz, 1H), 7.89 (s, 1H), 8.23 (dd, J = 8.0, 0.6 Hz, 1H), 8.51 (dd, J = 2.1, 0.6 Hz, 1H), 9.83 (s, 1H) |
| N-(2-Aminophenyl)-5-[1-(2-dimethylaminoethyl)-3-(2,2,2-trifluoroethyl)ureidomethyl]pyridine-2-carboxylic acid amide (Compound No.1-153)<br>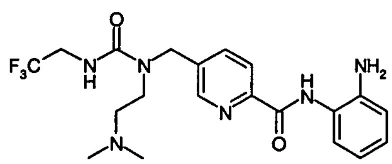 | $^1$H-NMR (400 MHz, $CDCl_3$)<br>δ 2.28 (s, 6H), 2.44 (t, J = 4.3 Hz, 2H), 3.25 (t, J = 4.3 Hz, 2H), 3.88 (m, 2H), 3.96 (s, 2H), 4.62 (s, 2H), 6.85 (dd, J = 7.7, 1.5 Hz, 1H), 6.86 (td, J = 7.7, 1.5 Hz, 1H), 7.09 (td, J = 7.7, 1.5 Hz, 1H), 7.50 (dd, J = 7.7, 1.5 Hz, 1H), 7.83 (dd, J = 8.0, 2.2 Hz, 1H), 8.23 (dd, J = 8.0, 0.6 Hz, 1H), 8.53 (dd, J = 2.2, 0.6 Hz, 1H), 9.45 (m, 1H), 9.82 (s, 1H) |

| | |
|---|---|
| N-(2-Aminophenyl)-5-[3-(2-cyanoethyl)-1-(2-dimethylaminoethyl)ureidomethyl]pyridine-2-carboxylic acid amide (Compound No.1-154) 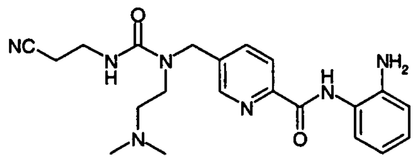 | $^1$H-NMR (500MHz, $CDCl_3$) δ 2.26 (s, 6H), 2.41 (t, J = 4.9 Hz, 2H), 2.57 (t, J = 6.3 Hz, 2H), 3.21 (t, J = 4.9 Hz, 2H), 3.50 (m, 2H), 3.69 (s, 2H), 4.59 (s, 2H), 6.85 (dd, J = 7.7, 1.5 Hz, 1H), 6.87 (td, J = 7.7, 1.5 Hz, 1H), 7.08 (td, J = 7.7, 1.5 Hz, 1H), 7.48 (dd, J = 7.7, 1.5 Hz, 1H), 7.83 (dd, J = 7.9, 2.1 Hz, 1H), 7.92 (s, 1H), 8.23 (d, J = 7.9 Hz, 1H), 8.52 (d, J = 1.8 Hz, 1H), 9.82 (s, 1H) |
| N-(2-Aminophenyl)-5-[1-(2-dimethylaminoethyl)-3-[3-(pyrrolidin-2-on-1-yl)propyl]ureidomethyl]pyridine-2-carboxylic acid amide (Compound No.1-155) 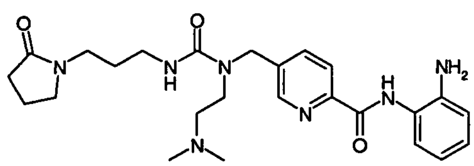 | $^1$H-NMR (400 MHz, $CDCl_3$) δ 1.70 (m, 2H), 2.04 (m, 2H), 2.27 (s, 6H), 2.39 (t, J = 8.2 Hz, 2H), 2.45 (t, J = 5.6 Hz, 2H), 3.20 (m, 2H), 3.30 (t, J = 5.6 Hz, 2H), 3.33 (t, J = 6.6 Hz, 2H), 3.40 (t, J = 7.1 Hz, 2H), 3.97 (s, 2H), 4.63 (s, 2H), 6.84 (dd, J = 7.7, 1.5 Hz, 1H), 6.85 (td, J = 7.7, 1.5 Hz, 1H), 7.08 (td, J = 7.7, 1.5 Hz, 1H), 7.36 (s, |

| | |
|---|---|
| | 1H), 7.48 (dd, J = 7.7, 1.5 Hz, 1H), 7.84 (dd, J = 8.0, 1.9 Hz, 1H), 8.23 (d, J = 8.0 Hz, 1H), 8.53 (d, J = 1.9 Hz, 1H), 9.83 (s, 1H) |
| N-(2-Aminophenyl)-5-[3-propargyl-1-(2-dimethylaminoethyl)ureidomethyl]pyridine-2-carboxylic acid amide (Compound No.1-156) 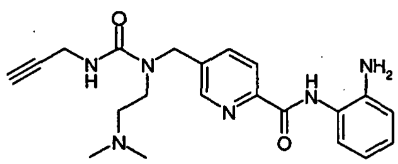 | $^{1}$H-NMR (500 MHz, $CDCl_3$) δ 2.20 (t, J = 2.5 Hz, 1H), 2.29 (s, 6H), 2.43 (t, J = 4.4 Hz, 2H), 3.22 (t, J = 4.4 Hz, 2H), 3.95 (s, 2H), 3.99 (dd, J = 4.7, 2.5 Hz, 2H), 4.61 (s, 2H), 6.84 (dd, J = 7.8, 1.6 Hz, 1H), 6.85 (td, J = 7.8, 1.6 Hz, 1H), 7.08 (td, J = 7.8, 1.6 Hz, 1H), 7.47 (dd, J = 7.8, 1.6 Hz, 1H), 7.84 (dd, J = 7.9, 2.1 Hz, 1H), 8.23 (dd, J = 7.9, 0.6 Hz, 1H), 8.52 (dd, J = 2.1, 0.6 Hz, 1H), 8.78 (s, 1H), 9.82 (s, 1H) |
| N-(2-Aminophenyl)-5-[1-(2-dimethylaminoethyl)-3-(2-methoxyethyl)ureidomethyl]pyridine-2-carboxylic acid amide (Compound No.1-157) | $^{1}$H-NMR (400MHz, $CDCl_3$) δ 2.26 (s, 6H), 2.41 (t, J = 4.8 Hz, 2H), 3.22 (t, J = 4.8 Hz, 2H), 3.36 (s, 3H), 3.42 (m, 2H), 3.49 (t, J = 4.8 Hz, 2H), 3.96 (s, 2H), 4.61 (s, 2H), 6.84 (dd, J = 7.7, 1.5 Hz, 1H), |

| | |
|---|---|
| 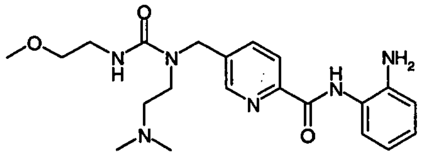 | 6.86 (td, J = 7.7, 1.5 Hz, 1H), 7.09 (td, J = 7.7, 1.5 Hz, 1H), 7.48 (dd, J = 7.7, 1.5 Hz, 1H), 7.85 (dd, J = 8.0, 2.2 Hz, 1H), 8.05 (s, 1H), 8.23 (dd, J = 8.0, 0.5 Hz, 1H), 8.53 (dd, J = 2.2, 0.5 Hz, 1H), 9.83 (s, 1H) |
| N-(2-Aminophenyl)-5-[1-(2-dimethylaminoethyl)-3-(2-methylthioethyl)ureidomethyl]pyridine-2-carboxylic acid amide (Compound No.1-158) 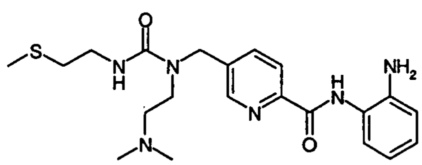 | $^1$H-NMR (400MHz, $CDCl_3$) δ 2.13 (s, 3H), 2.28 (s, 6H), 2.42 (t, J = 4.8 Hz, 2H), 2.66 (t, J = 6.5 Hz, 2H), 3.23 (t, J = 4.6 Hz, 2H), 3.44 (m, 2H), 3.96 (s, 2H), 4.61 (s, 2H), 6.84 (dd, J = 7.7, 1.5 Hz, 1H), 6.86 (td, J = 7.7, 1.5 Hz, 1H), 7.08 (td, J = 7.7, 1.5 Hz, 1H), 7.48 (dd, J = 7.7, 1.5 Hz, 1H), 7.85 (dd, J = 8.0, 2.2 Hz, 1H), 8.08 (m, 1H), 8.23 (dd, J = 8.0, 0.7 Hz, 1H), 8.53 (dd, J = 2.2, 0.7 Hz, 1H), 9.82 (s, 1H) |
| N-(2-Aminophenyl)-5-[1-(2-dimethylaminoethyl)-3-methylaminocarbonylmethylureidomethyl]pyridine-2-carboxylic acid amide | $^1$H-NMR (400 MHz, $CDCl_3$) δ 2.30 (s, 6H), 2.47 (br s, 2H), 2.84 (d, J = 4.9 Hz, 3H), 3.28 (br s, 2H), 3.86 (d, J = 5.6 |

| | |
|---|---|
| (Compound No.1-159)<br>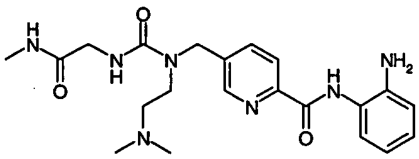 | Hz, 2H), 3.96 (s, 2H), 4.63 (s, 2H), 6.52 (s, 1H), 6.85 (dd, J = 7.7, 1.5 Hz, 1H), 6.86 (td, J = 7.7, 1.5 Hz, 1H), 7.09 (td, J = 7.7, 1.5 Hz, 1H), 7.50 (dd, J = 7.7, 1.5 Hz, 1H), 7.83 (dd, J = 8.0, 2.0 Hz, 1H), 8.24 (d, J = 8.0 Hz, 1H), 8.53 (d, J = 2.0 Hz, 1H), 8.63 (s, 1H), 9.82 (s, 1H) |
| N-(2-Aminophenyl)-5-[3-cyclopropyl-1-(2-dimethylaminoethyl)ureidomethyl]pyridine-2-carboxylic acid amide (Compound No.1-160)<br>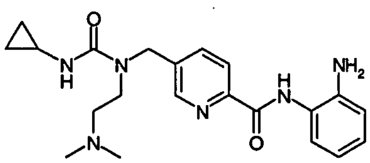 | $^1$H-NMR (400 MHz, $CDCl_3$)<br>δ 0.39 (m, 2H), 0.71 (m, 2H), 2.24 (s, 6H), 2.38 (t, J = 4.5 Hz, 2H), 2.70 (m, 1H), 3.15 (t, J = 4.5 Hz, 2H), 3.96 (s, 2H), 4.60 (s, 2H), 6.84 (dd, J = 7.7, 1.5 Hz, 1H), 6.86 (td, J = 7.7, 1.5 Hz, 1H), 7.09 (td, J = 7.7, 1.5 Hz, 1H), 7.49 (dd, J = 7.7, 1.5 Hz, 1H), 7.86 (dd, J = 8.0, 2.2 Hz, 1H), 8.23 (dd, J = 8.0, 0.7 Hz, 1H), 8.24 (s, 1H), 8.53 (dd, J = 2.2, 0.7 Hz, 1H), 9.83 (s, 1H) |
| N-(2-Aminophenyl)-5-[1-(2-dimethylaminoeth | $^1$H-NMR (400 MHz, $CDCl_3$) |

| | |
|---|---|
| yl)-3-[2-(pyridin-4-yl)ethyl]ureidomethyl]pyridine-2-carboxylic acid amide (Compound No.1-161) 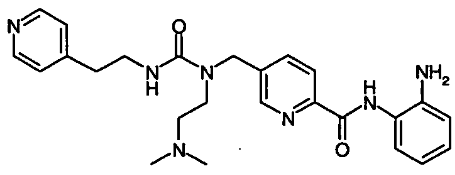 | δ 2.05 (s, 6H), 2.32 (t, J = 4.5 Hz, 2H), 2.86 (t, J = 6.8 Hz, 2H), 3.13 (t, J = 4.5 Hz, 2H), 3.54 (m, 2H), 3.99 (s, 2H), 4.58 (s, 2H), 6.85 (dd, J = 7.7, 1.4 Hz, 1H), 6.87 (td, J = 7.7, 1.4 Hz, 1H), 7.08 (td, J = 7.7, 1.4 Hz, 1H), 7.17 (d, J = 6.1 Hz, 2H), 7.50 (dd, J = 7.7, 1.4 Hz, 1H), 7.82 (dd, J = 7.9, 2.1 Hz, 1H), 7.97 (s, 1H), 8.23 (dd, J = 7.9, 0.6 Hz, 1H), 8.49 (dd, J = 2.1, 0.6 Hz, 1H), 8.54 (d, J = 6.1 Hz, 2H), 9.83 (s, 1H) |
| N-(2-Aminophenyl)-5-[1-(2-dimethylaminoethyl)-3-[2-(indol-3-yl)ethyl]ureidomethyl]pyridine-2-carboxylic acid amide (Compound No.1-162) 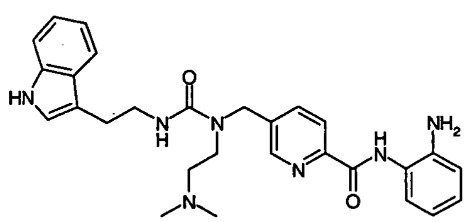 | $^1$H-NMR (400 MHz, $CDCl_3$) δ 1.97 (s, 6H), 2.27 (t, J = 4.9 Hz, 2H), 3.00 (t, J = 6.7 Hz, 2H), 3.13 (t, J = 4.9 Hz, 2H), 3.61 (m, 2H), 3.97 (s, 2H), 4.59 (s, 2H), 6.85 (dd, J = 7.7, 1.5 Hz, 1H), 6.87 (td, J = 7.7, 1.5 Hz, 1H), 7.03 (d, J = 2.2 Hz, 1H), 7.09 (td, J = 7.7, 1.5 Hz, 1H), 7.11 (t, J = 7.5 Hz, 1H), 7.19 (t, J = 7.5 Hz, 1H), 7.35 (d, J = |

| | |
|---|---|
| | 7.5 Hz, 1H), 7.49 (dd, J = 7.7, 1.5 Hz, 1H), 7.54 (s, 1H), 7.63 (d, J = 7.5 Hz, 1H), 7.79 (dd, J = 8.0, 2.2 Hz, 1H), 8.03 (s, 1H), 8.21 (dd, J = 8.0, 0.6 Hz, 1H), 8.50 (dd, J = 2.2, 0.6 Hz, 1H), 9.84 (s, 1H) |
| N-(2-Aminophenyl)-5-[3-isopropyl-1-[2-(4-methylpiperazin-1-yl)ethyl]ureidomethyl]pyridine-2-carboxylic acid amide (Compound No.1-163) 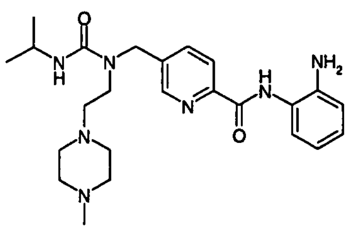 | $^1$H-NMR (400 MHz, $CDCl_3$) δ 1.22 (d, J = 6.6 Hz, 6H), 2.30 (s, 3H), 2.45 (t, J = 4.5 Hz, 2H), 2.47 (br s, 4H), 2.55 (br s, 4H), 3.21 (t, J = 4.5 Hz, 2H), 3.96 (br s, 2H), 3.98 (m, 1H), 4.59 (s, 2H), 6.84-6.88 (m, 2H), 7.04 (d, J = 7.7 Hz, 1H), 7.09 (t, J = 7.7 Hz, 1H), 7.48 (d, J = 7.7 Hz, 1H), 7.84 (dd, J = 8.0, 2.0 Hz, 1H), 8.23 (d, J = 8.0 Hz, 1H), 8.52 (d, J = 2.0 Hz, 1H), 9.82 (s, 1H) |
| N-(2-Aminophenyl)-5-[1-[2-(4-methylpiperazin-1-yl)ethyl]-3-phenethylureidomethyl]pyridine-2-carboxylic acid amide (Compound No.1-164) | $^1$H-NMR (400 MHz, $CDCl_3$) δ 2.21 (br s, 4H), 2.24 (s, 3H), 2.36 (br s, 4H), 2.39 (t, J = 4.5 Hz, 2H), 2.88 (t, J = 6.8 Hz, 2H), 3.16 (t, J = 4.5 Hz, 2H), 3.49 (m, |

| | |
|---|---|
| 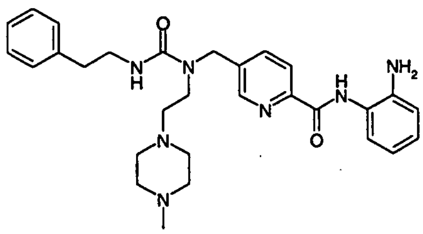 | 2H), 3.97 (br s, 2H), 4.60 (s, 2H), 6.84-6.89 (m, 2H), 7.09 (td, J = 7.6, 1.5 Hz, 1H), 7.20-7.32 (m, 5H), 7.49 (d, J = 7.6 Hz, 1H), 7.74 (br s, 1H), 7.82 (dd, J = 8.0, 2.1 Hz, 1H), 8.24 (d, J = 8.0 Hz, 1H), 8.52 (d, J = 2.1 Hz, 1H), 9.83 (s, 1H) |
| N-(2-Aminophenyl)-5-[3-(4-methylphenyl)-1-[2-(4-methylpiperazin-1-yl)ethyl]ureidomethyl]pyridine-2-carboxylic acid amide (Compound No.1-165) 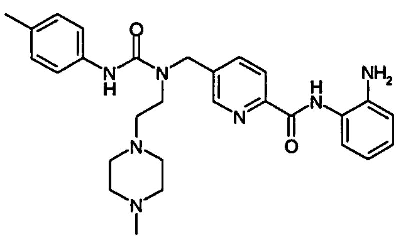 | $^1$H-NMR (400 MHz, $CDCl_3$) δ 2.31 (s, 3H), 2.32 (s, 3H), 2.53 (br s, 4H), 2.57 (t, J = 4.3 Hz, 2H), 2.65 (br s, 4H), 3.37 (t, J = 4.3 Hz, 2H), 3.95 (br s, 2H), 4.66 (s, 2H), 6.84-6.88 (m, 2H), 7.09 (td, J = 7.8, 1.5 Hz, 1H), 7.13 (d, J = 8.3 Hz, 2H), 7.35 (d, J = 8.3 Hz, 2H), 7.49 (d, J = 7.8 Hz, 1H), 7.92 (dd, J = 8.0, 2.2 Hz, 1H), 8.25 (d, J = 8.0 Hz, 1H), 8.59 (d, J = 2.2 Hz, 1H), 9.83 (s, 1H), 9.88 (s, 1H) |
| N-(2-Aminophenyl)-5-[3-isopropyl-1-[3-(4-methylpiperazin-1-yl)propyl]ureidomethyl]pyridin | $^1$H-NMR (400 MHz, $CDCl_3$) δ 1.21 (d, J = 6.6 Hz, 6H), 1.67 (m, 2H), |

| | |
|---|---|
| e-2-carboxylic acid amide (Compound No.1-166)<br>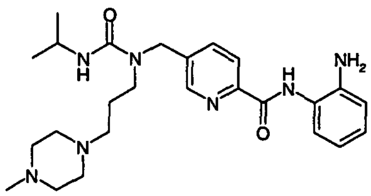 | 2.31 (s, 3H), 2.36 (t, J = 6.1 Hz, 2H), 2.48 (br s, 8H), 3.19 (t, J = 5.9 Hz, 2H), 3.96 (br s, 2H), 4.01 (m, 1H), 4.56 (s, 2H), 6.04 (d, J = 8.0 Hz, 1H), 6.83-6.87 (m, 2H), 7.08 (td, J = 7.8, 1.5 Hz, 1H), 7.48 (d, J = 7.8 Hz, 1H), 7.84 (dd, J = 8.0, 2.2 Hz, 1H), 8.22 (d, J = 8.0 Hz, 1H), 8.53 (d, J = 2.2 Hz, 1H), 9.82 (s, 1H) |
| N-(2-Aminophenyl)-5-[1-[3-(4-methylpiperazin-1-yl)propyl]-3-phenethylureidomethyl]pyridine-2-carboxylic acid amide (Compound No.1-167)<br>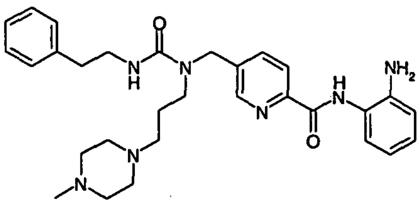 | $^1$H-NMR (400 MHz, $CDCl_3$)<br>δ 1.60 (m, 2H), 2.26 (s, 3H), 2.31 (t, J = 6.0 Hz, 2H), 2.33 (br s, 8H), 2.87 (t, J = 7.0 Hz, 2H), 3.14 (t, J = 5.7 Hz, 2H), 3.49 (m, 2H), 3.96 (br s, 2H), 4.58 (s, 2H), 6.84-6.88 (m, 2H), 7.08 (td, J = 7.6, 1.4 Hz, 1H), 7.20-7.35 (m, 6H), 7.49 (d, J = 7.6 Hz, 1H), 7.79 (dd, J = 8.0, 2.2 Hz, 1H), 8.23 (d, J = 8.0 Hz, 1H), 8.52 (d, J = 2.2 Hz, 1H), 9.84 (s, 1H) |
| N-(2-Aminophenyl)-5-[3-(4-methylphenyl)-1-[ | $^1$H-NMR (400 MHz, $CDCl_3$) |

| | |
|---|---|
| 3-(4-methylpiperazin-1-yl)propyl]ureidomethyl]pyridine-2-carboxylic acid amide (Compound No.1-168) 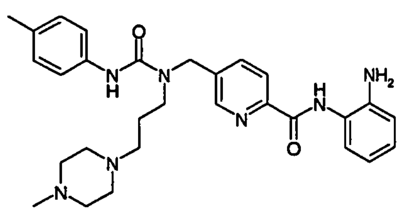 | δ 1.77 (m, 2H), 2.25 (s, 3H), 2.32 (s, 3H), 2.42 (br s, 4H), 2.48 (t, J = 5.9 Hz, 2H), 2.49 (br s, 4H), 3.37 (t, J = 5.6 Hz, 2H), 3.95 (br s, 2H), 4.64 (s, 2H), 6.83-6.88 (m, 2H), 7.08 (td, J = 7.8, 1.5 Hz, 1H), 7.12 (d, J = 8.3 Hz, 2H), 7.33 (d, J = 8.3 Hz, 2H), 7.48 (d, J = 7.8 Hz, 1H), 7.92 (dd, J = 8.0, 2.2 Hz, 1H), 8.23 (d, J = 8.0 Hz, 1H), 8.59 (d, J = 2.2 Hz, 1H), 8.99 (s, 1H), 9.83 (s, 1H) |
| N-(2-Aminophenyl)-5-[1-[2-(4-methylpiperazin-1-yl)ethyl]-3-(1,3-thiazol-2-yl)ureidomethyl] pyridine-2-carboxylic acid amide (Compound No.1-169) 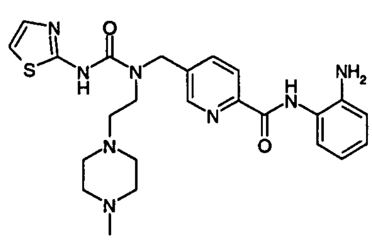 | $^{1}$H-NMR (500 MHz, $CDCl_3$) δ 2.37 (s, 3H), 2.58 (t, J = 4.3 Hz, 2H), 2.69 (br s, 4H), 2.75 (br s, 4H), 3.37 (t, J = 4.3 Hz, 2H), 3.96 (br s, 2H), 4.70 (s, 2H), 6.83-6.86 (m, 2H), 6.87 (d, J = 3.7 Hz, 1H), 7.08 (td, J = 7.9, 1.2 Hz, 1H), 7.38 (d, J = 3.7 Hz, 1H), 7.48 (d, J = 7.9 Hz, 1H), 7.88 (dd, J = 8.1, 2.0 Hz, 1H), 8.25 (d, J = 8.1 Hz, 1H), 8.57 (d, J = 2.0 Hz, 1H), 9.81 (s, 1H), |

| | |
|---|---|
| | 13.17 (br s, 1H) |
| N-(2-Aminophenyl)-5-[1-[3-(4-methylpiperazin-1-yl)propyl]-3-(1,3-thiazol-2-yl)ureidomethyl]pyridine-2-carboxylic acid amide (Compound No.1-170) 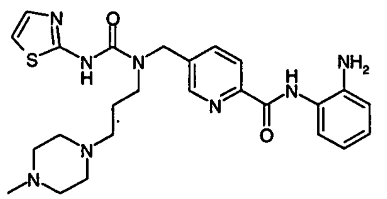 | $^1$H-NMR (500 MHz, $CDCl_3$) δ 1.79 (m, 2H), 2.39 (s, 3H), 2.45 (t, J = 6.1 Hz, 2H), 2.56 (br s, 4H), 2.76 (br s, 4H), 3.40 (t, J = 5.7 Hz, 2H), 3.95 (br s, 2H), 4.67 (s, 2H), 6.84-6.86 (m, 2H), 6.87 (d, J = 3.7 Hz, 1H), 7.09 (m, 1H), 7.37 (d, J = 3.7 Hz, 1H), 7.48 (d, J = 7.9 Hz, 1H), 7.90 (dd, J = 8.1, 2.0 Hz, 1H), 8.25 (d, J = 8.1 Hz, 1H), 8.59 (d, J = 1.8 Hz, 1H), 9.82 (s, 1H), 11.65 (br s, 1H) |
| N-(2-Aminophenyl)-5-[1-[4-(morpholin-4-yl)butyl]-3-(1,3-thiazol-2-yl)ureidomethyl]pyridine-2-carboxylic acid amide (Compound No.1-171) 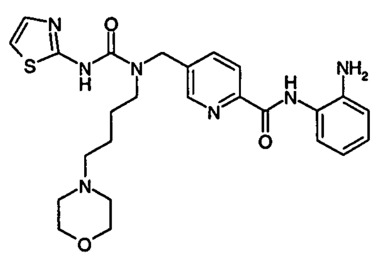 | $^1$H-NMR (500 MHz, $CDCl_3$) δ 1.49 (m, 2H), 1.67 (m, 2H), 2.37 (t, J = 6.9 Hz, 2H), 2.45 (br s, 4H), 3.35 (t, J = 8.1 Hz, 2H), 3.75 (t, J = 4.6 Hz, 4H), 3.97 (br s, 2H), 4.71 (s, 2H), 6.83-6.88 (m, 3H), 7.08 (td, J = 7.7, 1.5 Hz, 1H), 7.32 (d, J = 3.7 Hz, 1H), 7.50 (dd, J = 7.7, 1.5 Hz, 1H), 7.83 (dd, |

| | |
|---|---|
| | J = 8.1, 2.0 Hz, 1H), 8.25 (d, J = 8.1 Hz, 1H), 8.52 (d, J = 2.0 Hz, 1H), 9.03 (br s, 1H), 9.80 (s, 1H) |
| N-(2-Aminophenyl)-5-[1-[3-(4-methylpiperidin-1-yl)propyl]-3-phenethylureidomethyl]pyridine-2-carboxylic acid amide (Compound No.1-172) 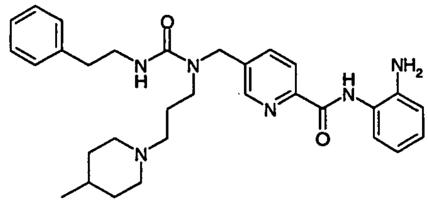 | $^1$H-NMR (500 MHz, $CDCl_3$) δ 0.92 (d, J = 6.4 Hz, 3H), 1.00 (m, 2H), 1.36 (m, 1H), 1.57-1.63 (m, 4H), 1.83 (t, J = 11.6 Hz, 2H), 2.27 (t, J = 6.0 Hz, 2H), 2.70 (d, J = 11.6 Hz, 2H), 2.85 (t, J = 7.0 Hz, 2H), 3.14 (t, J = 5.7 Hz, 2H), 3.47 (m, 2H), 3.97 (br s, 2H), 4.57 (s, 2H), 6.84-6.87 (m, 2H), 7.08 (td, J = 7.7, 1.2 Hz, 1H), 7.20-7.23 (m, 3H), 7.26-7.31 (m, 2H), 7.49 (d, J = 7.7 Hz, 1H), 7.67 (br s, 1H), 7.78 (dd, J = 8.2, 1.8 Hz, 1H), 8.22 (d, J = 8.2 Hz, 1H), 8.51 (d, J = 1.8 Hz, 1H), 9.84 (s, 1H) |
| N-(2-Aminophenyl)-5-[1-[5-(morpholin-4-yl)pentyl]-3-phenethylureidomethyl]pyridine-2-carboxylic acid amide (Compound No.1-173) | $^1$H-NMR (500 MHz, $CDCl_3$) δ 1.20 (m, 2H), 1.39-1.47 (m, 4H), 2.26 (t, J = 7.5 Hz, 2H), 2.40 (br s, 4H), 2.84 (t, J = |

| | |
|---|---|
| 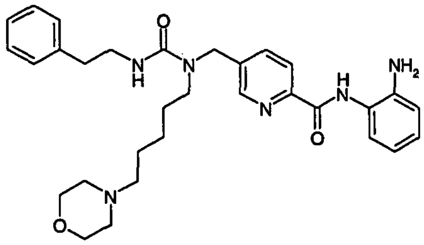 | 6.7 Hz, 2H), 3.07 (t, J = 7.6 Hz, 2H), 3.54 (m, 2H), 3.70 (t, J = 4.6 Hz, 4H), 3.97 (br s, 2H), 4.38 (t, J = 5.5 Hz, 1H), 4.55 (s, 2H), 6.84-6.88 (m, 2H), 7.09 (t, J = 7.7 Hz, 1H), 7.17 (d, J = 7.3 Hz, 2H), 7.22 (t, J = 7.3 Hz, 1H), 7.29 (t, J = 7.3 Hz, 2H), 7.49 (d, J = 7.7 Hz, 1H), 7.72 (dd, J = 7.9, 1.8 Hz, 1H), 8.21 (d, J = 7.9 Hz, 1H), 8.46 (d, J = 1.8 Hz, 1H), 9.82 (s, 1H) |
| N-(2-Aminophenyl)-5-[1-[3-(4-methylpiperidin-1-yl)propyl]-3-(1,3-thiazol-2-yl)ureidomethyl] pyridine-2-carboxylic acid amide (Compound No.1-174) 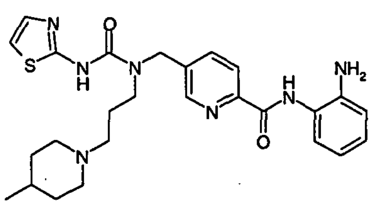 | $^1H$-NMR (400 MHz, $CDCl_3$) δ 1.00 (d, J = 6.6 Hz, 3H), 1.44 (m, 1H), 1.57 (m, 2H), 1.75-1.85 (m, 4H), 2.00 (t, J = 11.7 Hz, 2H), 2.39 (t, J = 6.1 Hz, 2H), 2.90 (d, J = 11.7 Hz, 2H), 3.39 (t, J = 5.6 Hz, 2H), 3.95 (br s, 2H), 4.66 (s, 2H), 6.83-6.88 (m, 3H), 7.08 (td, J = 7.7, 1.5 Hz, 1H), 7.36 (d, J = 3.7 Hz, 1H), 7.48 (d, J = 7.7 Hz, 1H), 7.90 (dd, J = 8.0, 2.2 Hz, 1H), 8.25 (d, J = 8.0 Hz, |

| | |
|---|---|
| | 1H), 8.59 (d, J = 2.2 Hz, 1H), 9.82 (s, 1H) |
| N-(2-Aminophenyl)-6-[1-(2-dimethylaminoethyl)-3-(3-methoxyphenyl)ureidomethyl]pyridine-3-carboxylic acid amide (Compound No.1-175) 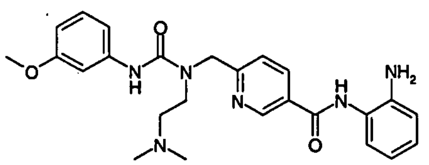 | $^1$H-NMR (500 MHz, $CDCl_3$) δ 2.40 (s, 6H), 2.59 (t, J = 4.2 Hz, 2H), 3.46 (t, J = 4.2 Hz, 2H), 3.79 (s, 3H), 3.85 (s, 2H), 4.72 (s, 2H), 6.54 (dd, J = 8.1, 1.9 Hz, 1H), 6.83 (dd, J = 8.1, 1.9 Hz, 1H), 6.86 (t, J = 7.6, 1H), 6.87 (m, 1H), 7.10 (dd, J = 7.6, 1.2 Hz, 1H), 7.13 (t, J = 1.9 Hz, 1H), 7.16 (t, J = 8.1 Hz, 1H), 7.37 (d, J = 7.6 Hz, 1H), 7.55 (d, J = 7.9 Hz, 1H), 8.02 (s, 1H), 8.17 (d, J = 7.9 Hz, 1H), 9.04 (s, 1H), 11.08 (s, 1H) |
| N-(2-Aminophenyl)-5-[1-(2-dimethylaminoethyl)-3-(5,5-dimethyl-4,5-dihydro-1,3-thiazol-4-on-2-yl)ureidomethyl]pyridine-2-carboxylic acid amide (Compound No.1-176) 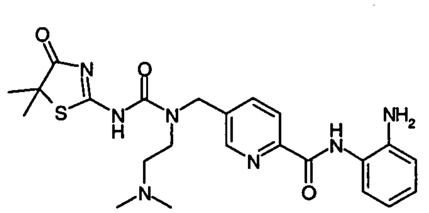 | $^1$H-NMR (500 MHz, $CDCl_3$) δ 2.16 (s, 6H), 2.32 (s, 6H), 2.50 (m, 2H), 3.47 (m, 2H), 3.92 (br s, 2H), 4.70 (s, 2H), 6.84 (dd, J = 7.7, 1.4 Hz, 1H), 6.85 (td, J = 7.7, 1.4 Hz, 1H), 7.08 (td, J = 7.7, 1.4 Hz, 1H), 7.47 (dd, J = 7.7, 1.4 Hz, 1H), 7.75 (s, 1H), 7.85 (dd, J = 7.9, 1.7 Hz, 1H), 8.24 (d, |

| | |
|---|---|
| | J = 8.1 Hz, 1H), 8.54 (d, J = 1.7 Hz, 1H), 9.80 (s, 1H) |
| N-(2-Aminophenyl)-5-[3-(4,5-dihydro-1,3-thiazol-2-yl)-1-(2-dimethylaminoethyl)ureidomethyl]pyridine-2-carboxylic acid amide (Compound No.1-177) 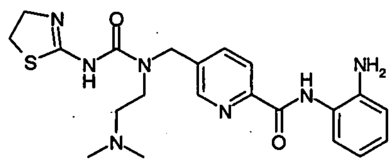 | $^1$H-NMR (400 MHz, $CDCl_3$) δ 2.24 (s, 6H), 2.46 (m, 2H), 3.24 (t, J = 7.4 Hz, 2H), 3.44 (m, 1H), 3.58 (m, 1H), 3.76 (m, 2H), 3.96 (s, 2H), 4.70 (m, 1H), 4.89 (m, 1H), 6.84 (m, 1H), 6.86 (td, J = 7.6, 1.4 Hz, 1H), 7.09 (td, J = 7.6, 1.4 Hz, 1H), 7.50 (dd, J = 7.6, 1.4 Hz, 1H), 7.83 (m, 1H), 8.22 (dd, J = 7.9, 0.6 Hz, 1H), 8.53 (m, 1H), 9.84 (s, 1H) |
| N-(2-Aminophenyl)-5-[3-(bicyclo[2.2.1]heptan-2-yl)-1-(2-dimethylaminoethyl)ureidomethyl]pyridine-2-carboxylic acid amide (Compound No.1-178) 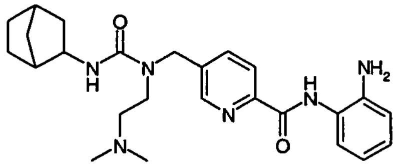 | $^1$H-NMR (500 MHz, $CDCl_3$) δ 0.69 (m, 1H), 1.16 (m, 1H), 1.32 (m, 1H), 1.40-1.47 (m, 2H), 1.58-1.61 (m, 2H), 2.07 (m, 1H), 2.20 (t, J = 4.1 Hz, 1H), 2.28 (s, 6H), 2.39 (m, 1H), 2.45 (t, J = 4.4 Hz, 2H), 3.21 (m, 2H), 3.96 (s, 2H), 4.02 (m, 1H), 4.53 (d, J = 15.6 Hz, 1H), 4.65 (d, J = 15.6 |

| | |
|---|---|
| | Hz, 1H), 6.84 (dd, J = 7.7, 1.4 Hz, 1H), 6.86 (td, J = 7.7, 1.4 Hz, 1H), 7.08 (td, J = 7.7, 1.4 Hz, 1H), 7.48 (dd, J = 7.7, 1.4 Hz, 1H), 7.85 (dd, J = 7.9, 1.8 Hz, 1H), 8.04 (d, J = 6.1 Hz, 1H), 8.23 (d, J = 7.9 Hz, 1H), 8.53 (d, J = 1.8 Hz, 1H), 9.83 (s, 1H) |
| N-(2-Aminophenyl)-5-[1-(2-dimethylaminoethyl)-3-(5-nitro-1,3-thiazol-2-yl)ureidomethyl]pyridine-2-carboxylic acid amide (Compound No.1-179) 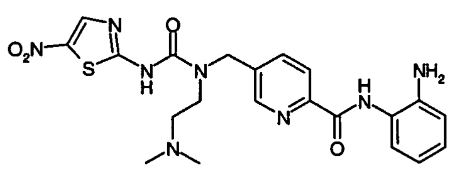 | $^1$H-NMR (400 MHz, DMSO-$d_6$) δ 2.79 (s, 6H), 3.17 (s, 2H), 3.70 (s, 2H), 4.69 (s, 2H), 4.88 (s, 2H), 6.65 (td, J = 7.8, 1.3 Hz, 1H), 6.82 (dd, J = 7.8, 1.3 Hz, 1H), 6.95 (td, J = 7.8, 1.3 Hz, 1H), 7.51 (dd, J = 7.8, 1.3 Hz, 1H), 7.70 (s, 1H), 7.94 (dd, J = 8.0, 1.9 Hz, 1H), 8.11 (dd, J = 8.0, 0.6 Hz, 1H), 8.40 (br s, 1H), 8.66 (dd, J = 1.9, 0.6 Hz, 1H), 10.03 (s, 1H) |
| N-(2-Aminophenyl)-5-[1-[5-(morpholin-4-yl)pentyl]-3-(pyrrolidin-1-yl)ureidomethyl]pyridine-2-carboxylic acid amide (Compound | $^1$H-NMR (500 MHz, $CDCl_3$) δ 1.30 (m, 2H), 1.50 (m, 2H), 1.60 (m, 2H), 1.78 (m, 4H), 2.31 (t, J = 7.6 Hz, 2H), 2.42 |

| | |
|---|---|
| No.1-180) 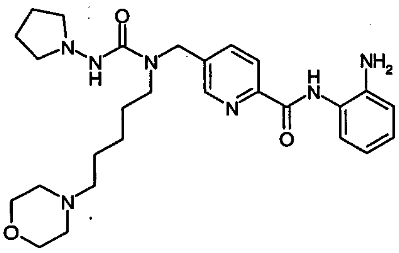 | (m, 4H), 2.83 (m, 4H), 3.21 (t, J = 7.6 Hz, 2H), 3.71 (t, J = 4.6 Hz, 4H), 3.96 (s, 2H), 4.61 (s, 2H), 5.07 (s, 1H), 6.85 (dd, J = 7.7, 1.4 Hz, 1H), 6.87 (td, J = 7.7, 1.4 Hz, 1H), 7.09 (td, J = 7.7, 1.4 Hz, 1H), 7.48 (dd, J = 7.7, 1.4 Hz, 1H), 7.83 (dd, J = 7.9, 2.0 Hz, 1H), 8.24 (d, J = 7.9 Hz, 1H), 8.52 (d, J = 2.0 Hz, 1H), 9.82 (s, 1H) |
| N-(2-Aminophenyl)-5-[3-(4-methoxyphenyl)-1-[5-(morpholin-4-yl)pentyl]ureidomethyl]pyridine-2-carboxylic acid amide (Compound No.1-181) 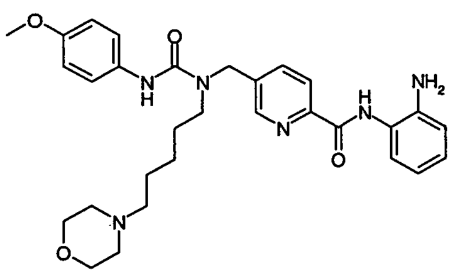 | $^1$H-NMR (500 MHz, $CDCl_3$) δ 1.36 (m, 2H), 1.53 (m, 2H), 1.68 (m, 2H), 2.32 (t, J = 7.6 Hz, 2H), 2.42 (s, 4H), 3.31 (t, J = 7.6 Hz, 2H), 3.68 (t, J = 4.7 Hz, 4H), 3.79 (s, 3H), 3.96 (s, 2H), 4.68 (s, 2H), 6.26 (s, 1H), 6.85 (d, J = 8.9 Hz, 2H), 6.84-6.88 (m, 2H), 7.09 (td, J = 7.8, 1.4 Hz, 1H), 7.26 (d, J = 8.9 Hz, 2H), 7.49 (dd, J = 7.8, 1.4 Hz, 1H), 7.87 (dd, J = 8.1, 2.0 Hz, 1H), 8.26 (d, J = 8.1 Hz, 1H), 8.58 (d, J = 2.0 Hz, 1H), |

| | |
|---|---|
| | 9.82 (s, 1H) |
| N-(2-Aminophenyl)-5-[3-(4-benzyloxyphenyl)-1-[5-(morpholin-4-yl)pentyl]ureidomethyl]pyridine-2-carboxylic acid amide (Compound No.1-182)<br>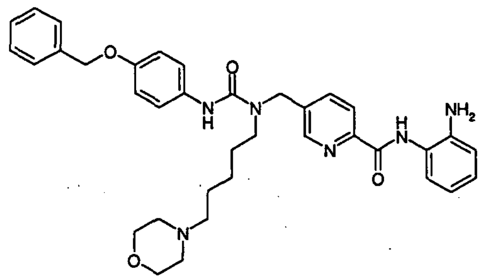 | $^1$H-NMR (500 MHz, $CDCl_3$)<br>δ 1.38 (m, 2H), 1.53 (m, 2H), 1.68 (m, 2H), 2.32 (t, J = 7.6 Hz, 2H), 2.42 (m, 4H), 3.31 (t, J = 7.6 Hz, 2H), 3.70 (t, J = 4.6 Hz, 4H), 3.95 (s, 2H), 4.68 (s, 2H), 5.05 (s, 2H), 6.24 (s, 1H), 6.85 (dd, J = 7.8, 1.4 Hz, 1H), 6.86 (td, J = 7.8, 1.4 Hz, 1H), 6.93 (d, J = 8.9 Hz, 2H), 7.09 (td, J = 7.8, 1.4 Hz, 1H), 7.25 (d, J = 8.9 Hz, 2H), 7.32 (m, 1H), 7.38 (d, J = 7.0 Hz, 2H), 7.42 (d, J = 7.0 Hz, 2H), 7.49 (dd, J = 7.8, 1.4 Hz, 1H), 7.87 (dd, J = 8.1, 2.0 Hz, 1H), 8.26 (d, J = 8.1 Hz, 1H), 8.58 (d, J = 2.0 Hz, 1H), 9.82 (s, 1H) |
| N-(2-Aminophenyl)-5-[3-(2-methoxyphenyl)-1-[5-(morpholin-4-yl)pentyl]ureidomethyl]pyridine-2-carboxylic acid amide (Compound No.1-183) | $^1$H-NMR (400 MHz, $CDCl_3$)<br>δ 1.40 (m, 2H), 1.55 (m, 2H), 1.71 (m, 2H), 2.33 (t, J = 7.6 Hz, 2H), 2.42 (m, 4H), 3.36 (t, J = 7.6 Hz, 2H), 3.71 (t, J = 4.6 Hz, 4H), |

| | |
|---|---|
| 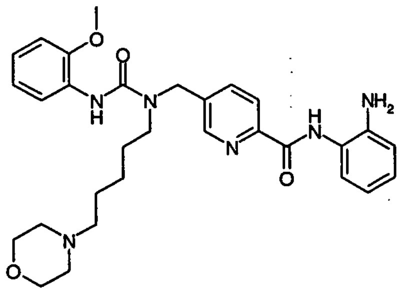 | 3.83 (s, 3H), 3.96 (s, 2H), 4.70 (s, 2H), 6.85 (dd, J = 7.7, 1.4 Hz, 1H), 6.85 (m, 1H), 6.87 (td, J = 7.7, 1.4 Hz, 1H), 6.95-7.00 (m, 2H), 7.09 (td, J = 7.7, 1.4 Hz, 1H), 7.16 (s, 1H), 7.49 (dd, J = 7.7, 1.4 Hz, 1H), 7.89 (dd, J = 8.0, 2.2 Hz, 1H), 8.17 (m, 1H), 8.27 (d, J = 8.0, 0.7 Hz, 1H), 8.60 (dd, J = 2.2, 0.7 Hz, 1H), 9.83 (s, 1H) |
| N-(2-Aminophenyl)-5-[1-[3-(morpholin-4-yl)propyl]-3-(pyrrolidin-1-yl)ureidomethyl]pyridine-2-carboxylic acid amide (Compound No.1-184) 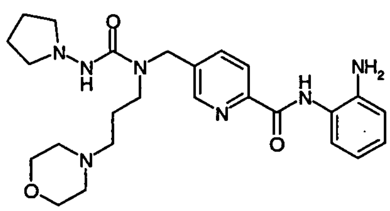 | $^1$H-NMR (500 MHz, $CDCl_3$) δ 1.70 (m, 2H), 1.83-1.88 (m, 4H), 2.38 (t, J = 6.1 Hz, 2H), 2.47 (br s, 4H), 2.98 (br s, 4H), 3.21 (t, J = 6.0 Hz, 2H), 3.78 (t, J = 4.6 Hz, 4H), 3.96 (br s, 2H), 4.56 (s, 2H), 6.83-6.88 (m, 2H), 7.09 (td, J = 7.8, 1.4 Hz, 1H), 7.48 (dd, J = 7.8, 1.4 Hz, 1H), 7.71 (s, 1H), 7.91 (dd, J = 7.9, 2.0 Hz, 1H), 8.23 (d, J = 7.9 Hz, 1H), 8.55 (d, J = 2.0 Hz, 1H), 9.83 (s, 1H) |

| | |
|---|---|
| N-(2-Aminophenyl)-5-[3-(morpholin-4-yl)-1-[3-(morpholin-4-yl)propyl]ureidomethyl]pyridine-2-carboxylic acid amide (Compound No.1-185) 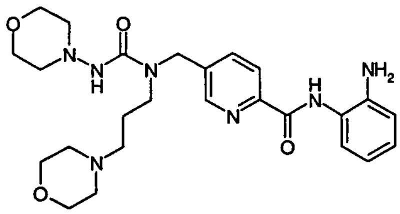 | $^1$H-NMR (500 MHz, $CDCl_3$) δ 1.72 (m, 2H), 2.39 (t, J = 6.0 Hz, 2H), 2.49 (br s, 4H), 2.97 (t, J = 4.6 Hz, 4H), 3.21 (t, J = 6.0 Hz, 2H), 3.77-3.84 (m, 8H), 3.96 (br s, 2H), 4.55 (s, 2H), 6.83-6.88 (m, 2H), 7.09 (td, J = 7.8, 1.5 Hz, 1H), 7.48 (dd, J = 7.8, 1.5 Hz, 1H), 7.90 (dd, J = 7.9, 2.0 Hz, 1H), 7.94 (br s, 1H), 8.23 (d, J = 7.9 Hz, 1H), 8.55 (d, J = 2.0 Hz, 1H), 9.82 (s, 1H) |
| N-(2-Aminophenyl)-5-[3-(1-methylpiperidin-4-yl)-1-[3-(morpholin-4-yl)propyl]ureidomethyl]pyridine-2-carboxylic acid amide (Compound No.1-186) 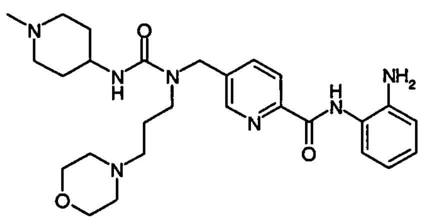 | $^1$H-NMR (500 MHz, $CDCl_3$) δ 1.48 (qd, J = 11.9, 3.7 Hz, 2H), 1.71 (m, 2H), 1.96 (d, J = 11.9 Hz, 2H), 2.04-2.11 (m, 2H), 2.27 (s, 3H), 2.37 (t, J = 6.3 Hz, 2H), 2.44 (br s, 4H), 2.84 (d, J = 11.9 Hz, 2H), 3.22 (t, J = 6.1 Hz, 2H), 3.66 (m, 1H), 3.75 (t, J = 4.7 Hz, 4H), 3.95 (br s, 2H), 4.57 (s, 2H), 5.82 (d, J = 7.6 Hz, 1H), 6.83-6.88 (m, 2H), 7.09 (td, J = 7.7, 1.5 Hz, 1H), 7.48 (dd, |

| | |
|---|---|
| | J = 7.7, 1.5 Hz, 1H), 7.84 (dd, J = 8.1, 2.1 Hz, 1H), 8.23 (dd, J = 8.1, 0.6 Hz, 1H), 8.53 (dd, J = 2.1, 0.6 Hz, 1H), 9.82 (s, 1H) |
| N-(2-Aminophenyl)-6-[1-(2-dimethylaminoethyl)-3-(3-fluorophenyl)ureidomethyl]pyridine-3-carboxylic acid amide (Compound No.1-187) 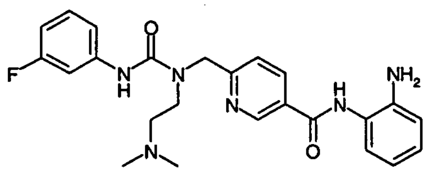 | $^1$H-NMR (400 MHz, $CDCl_3$) δ 2.41 (s, 6H), 2.62 (t, J = 4.2 Hz, 2H), 3.48 (t, J = 4.2 Hz, 2H), 3.84 (s, 2H), 4.72 (s, 2H), 6.66 (td, J = 7.8, 1.5 Hz, 1H), 6.87 (d, J = 7.8 Hz, 1H), 6.89 (m, 1H), 6.99 (m, 1H), 7.12 (td, J = 7.8, 1.5 Hz, 1H), 7.19 (td, J = 8.3, 6.6 Hz, 1H), 7.27 (m, 1H), 7.38 (d, J = 7.8 Hz, 1H), 7.57 (d, J = 8.2 Hz, 1H), 7.92 (s, 1H), 8.18 (d, J = 8.2 Hz, 1H), 9.05 (s, 1H), 11.30 (s, 1H) |
| N-(2-Aminophenyl)-6-[1-(2-dimethylaminoethyl)-3-(thiophen-3-yl)ureidomethyl]pyridine-3-carboxylic acid amide (Compound No.1-188) 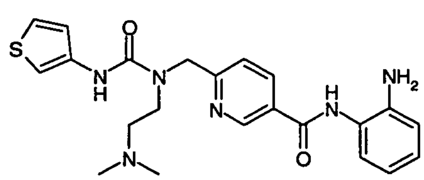 | $^1$H-NMR (400 MHz, $CDCl_3$) δ 2.39 (s, 6H), 2.59 (t, J = 4.3 Hz, 2H), 3.45 (t, J = 4.3 Hz, 2H), 3.84 (s, 2H), 4.74 (s, 2H), 6.85-6.90 (m, 2H), 6.87 (dd, J = 5.1, 1.4 Hz, 1H), 7.12 (td, J = 7.8, 1.4 Hz, 1H), 7.20 (dd, |

| | |
|---|---|
| | J = 5.1, 3.2 Hz, 1H), 7.28 (dd, J = 3.2, 1.4 Hz, 1H), 7.38 (d, J = 7.8 Hz, 1H), 7.56 (d, J = 7.8 Hz, 1H), 7.90 (s, 1H), 8.17 (d, J = 7.8 Hz, 1H), 9.04 (s, 1H), 11.45 (s, 1H) |
| N-(2-Aminophenyl)-6-[3-(2,3-dihydrobenzo[1,4]dioxin-6-yl)-1-(2-dimethylaminoethyl)ureido methyl]pyridine-3-carboxylic acid amide (Compound No.1-189) 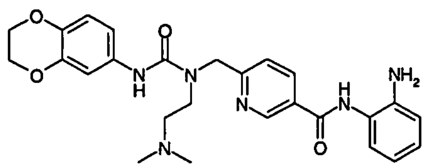 | $^{1}$H-NMR (500 MHz, $CDCl_3$) δ 2.38 (s, 6H), 2.57 (t, J = 4.1 Hz, 2H), 3.44 (t, J = 4.1 Hz, 2H), 3.84 (s, 2H), 4.20-4.25 (m, 4H), 4.71 (s, 2H), 6.76 (d, J = 8.8 Hz, 1H), 6.81 (dd, J = 8.8, 2.4 Hz, 1H), 6.85-6.90 (m, 2H), 6.88 (m, 1H), 6.92 (d, J = 2.4 Hz, 1H), 7.12 (td, J = 7.6, 1.5 Hz, 1H), 7.38 (d, J = 7.6 Hz, 1H), 7.58 (d, J = 7.9 Hz, 1H), 7.92 (s, 1H), 8.17 (d, J = 7.9 Hz, 1H), 9.04 (s, 1H), 10.80 (s, 1H) |
| N-(2-Aminophenyl)-6-[1-(2-dimethylaminoethyl)-3-(3-meth y l phenyl)ureidomethyl]pyridine-3-carboxylic acid amide (Compound No.1-190) | $^{1}$H-NMR (500 MHz, $CDCl_3$) δ 2.32 (s, 3H), 2.40 (s, 6H), 2.60 (t, J = 4.0 Hz, 2H), 3.47 (t, J = 4.0 Hz, 2H), 3.84 (s, 2H), 4.72 (s, 2H), 6.80 (d, J = 8.0 Hz, 1H), |

| | |
|---|---|
| 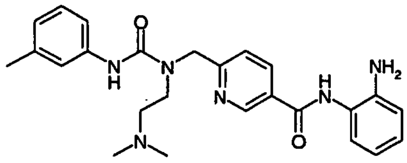 | 6.86 (d, J = 7.8 Hz, 1H), 6.87 (m, 1H), 7.05 (d, J = 8.0 Hz, 1H), 7.11 (t, J = 7.8 Hz, 1H), 7.15 (t, J = 8,0 Hz, 1H), 7.28 (s, 1H), 7.37 (d, J = 7.8 Hz, 1H), 7.57 (d, J = 7.8 Hz, 1H), 7.97 (s, 1H), 8.18 (d, J = 7.8 Hz, 1H), 9.05 (s, 1H), 10.91 (s, 1H) |

Example 2

N-(2-Aminophenyl)-5-[3-(4-dimethylaminophenyl)-1-[3-(morpholin-4-yl)propyl]ureidomethyl] pyridine-2-carboxylic acid amide (Compound No.2-1)

4-Dimethylaminophenylisocyanate (23 mg 0.14 mmol) was added to a solution of N-(2-t-butoxycarbonylaminophenyl)-5-[3-(morpholin-4-yl)propylaminomethyl)]pyridine-2-car boxylic acid amide (Reference Compound No.5-10, 25 mg, 0.053 mmol) in dichloromethane (1.0 mL), and then the mixture was stirred at room temperature for 2 hours. The reaction mixture was purified by silica gel column chromatography (NH-modified silica gel, hexane-ethyl acetate) to give a colorless compound. The obtained compound was dissolved in methanol (0.5 mL), 4.0 M hydrogen chloride-ethyl acetate solution (1.0 mL) was added thereto, and then the reaction mixture was stirred at room temperature for 4 hours. Saturated aqueous sodium hydrogen carbonate solution (30 mL) was added thereto, and then the whole was extracted with ethyl acetate (30 mL). The organic layer was dried over anhydrous magnesium sulfate, and then the solvent was evaporated under reduced pressure to give 27 mg of the title compound as a colorless amorphous product. (Yield 77%)

| | |
|---|---|
| 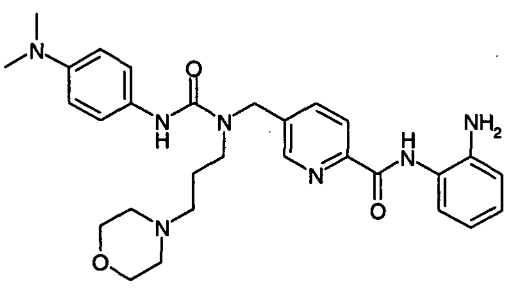 | $^1$H-NMR (500 MHz, DMSO-$d_6$)<br>δ 1.70 (m, 2H), 2.29-2.32 (m, 6H), 2.83 (s, 6H), 3.35 (t, J = 6.7 Hz, 2H), 3.52-3.54 (m, 4H), 4.66 (s, 2H), 4.89 (br s, 2H), 6.65 (m, 1H), 6.67 (d, J = 9.0 Hz, 2H), 6.82 (d, J = 7.6 Hz, 1H), 6.95 (td, J = 7.6, 1.2 Hz, 1H), 7.23 (d, J = 9.0 Hz, 2H), 7.50 (d, J = 7.6 Hz, 1H), 7.93 (dd, J = 8.2, 2.0 Hz, 1H), 8.12 (d, J = 8.2 Hz, 1H), 8.45 (br s, 1H), 8.64 (d, J = 2.0 Hz, 1H), 10.03 (br s, 1H) |

By using any compounds selected from Reference Compounds No.5-1, 5-2, 5-6, 5-7, 5-9, and 5-10, the following Compounds No.2-2~2-15 were obtained by a method similar to that of Compound No.2-1.

| | |
|---|---|
| N-(2-Aminophenyl)-5-[3-(4-cyanomethylphenyl)-1-[3-(morpholin-4-yl)propyl]ureidomethyl]pyridine-2-carboxylic acid amide (Compound No.2-2)<br>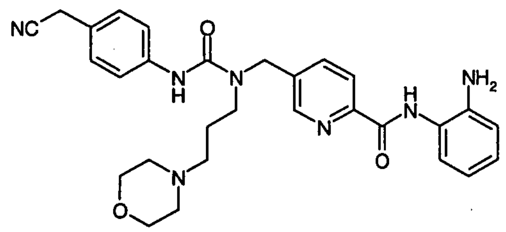 | $^1$H-NMR (400 MHz, DMSO-$d_6$)<br>δ 1.71 (m, 2H), 2.28-2.31 (m, 6H), 3.33 (s, 2H), 3.39 (t, J = 6.6 Hz, 2H), 3.54-3.56 (m, 4H), 4.70 (s, 2H), 4.89 (br s, 2H), 6.65 (t, J = 7.8 Hz, 1H), 6.82 (d, J = 7.8 Hz, 1H), 6.95 (td, J = 7.8, 1.1 Hz, 1H), 7.13 (m, 1H), 7.23 (d, J = 8.5 Hz, 1H), 7.42 (m, 1H), 7.49-7.53 (m, 2H), 7.93 (d, J = 8.2 Hz, 1H), 8.12 (d, J = 8.2 Hz, 1H), 8.62-8.69 (m, 2H), 10.04 (br s, 1H) |
| N-(2-Aminophenyl)-5-[3-(4-diethylaminophenyl)-1-(3-dimethylaminopropyl)ureidomethyl]pyridine-2-carboxylic acid amide (Compound No.2-3)<br>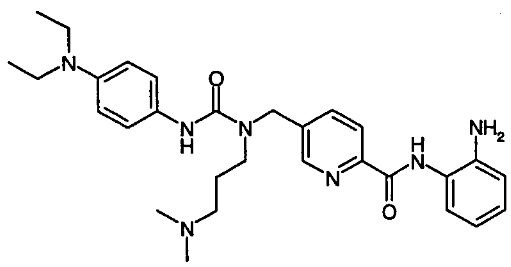 | $^1$H-NMR (500 MHz, DMSO-$d_6$)<br>δ 1.05 (t, J = 7.0 Hz, 6H), 1.69 (m, 2H), 2.18 (s, 6H), 2.26 (t, J = 6.4 Hz, 2H), 3.26 (q, J = 7.0 Hz, 4H), 3.32 (m, 2H), 4.61 (s, 2H), 4.89 (br s, 2H), 6.61 (d, J = 9.0 Hz, 2H), 6.65 (td, J = 7.9, 1.2 Hz, 1H), 6.82 (dd, J = 7.9, 1.2 Hz, 1H), 6.95 (td, J = 7.9, 1.2 Hz, 1H), 7.19 (d, J = 9.0 Hz, 2H), 7.51 (dd, J = 7.9, 1.2 Hz, 1H), 7.94 (dd, J = 8.2, 1.8 Hz, 1H), 8.11 (d, J = 8.2 Hz, 1H), 8.65 (d, J = 1.8 Hz, 1H), 9.15 (br s, 1H), 10.02 (br s, 1H) |
| N-(2-Aminophenyl)-5-[1-(3-diethylaminopropyl)-3-(4-dimethylaminophenyl)ureidomethyl]pyridine-2-carboxylic acid amide (Compound No.2-4)<br>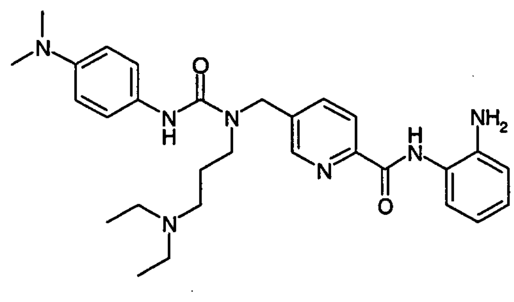 | $^1$H-NMR (400 MHz, DMSO-$d_6$)<br>δ 0.93 (t, J = 7.1 Hz, 6H), 1.68 (m, 2H), 2.39 (br s, 2H), 2.50 (m, 4H), 2.82 (s, 6H), 3.33 (m, 2H), 4.64 (s, 2H), 4.89 (br s, 2H), 6.63-6.69 (m, 3H), 6.82 (dd, J = 7.8, 1.2 Hz, 1H), 6.95 (td, J = 7.8, 1.2 Hz, 1H), 7.22 (d, J = 9.0 Hz, 2H), 7.50 (dd, J = 7.8, 1.2 Hz, 1H), 7.94 (dd, J = 8.1, 1.8 Hz, 1H), 8.12 (d, J = 8.1 Hz, 1H), 8.65 (d, J = 1.8 Hz, 1H), 8.78 (br s, 1H), 10.03 (br s, 1H) |
| N-(2-Aminophenyl)-5-[1-(3-diethylaminopropyl)-3-(4-methoxyphenyl)ureidomethyl]pyridine-2-carboxylic acid amide (Compound No.2-5)<br>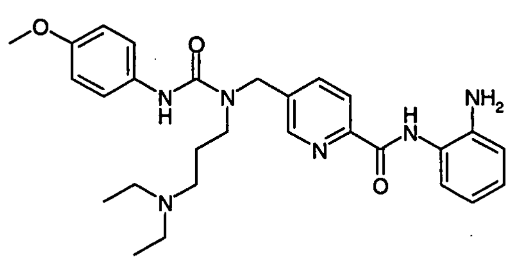 | $^1$H-NMR (400 MHz, DMSO-$d_6$)<br>δ 0.93 (t, J = 7.1 Hz, 6H), 1.69 (m, 2H), 2.39 (t, J = 6.6 Hz, 2H), 2.49 (m, 4H), 3.35 (m, 2H), 3.71 (s, 3H), 4.65 (s, 2H), 4.89 (br s, 2H), 6.65 (td, J = 7.8, 1.3 Hz, 1H), 6.81-6.86 (m, 3H), 6.95 (td, J = 7.8, 1.3 Hz, 1H), 7.32 (d, J = 9.0 Hz, 2H), 7.50 (dd, J = 7.8, 1.3 Hz, 1H), 7.94 (dd, J = 8.1, 1.8 Hz, 1H), 8.12 (d, J = 8.1 Hz, 1H), 8.65 (d, J = 1.8 Hz, 1H), 8.93 (br s, 1H), 10.03 (br s, 1H) |

(continued)

| | |
|---|---|
| 5-[3-(4-Acetylphenyl)-1-[3-(morpholin-4-yl)propyl] ureidomethyl]-N-(2-aminophenyl)pyridine-2-carboxylic acid amide (Compound No.2-6)<br>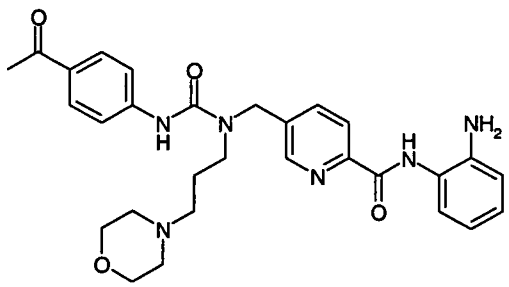 | $^1$H-NMR (500 MHz, DMSO-$d_6$)<br>δ 1.73 (m, 2H), 2.28-2.32 (m, 6H), 2.51 (s, 3H), 3.43 (t, J = 6.7 Hz, 2H), 3.54-3.57 (m, 4H), 4.74 (s, 2H), 4.89 (br s, 2H), 6.65 (td, J = 7.6, 1.2 Hz, 1H), 6.82 (dd, J = 7.6, 1.2 Hz, 1H), 6.95 (td, J = 7.6, 1.2 Hz, 1H), 7.51 (d, J = 7.6 Hz, 1H), 7.66 (d, J = 9.0 Hz, 2H), 7.88 (d, J = 9.0 Hz, 2H), 7.95 (dd, J = 8.1, 1.8 Hz, 1H), 8.13 (d, J = 8.1 Hz, 1H), 8.66 (d, J = 1.8 Hz, 1H), 8.93 (br s, 1H), 10.03 (br s, 1H) |
| N-(2-Aminophenyl)-5-[3-(3,5-dimethylisoxazol-4-yl)-1-[3-(morpholin-4-yl)propyl]ureidomethyl]pyridine-2-carboxylic acid amide (Compound No.2-7)<br>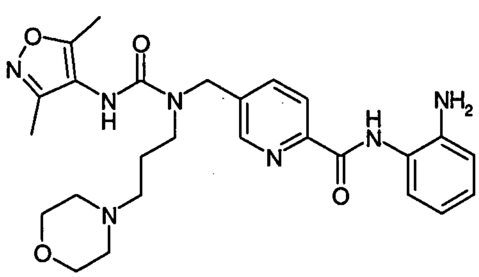 | $^1$H-NMR (500 MHz, DMSO-$d_6$)<br>δ 1.71 (m, 2H), 2.08 (s, 3H), 2.25 (s, 3H), 2.30-2.33 (m, 6H), 3.35 (t, J = 6.7 Hz, 2H), 3.49-3.50 (m, 4H), 4.66 (s, 2H), 4.89 (br s, 2H), 6.65 (td, J = 7.6, 1.2 Hz, 1H), 6.82 (dd, J = 7.6, 1.2 Hz, 1H), 6.96 (td, J = 7.6, 1.2 Hz, 1H), 7.49 (dd, J = 7.6, 1.2 Hz, 1H), 7.92 (dd, J = 8.1, 2.0 Hz, 1H), 8.13 (d, J = 8.1 Hz, 1H), 8.36 (br s, 1H), 8.63 (d, J = 2.0 Hz, 1H), 10.04 (br s, 1H) |
| N-(2-Aminophenyl)-5-[1-(2-carboxyethyl)-3-(2,3-dihydrobenzo[1,4]dioxin-6-yl)ureidomethyl]pyridine-2-carboxylic acid amide (Compound No.2-8)<br>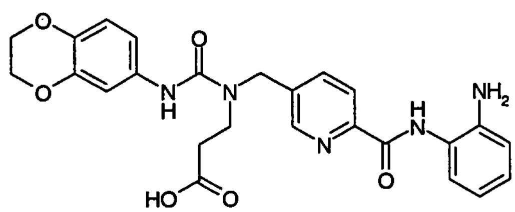 | 1H-NMR (500 MHz, DMSO-$d_6$)<br>δ 2.56 (t, J = 7.2 Hz, 2H), 3.54 (m, 2H), 4.17-4.26 (m, 4H), 4.67-4.72 (m, 4H), 6.65 (m, 1H), 6.73 (d, J = 8.7 Hz, 1H), 6.82 (dd, J = 7.9, 1.2 Hz, 1H), 6.87 (dd, J = 8.7, 2.6 Hz, 1H), 6.95 (td, J = 7.9, 1.2 Hz, 1H), 7.04 (d, J = 2.6 Hz, 1H), 7.51 (dd, J = 7.9, 1.2 Hz, 1H), 7.91 (dd, J = 7.9, 1.8 Hz, 1H), 8.12 (d, J = 7.9 Hz, 1H), 8.46 (br s, 1H), 8.62 (d, J = 1.8 Hz, 1H), 10.03 (br s, 1H) |
| N-(2-Aminophenyl)-5-[1-(2-carboxyethyl)-3-(3,4-difluorophenyl)ureidomethyl]pyridine-2-carboxylic acid amide (Compound No.2-9)<br>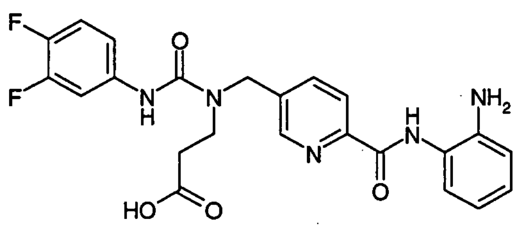 | $^1$H-NMR (500 MHz, DMSO-$d_6$)<br>δ 2.55 (t, J = 7.0 Hz, 2H), 3.56 (t, J = 7.0 Hz, 2H), 4.70 (s, 2H), 4.90 (br s, 2H), 6.65 (td, J = 7.6, 1.4 Hz, 1H), 6.82 (dd, J = 7.6, 1.4 Hz, 1H), 6.95 (td, J = 7.6, 1.4 Hz, 1H), 7.24 (m, 1H), 7.31 (dd, J = 19.7, 9.3 Hz, 1H), 7.51 (dd, J = 7.6, 1.4 Hz, 1H), 7.64 (ddd, J = 13.7, 7.6, 2.4 Hz, 1H), 7.93 (dd, J = 7.9, 2.0 Hz, 1H), 8.12 (d, J = 7.9 Hz, 1H), 8.63 (d, J = 2.0 Hz, 1H), 9.13 (br s, 1H), 10.16 (br s, 1H) |

(continued)

| | |
|---|---|
| N-(2-Aminophenyl)-5-[3-(2,3-dihydrobenzo[1,4]dioxin-6-yl)-1-(2-hydroxyethyl)ureidomethyl]pyridine-2-carboxylic acid amide (Compound No.2-10) 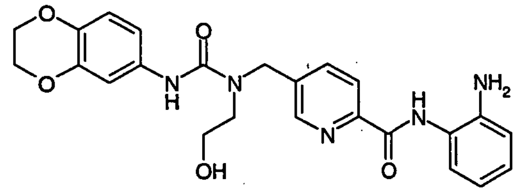 | $^1$H-NMR (400 MHz, DMSO-$d_6$) δ 3.43 (t, J = 5.1 Hz, 2H), 3.58 (t, J = 5.1 Hz, 2H), 4.16-4.21 (m, 4H), 4.69 (s, 2H), 4.90 (br s, 2H), 5.28 (br s, 1H), 6.65 (td, J = 7.7, 1.2 Hz, 1H), 6.72 (d, J = 8.8 Hz, 1H), 6.80 (dd, J = 8.8, 2.4 Hz, 1H), 6.83 (m, 1H), 6.95 (td, J = 7.7, 1.2 Hz, 1H), 7.02 (d, J = 2.4 Hz, 1H), 7.50 (dd, J = 7.7, 1.2 Hz, 1H), 7.93 (dd, J = 8.1, 1.8 Hz, 1H), 8.11 (dd, J = 8.1, 0.5 Hz, 1H), 8.55 (br s, 1H), 8.64 (d, J = 1.8 Hz, 1H), 10.03 (br s, 1H) |
| N-(2-Aminophenyl)-5-[1-(2-hydroxyethyl)-3-(4-methoxyphenyl)ureidomethyl]pyridine-2-carboxylic acid amide (Compound No.2-11) 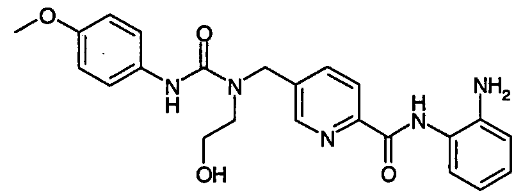 | $^1$H-NMR (400 MHz, DMSO-$d_6$) δ 3.44 (t, J = 5.1 Hz, 2H), 3.59 (m, 2H), 3.70 (s, 3H), 4.71 (s, 2H), 4.90 (br s, 2H), 5.27 (br s, 1H), 6.65 (td, J = 7.6, 1.2 Hz, 1H), 6.83 (m, 1H), 6.84 (d, J = 9.0 Hz, 2H), 6.95 (td, J = 7.6, 1.2 Hz, 1H), 7.30 (d, J = 9.0 Hz, 2H), 7.51 (dd, J = 7.6, 1.2 Hz, 1H), 7.94 (dd, J = 8.1, 1.8 Hz, 1H), 8.11 (d, J = 8.1 Hz ,1H), 8.57 (br s, 1H), 8.65 (d, J = 1.8 Hz, 1H), 10.03 (br s, 1H) |
| N-(2-Aminophenyl)-5-[3-(2,3-dihydrobenzo[1,4]dioxin-6-yl)-1-[2-(2,3-dihydrobenzo[1,4]dioxin-6-ylaminocarbonyloxy)ethyl]ureidomethyl] pyridine-2-carboxylic acid amide (Compound No.2-12) 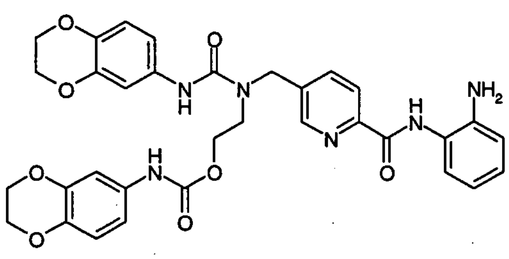 | $^1$H-NMR (400 MHz, DMSO-$d_6$) δ 3.65 (t, J = 5.5 Hz, 2H), 4.16-4.22 (m, 10H), 4.77 (s, 2H), 4.89 (br s, 2H), 6.65 (td, J = 7.6, 1.2 Hz, 1H), 6.71 (d, J = 8.5 Hz, 1H), 6.74 (d, J = 8.5 Hz, 1H), 6.82 (d, J = 7.6 Hz, 1H), 6.86 (m, 1H), 6.87 (dd, J = 8.5, 2.6 Hz, 1H), 6.95 (td, J = 7.6, 1.2 Hz, 1H), 7.01 (m, 1H), 7.04 (d, J = 2.6 Hz, 1H), 7.49 (dd, J = 7.6, 1.2 Hz, 1H), 7.92 (dd, J = 8.2, 1.6 Hz, 1H), 8.11 (d, J = 8.2 Hz, 1H), 8.36 (br s, 1H), 8.62 (d, J = 1.6 Hz, 1H), 9.43 (br s, 1H), 10.01 (br s, 1H) |
| N-(2-Aminophenyl)-5-[3-(benzo[1,3]dioxol-5-yl)-1-[2-(benzo[1,3]dioxol-5-ylaminocarbonyloxy)ethyl] ureidomethyl]pyridine-2-carboxylic acid amide (Compound No.2-13) 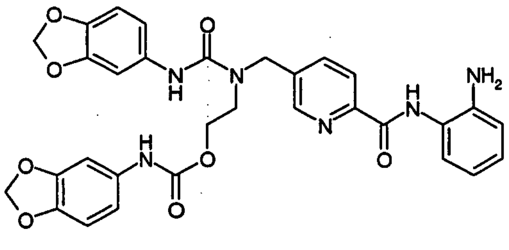 | $^1$H-NMR (500 MHz, DMSO-$d_6$) δ 3.66 (t, J = 5.8 Hz, 2H), 4.22 (t, J = 5.8 Hz, 2H), 4.77 (s, 2H), 4.88 (br s, 2H), 5.94 (s, 2H), 5.95 (s, 2H), 6.65 (td, J = 7.8, 1.2 Hz, 1H), 6.78 (d, J = 8.2 Hz, 1H), 6.81-6.84 (m, 4H), 6.95 (td, J = 7.8, 1.2 Hz, 1H), 7.08 (br s, 1H), 7.12 (d, J = 1.8 Hz, 1H), 7.50 (dd, J = 7.8, 1.2 Hz, 1H), 7.93 (dd, J = 8.0, 1.5 Hz, 1H), 8.11 (d, J = 8.0 Hz, 1H), 8.44 (br s, 1H), 8.63 (d, J = 1.5 Hz, 1H), 9.51 (br s, 1H), 10.01 (br s, 1H) |

(continued)

| | |
|---|---|
| N-(2-Aminophenyl)-5-[3-(4-methoxyphenyl)-1-[2-(4-methoxyphenylaminocarbonyloxy)ethyl]ureidomethyl] pyridine-2-carboxylic acid amide (Compound No.2-14) 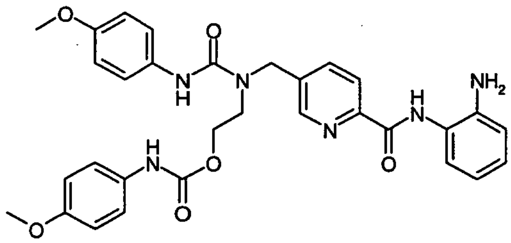 | $^1$H-NMR (400 MHz, DMSO-$d_6$) δ 3.67 (m, 2H), 3.69 (s, 3H), 3.70 (s, 3H), 4.22 (t, J = 5.5 Hz, 2H), 4.79 (s, 2H), 4.89 (br s, 2H), 6.65 (td, J = 7.8, 1.3 Hz, 1H), 6.81-6.86 (m, 5H), 6.95 (td, J = 7.8, 1.3 Hz, 1H), 7.32-7.36 (m, 4H), 7.50 (dd, J = 7.8, 1.3 Hz, 1H), 7.94 (d, J = 7.8 Hz, 1H), 8.12 (d, J = 7.8 Hz, 1H), 8.42 (br s, 1H), 8.64 (d, J = 1.2 Hz, 1H), 9.45 (br s, 1H), 10.02 (br s, 1H) |
| N-(2-Aminophenyl)-5-[1-(2-dimethylaminoethyl)-3-(1H-pyrazol-3-yl)ureidomethyl]pyddine-2-carboxylic acid amide (Compound No.2-15) 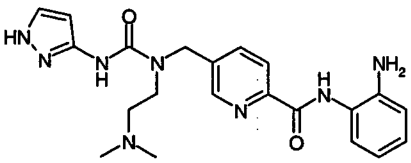 | $^1$H-NMR (500 MHz, $CDCl_3$) δ 2.40 (s, 6H), 2.54 (t, J = 4.4 Hz, 2H), 3.34 (t, J = 4.4 Hz, 2H), 3.95 (s, 2H), 4.67 (s, 2H), 6.01 (s, 1H), 6.84 (dd, J = 7.7, 1.4 Hz, 1H), 6.86 (td, J = 7.7, 1.4 Hz, 1H), 7.09 (td, J = 7.7, 1.4 Hz, 1H), 7.43 (d, J = 2.1 Hz, 1H), 7.49 (dd, J = 7.7, 1.4 Hz, 1H), 7.89 (dd, J = 7.9, 2.0 Hz, 1H), 8.26 (d, J = 7.9 Hz, 1H), 8.57 (d, J = 2.0 Hz, 1H), 9.82 (s, 1H), 12.18 (s, 1H) |

Example 3

N-(2-Aminophenyl)-5-[3-(4-hydroxyphenyl)-1-[5-(morpholin-4-yl)pentyl]ureidomethyl]pyridin e-2-carboxylic acid amide (Compound No.3-1)

Under a nitrogen atmosphere, 10% palladium on carbon (30 mg) was added to a solution of N-(2-aminophenyl)-5-[3-(4-benzyloxyphenyl)-1-[5-(morpholin-4-yl)pentyl]ureidomethyl]pyridi ne-2-carboxylic acid amide (Compound No. 1-182, 45 mg, 0.072 mmol) in a mixed solvent (ethyl acetate (2.0 mL) and methanol (3.0 mL)), and then the reaction mixture was stirred under a hydrogen atmosphere at room temperature for 2 hours. After the insoluble was filtered off with celite, the filtrate was concentrated. The residue was purified by silica gel column chromatography (chloroform-methanol) to give 32 mg of the title compound as a yellow oil. (Yield 84%)

| | |
|---|---|
| 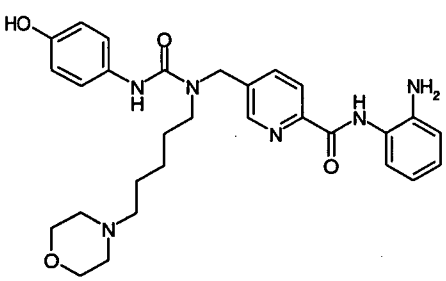 | $^1$H-NMR (500 MHz, $CDCl_3$) δ 1.34 (m, 2H), 1.52 (m, 2H), 1.63 (m, 2H), 2.33 (t, J = 7.6 Hz, 2H), 2.44 (m, 4H), 3.28 (t, J = 7.6 Hz, 2H), 3.71 (t, J = 4.7 Hz, 4H), 3.98 (s, 2H), 4.62 (s, 2H), 6.40 (s, 1H), 6.69 (d, J = 8.9 Hz, 2H), 6.84 (dd, J = 7.8, 1.4 Hz, 1H), 6.86 (td, J = 7.8, 1.4 Hz, 1H),7.09 (td, J = 7.8, 1.4 Hz, 1H), 7.10 (d, J = 8.9 Hz, 2H), 7.49 (dd, J = 7.8, 1.4 Hz, 1H), 7.80 (dd, J = 8.0, 2.0 Hz, 1H), 8.21 (d, J = 8.0 Hz, 1H), 8.52 (d, J = 2.0 Hz, 1H), 9.82 (s, 1H) |

[Preparation Examples]

Hereinafter, typical preparation examples of the present compound will be shown.

1) Tablet (in 150 mg)

| | |
|---|---|
| The present compound | 1 mg |
| Lactose | 100 mg |
| Cornstarch | 40 mg |
| Calcium carboxymethyl cellulose | 4.5 mg |
| Hydroxypropyl cellulose | 4 mg |
| Magnesium stearate | 0.5 mg |

A tablet of the above-mentioned formulation is coated using 3 mg of a coating agent (for example, a conventional coating agent such as hydroxypropylmethyl cellulose, macrogol or a silicone resin), whereby a desired tablet can be obtained. In addition, a desired tablet can be obtained by appropriately changing the kinds and/or amounts of the present compound and additives.

2) Capsule (in 150 mg)

| | |
|---|---|
| The present compound | 5 mg |
| Lactose | 135 mg |
| Calcium carboxymethyl cellulose | 4.5 mg |
| Hydroxypropyl cellulose | 4 mg |
| Magnesium stearate | 1.5 mg |

A desired capsule can be obtained by appropriately changing the kinds and/or amounts of the present compound and additives.

3) Eye drop (in 100 mL)

| | |
|---|---|
| The present compound | 100 mg |
| Sodium chloride | 900 mg |
| Polysorbate 80 | 500 mg |
| Sodium hydroxide | q.s. |
| Hydrochloric acid | q.s. |
| Sterile purified water | q.s. |

A desired eye drop can be obtained by appropriately changing the kinds and/or amounts of the present compound and additives.

[Pharmacological Test]

1. Test for Evaluation of an HDAC Inhibitory Effect

Using HDAC Fluorimetric Assay /Drug Discovery Kit (manufactured by Biomol), an HDAC inhibitory effect of the present compounds was measured according to the protocol included with the kit. The kit contains Buffer, HeLa nuclear extract (includes an HDAC), Substrate, Developer and Trichostatin A (an HDAC inhibitor).

(Preparation of Test Compound Solution)

A test compound was dissolved in dimethyl sulfoxide, whereby a 2 mg/mL solution was prepared. Then, the resulting solution was diluted with Buffer containing 5% dimethyl sulfoxide, whereby a 150 μM test compound solution was prepared.

(Test Method and Measurement Method)

1) To a 384-well culture plate, the test compound solution was added in an amount of 2 μL per well.
2) HeLa nuclear extract was diluted 30-fold with Buffer. The resulting solution was added in an amount of 3 μL per well and then, incubation was performed at 37°C for 2 hours.
3) Substrate was diluted 500-fold with Buffer. The resulting solution was added in an amount of 5 μL per well (the final concentration of the test compound was 30 μM) and then, incubation was performed at 37°C for 10 minutes.
4) Developer was diluted 20-fold with Buffer and Trichostatin A (2 μM) was added to it. The resulting solution was added in an amount of 10 μL per well and then, incubation was performed at room temperature for 15 minutes.
5) The fluorescence intensity of each well was measured using multilabel counter ARVO (manufactured by Wallac) with excitation at 360 nm and emission at 460 nm.
6) A control data was obtained from the same experiment except that Buffer containing 5% DMSO was added instead of the test compound solution as the above-mentioned procedure from 1) to 5).
7) A blank data was obtained from the same experiment except that Buffer containing 5% DMSO was added instead of the test compound solution and that Buffer was added instead of Hela nuclear extract as the above-mentioned procedure from 1) to 5).

(Calculation Equation for Enzyme Inhibitory Rate)

Enzyme inhibitory rate (%) was calculated using the following equation.

(Enzyme Inhibitory Rate(%)) = 100 x [1 − {(Fluorescence Intensity of Test Compound Solution) − (Florescence Intensity of Blank)} / {( Florescence Intensity of Control) − (Florescence Intensity of Blank)}]

(Test Results)

As an example of the test results, the enzyme inhibitory rate of the respective test compound {Trichostatin A (manufactured by Wako Pure Chemical Industries), SAHA(produced according to the method described in JP-A-2003-226680), M 344 (manufactured by Wako Pure Chemical Industries), Oxamflatin (manufactured by Alexis Biochemicals), CBHA (manufactured by Calbiochem), MC1293 (manufactured by Wako Pure Chemical Industries), Depudecin (manufactured by Sigma), MS 275 (manufactured by Calbiochem), Apicidin (manufactured by Alexis Biochemicals), MGCD-0103 free base (produced according to the method described in JP-A-2006-514998), Compound 1-2, Compound 1-3, Compound 1-4, Compound 1-6, Compound 1-8, Compound 1-10, Compound 1-13, Compound 1-14, Compound 1-20, Compound 1-21, Compound 1-24, Compound 1-25, Compound 1-27, Compound 1-30, Compound 1-31, Compound 1-38, Compound 1-40, Compound 1-41, Compound 1-42, Compound 1-44, Compound 1-46, Compound 1-51, Compound 1-53, Compound 1-55, Compound 1-56, Compound 1-58, Compound 1-63, Compound 1-68, Compound 1-79, Compound 1-90, Compound 1-96, Compound 1-115, Compound 1-116, Compound 1-141, Compound 2-1, Compound 2-3, Compound 2-10} are shown in Table I.

Table I

| Test Compound | Enzyme Inhibitory Rate (%) |
|---|---|
| Trichostatin A | 100 |
| SAHA | 97 |
| M 344 | 99 |

(continued)

| Test Compound | Enzyme Inhibitory Rate (%) |
|---|---|
| Oxamflatin | 100 |
| CBHA | 99 |
| MC1293 | 65 |
| Depudecin | 99 |
| MS 275 | 99 |
| Apicidin | 98 |
| MGCD-0103 free base | 96 |
| Compound 1-2 | 100 |
| Compound 1-3 | 100 |
| Compound 1-4 | 100 |
| Compound 1-6 | 100 |
| Compound 1-8 | 96 |
| Compound 1-10 | 98 |
| Compound 1-13 | 100 |
| Compound 1-14 | 100 |
| Compound 1-20 | 99 |
| Compound 1-21 | 98 |
| Compound 1-24 | 96 |
| Compound 1-25 | 97 |
| Compound 1-27 | 97 |
| Compound 1-30 | 100 |
| Compound 1-31 | 99 |
| Compound 1-38 | 97 |
| Compound 1-40 | 97 |
| Compound 1-41 | 96 |
| Compound 1-42 | 98 |
| Compound 1-44 | 98 |
| Compound 1-46 | 98 |
| Compound 1-51 | 98 |
| Compound 1-53 | 98 |
| Compound 1-55 | 98 |
| Compound 1-56 | 97 |
| Compound 1-58 | 98 |
| Compound 1-63 | 97 |
| Compound 1-68 | 99 |
| Compound 1-79 | 98 |
| Compound 1-90 | 98 |

(continued)

| Test Compound | Enzyme Inhibitory Rate (%) |
|---|---|
| Compound 1-96 | 98 |
| Compound 1-115 | 97 |
| Compound 1-116 | 100 |
| Compound 1-141 | 91 |
| Compound 2-1 | 98 |
| Compound 2-3 | 100 |
| Compound 2-10 | 99 |

If the enzyme inhibitory rate was more than 100%, the value is shown to be 100% in Table I.

2. Test for Evaluation of Effect of Morphological Change on Trabecular Meshwork Cells

As a method for evaluating a cellular morphological change, an evaluation system using the cell shape index (hereinafter referred to as "CSI") as an index has been reported in The Journal of Clinical Investigation, 103, 1141-1150 (1999). Therefore, according to the method described in the above document, an effect of morphological change by the present compounds on trabecular meshwork cells was evaluated.

(Used Cells)

A human trabecular meshwork cell line (hereinafter referred to as "TM-1 cells") reported in Investigative Ophthalmology & Visual Science, 43, 151-161 (2002) was used.

(Preparation of Reagents)

Culture medium 1: A reagent was prepared by adding fetal bovine serum (10%), L-glutamine (2 mM), amphotericin B (2.5 μg/mL), and gentamicin (25 μg/mL) to Dulbecco's Modified Eagle Medium (hereinafter referred to as "D-MEM").

Culture medium 2: Fetal bovine serum (3%), L-glutamine (2 mM), amphotericin B (2.5 μg/mL), and gentamicin (25 μg/mL) were added to D-MEM.

Cell staining liquid: A mixed liquid of Calcein-AM (16 μM) and Hoechst 33342 (40 μM) was prepared by diluting a Calcein-AM solution (cytoplasmic staining reagent, manufactured by Dojindo Laboratories) and a Hoechst 33342 solution (nuclear staining reagent, manufactured by Dojindo Laboratories) with D-MEM containing L-glutamine (2 mM), amphotericin B (2.5 μg/mL), and gentamicin (25 μg/mL).

(Preparation of Cells)

TM-1 cells subcultured at 37°C in a 8% carbon dioxide gas atmosphere were treated with a trypsin/EDTA solution (0.05% trypsin and 0.53 mM tetrasodium ethylenediaminetetraacetate) at 24 hours before performing a drug treatment mentioned below and seeded on a 96-well culture plate. The culture medium 1 was used for the subculture of the cells. The culture medium 2 was used for the cell culture after seeding the cells on the plate.

(Preparation of Test Compound Solution)

A test compound was dissolved in dimethyl sulfoxide, whereby a 5 mM solution was prepared. Then, the resulting solution was diluted with the culture medium 2, whereby a 200 μM test compound solution was prepared.

(Preparation of Positive Control Compound Solution)

It has been reported that Y-27632 which is a Rho kinase inhibitor induces a morphological change in trabecular meshwork cells in Investigative Ophthalmology & Visual Science, 42, 137-144 (2001). Therefore, Y-27632 (produced according to the method described in WO 90/05723) was used as a positive control, and dissolved in dimethyl sulfoxide

in the same manner as the test compound, whereby a 5 mM solution was prepared, and then, the resulting solution was diluted with the culture medium 2, whereby a 200 μM positive control compound solution was prepared.

(Test Method and Measurement Method)

1) To a 96-well culture plate, a solution of TM-1 cells adjusted to a cell density of 1.6 x $10^4$ cells/mL was added in an amount of 95 μL (1.5 x $10^4$ cells) per well.
2) Incubation was performed at 37°C in a 8% carbon dioxide gas atmosphere for 24 hours.
3) The test compound solution or positive control compound solution was added in an amount of 5 μL per well (the final concentration of the test compound or positive control compound was 10 μM). As a control, the culture medium 2 containing dimethyl sulfoxide (4%) was added in an amount of 5 μL per well.
4) Incubation was performed at 37°C in a 8% carbon dioxide gas atmosphere for 24 hours.
5) The cell staining liquid was added in an amount of 10 μL per well.
6) Incubation was performed at 37°C in a 8% carbon dioxide gas atmosphere for 1 hour to stain the cells.
7) A 37% formaldehyde solution was added in an amount of 10 μL per well.
8) Incubation was performed at room temperature for 1 hour to fix the cells.
9) Washing with phosphate-buffered saline was performed.
10) Using Array Scan Vti HCS reader (manufactured by Cellomics), images of stained cells magnified with a 20-fold objective lens were captured in 80 fields (10 fields x 8 wells) per test compound addition group.
11) CSI was calculated for each cell and an average value was obtained for each test compound addition group.

(Calculation Equation for CSI)

CSI was calculated using the following equation.

$$CSI = 4\pi \text{ x (Cell Area) / (Cell Perimeter)}^2$$

(Test Results)

As an example of the test results, the CSI values of the respective test compound {Trichostatin A (manufactured by Wako Pure Chemical Industries), SAHA(produced according to the method described in JP-A-2003-226680), M 344 (manufactured by Wako Pure Chemical Industries), Oxamflatin (manufactured by Alexis Biochemicals), CBHA (manufactured by Calbiochem), MC1293 (manufactured by Wako Pure Chemical Industries), Depudecin (manufactured by Sigma), MS 275 (manufactured by Calbiochem), Apicidin (manufactured by Alexis Biochemicals), MGCD-0103 free base (produced according to the method described in JP-A-2006-514998), Compound 1-2, Compound 1-3, Compound 1-4, Compound 1-6, Compound 1-8, Compound 1-10, Compound 1-13, Compound 1-14, Compound 1-20, Compound 1-21, Compound 1-24, Compound 1-25, Compound 1-27, Compound 1-30, Compound 1-31, Compound 1-38, Compound 1-40, Compound 1-41, Compound 1-42, Compound 1-44, Compound 1-46, Compound 1-51, Compound 1-53, Compound 1-55, Compound 1-56, Compound 1-58, Compound 1-63, Compound 1-68, Compound 1-79, Compound 1-90, Compound 1-96, Compound 1-115, Compound 1-116, Compound 1-141, Compound 2-1, Compound 2-3, Compound 2-10} addition groups and Y-27632 addition group are shown in Table II.

Table II

| Test Compound | CSI |
|---|---|
| Control | 0.679 |
| Trichostatin A | 0.496 |
| SAHA | 0.501 |
| M 344 | 0.500 |
| Oxamflatin | 0.551 |
| CBHA | 0.545 |

(continued)

| Test Compound | CSI |
|---|---|
| MC1293 | 0.628 |
| Depudecin | 0.618 |
| MS 275 | 0.552 |
| Apicidin | 0.489 |
| MGCD-0103 free base | 0.584 |
| Compound 1-2 | 0.516 |
| Compound 1-3 | 0.569 |
| Compound 1-4 | 0.556 |
| Compound 1-6 | 0.549 |
| Compound 1-8 | 0.538 |
| Compound 1-10 | 0.528 |
| Compound 1-13 | 0.545 |
| Compound 1-14 | 0.520 |
| Compound 1-20 | 0.516 |
| Compound 1-21 | 0.575 |
| Compound 1-24 | 0.597 |
| Compound 1-25 | 0.571 |
| Compound 1-27 | 0.624 |
| Compound 1-30 | 0.564 |
| Compound 1-31 | 0.551 |
| Compound 1-38 | 0.575 |
| Compound 1-40 | 0.572 |
| Compound 1-41 | 0.499 |
| Compound 1-42 | 0.536 |
| Compound 1-44 | 0.554 |
| Compound 1-46 | 0.547 |
| Compound 1-51 | 0.518 |
| Compound 1-53 | 0.502 |
| Compound 1-55 | 0.525 |
| Compound 1-56 | 0.526 |
| Compound 1-58 | 0.518 |
| Compound 1-63 | 0.521 |
| Compound 1-68 | 0.502 |
| Compound 1-79 | 0.497 |
| Compound 1-90 | 0.480 |
| Compound 1-96 | 0.489 |
| Compound 1-115 | 0.467 |
| Compound 1-116 | 0.455 |

(continued)

| Test Compound | CSI |
|---|---|
| Compound 1-141 | 0.515 |
| Compound 2-1 | 0.574 |
| Compound 2-3 | 0.550 |
| Compound 2-10 | 0.582 |
| Y-27632 | 0.543 |

3. Test for Evaluation of Intraocular Pressure-Lowering Effect

In order to evaluate an intraocular pressure-lowering effect of the present compounds, a test for evaluation of intraocular pressure-lowering effect by intracameral administration of a drug using male Japanese White rabbits was performed.

(Preparation of Test Compound Administration Liquid)

A test compound was dissolved or suspended in physiological saline containing 0.5% dimethyl sulfoxide, whereby a 0.1 mM or 1 mM test compound administration liquid was prepared.

(Test Method and Measurement Method)

One drop of 0.4% oxybuprocaine hydrochloride eye drop was instilled into both eyes of each male Japanese White rabbit to achieve local anesthesia, and thereafter, the intraocular pressure was measured using an applanation tonometer. Then, by using a syringe fitted with a 30-gauge needle, the test compound administration liquid (20 μL) was intracamerally administered to one eye. As a control, 20 μL of the vehicle (physiological saline containing 0.5% dimethyl sulfoxide) for the test compound was intracamerally administered. After the lapse of a certain period of time from the administration of the test compound or vehicle, one drop of 0.4% oxybuprocaine hydrochloride eye drop was instilled into the administered eye to achieve local anesthesia, and thereafter, the intraocular pressure was measured using an applanation tonometer.

(Calculation Equation for Intraocular Pressure Reduction Rate)

The intraocular pressure-lowering effect of each test compound was evaluated by calculating an intraocular pressure reduction rate. The intraocular pressure reduction rate (%) was calculated using the following equation.

(Intraocular Pressure Reduction Rate(%)) = 100 x [{(Average Value of Intraocular Pressure of Control Group) – (Average Value of Intraocular Pressure of Each Test Compound Administration Group)} / (Average Value of Intraocular Pressure of Control Group)]

(Test Results and Discussion)

As an example of the test results, the intraocular pressure reduction rates of the respective test compound administration groups at 8 or 9 hours after administering the respective test compounds {Trichostatin A (manufactured by Alexis Biochemicals), SAHA (manufactured by Alexis Biochemicals), Oxamflatin (manufactured by Alexis Biochemicals), MS 275 (manufactured by Calbiochem), Apicidin (manufactured by Calbiochem), MGCD-0103 free base (produced according to the method described in JP-A-2006-514998) and Compound 1-96} are shown in Table III (one group consisting of 6 cases).

Table III

| Test Compound | Concentration of Test Compound | Intraocular Pressure Reduction Rate (%) |
|---|---|---|
| Trichostatin A | 1 mM | 18 |
| SAHA | 1 mM | 17 |
| Oxamflatin | 1 mM | 26 |
| MS 275 | 1 mM | 13 |
| Apicidin | 0.1 mM | 13 |
| MGCD-0103 free base | 1 mM | 23 |
| Compound 1-96 | 1 mM | 18 |

As shown in Table II, the present compounds have an excellent effect of morphological change on trabecular meshwork cells equal to or greater than that of Y-27632 used as the positive control (lower CSI indicates greater morphological changes in Table II). Further, as shown in Table III, the present compounds have an excellent intraocular pressure-lowering effect also in the test using actual animal models. Accordingly, the present compounds can be used as an intraocular pressure-lowering agent and are expected to be useful as a preventive and/or therapeutic agent for diseases associated with aqueous humor circulation and/or intraocular pressure, particularly as a preventive and/or therapeutic agent for glaucoma, ocular hypertension, etc.

Industrial Applicability

The compound having an HDAC inhibitory effect of the invention has an excellent effect of morphological change on trabecular meshwork cells and/or effect of intraocular pressure reduction, and is therefore useful as a preventive and/or therapeutic agent for a disease considered to be associated with aqueous humor circulation and/or intraocular pressure, particularly as a preventive and/or therapeutic agent for glaucoma or ocular hypertension.

## Claims

1. An intraocular pressure-lowering agent comprising at least one compound having a histone deacetylase inhibitory effect as an active ingredient.

2. The intraocular pressure-lowering agent according to claim 1, wherein the compound having a histone deacetylase inhibitory effect is a compound selected from the following compounds or a salt thereof:

   ·(2E,4E,6R)-7-(4-Dimethylaminophenyl)-N-hydroxy-4,6-dimethyl-7-oxo-2,4-heptadienamide (Trichostatin A),
   ·N-Hydroxy-N'-phenyloctanediamide (SAHA),
   ·4-Dimethylamino-N-(6-hydroxycarbamoylhexyl)benzamide (M 344),
   .(E)-N-Hydroxy-5-[3-(phenylsulfonylamino)phenyl]-2-penten-4-ynamide (Oxamflatin),
   ·N-Hydroxy-3-[3-hydroxyamino-3-oxo-1-propenyl]benzamide (CBHA),
   ·(E)-N-Hydroxy-3-[1-methyl-4-(4-methylbenzoyl)-1H-pyrrol-2-yl]-2-propenamide (MC1293),
   ·(1R)-[(2S,3S)-3-[(E)-2-[(2S,3S)-3-[(1R)-Hydroxyethyl]oxiran-2-yl] vinyl]oxiran-2-yl]-2-propen-1-ol (Depudecin),
   ·N-(2-Aminophenyl)-4-(pyridin-3-ylmethyloxycarbonylaminomethyl) benzamide (MS 275),
   *cyclo*[L-(2-Amino-8-oxodecanoyl)-(1'-methoxy-L-tryptophyl)-L-isoleucyl-D-pipecolinyl] (Apicidin); and
   ·N-(2-Aminophenyl)-4-[4-(pyridin-3-yl)pyrimidin-2-ylaminomethyl]benzamide (free base of MGCD-0103).

3. The intraocular pressure-lowering agent according to claim 1, wherein the compound having a histone deacetylase inhibitory effect is a compound represented by the following general formula (1) or a salt thereof:

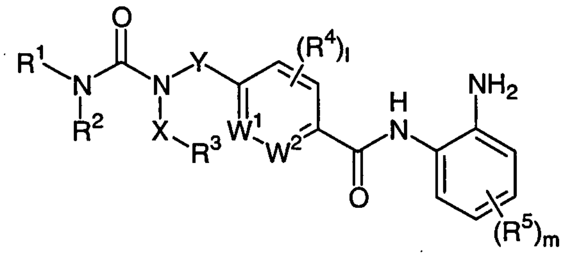

( 1 )

[wherein $R^1$ and $R^2$ are the same or different and represent a hydrogen atom, a lower alkyl group which may have a substituent, a lower alkenyl group which may have a substituent, a lower alkynyl group which may have a substituent or a group represented by the following general formula (2);

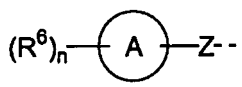

(2)

$R^3$ represents a hydroxy group, a lower alkoxy group which may have a substituent, a lower cycloalkyloxy group which may have a substituent, an aryloxy group which may have a substituent, a carboxy group, a lower alkoxycarbonyl group which may have a substituent, $-OCONR^aR^b$, $-NR^cR^d$ or a group represented by the following general formula (3);

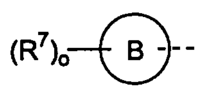

(3)

$R^4$ and $R^5$ are the same or different and represent a halogen atom, a lower alkyl group which may have a substituent, a hydroxy group, or a lower alkoxy group which may have a substituent;
$R^6$ represents a halogen atom, a lower alkyl group which may have a substituent, a lower cycloalkyl group which may have a substituent, an aryl group which may have a substituent, a heterocyclic group which may have a substituent, a hydroxy group, a lower alkoxy group which may have a substituent, a lower cycloalkyloxy group which may have a substituent, an aryloxy group which may have a substituent, a mercapto group, a lower alkylthio group which may have a substituent, a lower cycloalkylthio group which may have a substituent, an arylthio group which may have a substituent, a formyl group, a lower alkylcarbonyl group which may have a substituent, a carboxy group, a lower alkoxycarbonyl group which may have a substituent, a nitro group, a cyano group or $-NR^eR^f$;
$R^7$ represents a lower alkyl group which may have a substituent, a lower cycloalkyl group which may have a substituent, an aryl group which may have a substituent, a hydroxy group, a lower alkoxy group which may have a substituent, a lower cycloalkyloxy group which may have a substituent or an aryloxy group which may have a substituent;
$R^a$ and $R^b$ are the same or different and represent a hydrogen atom, a lower alkyl group which may have a substituent, a lower cycloalkyl group which may have a substituent, an aryl group which may have a substituent or a heterocyclic group which may have a substituent;
$R^c$, $R^d$, $R^e$ and $R^f$ are the same or different and represent a hydrogen atom, a lower alkyl group which may have a substituent, a lower cycloalkyl group which may have a substituent or an aryl group which may have a substituent;
the ring A represents a cyclic hydrocarbon or a heterocyclic ring;
the ring B represents a heterocyclic ring having one or plural heteroatoms selected from the group consisting of a nitrogen atom and an oxygen atom in the ring;
X represents a lower alkylene group which may have a substituent;
Y and Z are the same or different and represent a single bond or a lower alkylene group which may have a substituent;
$W^1$-$W^2$ represents N-C or C-N; and
l, m, n and o are the same or different and represent 0, 1, 2 or 3].

**4.** The intraocular pressure-lowering agent according to claim 3, wherein in the general formula (1), $R^1$ and $R^2$ are the same or different and represent a hydrogen atom, a lower alkyl group, a lower alkyl group having

a halogen atom as a substituent, a lower alkyl group having a methoxy group as a substituent, a lower alkyl group having a methylthio group as a substituent, a lower alkyl group having a cyano group as a substituent, a lower alkyl group having a methylaminocarbonyl group as a substituent, a lower alkyl group having a diisopropylamino group as a substituent, a lower alkenyl group, a lower alkynyl group or a group represented by the following general formula (2);

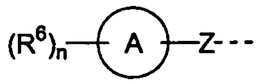 (2)

$R^3$ represents a hydroxy group, a lower alkoxy group, a lower cycloalkyloxy group, an aryloxy group, a carboxy group, a lower alkoxycarbonyl group, $-OCONR^aR^b$, $-NR^cR^d$ or a group represented by the following general formula (3);

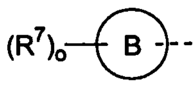 (3)

$R^4$ and $R^5$ are the same or different and represent a halogen atom, a lower alkyl group, a hydroxy group or a lower alkoxy group;
$R^6$ represents a halogen atom, a lower alkyl group, a lower alkyl group having a halogen atom as a substituent, a lower alkyl group having a cyano group as a substituent, a lower cycloalkyl group, an aryl group, a heterocyclic group, a hydroxy group, a lower alkoxy group, a lower alkoxy group having a halogen atom as a substituent, a lower alkoxy group having an aryl group as a substituent, a lower cycloalkyloxy group, an aryloxy group, a mercapto group, a lower alkylthio group, a lower cycloalkylthio group, an arylthio group, a formyl group, a lower alkylcarbonyl group, a carboxy group, a lower alkoxycarbonyl group, a nitro group, a cyano group or $-NR^eR^f$;
$R^7$ represents a lower alkyl group, a lower cycloalkyl group, an aryl group, a hydroxy group, a lower alkoxy group, a lower cycloalkyloxy group or an aryloxy group;
$R^a$ and $R^b$ are the same or different and represent a hydrogen atom, a lower alkyl group, a lower cycloalkyl group, an aryl group or a heterocyclic group;
$R^c$, $R^d$, $R^e$ and $R^f$ are the same or different and represent a hydrogen atom, a lower alkyl group, a lower cycloalkyl group or an aryl group;
the ring A represents a cyclic hydrocarbon or a heterocyclic ring;
the ring B represents a heterocyclic ring having one or plural heteroatoms selected from the group consisting of a nitrogen atom and an oxygen atom in the ring;
X represents a lower alkylene group;
Y and Z are the same or different and represent a single bond, a lower alkylene group or a lower alkylene group having an oxo group as a substituent;
$W^1$-$W^2$ represents N-C or C-N; and
1, m, n and o are the same or different and represent 0, 1, 2 or 3.

**5.** The intraocular pressure-lowering agent according to claim 3, wherein in the general formula (1),
$R^1$ represents a lower alkyl group, a lower alkyl group having a halogen atom as a substituent, a lower alkyl group having a methoxy group as a substituent, a lower alkyl group having a methylthio group as a substituent, a lower alkyl group having a cyano group as a substituent, a lower alkyl group having a methylaminocarbonyl group as a substituent, a lower alkyl group having a diisopropylamino group as a substituent, a lower alkynyl group or a group represented by the following general formula (2);

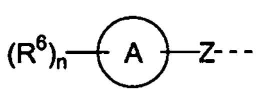 (2)

$R^2$ represents a hydrogen atom;
$R^3$ represents a hydroxy group, a carboxy group, a lower alkoxycarbonyl group, $-OCONR^aR^b$, $-NR^cR^d$ or a group represented by the following general formula (3);

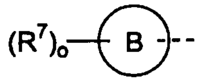 (3)

$R^6$ represents a halogen atom, a lower alkyl group, a lower alkyl group having a halogen atom as a substituent, a lower alkyl group having a cyano group as a substituent, an aryl group, a morpholino group, a hydroxy group, a lower alkoxy group, a lower alkoxy group having a halogen atom as a substituent, a lower alkoxy group having an aryl group as a substituent, a lower alkylthio group, a lower alkylcarbonyl group, a nitro group, a cyano group or $-NR^eR^f$;
$R^7$ represents a lower alkyl group or an alkoxy group;
$R^a$ and $R^b$ are the same or different and represent a hydrogen atom, an aryl group or a heterocyclic group;
$R^c$, $R^d$, $R^e$ and $R^f$ represent a lower alkyl group;
the ring A represents a cyclic hydrocarbon or a heterocyclic ring;
the ring B represents a heterocyclic ring having plural heteroatoms selected from the group consisting of a nitrogen atom and an oxygen atom in the ring;
X and Y represent a lower alkylene group;
Z represents a single bond, a lower alkylene group or a lower alkylene group substituted with an oxo group;
$W^1$-$W^2$ represents C-N or N-C;
l and m represent 0;
o represents 0 or 1; and
n represents 0, 1 or 2.

**6.** The intraocular pressure-lowering agent according to claim 3, wherein in the general formula (1), the ring A represents benzene, indan, thiophene, benzo[1,3]dioxole, 2,3-dihydrobenzofuran, 1H-benzimidazole, isoxazole, thiazole, benzothiazole, 2,3-dihydrobenzo[1,4]dioxin or pyridine.

**7.** The intraocular pressure-lowering agent according to any one of claim 3, wherein in the general formula (1), the ring B represents pyrrolidine or morpholine.

**8.** The intraocular pressure-lowering agent according to claim 1, wherein the compound having a histone deacetylase inhibitory effect is a compound selected from the following compounds or a salt thereof:

· N-(2-AminophenyD-5-[3-(2,3-dihydrobenzo[1,4]dioxin-6-yD-1-(3-dimethylaminopropyl)ureidomethyl]pyridine-2-carboxylic acid amide,
· N-(2-Aminophenyl)-5-[3-(4-dimethylaminophenyl)-1-(3-(morpholin-4-yl) propyl)ureidomethyl]pyridine-2-carboxylic acid amide,
· N-(2-Aminophenyl)-5-[1-(3-(morpholin-4-yl)propyl)-3-phenethylureidomethyl] pyridine-2-carboxylic acid amide,
· N-(2-Aminophenyl)-5-[3-(3,4-difluorophenyl)-1-(3-dimethylaminopropyl) ureidomethyflpyridine-2-carboxyhc acid amide,
· N-(2-Aminophenyl)-5-[1-(3-dimethylaminopropyl)-3-(4-methoxyphenyl) ureidomethyl]pyridine-2-carboxylic acid amide,
· N-(2-Aminophenyl)-5-[3-(4-diethylaminophenyl)-1-(3-dimethylaminopropyl) ureidomethyllpyridine-2-carboxylic acid amide,
· N-(2-Aminophenyl)-5-[1-(3-dimethylaminopropyl)-3-(3-fluorophenyl) ureidomethyflpyridine-2-carboxylic acid amide,
· N-(2-AminophenyD-5-[1-(3-dimethylaminopropyl)-3-(3-fluoro-4-methylphenyl)ureidomethyflpyridine-2-carboxylic acid amide,
· N-(2-AminophenyD-5-[1-(3-dimethylaminopropyl)-3-(4-fluoro-3-methylphenyl)ureidomethyl]pyridine-2-carboxylic acid amide,
· N-(2-Aminophenyl)-5-[3-(4-cyanophenyl)-1-(3-dimethylaminopropyl) ureidomethyl]pyridine-2-carboxylic acid amide,
· N-(2-Aminophenyl)-5-[1-(3-dimethylaminopropyl)-3-(4-trifluoromethoxyphenyl) ureidomethyl]pyridine-2-carboxylic acid amide,
· N-(2-Aminophenyl)-5-[3-(benzo[1,3]dioxol-5-yl)-1-(3-hydroxypropyl) ureidomethyl]pyridine-2-carboxylic acid amide,
· N-(2-Aminophenyl)-5-[3-(4-dimethylaminophenyl)-1-(2-ethoxycarbonylethyl)ureidomethyl]pyridine-2-carboxylic acid amide,

· N-(2-Aminophenyl)-5-[3-(1,3-benzothiazol-2-yl)-1-(3-dimethylaminopropyl)ureidomethyl]pyridine-2-carboxylic acid amide,
· N-(2-Aminophenyl)-5-[3-(1H-benzimidazol-2-yl)-1-(3-dimethylaminopropyl)ureidomethyl]pyridine-2-carboxylic acid amide,
· N-(2-Aminophenyl)-5-[3-(2,3-dihydro-1-benzofuran-5-yl)-1-(3-(morpholin-4-yl)propyl)ureidomethyl]pyridine-2-carboxylic acid amide,
· N-(2-Aminophenyl)-5-[3-(2,3-dihydrobenzo[1,4]dioxin-6-yl)-1-(2-hydroxyethyl)ureidomethyl]pyridine-2-carboxylic acid amide,
· N-(2-Aminophenyl-5-[1-(3-dimethylaminopropyl)-3-(phenylcarbonylmethyl)ureidomethyl]pyridine-2-carboxylic acid amide,
· N-(2-Aminophenyl)-5-[1-(3-dimethylaminopropyl)-3-(1,3-thiazol-2-yl)ureidomethyl)pyridine-2-carboxylic acid amide,
· N-(2-Aminophenyl)-5-[1-(3-dimethylaminopropyl)-3-(6-methoxy-1,3-benzothiazol-2-yl)ureidomethyl]pyridine-2-carboxylic acid amide,
· N-(2-Aminophenyl)-5-[1-(3-dimethylaminopropyl)-3-(6-fluoro-1,3-benzothiazol-2-yl)ureidomethyl]pyridine-2-carboxylic acid amide,
· N-(2-Aminophenyl)-5-[1-(3-diethylaminopropyl)-3-(3,4-difluorophenyl) ureidomethyl]pyridine-2-carboxylic acid amide,
· N-(2-Aminophenyl)-5-[1-(3-(morpholin-4-yl)propyl)-3-(pyridin-3-yl)ureidomethyl]pyridine-2-carboxylic acid amide,
· N-(2-Aminophenyl)-5-[1-(3-dimethylaminopropyl)-3-(thiophen-3-yl)ureidomethyl]pyridine-2-carboxylic acid amide,
· N-(2-Aminophenyl)-5-[1-(3-dimethylaminopropyl)-3-(3-fluoro-4-methoxyphenyl)ureidomethyl]pyridine-2-carboxylic acid amide,
· N-(2-Aminophenyl)-5-[3-(3-chloro-4-fluorophenyl)-1-(3-diethylaminopropyl)ureidomethyl]pyridine-2-carboxylic acid amide,
· N-(2-Aminophenyl)-5-[1-(3-diethylaminopropyl)-3-(4-fluoro-3-nitrophenyl)ureidomethyl]pyridine-2-carboxylic acid amide,
· N-(2-Aminophenyl)-5-[3-(2,3-dihydrobenzo[1,4]dioxin-6-yl)-1-(2-dimethylaminoethyl)ureidomethyl]pyridine-2-carboxylic acid amide,
· N-(2-Aminophenyl)-5-[1-(2-dimethylaminoethyl)-3-(3-methoxyphenyl) ureidomethyl]pyridine-2-carboxylic acid amide,
· N-(2-Aminophenyl)-5-[1-(2-dimethylaminoethyl)-3-(3-fluorophenyl) ureidomethyl]pyridine-2-carboxylic acid amide,
· N-(2-Aminophenyl)-5-[1-(2-dimethylaminoethyl)-3-(thiophen-3-yl) ureidomethyl]pyridine-2-carboxylic acid amide,
· N-(2-Aminophenyl)-5-[3-(pyridin-3-yl)-1-[3-(pyrrohdin-1-yl)propyl] ureidomethyl]pyridine-2-carboxylic acid amide,
· N-(2-Aminophenyl)-5-[1-(3-(morpholin-4-yl)propyl)-3-(phenylcarbonylmethyl) ureidomethyl]pyridine-2-carboxylic acid amide,
· N-(2-Aminophenyl)-5-[3-(3-chlorophenyl)-1-(3-(morpholin-4-yl)propyl) ureidomethyl]pyridine-2-carboxylic acid amide,
· N-(2-Aminophenyl)-5-[3-(4-fluorophenethyl)-1-(3-(morpholin-4-yl) propyl)ureidomethyl]pyridine-2-carboxylic acid amide,
· N-(2-Aminophenyl)-5-[3-(4-cyanophenyl)-1-(3-hydroxypropyl) ureidomethyl]pyridine-2-carboxylic acid amide,
· N-(2-Aminophenyl)-5-[3-(3-fluorophenyl)-1-[2-(4-methylpiperazin-1-yl)ethyl]ureidomethyl]pyridine-2-carboxylic acid amide,
· N-(2-Aminophenyl)-5-[3-(2,3-dihydrobenzo[1,4]dioxin-6-yl)-1-[3-(4-methylpiperazin-1-yl)propyl]ureidomethyl] pyridine-2-carboxylic acid amide,
· N-(2-Aminophenyl)-5-[1-(2-dimethylaminoethyl)-3-(3-methylphenyl) ureidomethyl]pyridine-2-carboxylic acid amide,
· N-(2-Aminophenyl)-5-[3-(2,3-dihydrobenzo[1,4]dioxin-6-yl)-1-[4-(morpholin-4-yl)butyl]ureidomethyl]pyridine-2-carboxylic acid amide,
· N-(2-Aminophenyl)-5-[1-(2-dimethylaminoethyl)-3-(1,3-thiazol-2-yl)ureidomethyl]pyridine-2-carboxylic acid amide,
· N-(2-Aminophenyl)-5-[3-(2-methoxyphenyl)-1-[4-(morpholin-4-yl)butyl]ureidomethyl]pyridine-2-carboxylic acid amide,
· N-(2-Aminophenyl)-5-[3-(3-methylphenyl)-1-[3-(4-methylpiperidin-1-yl)propyl]ureidomethyl]pyridine-2-carboxylic acid amide; and

· N-(2-Aminophenyl)-5-[3-cyclopentyl-1-[5-(morpholin-4-yl)pentyl] ureidomethyl]pyridine-2-carboxylic acid amide.

**9.** A method for changing morphology of trabecular meshwork cells and/or a method for reducing intraocular pressure, comprising administering to a patient an effective amount of the intraocular pressure-lowering agent according to any one of claims 1 to 8.

**10.** A method for preventing and/or treating a disease associated with aqueous humor circulation and/or intraocular pressure, comprising administering to a patient an effective amount of the intraocular pressure-lowering agent according to any one of claims 1 to 8.

**11.** A method for preventing and/or treating glaucoma and/or ocular hypertension, comprising administering to a patient an effective amount of the intraocular pressure-lowering agent according to any one of claims 1 to 8.

**INTERNATIONAL SEARCH REPORT**

International application No.
PCT/JP2008/056014

A. CLASSIFICATION OF SUBJECT MATTER
*A61K45/00*(2006.01)i, *A61K31/44*(2006.01)i, *A61K31/443*(2006.01)i, *A61K31/4433*(2006.01)i, *A61K31/4436*(2006.01)i, *A61K31/4439*(2006.01)i, *A61K31/444*(2006.01)i, *A61K31/5377*(2006.01)i, *A61P27/02*(2006.01)i,
According to International Patent Classification (IPC) or to both national classification and IPC

B. FIELDS SEARCHED

Minimum documentation searched (classification system followed by classification symbols)
A61K45/00, A61K31/44, A61K31/443, A61K31/4433, A61K31/4436, A61K31/4439, A61K31/444, A61K31/5377, A61P27/02, A61P43/00, C07D213/81, C07D401/12, C07D405/12, C07D405/14, C07D409/12, C07D413/12, C07D417/12

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2008 |
| Kokai Jitsuyo Shinan Koho | 1971-2008 | Toroku Jitsuyo Shinan Koho | 1994-2008 |

Electronic data base consulted during the international search (name of data base and, where practicable, search terms used)
BIOSIS(STN), CAplus(STN), EMBASE(STN), MEDLINE(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| A | COFFEY, D.C. et al, The histone deacetylase inhibitor, CBHA, inhibits growth of human neuroblastoma xenografts in vivo, alone and synergistically with all-trans retinoic acid, Cancer Res, 2001, Vol.61, No.9, p.3591-4 Abstract | 1,2 |
| A | TAKAI, N. et al, M344 is a novel synthesized histone deacetylase inhibitor that induces growth inhibition, cell cycle arrest, and apoptosis in human endometrial cancer and ovarian cancer cells, Gynecol Oncol, 2006, Vol.101, No.1, p.108-13 Abstract | 1,2 |
| A | MONNERET C., Histone deacetylase inhibitors, European Journal of Medicinal Chemistry, 2005, Vol.40, p.1-13 Abstract | 1,2 |

☒ Further documents are listed in the continuation of Box C.  ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>29 May, 2008 (29.05.08) | Date of mailing of the international search report<br>17 June, 2008 (17.06.08) |
| Name and mailing address of the ISA/<br>Japanese Patent Office<br>Facsimile No. | Authorized officer<br><br>Telephone No. |

Form PCT/ISA/210 (second sheet) (April 2007)

INTERNATIONAL SEARCH REPORT

International application No.
PCT/JP2008/056014

C (Continuation). DOCUMENTS CONSIDERED TO BE RELEVANT

| Category* | Citation of document, with indication, where appropriate, of the relevant passages | Relevant to claim No. |
|---|---|---|
| A | JP 2006-514998 A (METHYLGENE INC.), 18 May, 2006 (18.05.06), Par. No. [0506], example 426 & WO 2004/069823 A1 & US 6897220 B2 & AU 2004210016 A1 & EP 1590340 A1 & TW 200418826 A & BR 200407195 A & MX 2005008246 A1 & CN 1723207 A & KR 2005098228 A & IN 200501604 P2 | 1,2 |
| A | JP 2006-509021 A (F. Hoffmann-La Roche AG.), 16 March, 2006 (16.03.06), Claims; example Nos. 6-38 to 6-41 & US 2004/0138270 A1 & EP 1572625 A1 & WO 2004/052838 A1 & CA 2507137 A & BR 317027 A & KR 10-2005-0088421 A & CN 1723192 A | 1,3-8 |
| A | JP 11-302173 A (Mitsui Chemicals, Inc.), 02 November, 1999 (02.11.99), Claims; compounds 22, 28, 34, 39 (Family: none) | 1,3-8 |

Form PCT/ISA/210 (continuation of second sheet) (April 2007)

**INTERNATIONAL SEARCH REPORT**

International application No.
PCT/JP2008/056014

Continuation of A. CLASSIFICATION OF SUBJECT MATTER
(International Patent Classification (IPC))

*A61P43/00*(2006.01)i, *C07D213/81*(2006.01)i, *C07D401/12*(2006.01)i, *C07D405/12*(2006.01)i, *C07D405/14*(2006.01)i, *C07D409/12*(2006.01)i, *C07D413/12*(2006.01)i, *C07D417/12*(2006.01)i

(According to International Patent Classification (IPC) or to both national classification and IPC)

Form PCT/ISA/210 (extra sheet) (April 2007)

**INTERNATIONAL SEARCH REPORT**

International application No.
PCT/JP2008/056014

**Box No. II Observations where certain claims were found unsearchable (Continuation of item 2 of first sheet)**

This international search report has not been established in respect of certain claims under Article 17(2)(a) for the following reasons:

1. ☒ Claims Nos.: 9-11
because they relate to subject matter not required to be searched by this Authority, namely:
The invention of the claims pertains to a method for treatment of a human body by therapy and thus relates to a subject matter which this International Searching Authority is not required, under the provisions of PCT Article 17(2)(a)(i) and PCT Rule 39.1(iv), to search.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.
2. ☐ As all searchable claims could be searched without effort justifying additional fees, this Authority did not invite payment of additional fees.
3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** the

☐ The additional search fees were accompanied by the applicant's protest and, where applicable, payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 1997030701 A
- WO 2000009162 A
- JP 2005272419 A
- JP 2006517532 T
- JP 2002332267 A
- JP 2001064177 A
- JP 2001081031 A
- JP 10152462 A
- JP 11335375 A
- JP 11269140 A
- JP 11269146 A
- JP 11302173 A
- JP 11335373 A
- US 20040162317 A
- WO 2004069133 A
- WO 2004052838 A
- WO 2004087693 A
- WO 2003087057 A
- WO 2003092686 A
- WO 2004035525 A
- WO 2004069823 A
- JP 2001316283 B
- WO 2002030879 A
- WO 2002022577 A
- WO 2004092115 A
- WO 2005019174 A
- JP 2007001885 B
- JP 2003226680 A
- JP 2006514998 A
- WO 9005723 A

### Non-patent literature cited in the description

- *Protein, Nucleic Acid and Enzyme,* 2006, vol. 51 (14
- *J. Biol. Chem.,* 1979, vol. 254, 1716-1723
- *Cancer Res.,* 1987, vol. 47, 3688-3691
- *Exp. Cell Res.,* 1988, vol. 177, 122-131
- *J. Biol. Chem.,* 1990, vol. 265, 17174-17179
- *J. Antibiotics,* 1990, vol. 43, 1524-1534
- *J. Biol. Chem.,* 1993, vol. 268, 22429-22435
- *Journal of Medicinal Chemistry,* 2002, vol. 45, 753
- *European Journal of Medicinal Chemistry,* 2006, vol. 41, 697
- *Journal of Clinical Investigation,* 1999, vol. 103, 1141-1150
- *The Journal of Clinical Investigation,* 1999, vol. 103, 1141-1150
- *Investigative Ophthalmology & Visual Science,* 2002, vol. 43, 151-161
- *Investigative Ophthalmology & Visual Science,* 2001, vol. 42, 137-144